# EUROPEAN PATENT APPLICATION

(11) **EP 1 557 165 A1**
(43) Date of publication of application: **27.07.2005**
(21) Application number: 03798438.2
(22) Date of filing: 22.09.2003
(51) Int. Cl.: A61K 31/155, A61K 45/00, A61K 31/496, A61K 31/5025, A61K 31/522, A61P 1/00, A61P 3/04, A61P 3/06, A61P 3/10, A61P 5/00, A61P 5/50, A61P 43/00

(54) **COMBINATION DRUG**

(30) Priority: 26.09.2002 JP 2002280137; 23.04.2003 JP 2003117927
(71) Applicant: Eisai Co., Ltd., Tokyo 112-8088 (JP)
(72) Inventor: YASUDA, Nobuyuki, Ushiku-shi, Ibaraki 300-1237 (JP); YAMAZAKI, Kazuto, Tsukuba-shi, Ibaraki 305-0031 (JP)
(74) Representative: VOSSIUS & PARTNER
(86) International application number: PCT/JP2003/012075
(87) International publication number: WO 2004/028524

(57) **Abstract**

The present invention provides pharmaceutical agents comprising a dipeptidyl peptidase IV (DPPIV) inhibitor and a biguanide agent in combination, which enhance the effects of active circulating glucagon-like peptide-1 (GLP-1) and/or active circulating glucagon-like peptide-2 (GLP-2).

## Description

### Technical Field

The present invention relates to pharmaceutical agents comprising a dipeptidyl peptidase IV (DPPIV) inhibitor and a biguanide agent, which enhance the effects of active circulating glucagon-like peptide-1 (GLP-1) and/or active circulating glucagon-like peptide-2 (GLP-2).

### Background Art

Glucagon-like peptide-1 (GLP-1) is a hormone known to be secreted in response to food intake from L cells in the distal part of the small intestine. It enhances the secretion of insulin from pancreatic β cells in a glucose-dependent manner. GLP-1 is degraded and rapidly inactivated by dipeptidyl peptidase IV (DPPIV). Thus, DPPIV inhibitors can be used as preventive and/or therapeutic agents for diseases such as diabetes (particularly typeII diabetes) and obesity, with which GLP-1 levels are associated. DPPIV inhibitors have been under development in clinical trials and are disclosed in Patent documents 1, 2, and 3.

Metformin, a biguanide agent, has commonly been used as a preventive and/or therapeutic agent for diabetes.

In recent years, new findings have been reported successively: GLP-1 levels are increased in obese non-diabetic patients upon administration of metformin (Non-patent document 1); and a combination of metformin and GLP-1 is effective to treat typeII diabetes (Non-patent document 2). However, even if the level of GLP-1 is elevated transiently by metformin, GLP-1 is rapidly degraded and inactivated by DPPIV as described above. Therefore, the elevated level of GLP-1 does not have a long duration, and thus GLP-1 effects are extremely reduced. This is a problem to be solved.

Non-patent documents 3 and 4 suggest the applicability of the combined use of a DPPIV inhibitor and metformin. Patent documents 4 to 8 describe the combined use of a DPPIV inhibitor and a biguanide agent. However, these documents have not disclosed particular test results for the combined use of these agents. In other words, there is no combination drug that contains a DPPIV inhibitor and metformin, which is known to enhance the effects of GLP-1.

It has been reported that glucagon-like peptide-2 (GLP-2) is a hormone secreted in response to food intake from L cells in the distal part of the small intestine like GLP-1, and that it can be used for preventing and/or treating gastrointestinal diseases (Non-patent documents 5 to 9). However, like GLP-1, GLP-2 is rapidly degraded and inactivated by DPPIV
Consequently, there has been demand to develop agents suppressing the degradation of GLP-2, and therefore enhancing GLP-2 effects. However, there are no reports describing increases in the GLP-2 level upon administration of metformin or the enhancement of GLP-2 effects by the combined use of a DPPIV inhibitor and metformin.
[Patent document 1]
US Patent No. 6166063
[Patent document 2]
US Patent No. 6011155
[Patent document 3]
US Patent No. 6548481
[Patent document 4]
WO 01/52825
[Patent document 5]
WO 01/97808
[Patent document 6]
US Patent Application NO. 2002/0161001
[Patent document 7]
US Patent Application NO. 2002/0198205
[Patent document 8]
US Patent Application NO. 2003/0105077
[Non-patent document 1]
Edoardo Mannucci, and eight other authors, "Diabetes Care", 24(3): 489-494 (2001) Mar.
[Non-patent document 2]
Mette Zander, and four other authors, "Diabetes Care", 24(4): 720-725 (2001) Apr.
[Non-patent document 3]
Simon A. Hinke, and five other authors, "Biochemical and Biophysical Research Communications", 291(5): 1302-1308 (2002) Mar.
[Non-patent document 4]
Simon A. Hinke, and nine other authors, "Diabetes Care", 25(8): 1490-1491 (2002) Aug.
[Non-patent document 5]
Robin P. Boushey, and two other authors, "American Journal of Physiology", 277(8): E937-E947 (1999)
[Non-patent document 6]
D. L. Sigalet, "Current Opinion in Investigational Drugs", 2(4): 505-509 (2001) Apr.
[Non-patent document 7]
Daniel J. Drucker, "Gut", 50(3): 428-435 (2002)
[Non-patent document 8]
Daniel J. Drucker "Gastroenterology", 122(2): 531-544 (2002) Feb.
[Non-patent document 9]
Robin P. Boushey, and two other authors, "Cancer Research", 61: 687-693 (2001) Jan.

### Disclosure of the Invention

An objective of the present invention is to provide pharmaceutical agents that enhance the pharmacological actions of active circulating GLP-1 and/or active circulating GLP-2, by suppressing the degradation of GLP-1 and/or GLP-2 when levels have been elevated by a biguanide agent.

The present inventors conducted extensive studies in view of the above background, and revealed that the combined use of a DPPIV inhibitor and a biguanide agent enhanced the pharmacological actions of active circulating GLP-1 and/or active circulating GLP-2. This is because the DPPIV inhibitor suppresses the degradation of active circulating GLP-1 and/or active circulating GLP-2, when levels are increased by the biguanide agent. Thus, the inventors completed the present invention.

Specifically, the present invention provides:
<1> a pharmaceutical agent comprising a dipeptidyl peptidase IV inhibitor and a biguanide agent in combination;
<2> the pharmaceutical agent according to <1>, which enhances the effects of active circulating glucagon-like peptide-1 (GLP-1) and/or active circulating glucagon-like peptide-2 (GLP-2);
<3> a pharmaceutical agent that enhances the effects of active circulating GLP-2;
<4> a pharmaceutical agent comprising a dipeptidyl peptidase IV inhibitor and the pharmaceutical agent according to <3> in combination;
<5> the pharmaceutical agent according to <1> or <4>, wherein the dipeptidyl peptidase IV inhibitor is any one compound selected from:
   (S)-1-((3-hydroxy-1-adamantyl)amino)acetyl-2-cyanopyrrolidine;
   (S)-1-(2-((5-cyanopyridin-2-yl)amino)ethyl-aminoacetyl)-2-cyanopyrrolidine; isoleucine thiazolidide; isoleucine pyrrolidide; and valine pyrrolidide;
   or a salt or hydrate thereof;
<6> the pharmaceutical agent according to <1> or <4>, wherein the dipeptidyl peptidase IV inhibitor is a compound represented by the following formula, or a salt or hydrate thereof, 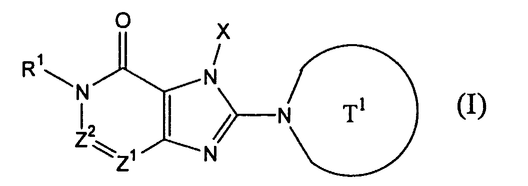 (wherein,
   T¹ represents a monocyclic or bicyclic 4- to 12-membered heterocyclic group containing one or two nitrogen atoms in the ring, that may have one or more substituents;
   X represents a C₁₋₆ alkyl group which may have one or more substituents, a C₂₋₆ alkenyl group which may have one or more substituents, a C₂₋₆ alkynyl group which may have one or more substituents, a C₆₋₁₀ aryl group which may have one or more substituents, a 5 to 10-membered heteroaryl group which may have one or more substituents, a C₆₋₁₀ aryl C₁₋₆ alkyl group which may have one or more substituents, or a 5 to 10-membered heteroaryl C₁₋₆ alkyl group which may have one or more substituents;
   Z¹ and Z² each independently represent a nitrogen atom or a group represented by the formula -CR²=;
   R¹ and R² each independently represent a group according to the formula -A⁰-A¹-A² (wherein A⁰ represents a single bond or a C₁₋₆ alkylene group, which may have 1 to 3 substituents selected from group B consisting of the substituents described below;
   A¹ represents a single bond, an oxygen atom, a sulfur atom, a sulfinyl group, a sulfonyl group, a carbonyl group, a group represented by the formula -O-CO-, a group represented by the formula -CO-O-, a group represented by the formula -NR^{A}-, a group represented by the formula -CO-NR^{A}-, a group represented by the formula -NR^{A}-CO-, a group represented by the formula -SO₂-NR^{A}-, or a group represented by the formula -NR^{A}-SO₂-;
   A² and R^{A} each independently represent a hydrogen atom, a halogen atom, a cyano group, a C₁₋₆ alkyl group, a C₃₋₈ cycloalkyl group, a C₂₋₆ alkenyl group, a C₂₋₆ alkynyl group, C₆₋₁₀ aryl group, a 5 to 10-membered heteroaryl group, a 4 to 8-membered heterocyclic group, a 5 to 10-membered heteroaryl C₁₋₆ alkyl group, a C₆₋₁₀ aryl C₁₋₆ alkyl group, or a C₂₋₇ alkylcarbonyl group;
   however, A² and R^{A} each independently may have 1 to 3 substituents selected from the substituent group B described below:
   when Z² is a group represented by the formula -CR²=, R¹, and R² may in combination form a 5 to 7-membered ring;
   except in cases where: [1] R¹ is a hydrogen atom; Z¹ is a nitrogen atom; and Z² is -CH=; and [2] Z¹ is a nitrogen atom; and Z² is -C(OH)=;
   <Substituent group B>
   Substituent group B represents the group consisting of: a hydroxyl group, a mercapto group, a cyano group, a nitro group, a halogen atom, a trifluoromethyl group, a C₁₋₆ alkyl group which may have one or more substituents, a C₃₋₈ cycloalkyl group, a C₂₋₆ alkenyl group, a C₂₋₆ alkynyl group, a C₆₋₁₀ aryl group, a 5 to 10-membered heteroaryl group, a 4 to 8-membered heterocyclic group, a C₁₋₆ alkoxy group, a C₁₋₆ alkylthio group, a group represented by the formula -SO₂-NR^{B1}-R^{B2}, a group represented by the formula -NR^{B1}-CO-R^{B2}, a group represented by the formula -NR^{B1}-R^{B2} (where R^{B1} and R^{B2} each independently represent a hydrogen atom or a C₁₋₆ alkyl group), a group represented by the formula -CO-R^{B3} (where R^{B3} represents a 4 to 8-membered heterocyclic group), a group represented by the formula -CO-R^{B4}-R^{B5} and a group represented by the formula -CH₂-CO-R^{B4}-R^{B5} (where R^{B4} represents a single bond, an oxygen atom, or a group represented by the formula -NR^{B6}-; R^{B5} and R^{B6} each independently represent a hydrogen atom, a C₁₋₆ alkyl group, a C₃₋₈ cycloalkyl group, a C₂₋₆ alkenyl group, a C₂₋₆ alkynyl group, a C₆₋₁₀ aryl group, a 5 to 10-membered heteroaryl group, a 4 to 8-membered heterocyclic C₁₋₆ alkyl group, a C₆₋₁₀ aryl C₁₋₆ alkyl group, or a 5 to 10-membered heteroaryl C₁₋₆ alkyl group));
<7> the pharmaceutical agent according to <6>, wherein T¹ is a piperazin-1-yl group or a 3-amino-piperidin-1-yl group;
<8> the pharmaceutical agent according to <6>, wherein T¹ is a piperazin-1-yl group;
<9> the pharmaceutical agent according to any one of <6> to <8>, wherein X is a 3-methyl-2-buten-1-yl group, a 2-butynyl group, a benzyl group, or a 2-chlorophenyl group;
<10> the pharmaceutical agent according to any one of <6> to <8>, wherein X is a 3-methyl-2-buten-1-yl group or a 2-butyn-1-yl group;
<11> the pharmaceutical agent according to any one of <6> to <8>, wherein X is a 2-butyn-1-yl group;
<12> the pharmaceutical agent according to any one of <6> to <11>, wherein,
   Z¹ is a nitrogen atom; and
   Z² is a group represented by the formula -CR₂=
   (where R² is as defined in <6>);
<13> the pharmaceutical agent according to any one of <6> to <11>, wherein,
   Z² is a nitrogen atom; and
   Z¹ is a group represented by the formula -CR₂=
   (where R² is as defined in <6>);
<14> the pharmaceutical agent according to any one of <6> to <13>, wherein R¹ is either a methyl group, a cyanobenzyl group, a fluorocyanobenzyl group, a phenethyl group, a 2-methoxyethyl group, or a 4-methoxycarbonylpridin-2-yl group;
<15> the pharmaceutical agent according to any one of <6> to <13>, wherein R¹ is a methyl group, or a 2-cyanobenzyl group;
<16> the pharmaceutical agent according to any one of <6> to <15>, wherein R² is either a hydrogen atom, a cyano group, a methoxy group, a carbamoylphenyloxy group, or a group represented by the formula: 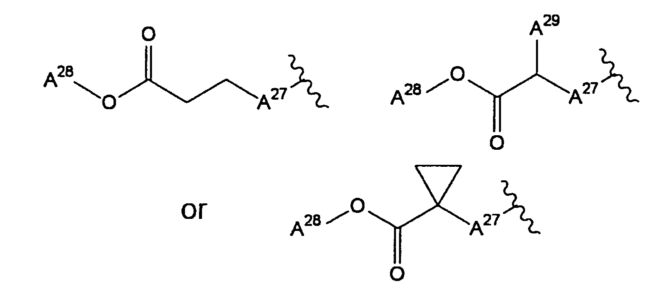 (where,
   A²⁷ represents an oxygen atom, a sulfur atom, or -NH-;
   A²⁸ and A²⁹ each independently represent a hydrogen atom or a C₁₋₆ alkyl group);
<17> the pharmaceutical agent according to any one of <6> to <15>, wherein R² is a hydrogen atom, a cyano group, or a 2-carbamoylphenyloxy group;
<18> the pharmaceutical agent according to <6>, wherein the compound represented by formula (I) is any one compound selected from:
   (1) 7-(2-butynyl)-2-cyano-1-methyl-8-(piperazin-1-yl)-1,7-dihydropurin-6-one; (2)
   3-(2-butynyl)-5-methyl-2-(piperazin-1-yl)-3,5-dihydroimidazo[4,5-d]pyridazin-4-one; (3)
   2-(3-aminopiperidin-1-yl)-3-(2-butynyl)-5-methyl-3,5-dihydroimidazo[4,5-d]pyridazin-4-one;
   (4) 2-[7-(2-butynyl)-1-methyl-6-oxo-8-(piperazin-1-yl)-6,7-dihydro-1H-purin-2-yloxy] benzamide;
   (5) 7-(2-butynyl)-1-(2-cyanobenzyl)-6-oxo-8-(piperazin-1-yl)-6,7-dihydro-1H-purine-2-car bonitrile; and
   (6) 2-[3-(2-butynyl)-4-oxo-2-(piperazin-1-yl)-3,4-dihydroimidazo[4,5-d] pyridazin-5-ylmethyl] benzonitrile;
   or a salt or hydrate thereof;
<19> the pharmaceutical agent according to <1> or <4>, wherein the dipeptidyl peptidase IV inhibitor is a compound represented by the following formula, or a salt or hydrate thereof, 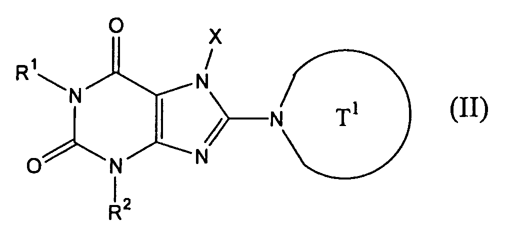 (wherein T¹, X, R¹, and R² are as defined in <6>);
<20> the pharmaceutical agent according to <19>, wherein T¹ is a piperazin-1-yl group;
<21> the pharmaceutical agent according to <19> or <20>, wherein X is a 2-butynyl group or a 2-chlorophenyl group;
<22> the pharmaceutical agent according to <19> or <20>, wherein X is a 2-butynyl group;
<23> the pharmaceutical agent according to any one of <19> to <22>, wherein R¹ is a hydrogen atom, a methyl group, a 2-propynyl group, a 2-butynyl group, a cyanomethyl group, a phenethyl group, a phenoxyethyl group, or a group represented by the formula: 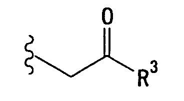 (where R³ represents a hydroxyl group, a C₁₋₆ alkoxy group, or a phenyl group);
<24> the pharmaceutical agent according to any one of <19> to <23>, wherein R² is a hydrogen atom, a C₁₋₆ alkyl group, an ethoxyethyl group, a tetrahydrofuranylmethyl group, or a group represented by the formula: 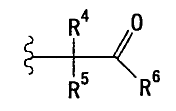 (where,
   R⁴ and R⁵ are identical to or different from each other, and independently represent a hydrogen atom, a methyl group, or a phenyl group; and
   R⁶ represents a hydroxyl group, a C₁₋₆ alkoxy group, or a phenyl group),
   or a group represented by the formula: 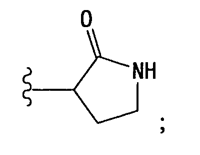
<25> the pharmaceutical agent according to <19>, wherein the compound represented by formula (II) is any one compound selected from:
   (1) 7-(2-butynyl)-1,3-dimethyl-8-(piperazin-1-yl)-3,7-dihydropurine-2,6-dione;
   (2) 7-(2-butynyl)-3-methyl-8-(piperazin-1-yl)-3,7-dihydropurine-2,6-dione;
   (3) methyl [7-(2-butynyl)-3-methyl-2,6-dioxo-8-(piperazin-1-yl)-2,3,6,7-tetrahydropurin-1-yl] acetate;
   (4) 7-(2-butynyl)-3-methyl-8-(piperazin-1-yl)-1-(2-propynyl)-3,7-dihydropurine-2,6-dione;
   (5) 1,7-bis(2-butynyl)-3-methyl-8-(piperazin-1-yl)-3,7-dihydropurine-2,6-dione;
   (6) [7-(2-butynyl)-3-methyl-2,6-dioxo-8-(piperazin-1-yl)-2,3,6,7-tetrahydropurin-1-yl] acetonitrile;
   (7) 7-(2-butynyl)-3-methyl-1-[(2-oxo-2-phenyl)ethyl]-8-(piperazin-1-yl)-3,7-dihydropurine-2,6-dione;
   (8) 7-(2-butynyl)-3-ethyl-1-methyl-8-(piperazin-1-yl)-3,7-dihydropurine-2,6-dione;
   (9) methyl [7-(2-butynyl)-1-methyl-2,6-dioxo-8-(piperazin-1-yl)-1,2,6,7-tetrahydropurin-3-yl] acetate;
   (10) 7-(2-butynyl)-3-(2-tetrahydrofuranyl)methyl-1-methyl-8-(piperazin-1-yl)-3,7-dihydropu rine-2,6-dione;
   (11) methyl [7-(2-butynyl)-1-methyl-2,6-dioxo-8-(piperazin-1-yl)-1,2,6,7-tetrahydropurin-3-yl]phen ylacetate;
   (12) 7-(2-butynyl)-3-propyl-1-methyl-8-(piperazin-1-yl)-3,7-dihydropurine-2,6-dione;
   (13) 7-(2-butynyl)-3-(2-oxo-2-phenethyl)-1-methyl-8-(piperazin-1-yl)-3,7-dihydropurine-2,6 -dione;
   (14) ethyl 2-[7-(2-butynyl)-1-methyl-2,6-dioxo-8-(piperazin-1-yl)-1,2,6,7-tetrahydropurin-3-yl] propionate;
   (15) 7-(2-butynyl)-3-(2-ethoxyethyl)-1-methyl-8-(piperazin-1-yl)-3,7-dihydropurine-2,6-dio ne;
   (16) 7-(2-butynyl)-3-isopropyl-1-methyl-8-(piperazin-1-yl)-3,7-dihydropurine-2,6-dione;
   (17) 7-(2-butynyl)-3-(3,3-dimethyl-2-oxobutyl)-1-methyl-8-(piperazin-1-yl)-3,7-dihydropuri ne-2,6-dione;
   (18) 7-(2-butynyl)-1-methyl-3-(2-oxopyrrolidin-3-yl)-8-(piperazin-1-yl)-3,7-dihydropurine-2 ,6-dione;
   (19) 7-(2-butynyl)-3-(2-ethoxyethyl)-1-(2-oxo-2-phenylethyl)-8-(piperazin-1-yl)-3,7-dihydro purine-2,6-dione;
   (20) methyl [7-(2-butynyl)-2,6-dioxo-1-(2-oxo-2-phenylethyl)-8-(piperazin-1-yl)-1,2,6,7-tetrahydro purin-3-yl] acetate;
   (21) ethyl [7-(2-butynyl)-2,6-dioxo-1-(2-phenethyl)-8-(piperazin-1-yl)-1,2,6,7-tetrahydropurin-3-y 1] acetate;
   (22) [7-(2-butynyl)-2,6-dioxo-1-(2-phenethyl)-8-(piperazin-1-yl)-1,2,6,7-tetrahydropurin-3-y 1] acetate;
   (23) 7-(2-butynyl)-3-[2-oxo-2-(pyrrolidin-1-yl)ethyl]-1-(2-phenethyl)-8-(piperazin-1-yl)-3,7-dihydropurine-2,6-dione;
   (24) 2-[7-(2-butynyl)-2,6-dioxo-1-(2-phenethyl)-8-(piperazin-1-yl)-1,2,6,7-tetrahydropurin-3 -yl]-N-methylacetamide;
   (25) 2-[7-(2-butynyl)-2,6-dioxo-1-(2-phenethyl)-8-(piperazin-1-yl)-1,2,6,7-tetrahydropurin-3 -yl]-N-cyclopropyl acetamide;
   (26) 2-[7-(2-butynyl)-2,6-dioxo-1-(2-phenethyl)-8-(piperazin-1-yl)-1,2,6,7-tetrahydropurin-3 -yl]-N-phenylacetamide; and
   (27) 2-[7-(2-butynyl)-2,6-dioxo-1-(2-phenethyl)-8-(piperazin-1-yl)-1,2,6,7-tetrahydropurin-3 -yl]-N-(2-propynyl) acetamide;
   or a salt or hydrate thereof;
<26> the pharmaceutical agent according to <1>, wherein the biguanide agent is metformin;
<27> the pharmaceutical agent according to <1> or <2>, which is a preventive or therapeutic agent for a disease which is associated with active circulating GLP-1 and/or active circulating GLP-2;
<28> the pharmaceutical agent according to <27>, wherein the disease is at least any one selected from the group consisting of: diabetes, obesity, hyperlipidemia, and gastrointestinal diseases;
<29> the pharmaceutical agent according to <3> or <4>, which is a preventive or therapeutic agent for a disease which is associated with active circulating GLP-2;
<30> the pharmaceutical agent according to <29>, wherein the disease is a gastrointestinal disease;
<31> a method for preventing or treating a disease which is associated with active circulating GLP-1 and/or active circulating GLP-2, which comprises administering the pharmaceutical agent according to <1> or <2> at an effective amount;
<32> the use of the pharmaceutical agent according to <1> or <2> for producing a preventive or therapeutic agent for a disease which is associated with active circulating GLP-1 and/or active circulating GLP-2;
<33> a method for preventing or treating a disease which is associated with active circulating GLP-2, which comprises administering the pharmaceutical agent according to <3> or <4> at an effective amount;
<34> the use of the pharmaceutical agent according to <3> or <4> for producing a preventive or therapeutic agent for a disease which is associated with active circulating GLP-2;
<35> a method for enhancing the effects of active circulating GLP-1 and/or active circulating GLP-2, which comprises using the pharmaceutical agent according to <1> or <2>; and
<36> a method for enhancing the effects of active circulating GLP-2, which comprises using the pharmaceutical agent according to <3> or <4>.

The present invention also includes:
<37> an agent for enhancing the effects of active circulating glucagon-like peptide-1 (GLP-1) and/or active circulating glucagon-like peptide-2 (GLP-2), which comprises a dipeptidyl peptidase IV inhibitor and a biguanide agent in combination;
<38> an agent for enhancing the effects of active circulating glucagon-like peptide-2 (GLP-2), which comprises a biguanide agent as an active ingredient;
<39> an agent for enhancing the effects of active circulating glucagon-like peptide-2 (GLP-2), which comprises a dipeptidyl peptidase IV inhibitor and a biguanide agent in combination;
<40> a preventive or therapeutic agent for diabetes, obesity, hyperlipidemia, or gastrointestinal diseases, which enhances the effects of active circulating glucagon-like peptide-1 (GLP-1), and which comprises a dipeptidyl peptidase IV inhibitor and a biguanide agent as active ingredients;
<41> a preventive or therapeutic agent for gastrointestinal diseases, which enhances the effects of active circulating glucagon-like peptide-2 (GLP-2), and which comprises a dipeptidyl peptidase IV inhibitor and a biguanide agent as active ingredients; and
<42> a preventive or therapeutic agent for diabetes, obesity, hyperlipidemia, or gastrointestinal diseases, which comprises a dipeptidyl peptidase IV inhibitor and a biguanide agent as active ingredients.

In items <37> to <42>, it is preferred that the dipeptidyl peptidase IV inhibitor is as defined by any one of <5> to <25> listed above and the biguanide agent is as defined above in <26>.

### Best Mode for Carrying Out the Invention

Herein, a structural formula of a compound sometimes represents a certain isomer for convenience of description. However, compounds of the present invention may include all possible isomers, such as structurally possible geometric isomers, optical isomers generated due to the presence of asymmetric carbons, stereoisomers, tautomers, and mixtures of isomers, and are not limited to formulae being used for the convenience of description, and may be either of two isomers or a mixture of both isomers. Thus, compounds of the present invention may be either optically active compounds having an asymmetric carbon atom in their molecules or their racemates, and are not restricted to either of them but include both. Furthermore, compounds of the present invention may exhibit crystalline polymorphism, but likewise are not restricted to any one of these but may be in any one of these crystal forms or exist as a mixture of two or more crystal forms. Compounds of the present invention also include both anhydrous and hydrated forms. Substances produced through *in vivo* metabolism of compounds of the invention are also within the scope of claims.

The terms and symbols used herein are defined and the present invention is described in detail below.

As used herein, the phrase "C₁₋₆ alkyl group" refers to a linear or branched alkyl group containing 1 to 6 carbon atoms, which is a monovalent group obtained by removal of any one of the hydrogen atoms from an aliphatic hydrocarbon containing 1 to 6 carbons, and specifically, includes, for example, a methyl group, an ethyl group, a 1-propyl group, a 2-propyl group, a 2-methyl-1-propyl group, a 2-methyl-2-propyl group, a 1-butyl group, a 2-butyl group, a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2-hexyl group, a 3-hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3-dimethyl-1-butyl group, a 3,3-dimethyl-1-butyl group, a 2,2-dimethyl-1-butyl group, a 2-ethyl-1-butyl group, a 3,3-dimethyl-2-butyl group, and a 2,3-dimethyl-2-butyl group.

As used herein, the phrase "C₂₋₆ alkenyl group" refers to a linear or branched alkenyl group containing 2 to 6 carbons, and specifically includes, for example, a vinyl group, an allyl group, a 1-propenyl group, a 2-propenyl group, a 1-butenyl group, a 2-butenyl group, a 3-butenyl group, a pentenyl group, and a hexenyl group.

As used herein, the phrase "C₂₋₆ alkynyl group" refers to a linear or branched alkynyl group containing 2 to 6 carbons, and specifically includes, for example, an ethynyl group, a 1-propynyl group, a 2-propynyl group, a butynyl group, a pentynyl group, and a hexynyl group.

As used herein, the phrase "C₃₋₈ cycloalkyl group" refers to a cyclic aliphatic hydrocarbon group containing 3 to 8 carbon atoms, and specifically includes, for example, a cyclopropyl group, a cyclobutyl group, a cyclopentyl group, a cyclohexyl group, a cycloheptyl group, and a cyclooctyl group.

As used herein, the phrase "C₁₋₆ alkylene group" refers to a divalent group obtained by removal of another arbitrary hydrogen atom from a "C₁₋₆ alkyl group" defined above, and specifically includes, for example, a methylene group, a 1,2-ethylene group, a 1,1-ethylene group, a 1,3-propylene group, a tetramethylene group, a pentamethylene group, and a hexamethylene group.

As used herein, the phrase "C₃₋₈ cycloalkylene group" refers to a divalent group obtained by removal of another arbitrary hydrogen atom from a "C₃₋₈ cycloalkyl group" defined above.

As used herein, the phrase "C₁₋₆ alkoxy group" refers to an oxy group linked to a "C₁₋₆ alkyl group" defined above, and specifically includes, for example, a methoxy group, an ethoxy group, a 1-propyloxy group, a 2-propyloxy group, a 2-methyl-1-propyloxy group, a 2-methyl-2-propyloxy group, a 1-butyloxy group, a 2-butyloxy group, a 1-pentyloxy group, a 2-pentyloxy group, a 3-pentyloxy group, a 2-methyl-1-butyloxy group, a 3-methyl-1-butyloxy group, a 2-methyl-2-butyloxy group, a 3-methyl-2-butyloxy group, a 2,2-dimethyl-1-propyloxy group, a 1-hexyloxy group, a 2-hexyloxy group, a 3-hexyloxy group, a 2-methyl-1-pentyloxy group, a 3-methyl-1-pentyloxy group, a 4-methyl-1-pentyloxy group, a 2-methyl-2-pentyloxy group, a 3-methyl-2-pentyloxy group, a 4-methyl-2-pentyloxy group, a 2-methyl-3-pentyloxy group, a 3-methyl-3-pentyloxy group, a 2,3-dimethyl-1-butyloxy group, a 3,3-dimethyl-1-butyloxy group, a 2,2-dimethyl-1-butyloxy group, a 2-ethyl-1-butyloxy group, a 3,3-dimethyl-2-butyloxy group, and a 2,3-dimethyl-2-butyloxy group.

As used herein, the phrase "C₁₋₆ alkylthio group" refers to a thio group linked to a "C₁₋₆ alkyl group" defined above, and specifically includes, for example, a methylthio group, an ethylthio group, a 1-propylthio group, a 2-propylthio group, a butylthio group, and a pentylthio group.

As used herein, the phrase "C₂₋₇ alkoxycarbonyl group" refers to a carbonyl group linked to a "C₁₋₆ alkoxy group" defined above, and specifically includes, for example, a methoxycarbonyl group, an ethoxycarbonyl group, a 1-propyloxycarbonyl group, and a 2-propyloxycarbonyl group.

As used herein, the phrase "C₂₋₇ alkylcarbonyl group" refers to a carbonyl group linked to a "C₁₋₆ alkyl group" defined above, and specifically includes, for example, a methylcarbonyl group, an ethylcarbonyl group, a 1-propylcarbonyl group, and a 2-propylcarbonyl group.

As used herein, the term "halogen atom" refers to a fluorine atom, a chlorine atom, a bromine atom, or an iodine atom.

As used herein, the phrase "C₆₋₁₀ aryl group" refers to an aromatic cyclic hydrocarbon group containing 6 to 10 carbon atoms, and specifically includes, for example, a phenyl group, a 1-naphthyl group, and a 2-naphthyl group.

As used herein, the term "heteroatom" refers to a sulfur atom, an oxygen atom, or a nitrogen atom.

As used herein, the phrase "5 to 10-membered heteroaryl ring" refers to an aromatic 5 to 10-membered ring containing one or more heteroatoms, and specifically includes, for example, a pyridine ring, a thiophene ring, a furan ring, a pyrrole ring, an oxazole ring, an isoxazole ring, a thiazole ring, a thiadiazole ring, an isothiazole ring, an imidazole ring, a triazole ring, a pyrazole ring, a furazan ring, a thiadiazole ring, an oxadiazole ring, a pyridazine ring, a pyrimidine ring, a pyrazine ring, a triazine ring, indole ring, an isoindole ring, an indazole ring, a chromene ring, a quinoline ring, an isoquinoline ring, a cinnoline ring, a quinazoline ring, a quinoxaline ring, a naphthyridine ring, a phthalazine ring, a purine ring, a pteridine ring, a thienofuran ring, an imidazothiazole ring, a benzofuran ring, a benzothiophene ring, a benzoxazole ring, a benzothiazole ring, a benzothiadiazole ring, a benzimidazole ring, an imidazopyridine ring, a pyrrolopyridine ring, a pyrrolopyrimidine ring, and a pyridopyrimidine ring. Preferable "5 to 10-membered heteroaryl rings" include a pyridine ring, a thiophene ring, a furan ring, a pyrrole ring, an imidazole ring, a 1,2,4-triazole ring, a thiazole ring, a thiadiazole ring, a pyrazole ring, a furazan ring, a thiadiazole ring, a pyridazine ring, a pyrimidine ring, a pyrazine ring, an isoquinoline ring, a benzoxazole ring, a benzothiazole ring, and a benzimidazole ring. The most preferable example is a pyridine ring.

As used herein, the phrase "5 to 10-membered heteroaryl group" refers to a monovalent or divalent group obtained by removal of any one or two hydrogen atoms from a "5 to 10-membered heteroaryl ring" described above.

As used herein, the phrase "4 to 8-membered heterocyclic ring" refers to a non-aromatic ring in which:
(i) the number of atoms constituting the ring is 4 to 8;
(ii) the atoms constituting the ring include 1 to 2 heteroatoms;
(iii) the ring may contain 1 to 2 double bonds;
(iv) the ring may contain 1 to 3 carbonyl groups; and
(v) the ring is monocyclic.

Specifically, the 4 to 8-membered heterocyclic ring includes, for example, an azetidine ring, a pyrrolidine ring, a piperidine ring, an azepan ring, an azocane ring, a tetrahydrofuran ring, a tetrahydropyran ring, a morpholine ring, a thiomorpholine ring, a piperazine ring, a thiazolidine ring, a dioxane ring, an imidazoline ring, a thiazoline ring, and a ring represented by one of the formulae: 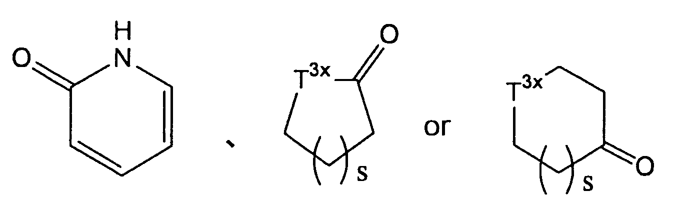 (where s represents an integer from 1 to 3; T^{3x} represents a methylene group, an oxygen atom or a group represented by the formula -NT^{4x}-, wherein T^{4x} represents a hydrogen atom or C₁₋₆ alkyl group. Preferably the "4- to 8-membered heterocyclic rings" include a pyrrolidine ring, a piperidine ring, an azepan ring, a morpholine ring, a thiomorpholine ring, a piperazine ring, a dihydrofuran-2-one ring, and a thiazolidine ring.

As used herein, the phrase "4 to 8-membered heterocyclic group" refers to a monovalent or divalent group obtained by removal of any one or two hydrogen atoms from a "4 to 8-membered heterocycle" described above. Preferably, the "4 to 8-membered heterocyclic groups" include a piperidin-1-yl group, a pyrrolidin-1-yl group, and a morpholin-4-yl group.

As used herein, the phrase "C₆₋₁₀ aryl C₁₋₆ alkyl group" refers to a group obtained by substitution of a "C₆₋₁₀ aryl group" defined above for an arbitrary hydrogen atom in a "C₁₋₆ alkyl group" defined above, and specifically includes, for example, a benzyl group, a phenethyl group, and a 3-phenyl-1-propyl group.

As used herein, the phrase "5 to 10-membered heteroaryl C₁₋₆ alkyl group" refers to a group obtained by substitution of a "5 to 10-membered heteroaryl group" defined above for an arbitrary hydrogen atom in a "C₁₋₆ alkyl group" defined above, and specifically, includes for example, a 2-pyridylmethyl and a 2-thienylmethyl group.

As used herein, the phrase "4 to 8-membered heterocyclic C₁₋₆ alkyl group" refers to a group obtained by substitution of a "4 to 8-membered heterocyclic group" defined above for an arbitrary hydrogen atom in a "C₁₋₆ alkyl group" defined above.

As used herein, the phrase "monocyclic or bicyclic 4 to 12-membered heterocyclic group containing one or two nitrogen atoms in the ring, that may have one or more substituents" refers to a non-aromatic cyclic group which may have one or more substituents. In the non-aromatic cyclic groups:
(i) the number of atoms constituting the ring of the cyclic group is 4 to 12;
(ii) the atoms constituting the ring of the cyclic group include one or two nitrogen atoms; and
(iii) the group is a monocyclic or bicyclic structure.

Specifically, the group is represented by the formula: 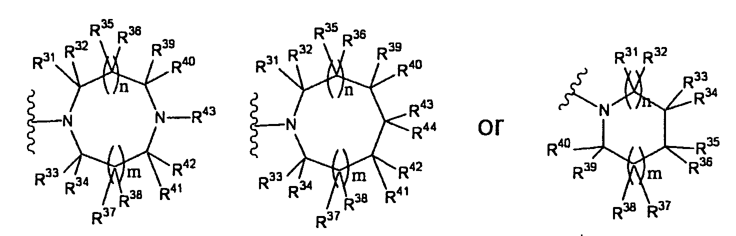 (where n and m each independently represent 0 or 1; R³¹ to R⁴⁴ independently represent a hydrogen atom or a substituent selected from substituents referred to in the phrase "which may have one or more substituents" (the substituent group S defined below); any two of R³¹ to R⁴⁴ may in combination form a C₁₋₆ alkylene group).

As used herein, the phrase "which may have one or more substituents" means that a group may have one or more substituents in any combination at replaceable positions. Specifically, such substituents include, for example, a substituent selected from the substituent group S defined below.

### <Substituent group S>

This group consists of:
(1) a halogen atom,
(2) a hydroxyl group,
(3) a mercapto group,
(4) a nitro group,
(5) a cyano group,
(6) a formyl group,
(7) a carboxyl group,
(8) a trifluoromethyl group,
(9) a trifluoromethoxy group,
(10) an amino group,
(11) an oxo group,
(12) an imino group, and
(13) a group represented by the formula -T^{1x}-T^{2x} (where T^{1x} is a single bond, a C₁₋₆ alkylene group, an oxygen atom, a group represented by the formula -CO-, a group represented by the formula -S-, a group represented by the formula -S(O)-, a group represented by the formula -S(O)₂-, a group represented by the formula -O-CO-, a group represented by the formula -CO-O-, a group represented by the formula -NR^{T}-, a group represented by the formula -CO-NR^{T}-, a group represented by the formula -NR^{T}-CO-, a group represented by the formula -SO₂-NR^{T}-, a group represented by the formula -NR^{T}-SO₂-, a group represented by the formula -NH-CO-NR^{T}- or a group represented by the formula -NH-CS-NR^{T}-;
T^{2x} represents a hydrogen atom, a C₁₋₆ alkyl group, a C₃₋₈ cycloalkyl group, a C₂₋₆ alkenyl group, a C₂₋₆ alkynyl group, a phenyl group, a 1-naphthyl group, a 2-naphthyl group, a 5 to 10-membered heteroaryl group or a 4 to 8-membered heterocyclic group;
R^{T} represents a hydrogen atom, a C₁₋₆ alkyl group, a C₃₋₈ cycloalkyl group, a C₂₋₆ alkenyl group or a C₂₋₆ alkynyl group;
provided that T^{2x} and R^{T} each may independently have 1 to 3 substituents selected from the substituent group T defined below).

### <Substituent group T>

This group consists of: hydroxyl, cyano, a halogen atom, C₁₋₆ alkyl, C₃₋₈ cycloalkyl, C₂₋₆ alkenyl, C₂₋₆ alkynyl, phenyl, 1-naphthyl, 2-naphthyl, 5 to 10-membered heteroaryl, 4 to 8-membered heterocyclic ring, C₁₋₆ alkoxy, C₁₋₆ alkylthio, C₂₋₇ alkoxycarbonyl group, etc.

The <substituent group S> preferably consists of:
(1) a halogen atom,
(2) a hydroxyl group,
(3) a cyano group,
(4) a carboxyl group,
(5) a trifluoromethyl group,
(6) a trifluoromethoxy group,
(7) an amino group,
(8) a C₁₋₆ alkyl group,
(9) a C₃₋₈ cycloalkyl group,
(10) a C₂₋₆ alkenyl group,
(11) a C₂₋₆ alkynyl group,
(12) a phenyl group, and
(13) a C₁₋₆ alkoxy group.

As used herein, the phrase "C₁₋₆ alkyl group which may have one or more substituents" in the substituent group B defined above refers to a "C₁₋₆ alkyl group" which may have one or more groups selected from the substituents referred to in the phrase "which may have one or more substituents" at replaceable positions. Preferably, the "C₁₋₆ alkyl group which may have one or more substituents" refers to a C₁₋₆ alkyl group which may have one or two substituents selected from the group consisting of a cyano group, a carboxyl group, a C₂₋₇ alkoxycarbonyl group, a group represented by the formula -NR^{3T}COR^{4T}, a group represented by the formula -CONR^{3T}R^{4T} (where R^{3T} and R^{4T} each independently represent a hydrogen atom or a C₁₋₆ alkyl group), and a C₁₋₆ alkoxy group.

In formula (I) indicated above, the phrase "when Z² represents a group of the formula -CR²=, R¹, and R² may in combination form a 5 to 7-membered ring" means that compounds represented by formula (I) indicated above includes compounds (III) represented by the formula: 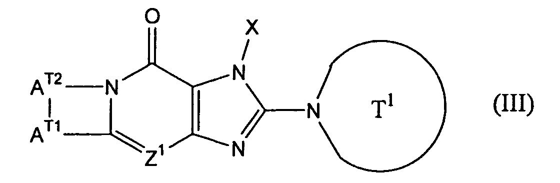 (where Z¹, X, and T¹ are as defined above; A^{T1} represents an oxygen atom, a sulfur atom, a sulfinyl group, a sulfonyl group, a carbonyl group, a methylene group which may have one or more substituents, or a nitrogen atom which may have one or more substituents; A^{T2} represents a C₂₋₆ alkylene group which may have one or more substituents). In formula (III) shown above, A^{T1} preferably represents an oxygen atom, and A^{T2} preferably represents a C₂₋₄ alkylene group.

As used herein, the phrase "cyanobenzyl group" refers to a benzyl group having one cyano group, and specifically, includes, for example, a 2-cyanobenzyl group, a 3-cyanobenzyl group, and a 4-cyanobenzyl group.

As used herein, the phrase "fluorocyanobenzyl group" refers to a benzyl group having one fluorine atom and one cyano group, and specifically, includes, for example, a 2-cyano-4-fluorobenzyl group and a 2-cyano-6-fluorobenzyl group.

As used herein, the phrase "carbamoylphenyloxy group" refers to a phenyloxy group having a group represented by the formula -CONH₂, and specifically, includes, for example, a 2-carbamoylphenyloxy group, a 3-carbamoylphenyloxy group, and a 4-carbamoylphenyloxy group.

As used herein, the phrase "phenyloxy" and "phenoxy" are equivalent.

Herein, there is no limitation on the type of "salts" as long as salts are pharmaceutically acceptable and derived from any compound of the present invention. Such salts include, for example, inorganic acid salts, organic acid salts, inorganic base salts, organic base salts, and acidic or basic amino acid salts.

Examples of preferred inorganic acid salts include hydrochloride, hydrobromide, sulfate, nitrate, and phosphate. Examples of preferred organic salts include acetate, succinate, fumarate, maleate, tartrate, citrate, lactate, stearate, benzoate, methanesulfonate, and p-toluene sulfonate.

Examples of preferred inorganic base salts include: alkali metal salts such as sodium salts and potassium salts; alkaline earth metal salts such as calcium salts and magnesium salts; aluminum salts; and ammonium salts. Examples of preferred organic base salts include diethylamine salts, diethanolamine salts, meglumine salts, and N,N'-dibenzylethylenediamine salts.

Examples of preferred acidic amino acid salts include aspartate and glutamate. Examples of preferred basic amino acid salts include arginine salts, lysine salts, and ornithine salts.

As used herein, the phrase "enhancing the effects of active circulating GLP-1 and/or active circulating GLP-2" means that the effects of active circulating GLP-1 and/or active circulating GLP-2 are enhanced due to increased blood levels of these peptides, which results from their enhanced secretion or suppressed degradation.

As used herein, the phrase "enhancing the effects of active circulating GLP-2" means that the effects of active circulating GLP-2 are enhanced due to the increased blood level of this peptide, which results from its enhanced secretion or suppressed degradation.

The effects of active circulating GLP-1 include: enhancing secretion of insulin in a glucose-dependent manner; enhancing biosynthesis of insulin; suppressing secretion of glucagon; promoting β cell renewal; activating glycogen synthase in the liver; suppressing food intake; suppressing weight gain; suppressing gastric emptying; and suppressing gastric acid secretion.

The effects of active circulating GLP-2 include: promoting growth of intestinal epithelial cells; promoting growth of epithelial cells in the gastrointestinal tract; suppressing apoptosis of epithelial cells in the gastrointestinal tract; maintaining the gastrointestinal barrier function; enhancing glucose absorption; suppressing secretion of gastric acid; and enhancing blood flow in the gastrointestinal tract.

The phrase "enhancing the effect(s)" means enhancing the effects described above.

As used herein, the "biguanide agent" refers, for example, to phenformin, metformin, and buformin, which are agents that have the following effects: suppressing gluconeogenesis and glycogenolysis in the liver; potentiating the susceptibility of skeletal muscle to insulin; suppressing glucose absorption in the intestinal tract; and decreasing weight by suppressing food intake. A preferred biguanide agent is metformin.

Herein, the "disease which is associated with active circulating GLP-1 and/or active circulating GLP-2" include, for example, diabetes, obesity, hyperlipidemia, hypertension, arteriosclerosis, and gastrointestinal diseases.

Herein, the "disease which is associated with active circulating GLP-2" includes, for example, gastrointestinal diseases.

As used herein, the phrase "and/or" means both "and" and "or".

(S)-1-((3-hydroxy-1-adamantyl)amino)acetyl-2-cyanopyrrolidine can be produced by the method described in US Patent No. 6166063.
(S)-1-(2-((5-cyanopyridin-2-yl)amino)ethyl-aminoacetyl)-2-cyanopyrrolidine can be produced by the method described in US Patent No. 6011155.

Isoleucine thiazolidide, isoleucine pyrrolidide, and valine pyrrolidide can be produced according to the method described in US Patent No. 6548481.

The compound represented by formula (II) indicated herein can be produced by the method described below in [Typical synthesis methods] or any one of the methods described in US Patent Application Publication No. 2002/0161001; US Patent Application Publication No. 2003/0105077; and US Patent Application Publication No. 2002/0198205.

### [Typical synthesis methods]

Representative methods for producing compounds of the present invention, represented by formulae (I) and (II) above are described below.

Each symbol in the production methods is defined below. R³¹ to R⁴², n, m, R¹, R², X, A⁰, A¹, A², R^{A}, and T¹ are the same as defined above.

U¹, U³ and Hal each independently represent a leaving group such as a chlorine atom, a bromine atom, an iodine atom, a methanesulfonyloxy group, or a *p*-toluenesulfonyloxy group.

R^{p1}, R^{p2}, and R^{p3} each independently represent an -NH-protecting group such as a pivalyloxymethyl group and a trimethylsilylethoxymethyl group.

R^{p4} represents a hydroxyl group-protecting group such as a *t*-butyldimethylsilyl group and a *t*-butyldiphenylsilyl group.

R^{p5} represents an NH-protecting group such as N,N-dimethylsulfamoyl, trityl, benzyl, and *t*-butoxycarbonyl.

U² and U⁴ each independently represent a chlorine atom, a bromine atom, an iodine atom, a methanesulfonyloxy group, a *p*-toluenesulfonyloxy group, a group represented by the formula -B(OH)₂, a 4,4,5,5-tetramethyl-1,3,2-dioxaboran-2-yl group, or a group represented by the formula -Sn(R^{z})₃ (where R^{z} represents a C₁₋₆ alkyl group).

R^{x2} is a group represented by the formula -O-A², a group represented by the formula -S-A², a group represented by the formula -N(R^{A})A², or a 4- to 8-membered heterocyclic group which may have one or more substituents (for example, 1-pyrrolidinyl, 1-morpholinyl, 1-piperazinyl, or 1-piperidyl), etc.

R^{x3} represents a group of the formula -A⁰-A¹-A², such as a cyano group, a C₁₋₆ alkyl group which may have one or more substituents, a C₃₋₈ cycloalkyl group which may have one or more substituents, a C₂₋₆ alkenyl group which may have one or more substituents, a C₂₋₆ alkynyl group which may have one or more substituents, and a C₆₋₁₀ aryl group which may have one or more substituents.

A^{2COOR} represents a C₁₋₆ alkyl group, a C₃₋₈ cycloalkyl group, a C₂₋₆ alkenyl group, a C₂₋₆ alkynyl group, a C₆₋₁₀ aryl group, a 5- to 10-membered heteroaryl group, a 4- to 8-membered heterocyclic group, a 5- to 10-membered heteroaryl C₁₋₆ alkyl group, or a C₆₋₁₀ aryl C₁₋₆ alkyl group, each of which contains an ester group.

A^{2COOH} represents a C₁₋₆ alkyl group, a C₃₋₈ cycloalkyl group, a C₂₋₆ alkenyl group, a C₂₋₆ alkynyl group, C₆₋₁₀ aryl group, a 5- to 10-membered heteroaryl group, a 4- to 8-membered heterocyclic group, a 5- to 10-membered heteroaryl C₁₋₆ alkyl group, or a C₆₋₁₀ aryl C₁₋₆ alkyl group, each of which contains a carboxylic acid.

A^{2NO2} represents a C₁₋₆ alkyl group, a C₃₋₈ cycloalkyl group, a C₂₋₆ alkenyl group, a C₂₋₆ alkynyl group, a C₆₋₁₀ aryl group, a 5- to 10-membered heteroaryl group, a 4- to 8-membered heterocyclic group, a 5- to 10-membered heteroaryl C₁₋₆ alkyl group, or a C₆₋₁₀ aryl C₁₋₆ alkyl group, each of which contains a nitro group.

A^{2NH2} represents a C₁₋₆ alkyl group, a C₃₋₈ cycloalkyl group, a C₂₋₆ alkenyl group, a C₂₋₆ alkynyl group, a C₆₋₁₀ aryl group, a 5- to 10-membered heteroaryl group, a 4- to 8-membered heterocyclic group, a 5- to 10-membered heteroaryl C₁₋₆ alkyl group, or a C₆₋₁₀ aryl C₁₋₆ alkyl group, each of which contains an amino group.

A^{2CN} represents a C₁₋₆ alkyl group, a C₃₋₈ cycloalkyl group, a C₂₋₆ alkenyl group, a C₂₋₆ alkynyl group, a C₆₋₁₀ aryl group, a 5- to 10-membered heteroaryl group, a 4- to 8-membered heterocyclic group, a 5- to 10-membered heteroaryl C₁₋₆ alkyl group, or a C₆₋₁₀ aryl C₁₋₆ alkyl group, each of which contains a nitrile group.

A^{CONH2} represents a C₁₋₆ alkyl group, a C₃₋₈ cycloalkyl group, a C₂₋₆ alkenyl group, a C₂₋₆ alkynyl group, C₆₋₁₀ aryl group, a 5- to 10-membered heteroaryl group, a 4- to 8-membered heterocyclic group, a 5- to 10-membered heteroaryl C₁₋₆ alkyl group, or a C₆₋₁₀ aryl C₁₋₆ alkyl group, each of which contains a carboxylic amide group.

M represents -MgCl, -MgBr, -Sn(R^{z})₃ (where R^{z} is as defined above), etc.

The term "room temperature" refers to a temperature of about 20 to about 30°C.

T^{1a} is defined as the group represented by T¹, or represents a group of the formula: 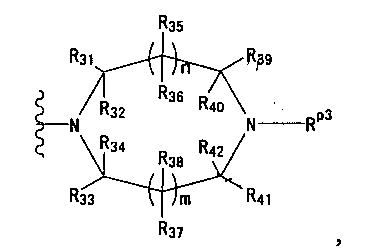 a group represented by the formula: 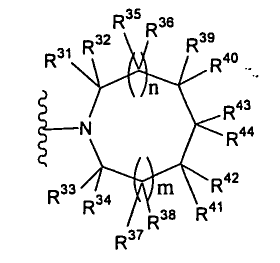 (where R³¹ to R⁴⁴ are as defined above, except that any one of R³¹ to R⁴⁴ represents -NH-R^{p3}), or a group represented by the formula: 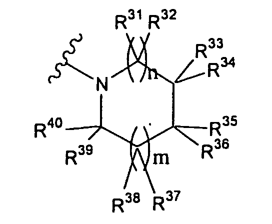 (where R³¹ to R⁴⁰ are as defined above, except that any one of R³¹ to R⁴⁰ represents -NH-R^{p3}).

s represents 1 to 4.

R⁵¹ to R⁵⁴ each independently represent a hydrogen atom, a C₁₋₆ alkyl group, or a C₆₋₁₀ aryl group.

In examples of reactions represented by the following reaction schemes, unless otherwise specified, quantities of reagents, catalysts, and others, to be used (equivalent, weight%, and weight ratio) are represented as ratios to a main compound in each reaction scheme. A main compound refers to a compound represented by a chemical formula in the reaction scheme and having the backbone of compounds of the present invention.

### Production method A

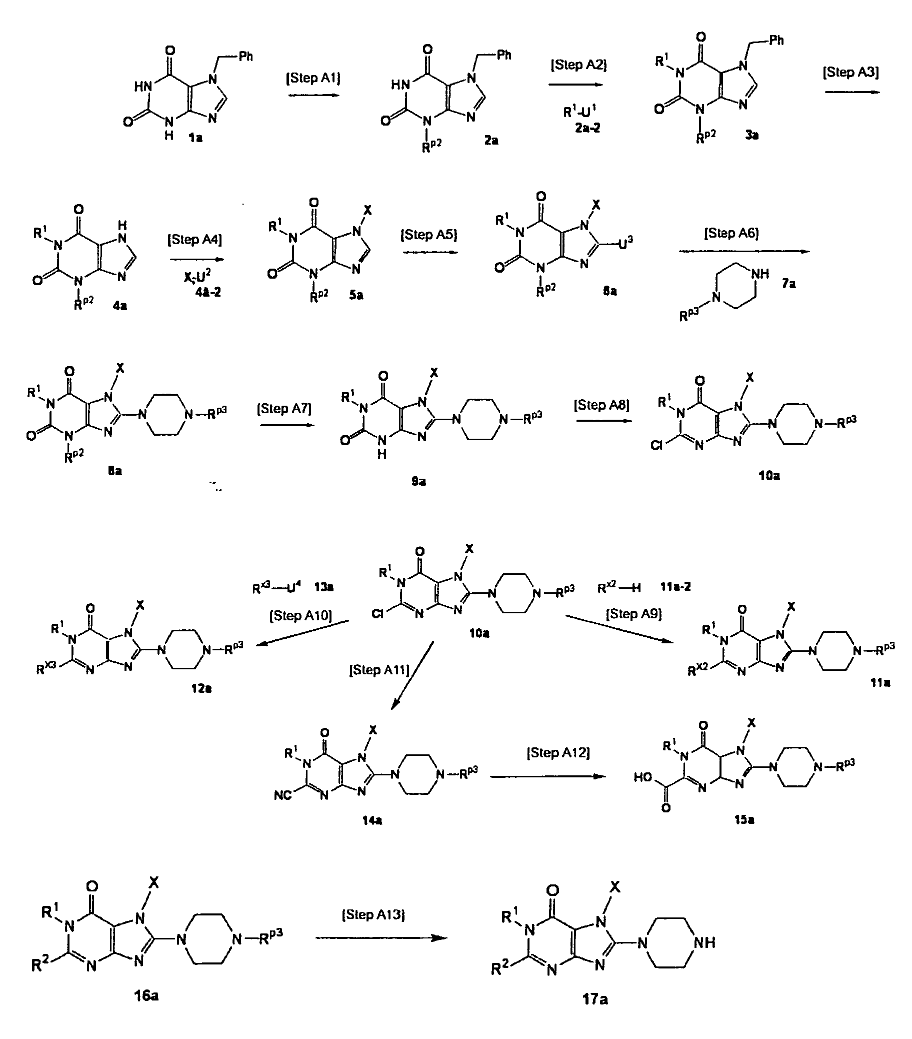

### [Step A1]

In this step, an -NH-protecting reagent is reacted with compound (1a) [CAS No. 56160-64-6] to give compound (2a). The reaction conditions are selected depending on the type of -NH-protecting reagent to be used. The reaction may be performed under conditions that are generally used to introduce a protecting group using the reagent.

An -NH-protecting reagent can be a reagent that is generally used to introduce an -NH-protecting group. Specifically, such -NH-protecting reagents include, for example, chloromethyl pivalate. It is preferable to use 1 to 2 equivalents of a protecting reagent. Solvents for the reaction include acetonitrile, N,N-dimethylformamide, N-methylpyrrolidone, 1,4-dioxane, tetrahydrofuran, and dimethoxyethane. N,N-dimethylformamide is preferably used.

The reaction can be achieved in the presence of a base. Examples of bases to be used in the reaction include cesium carbonate, lithium carbonate, sodium carbonate, potassium carbonate, and sodium hydride. Sodium hydride is preferably used. In this case, a base is preferably used in an amount of 1 to 5 equivalents. The reaction can be conducted at a temperature ranging from 0°C to 150°C. A preferred reaction temperature is room temperature.

### [Step A2]

In this step, compound (2a) is reacted with compound (2a-2) to give compound (3a).

Compound (2a-2) can be any compound that is an electrophilic reagent such as an alkyl halide. Specific examples include alkyl halides such as iodomethane, iodoethane, iodopropane, and benzyl bromide; alkenyl halides such as allyl bromide and 1-bromo-3-methyl-2-butene; and alkynyl halides such as propargyl bromide and 1-bromo-2-butyne. One to two equivalents of an electrophilic reagent are preferably used.

Solvents for the reaction include, for example, dimethyl sulfoxide, N,N-dimethylformamide, N-methylpyrrolidone, dioxane, tetrahydrofuran, and toluene.

The reaction can be achieved in the presence or absence of a base. Examples of bases to be used in the reaction include lithium hydroxide, sodium hydroxide, potassium hydroxide, lithium carbonate, sodium carbonate, potassium carbonate, cesium carbonate, lithium hydride, sodium hydride, potassium hydride, butyllithium, methyllithium, lithium bis(trimethylsilyl)amide, sodium bis(trimethylsilyl)amide, and potassium bis(trimethylsilyl)amide. In this case, one to two equivalents of a base are preferably used. The reaction can be conducted at a temperature ranging from 0°C to 150°C.

### [Step A3]

In this step, the benzyl group at the 7-position is removed from compound (3a) to give compound (4a).

Specifically, compound (4a) can be prepared from compound (3a), for example, by catalytic reduction under a hydrogen atmosphere in the presence of a metal catalyst, but the reaction conditions are not limited thereto.

Specific solvents for the reaction include, for example, methanol, ethanol, propanol, acetic acid, dimethyl sulfoxide, N,N-dimethylformamide, N-methylpyrrolidone, dioxane, tetrahydrofuran, and toluene. Examples of metal catalysts include palladium carbon, platinum oxide, and Raney nickel. A metal catalyst is preferably used at 0.5 to 50 weight%. A preferred hydrogen pressure is 1 to 5 atm. The reaction can be conducted at a temperature ranging from 0°C to 150°C.

### [Step A4]

In this step, compound (4a) is reacted with compound (4a-2) to give compound (5a).

Specific examples of compound (4a-2) are: alkyl halides such as iodomethane, iodoethane, iodopropane, and benzyl bromide; alkenyl halides such as allyl bromide and 1-bromo-3-methyl-2-butene; or alkynyl halides such as propargyl bromide and 1-bromo-2-butyne. These halides are preferably used in an amount of one to two equivalents.

Solvents for the reaction include dimethyl sulfoxide, N,N-dimethylformamide, N-methylpyrrolidone, dioxane, tetrahydrofuran, and toluene.

The reaction can be carried out in the presence or absence of a base. Examples of bases to be used in the reaction include lithium hydroxide, sodium hydroxide, potassium hydroxide, lithium carbonate, sodium carbonate, potassium carbonate, cesium carbonate, lithium hydride, sodium hydride, potassium hydride, butyllithium, methyllithium, lithium bis(trimethylsilyl)amide, sodium bis(trimethylsilyl)amide, and potassium bis(trimethylsilyl)amide. In this case, 1 to 4 equivalents of a base are preferably used. The reaction can be conducted at a temperature ranging from 0°C to 150°C.

Compound (5a) can be obtained by reacting compound (4a) with compound (4a-2) in the presence of a copper catalyst and a base. In this case, it is preferable to use 0.1 to 2 equivalents of a copper catalyst and 1 to 10 equivalents of a base.

In this reaction, compound (4a-2) may be arylboronic acid, heteroarylboronic acid, or such, in which X is a C₆₋₁₀ aryl group which may have one or more substituents or a 5- to 10-membered heteroaryl group which may have one or more substituents, and U² is -B(OH)₂ or such. One to three equivalents of compound (4a-2) are preferably used.

In this case, reaction solvents include dichloromethane, chloroform, 1,4-dioxane, tetrahydrofuran, toluene, pyridine, N,N-dimethylformamide, and N-methylpyrrolidone.

Bases include triethylamine, diisopropylethylamine, pyridine, and N,N-dimethylaminopyridine. Copper catalysts include copper (II) acetate, copper (II) trifluoroacetate, copper (II) chloride, and copper (II) iodide. The reaction can be conducted at a temperature ranging from 0°C to 150°C.

### [Step A5]

In this step, compound (5a) is reacted with a halogenating agent to give compound (6a).

Specific examples of halogenating agents include, for example, N-chlorosuccinimide, N-bromosuccinimide, and N-iodosuccinimide. A halogenating agent is preferably used in an amount of 1 to 4 equivalents.

Solvents for the reaction include acetonitrile, N,N-dimethylformamide, N-methylpyrrolidone, 1,4-dioxane, tetrahydrofuran, and dimethoxyethane. The reaction can be conducted at a temperature ranging from 0°C to 150°C.

### [Step A6]

In this step, compound (6a) is reacted with compound (7a) to give compound (8a). In this case, 1 to 4 equivalents of compound (7a) are preferably used.

The reaction can be carried out, for example, in a solvent such as tetrahydrofuran, acetonitrile, N,N-dimethylformamide, N-methylpyrrolidone, methanol, ethanol, 1,4-dioxane, toluene, and xylene, or in the absence of a solvent. The reaction can be conducted at a temperature ranging from 0°C to 200°C in the presence or absence of a base. Examples of a base include triethylamine, potassium carbonate, and 1,8-diazabicyclo[5,4,0]undecene. In this case, 1 to 4 equivalents of a base are preferably used.

### [Step A7]

In this step, the -NH-protecting group at the 3-position of compound (8a) is removed to give compound (9a). The reaction conditions are selected depending on the type of -NH-protecting group to be removed. The deprotection reaction may be preformed under conditions that are generally used for the protecting group.

For example, when R^{p2} is a pivalyloxymethyl group, the reaction can be carried out in methanol, or a mixed solution of methanol and tetrahydrofuran, using a base such as sodium methoxide, sodium hydride, or 1,8-diazabicyclo[5,4,0]-7-undecene at a temperature of 0°C to 150°C. In this case, 0.1 to 2 equivalents of a base are preferably used.

Alternatively, when R^{p2} is a trimethylsilylethoxymethyl group, the reaction can be carried out in a solvent such as acetonitrile, N,N-dimethylformamide, N-methylpyrrolidone, 1,4-dioxane, tetrahydrofuran, or dimethoxyethane, using a fluoride reagent such as tetrabutyl ammonium fluoride or cesium fluoride at a temperature of 0°C to 150°C. In this case, 1 to 5 equivalents of a fluoride reagent are preferably used.

### [Step A8]

In this step, compound (9a) is chlorinated to give compound (10a).

There are no particular limitations on the reaction conditions, and the reaction can be conducted under standard conditions for chlorination. For example, the reaction can be carried out at a temperature ranging from 0°C to 150°C in a solvent such as phosphorus oxychloride. In this case, it is preferable to use a 10 to 200 times amount of halogenating agent by weight.

When R^{p3} is a *t*-butoxycarbonyl group or such, which is removed under the above-described conditions using phosphorus oxychloride or such, the protecting group should be reintroduced.

There are no particular limitations on the reaction conditions for the protection. In the case of the *t*-butoxycarbonyl group, the reaction can be carried out using an -NH- protection reagent such as di-*t*-butyl dicarbonate, in a solvent such as acetonitrile, N,N-dimethylformamide, N-methylpyrrolidone, 1,4-dioxane, tetrahydrofuran, or dimethoxyethane in the presence of a base such as lithium hydroxide, sodium hydroxide, potassium hydroxide, lithium carbonate, sodium carbonate, potassium carbonate, cesium carbonate, potassium bicarbonate, sodium bicarbonate, or triethylamine at 0 to 150°C.

### [Step A9]

In this step, compound (10a) is reacted with compound (11a-2) to give compound (11a).

Compound (11a-2) includes alcohol compounds or phenol compounds represented by A²-OH, amine compounds represented by A²(R^{A})NH or such, and thiol compounds represented by A²-SH. In this case, compound (11a-2) is preferably used in an amount of 1 to 10 equivalents or 5 to 100 times by weight.

Solvents for the reaction include acetonitrile, N,N-dimethylformamide, N-methylpyrrolidone, 1,4-dioxane, tetrahydrofuran, dimethoxyethane, methanol, and ethanol.

The reaction can be carried out in the presence or absence of a base. Bases to be used in the reaction include lithium hydroxide, sodium hydroxide, potassium hydroxide, lithium carbonate, sodium carbonate, potassium carbonate, cesium carbonate, lithium hydride, sodium hydride, potassium hydride, butyllithium, methyllithium, lithium bis(trimethylsilyl)amide, sodium bis(trimethylsilyl)amide, potassium bis(trimethylsilyl)amide, and triethylamine. In this case, 1 to 10 equivalents of a base is preferably used. The reaction can be conducted at a temperature ranging from 0°C to 150°C.

### [Step A10]

In this step, compound (10a) is reacted with compound (13a) in the presence of a metal catalyst to give compound (12a). In this case, 1 to 50 equivalents of compound (13a) are preferably used.

Solvents for the reaction include acetonitrile, N,N-dimethylformamide, N-methylpyrrolidone, 1,4-dioxane, tetrahydrofuran, dimethoxyethane, methanol, and ethanol.

Metal catalysts include palladium catalyst and copper catalyst. Palladium catalysts include tetrakis triphenylphosphine palladium, palladium acetate, and dibenzylideneacetone palladium. Copper catalyst include copper iodide. It is preferable to use 0.01 to 2 equivalents of a metal catalyst.

The reaction can be conducted in the presence of an organophosphorous ligand. When the reaction is carried out in the presence of an organophosphorous ligand, examples of the ligands include o-tolyl phosphine and diphenylphosphinoferrocene. In this case, it is preferable to use 1 to 5 equivalents of an organophosphorous ligand to the metal catalyst.

The reaction can be carried out in the presence or absence of a base. Bases to be used in the reaction include lithium hydroxide, sodium hydroxide, potassium hydroxide, lithium carbonate, sodium carbonate, potassium carbonate, cesium carbonate, lithium hydride, sodium hydride, potassium hydride, potassium phosphate, lithium bis trimethylsilyl amide, sodium bis trimethylsilyl amide, potassium bis trimethylsilyl amide, and triethylamine. The reaction can be conducted at a temperature ranging from 0°C to 150°C.

### [Step A11]

In this step, compound (10a) is reacted with a cyanidation reagent to give compound (14a).

Specifically, cyanidation reagents include, for example, sodium cyanide and potassium cyanide. It is preferably used in an amount of 1 to 20 equivalents.

Solvents for the reaction include, for example, acetonitrile, N,N-dimethylformamide, N-methylpyrrolidone, 1,4-dioxane, tetrahydrofuran, dimethoxyethane, methanol, and ethanol. The reaction can be conducted at a temperature ranging from 0°C to 150°C.

### [Step A12]

In this step, the cyano group of compound (14a) is hydrolyzed to give compound (15a). There are no particular limitations on the reaction conditions, and the reaction can be carried out under conditions generally used for the conversion of a cyano group to a carbamoyl group by hydrolysis.

Solvents for the reaction include N,N-dimethylformamide, N-methylpyrrolidone, 1,4-dioxane, tetrahydrofuran, dimethoxyethane, methanol, ethanol, and a mixed solvent of tetrahydrofuran and methanol.

The reaction can be carried out in the presence or absence of a base. When a base is used, the reaction can be carried out using an aqueous solution of a base such as potassium hydroxide, sodium hydroxide, lithium hydroxide, or ammonia. The reaction can be achieved after adding an aqueous solution of hydrogen peroxide (preferably an aqueous solution of 30% hydrogen peroxide).

The reaction can be conducted at a temperature ranging from 0°C to 150°C.

### [Step A13]

In this step, R^{p3} of compound (16a) is removed to give compound (17a). Compounds (11a), (12a), (14a), (15a), and others can be used as compound (16a).

The deprotection reaction for R^{p3} can be carried out under standard reaction conditions for removing an -NH-protecting group.

For example, when R^{p3} is a *t*-butoxycarbonyl group, the reaction can be carried out in the presence of an acid such as an anhydrous methanol solution of hydrogen chloride, an anhydrous ethanol solution of hydrogen chloride, an anhydrous dioxane solution of hydrogen chloride, trifluoroacetic acid, or formic acid.

An alternative method for producing compound (10a) is described below.

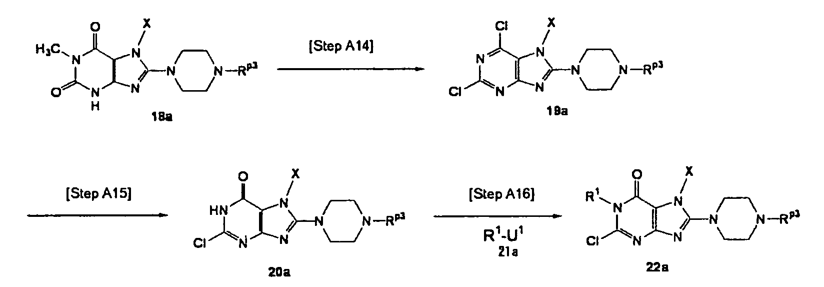

### [Step A14]

In this step, compound (18a) is chlorinated to give compound (19a). There are no particular limitations on the reaction conditions, and the reaction can be conducted under standard conditions for chlorination. For example, the reaction can be carried out in a solvent such as phosphorus oxychloride at a temperature ranging from 0°C to 150°C. Preferably 10 to 200 times by weight of chlorination reagent is used.

When R^{p3} is a *t*-butoxycarbonyl group or such, which is removed under the above-described condition using phosphorus oxychloride or such, the protecting group should be reintroduced.

There are no particular limitations on the reaction conditions for the protection, and when R^{p3} is a *t*-butoxycarbonyl group, the reaction can be carried out using an -NH- protection reagent such as di-*t*-butyl dicarbonate, in a solvent such as acetonitrile, N,N-dimethylformamide, N-methylpyrrolidone, 1,4-dioxane, tetrahydrofuran, and dimethoxyethane, in the presence of a base such as lithium hydroxide, sodium hydroxide, potassium hydroxide, lithium carbonate, sodium carbonate, potassium carbonate, cesium carbonate, potassium bicarbonate, sodium bicarbonate, or triethylamine at a temperature ranging from 0°C to 150°C.

### [Step A15]

In this step, compound (19a) is partially hydrolyzed to give compound (20a). The reaction is carried out in the presence of a base such as sodium acetate, potassium carbonate, or sodium hydroxide. One to ten equivalents of a base are preferably used. Solvents for the reaction include dimethyl sulfoxide, N-methylpyrrolidone, tetrahydrofuran, water, and mixtures thereof. The reaction can be conducted at a temperature ranging from 0°C to 100°C.

### [Step A16]

In this step, compound (20a) is reacted with compound (21 a) to give compound (22a). The reaction can be conducted under the same conditions as used in [Step A2] of production method A.

An alternative method for producing compound (19a) is described below.

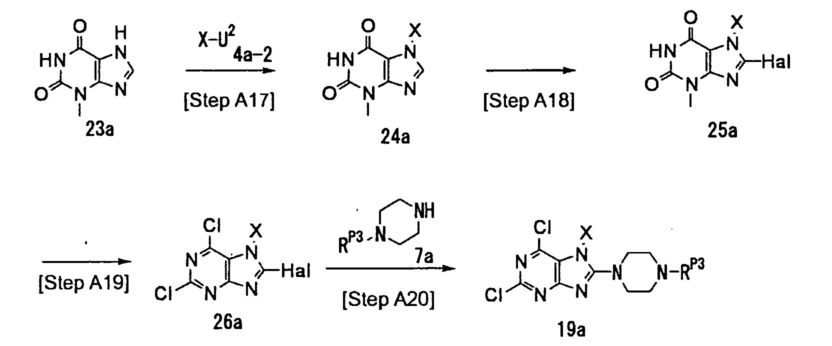

### [StepA17]

In this step, a substitution reaction is carried out using compound (23a) [CAS No. 1076-22-8] and compound (4a-2) to give compound (24a).

The reaction can be conducted under the same conditions as used in [Step A4] of production method A.

### [Step A18]

In this step, compound (24a) is reacted with a halogenating agent to give compound (25a).

The reaction can be conducted under the same conditions as used in [Step A5] of production method A.

### [Step A19]

In this step, compound (25a) is chlorinated to give compound (26a).

There are no particular limitations on the reaction conditions, and compound (25a) can be reacted with phosphorus oxychloride, phosphorus pentachloride, or a mixture thereof in a solvent or in the absence of a solvent at a temperature of 0°C to 150°C. Solvents include, for example, toluene, acetonitrile, and dichloroethane.

### [Step A20]

In this step, compound (26a) is reacted with compound (7a) to give compound (19a).

The reaction can be conducted under the same conditions as used in [Step A6] of production method A.

### Production method B

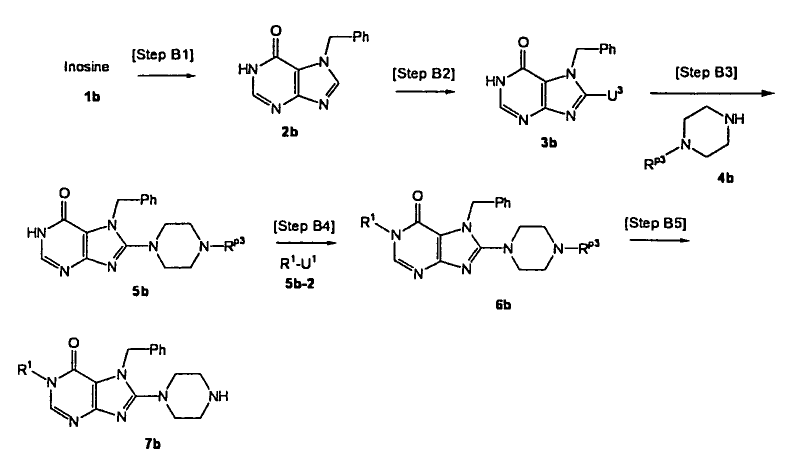

### [Step B1]

In this step, compound (1b) is benzylated and the sugar chain is cleaved to give compound (2b).

There are no particular limitations on the reaction conditions. Compound (2b) can be obtained by reacting compound (1b) with benzyl bromide in a solvent such as acetonitrile, N,N-dimethylformamide, N-methylpyrrolidone, dimethyl sulfoxide, 1,4-dioxane, tetrahydrofuran, dimethoxyethane, methanol, or ethanol, at a temperature of 0°C to 150°C, adding 3 to 10 equivalents of hydrochloric acid, and incubating the mixture at a temperature of 0°C to 150°C to cleave the sugar moiety. It is preferable to use 1 to 3 equivalents of benzyl bromide.

### [Step B2]

In this step, compound (2b) is reacted with a halogenating agent to give compound (3b). The halogenation reaction can be conducted under the same conditions as used in [Step A5] of production method A.

### [Step B3]

In this step, compound (3b) is reacted with compound (4b) to give compound (5b). The reaction can be conducted under the same conditions as used in [Step A6] of production method A.

### [Step B4]

In this step, compound (5b) is reacted with compound (5b-2) to give compound (6b). The reaction can be conducted under the same condition as used in [Step A2] of production method A.

### [Step B5]

In this step, R^{p3} of compound (6b) is removed to give compound (7b). The reaction can be conducted under the same conditions as used in [Step A13] of production method A. Production method B-2

Compound (9b) represented by the formula: 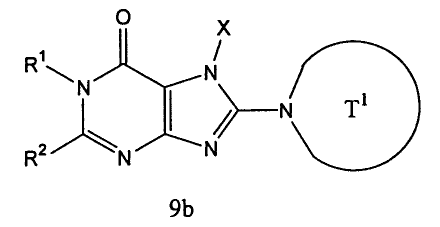 can be obtained by using compound (8b) represented by H-T^{1a}, instead of compound (7a) in [Step A6] of production method A described above under the same reaction conditions as used in [Step A6], and then appropriately applying [Step A7] to [Step A13] described above.

Compound (10b) represented by the formula: 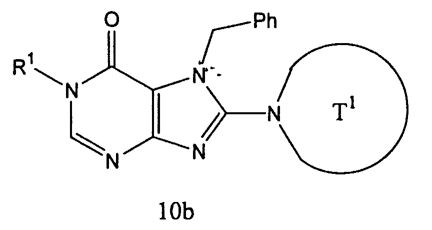 can be obtained by using compound (8b) represented by H-T^{1a}, instead of compound (3b) in [Step B3] of production method B described above under the same reaction conditions as used in [Step B3] and then appropriately applying [Step B4] to [Step B6] described above. Preferable examples of compound (8b) include piperidin-3-yl carbamic acid *t*-butyl ester.

### Production method C

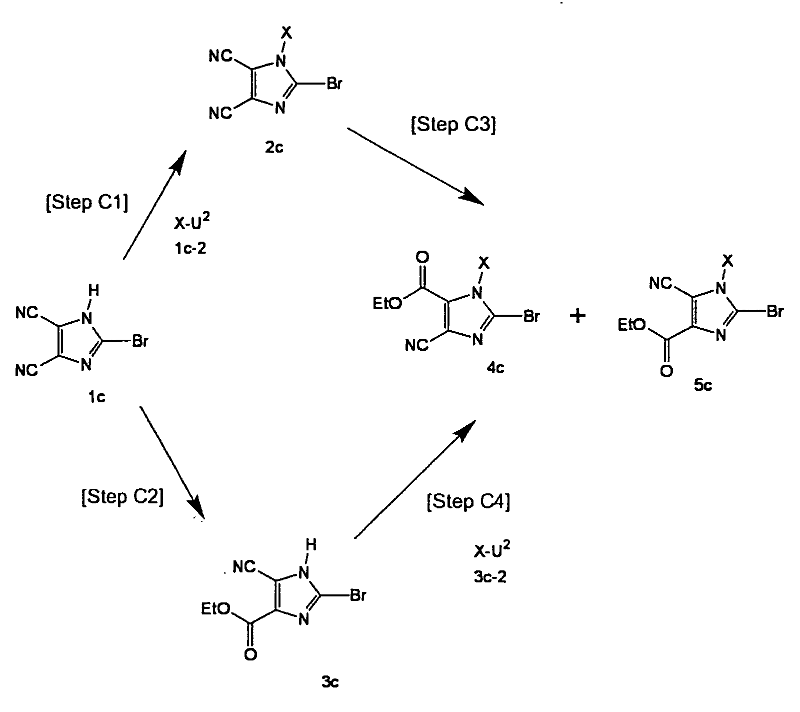

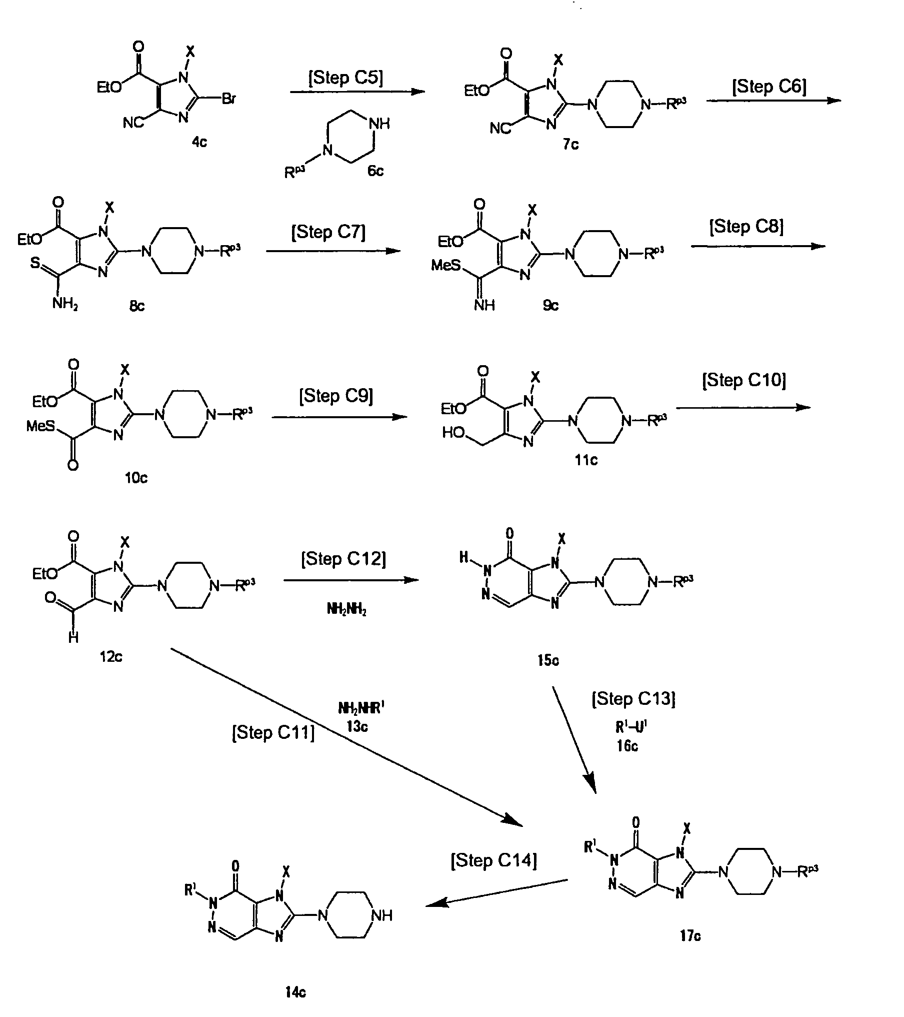

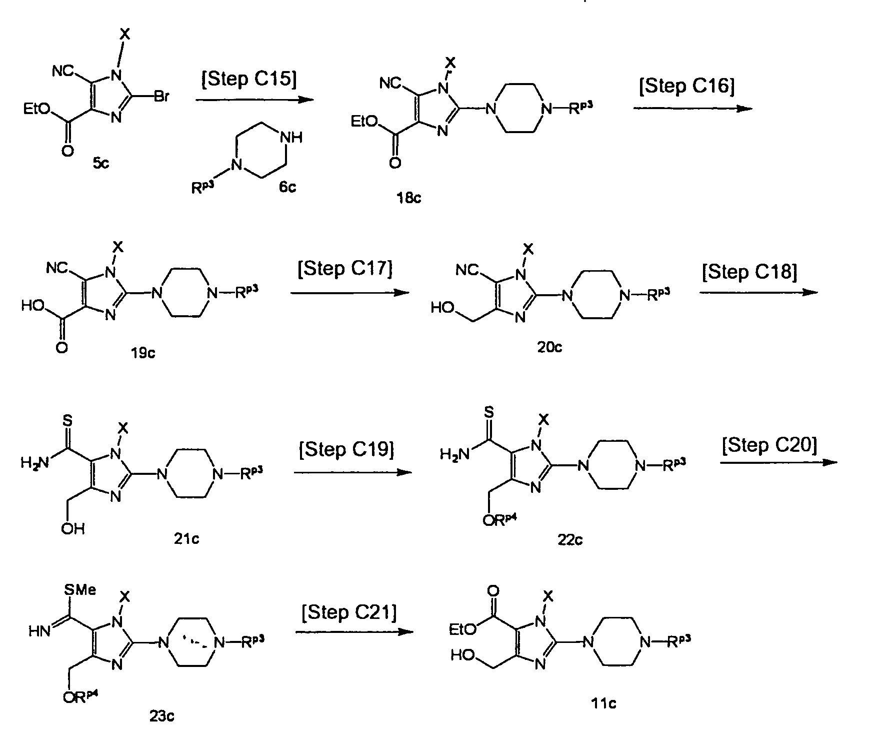

### [Step C1]

In this step, compound (1c) is reacted with compound (1c-2) to give compound (2c). The reaction can be conducted under the same conditions as used in [Step A4] of production method A.

### [Step C2]

In this step, compound (1c) is reacted with ethanol to give compound (3c).

Compound (3c) can be obtained, for example, by heating an ethanol solution of compound (2c) under reflux in the presence of an acid such as sulfuric acid or hydrochloric acid. However, the reaction conditions are not limited thereto. In this reaction, it is preferable to use one to two equivalents of an acid.

### [Step C3]

In this step, compound (2c) is reacted with ethanol to give compounds (4c) and (5c). The reaction can be conducted under the same conditions as used in [Step C2] of production method C.

### [Step C4]

In this step, compound (3c) is reacted with compound (3c-2) to give compounds (4c) and (5c). The reaction can be conducted under the same conditions as used in [Step A4] of production method A.

### [Step C5]

In this step, compound (4c) is reacted with compound (6c) to give compound (7c). The reaction can be conducted under the same conditions as used in [Step A6] of production method A.

### [Step C6]

In this step, compound (7c) is thioamidated to give compound (8c). Solvents for the reaction include methanol, ethanol, N,N-dimethylformamide, N-methylpyrrolidone, 1,4-dioxane, tetrahydrofuran, and dimethoxyethane. Thioamidation reagents include ammonium sulfide, sodium sulfide, and hydrogen sulfide. It is preferable to use 2 to 10 equivalents of a thioamidation reagent. When hydrogen sulfide is used as the thioamidation reagent, the reaction is carried out in the presence of a base such as triethylamine or N,N-diisopropylethylamine. The reaction can be conducted at a temperature ranging from 0°C to 150°C.

### [Step C7]

In this step, compound (8c) is reacted with a methylating reagent to give compound (9c). Methylating reagents include trimethyl oxonium tetrafluoroborate, methyl sulfate, methyl iodide, and trimethyl phosphite. It is preferable to use 1.0 to 1.5 equivalent of the methylating reagent.

When trimethyl oxonium tetrafluoroborate is used as the methylating reagent, compound (9c) can be obtained by carrying out the reaction in a halogenated solvent such as dichloromethane at a temperature ranging from 0°C to 50°C.

When methyl sulfate, methyl iodide, or trimethyl phosphite is used as the methylating reagent, compound (9c) can be obtained by carrying out the reaction in the presence of a base such as potassium carbonate, triethylamine, or N,N-diisopropylethylamine. In this case, it is preferable to use 1.0 to 1.5 equivalent of a base. Solvents for the reaction include acetone, N,N-dimethylformamide, N-methylpyrrolidone, 1,4-dioxane, tetrahydrofuran, and dimethoxyethane. The reaction can be performed at a temperature ranging from 0°C to 100°C.

### [Step C8]

In this step, compound (9c) is hydrolyzed to give compound (10c).

There are no particular limitations on the reaction conditions for the hydrolysis. The reaction can be carried out in a mixed solvent of ethanol and water in the presence of an acid such as sulfuric acid, hydrochloric acid, or p-toluenesulfonic acid, at a temperature ranging from 0°C to 80°C. In this case, it is preferable to use 5 to 50 equivalents of the acid.

When R^{p3} is a group, such as a *t*-butoxycarbonyl group, which is removed under the above-described condition, the protecting group should be reintroduced. There are no particular limitations on the reaction conditions for the introduction of this protecting group. When R^{p3} is a *t*-butoxycarbonyl group, the reaction can be carried out using a reagent such as t-butyl dicarbonate in a solvent such as dichloromethane, chloroform, N,N-dimethylformamide, or tetrahydrofuran, in the presence of a base such as pyridine, 4-aminopyridine, triethylamine, and N,N-diisopropylethylamine, at a temperature ranging from 0°C to 80°C. In this case, it is preferable to use 2 to 3 equivalents of a base.

### [Step C9]

In this step, compound (10c) is reacted with a reducing agent to give compound (11c).

There are no particular limitations on the reaction conditions for the reduction. The reaction can be achieved by reacting compound (10c) with hydrogen in the presence of Raney nickel in a solvent such as benzene, ethanol, 2-propanol, or acetone, at a temperature ranging from 0°C to 50°C, or alternatively reacting compound (10c) with a reducing agent such as sodium borohydride, in a solvent such as methanol, ethanol, or 2-methyl-2-propanol, or in a mixed solvent of water and tetrahydrofuran at a temperature ranging from 0°C to 50°C, or alternatively reacting compound (10c) with a reducing agent such as sodium borohydride, in the presence of 1 to 5 equivalents of a mercury salt such as mercuric acetate in a solvent such as methanol, ethanol, or 2-methyl-2-propanol at a temperature ranging from 0°C to 50°C. It is preferable to use two to three equivalents of a reducing agent.

### [Step C10]

In this step, compound (11c) is subjected to an oxidation reaction to give compound (12c).

When an oxidant such as manganese dioxide, pyridinium chlorochromate, or pyridinium dichromate is used in the oxidation reaction, compound (12c) can be obtained by carrying out the reaction in a solvent such as dichloromethane or chloroform, at a temperature ranging from 20°C to 80°C. Alternatively, compound (12c) can also be obtained by carrying out the reaction under standard conditions for the oxidation of a primary alcohol to aldehyde, such as Swern oxidation. It is preferable to use 5 to 20 equivalents of an oxidant.

### [Step C11]

In this step, compound (12c) is reacted with compound (13c) to give compound (17c). In this case, it is preferable to use 2 to 10 equivalents of compound (13c).

Compound (17c) can be obtained, for example, by combining compounds (12c) and (13c) in a solvent such as methanol, ethanol, 1-methyl-2-pyrrolidone, 1,4-dioxane, tetrahydrofuran, or dimethoxyethane, or in the absence of solvent, and reacting the mixture at a temperature of 20°C to 150°C. However, the reaction conditions are not limited thereto.

### [Step C12]

In this step, compound (12c) is reacted with hydrazine to give compound (15c). The reaction can be conducted under the same conditions as used in [Step C11] of production method C. It is preferable to use 2 to 10 equivalents of hydrazine.

### [Step C13]

In this step, a substitution reaction is carried out using compound (15c) and compound (16c) to give compound (17c). The reaction can be conducted under the same conditions as used in [Step A2] of production method A. It is preferable to use 1 to 3 equivalents of compound (16c).

### [Step C14]

In this step, R^{p3} of compound (17c) is removed to give compound (14c). The reaction can be conducted under the same conditions as used in [Step A13] of production method A.

### [Step C15]

In this step, compound (5c) is reacted with compound (6c) to give compound (18c). The reaction can be conducted under the same conditions as used in [Step A6] of production method A.

### [Step C16]

In this step, compound (18c) is hydrolyzed to give compound (19c).

There are no particular limitations on the reaction conditions for the hydrolysis. For example, compound (19c) can be obtained by incubating compound (18c) in the presence of a base at a temperature ranging from 0°C to 100°C.

Solvents for the reaction include methanol, ethanol, tetrahydrofuran, water, or mixtures thereof. Bases include lithium hydroxide, sodium hydroxide, and potassium hydroxide. It is preferable to use 1 to 2 equivalents of a base.

### [Step C17]

In this step, compound (19c) is reacted with a reducing agent to give compound (20c). The reduction can be achieved under a standard condition for the reduction of carboxylic acid to methyl alcohol.

Reducing agents include borane derivatives such as borane-tetrahydrofuran complex and borane-methyl sulfide complex, and sodium borohydride. It is preferable to use 5 to 30 equivalents of a reducing agent.

When a borane derivative is used as a reducing agent, compound (20c) can be obtained by carrying out the reaction using a solvent such as 1,4-dioxane, tetrahydrofuran, or dimethoxyethane at a temperature ranging from -78°C to 35°C.

Alternatively, when sodium borohydride is used as a reducing agent, first, compound (19c) is reacted with an activator such as isobutyl chloroformate, at a temperature ranging from -78°C to 20°C, then reacted with a reducing agent such as sodium borohydride at a temperature ranging from -78°C to 35°C, to obtain compound (20c). Solvents for the reaction include 1,4-dioxane, tetrahydrofuran, and dimethoxyethane.

### [Step C18]

In this step, compound (20c) is thioamidated to give compound (21c). The reaction can be conducted under the same conditions as used in [Step C6] of production method C.

### [Step C19]

In this step, compound (21 c) is reacted with a silylating agent in the presence of a base to give compound (22c).

Solvents for the reaction include dichloromethane, N,N-dimethylformamide, 1,4-dioxane, tetrahydrofuran, and dimethoxyethane. Bases include imidazole, pyridine, 4-dimethylaminopyridine, triethylamine, and N,N-diisopropylethylamine. Silylating agents include *t*-butyldimethylchlorosilane, and *t*-butylchlorodiphenylsilane. It is preferable to use 1.0 to 1.5 equivalent of a base and 1.0 to 1.5 equivalent of a silylating agent. The reaction can be conducted at a temperature ranging from 0°C to 80°C.

### [Step C20]

In this step, compound (22c) is methylated to give compound (23c).

The reaction can be conducted under the same condition as used in [Step C7] of production method C.

### [Step C21]

In this step, compound (23c) is hydrolyzed to give compound (24c).

There are no particular limitations on the reaction conditions for the hydrolysis. Compound (24c) can be obtained by carrying out the reaction in a mixed solvent of ethanol and water in the presence of an acid such as sulfuric acid, hydrochloric acid, or *p*-toluenesulfonic acid, at a temperature ranging from 50°C to 100°C.

When such a reaction results in removal of -R^{p3}, -NH- is re-protected through a protection reaction. Specifically, for example, when R^{p3} is a *t*-butoxycarbonyl group, the reaction can be carried out using a reagent such as *t*-butyl dicarbonate, in a solvent such as dichloromethane, chloroform, N,N-dimethylformamide, or tetrahydrofuran, in the presence of a base such as pyridine, 4-aminopyridine, triethylamine, or N,N-diisopropyl ethylamine, at a temperature ranging from 0°C to 80°C. However, the reaction is not limited thereto.

### Production method D

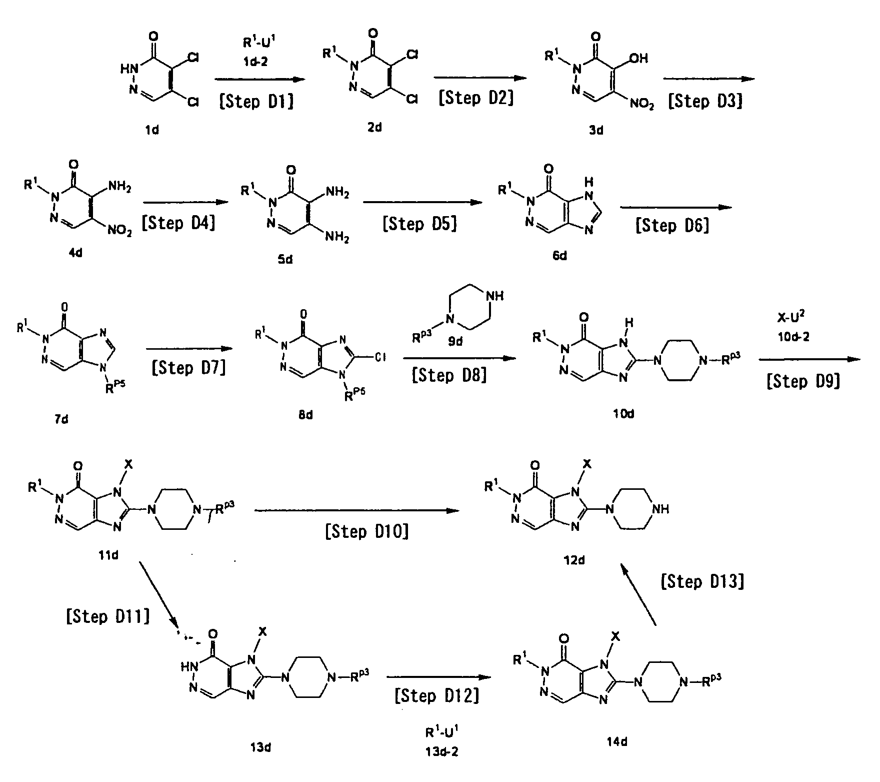

### [Step D1]

In this step, compound (1d) is reacted with compound (1d-2) to give compound (2d).

Specifically, compound (1d-2) includes, for example, alkyl halides such as iodomethane, iodoethane, iodopropane, benzyl bromide, 2-bromoacetophenone, chloromethyl benzyl ether, and bromoacetonitrile; alkenyl halides such as allyl bromide and 1-bromo-3-methyl-2-butene; and alkynyl halides such as propargyl bromide and 1-bromo-2-butyne. It is preferable to use 1 to 1.5 equivalent of compound (1d-2).

Solvents for the reaction include N,N-dimethylformamide, N-methylpyrrolidone, tetrahydrofuran, 1,2-dimethoxyethane, 1,4-dioxane, and dichloromethane. The reaction can be carried out in the presence or absence of a base. Bases to be used in the reaction include 1,8-diazabicyclo[5,4,0]undecene, triethylamine, N,N-diisopropylethylamine, and sodium hydride. In this case, it is preferable to use 1 to 1.5 equivalent of the base. The reaction can be conducted at a temperature ranging from 0°C to 150°C.

### [Step D2]

In this step, compound (2d) is reacted with a nitrite salt to give compound (3d).

Solvents for the reaction include a mixed solvent of water and a solvent from N,N-dimethylformamide, N-methylpyrrolidone, tetrahydrofuran, 1,2-dimethoxyethane, and 1,4-dioxane. Nitrite salts include sodium nitrite and potassium nitrite. It is preferable to use 3 to 5 equivalents of a nitrite. The reaction can be conducted at a temperature ranging from 20°C to 120°C.

### [Step D3]

In this step, compound (3d) is reacted with ammonia to give compound (4d). It is preferable to use 10 to 20 equivalents of ammonia.

The reaction can be carried out in a solvent such as methanol, ethanol, or 1,4-dioxane at a temperature ranging from 20°C to 200°C.

### [Step D4]

In this step, compound (4d) is subjected to catalytic reduction under hydrogen atmosphere or in the presence of 2 to 3 equivalents of hydrazine using a metal catalyst to give compound (5d).

Solvents for the reaction include methanol, ethanol, N,N-dimethylformamide, tetrahydrofuran, 1,2-dimethoxyethane, 1,4-dioxane, water, or a mixed solvent thereof. Metal catalysts include palladium carbon, platinum oxide, and Raney nickel. It is preferable to use a metal catalyst in the amount of 0.5 to 10% by weight. The reaction can be conducted at a temperature ranging from 0°C to 150°C.

### [Step D5]

In this step, compound (5d) is reacted with an orthoformate ester to give compound (6d).

The reaction is carried out in the presence of a carboxylic anhydride such as acetic anhydride. Orthoformate esters include methyl orthoformate, and ethyl orthoformate. It is preferable to use 1 to 20 times as much orthoformate ester by weight and 3 to 10 equivalents of carboxylic anhydride. The reaction can be conducted at a temperature ranging from 20°C to 200°C.

### [Step D6]

In this step, the NH group at the 1-position of compound (6d) is protected to give compound (7d).

Protecting reagents include N,N-dimethylsulfamoyl chloride, trityl chloride, di-*t*-butyl dicarbonate, and benzyl bromide. It is preferable to use 1 to 1.5 equivalent of a protecting reagent. Solvents for the reaction include dichloromethane, chloroform, carbon tetrachloride, toluene, N,N-dimethylformamide, and tetrahydrofuran. Bases include pyridine, 4-dimethylaminopyridine, 1,8-diazabicyclo[5,4,0]undecene, triethylamine, and N,N-diisopropylethylamine. In typical cases, it is preferable to use 1.2 equivalents of a base. However, when the protecting reagent is di-*t*-butyl dicarbonate, 0.005 to 0.1 equivalent of 4-dimethylaminopyridine is used preferably. The reaction can be conducted at a temperature ranging from 20°C to 200°C.

### [Step D7]

In this step, compound (7d) is chlorinated to give compound (8d).

There are no particular limitations on the reaction conditions. For example, the reaction is carried out as follows. Compound (7d) is reacted with a base at a temperature ranging from -100°C to 20°C, and then a chlorinating reagent is reacted thereto. This reaction produces compound (8d). Compound (8d) can also be obtained by reacting compound (7d) with a base in the presence of a chlorination reagent. Solvents for the reaction include, for example, diethyl ether, tetrahydrofuran, 1,2-dimethoxyethane, and 1,4-dioxane. Bases include *n*-butyllithium, *t*-butyllithium, lithium diisopropylamide, lithium bis(trimethylsilyl)amide, and magnesium diisopropylamide. It is preferable to use 1 to 1.5 equivalent of a base. Chlorinating reagents include hexachloroethane, and N-chloro succinimide. It is preferable to use 1 to 3 equivalents of a chlorination reagent.

### [Step D8]

In this step, compound (8d) is reacted with compound (9d) to give compound (10d). The reaction can be conducted under the same conditions as used in [Step A6] of production method A.

### [Step D9]

In this step, a substitution reaction is carried out using compound (10d) and compound (10d-2) to give compound (11d). The reaction can be conducted under the same conditions as used in [Step A4] of production method A.

### [Step D10]

In this step, R^{p3} of compound (11d) is removed to give compound (12d). The reaction can be conducted under the same condition as used in [Step A13] of production method A.

### [Step D11]

In this step, the group at the 5-position of compound (11d) is obtained by dealkylation to give compound (13d). There are no particular limitations on the reaction conditions for the dealkylation. For example, such a reaction can be achieved as follows:

When R¹ is a benzyloxymethyl group, compound (11d) is reacted with 3 to 10 equivalents of boron tribromide, boron trichloride, or such in a solution such as dichloromethane at a temperature ranging from -100°C to 20°C. This reaction produces compound (13d).

When such a reaction results in removal of R^{p3}, -NH- is re-protected through a protection reaction. Specifically, for example, when R^{p3} is a *t*-butoxycarbonyl group, the reaction can be carried out using a reagent such as di-*t*-butyl dicarbonate, in a solvent such as dichloromethane, chloroform, N,N-dimethylformamide, or tetrahydrofuran, in the presence of a base such as pyridine, 4-aminopyridine, triethylamine, or N,N-diisopropylethylamine, at a temperature ranging from 0°C to 80°C. However, the reaction is not limited thereto.

### [Step D12]

In this step, compound (13d) is reacted with compound (13d-2) to give compound (14d). The reaction can be conducted under the same conditions as used in [Step D1] of production method D.

### [Step D13]

In this step, R^{p3} of compound (14d) is removed to give compound (12d). The reaction can be conducted under the same conditions as used in [Step A13] of production method A.

An alternative method for producing compound (11d) is described below.

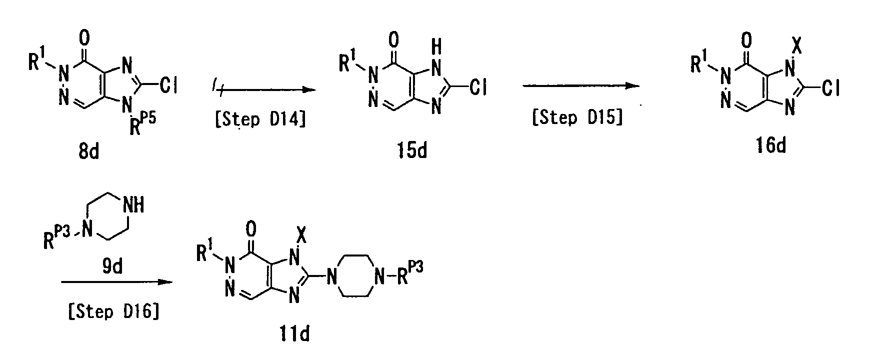

### [Step D14]

In this step, compound (8d) is deprotected to give compound (15d).

The deprotection can be achieved under standard reaction conditions depending on the type of protecting group. For example, in the case of a *t*-butoxycarbonyl group, the deprotection can be achieved by carrying out the reaction using a base such as sodium hydroxide, potassium carbonate, and ammonia, in tetrahydrofuran, N,N-dimethylformamide, methanol, ethanol, water, or a mixed solvent thereof at a temperature ranging from 0°C to 100°C. When a solvent and a base are added after chlorination in the previous step, the deprotection can be achieved without isolating compound (8d).

### [Step D15]

In this step, X is introduced into compound (15d) to give compound (16d). The reaction can be conducted using X-U² under the same conditions as used in [Step A4] of production method A.

An alcohol (X-OH) can be introduced using Mitsunobu's reaction. Specifically, compound (16d) can be obtained by reacting an alcohol (X-OH) with an azodicarboxylic acid dialkyl ester and triphenylphosphine in a solvent such as tetrahydrofuran, at a temperature ranging from -70°C to 50°C.

### [Step D16]

In this step, compound (16d) is reacted with compound (9d) to give compound (11d).

The reaction can be conducted under the same conditions as used in [Step A6] of production method A.

### Production method E

Compound (1e) represented by the formula: 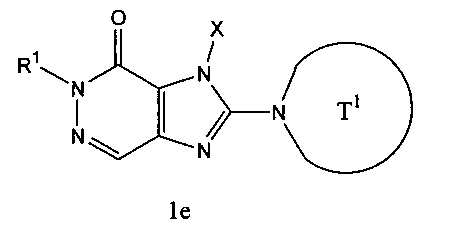 can be obtained by using compound (8b) represented by H-T^{1a}, instead of compound (6c), in [Step C5] or [Step C15] of production method C described above under the same reaction conditions as used in [Step C5], and then appropriately applying [Step C6] to [Step C21] described above.

Compound (1e) represented by the formula: 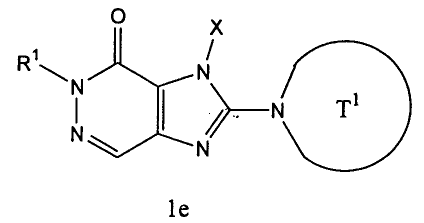 can be obtained by using compound (8b) represented by H-T^{1a}, instead of compound (9d) in [Step D8] of production method D described above under the same reaction conditions as used in [Step D8], and then appropriately applying [Step D9] to [Step D13] described above.

### Production method F

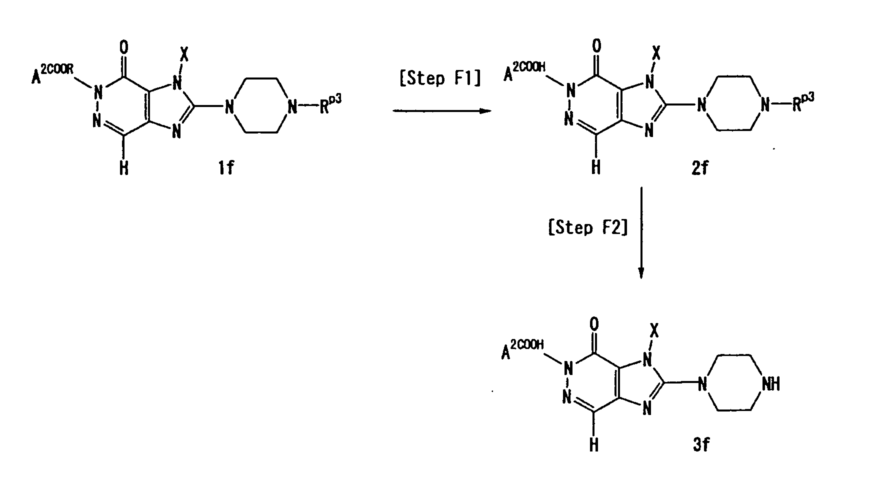

### [Step F1]

In this step, the ester group of compound (1f) is hydrolyzed to give compound (2f). The reaction can be conducted under the same conditions as used in [Step C16] of production method C.

### [Step F2]

In this step, R^{p3} of compound (2f) is removed to give compound (3f). The reaction can be conducted under the same conditions as used in [Step A13] of production method A. Production method G

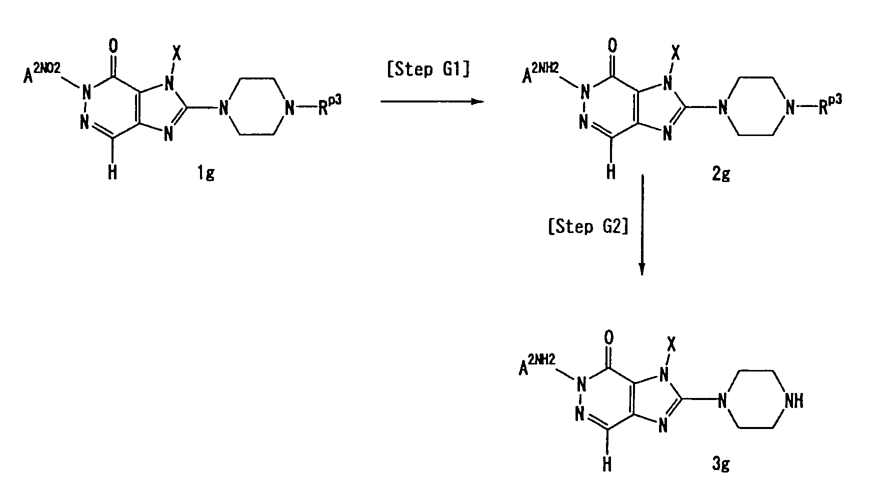

### [Step G1]

In this step, the nitro group of compound (1g) is reduced to give compound (2g).

Solvents for the reaction include methanol, ethanol, tetrahydrofuran, water, or mixtures thereof. Reducing agents includes, iron, tin, and zinc. Catalysts include hydrochloric acid and ammonium salts such as ammonium chloride. The reaction can be conducted at a temperature ranging from 20°C to 120°C.

### [Step G2]

In this step, R^{p3} of compound (2g) is removed to give compound (3g). The reaction can be conducted under the same conditions as used in [Step A13] of production method A. Production method H

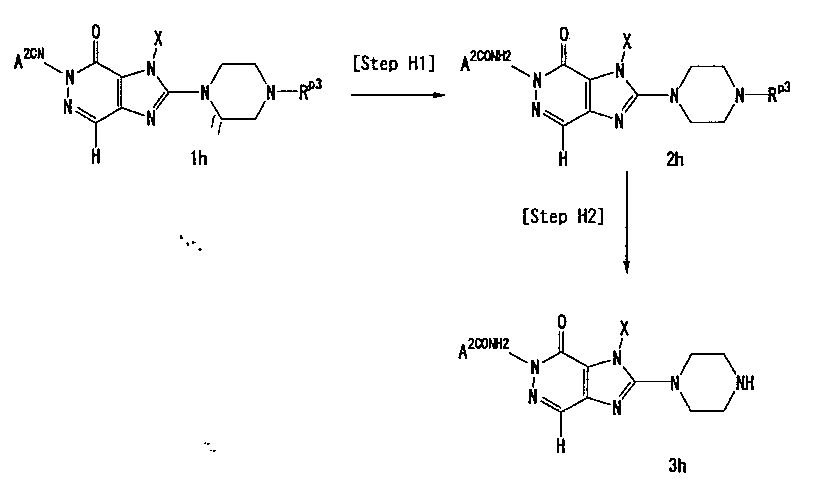

### [Step H1]

In this step, the nitrile group of compound (1h) is hydrolyzed to give compound (2h).

There are no particular limitations on the reaction conditions. For example, the reaction is carried out as follows. Compound (2h) can be obtained by reacting compound (1h) with hydrogen peroxide in the presence of a base at a temperature ranging from -20°C to 50°C. Solvents include methanol, ethanol, tetrahydrofuran, water, or a solvent mixture thereof. Bases include ammonia and alkyl amines such as triethylamine.

### [Step H2]

In this step, R^{p3} of compound (2h) is removed to give compound (3h). The reaction can be conducted under the same conditions as used in [Step A13] of production method A. Production method I

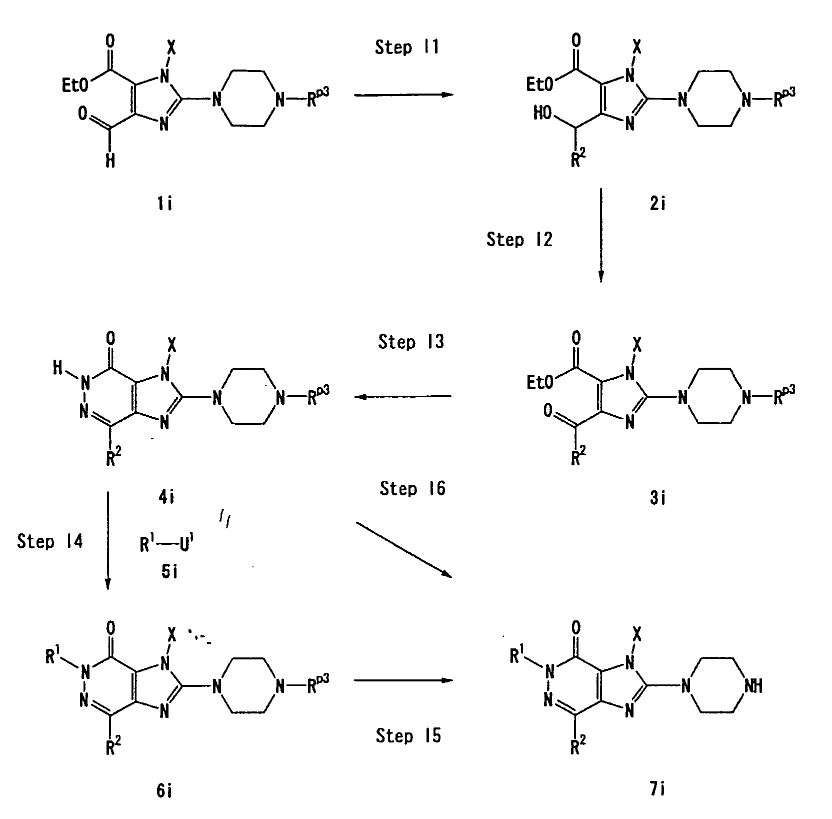

### [Step I1]

In this step, compound (1i) is reacted with an alkyl metal agent or an aryl metal agent to give compound (2i).

There are no particular limitations on the reaction conditions. For example, the reaction is carried out as follows. Compound (1i) may be reacted with an agent such as alkyllithium, aryllithium, alkyl Grignard reagent, or aryl Grignard reagent, in a solvent such as diethyl ether or tetrahydrofuran, at a temperature ranging from -100°C to 100°C. Alternatively, the compound may be reacted with alkylzinc or arylzinc in a solvent such as N,N-dimethylformamide or 1-methyl-2-pyrrolidone, at a temperature ranging from 0°C to 50°C.

### [Step I2]

In this step, compound (2i) is oxidized to give compound (3i). A typical reagent that is generally used in the oxidation of an alcohol can be used as the oxidant. Specifically, for example, manganese dioxide can be used as the oxidant in a solvent such as dichloromethane or chloroform, at a temperature within the range of 20°C to 100°C. Alternatively, sulfur trioxide pyridine can be used as the oxidant in a solvent such as dimethyl sulfoxide, at a temperature within the range of 20°C to 100°C. Alternatively, Dess-Martin periodinane may be used in a solvent such as dichloromethane or chloroform, at a temperature within the range of -50 to 50°C.

### [Step I3]

In this step, compound (3i) is reacted with hydrazine to give compound (4i). The reaction can be conducted under the same conditions as used in [Step C12] of production method C.

### [Step I4]

In this step, a substitution reaction is carried out using compound (4i) and compound (5i) to give compound (6i). The reaction can be conducted under the same conditions as used in [Step A2] of production method A.

### [Step I5]

In this step, R^{p3} of compound (6i) is removed to give compound (7i). The reaction can be conducted under the same conditions as used in [Step A13] of production method A.

### [Step I6]

In this step, R^{p3} of compound (4i) is removed to give compound (7i) when R¹ of compound (7i) is H. The reaction can be conducted under the same conditions as used in [Step A13] of production method A.

### Production method J

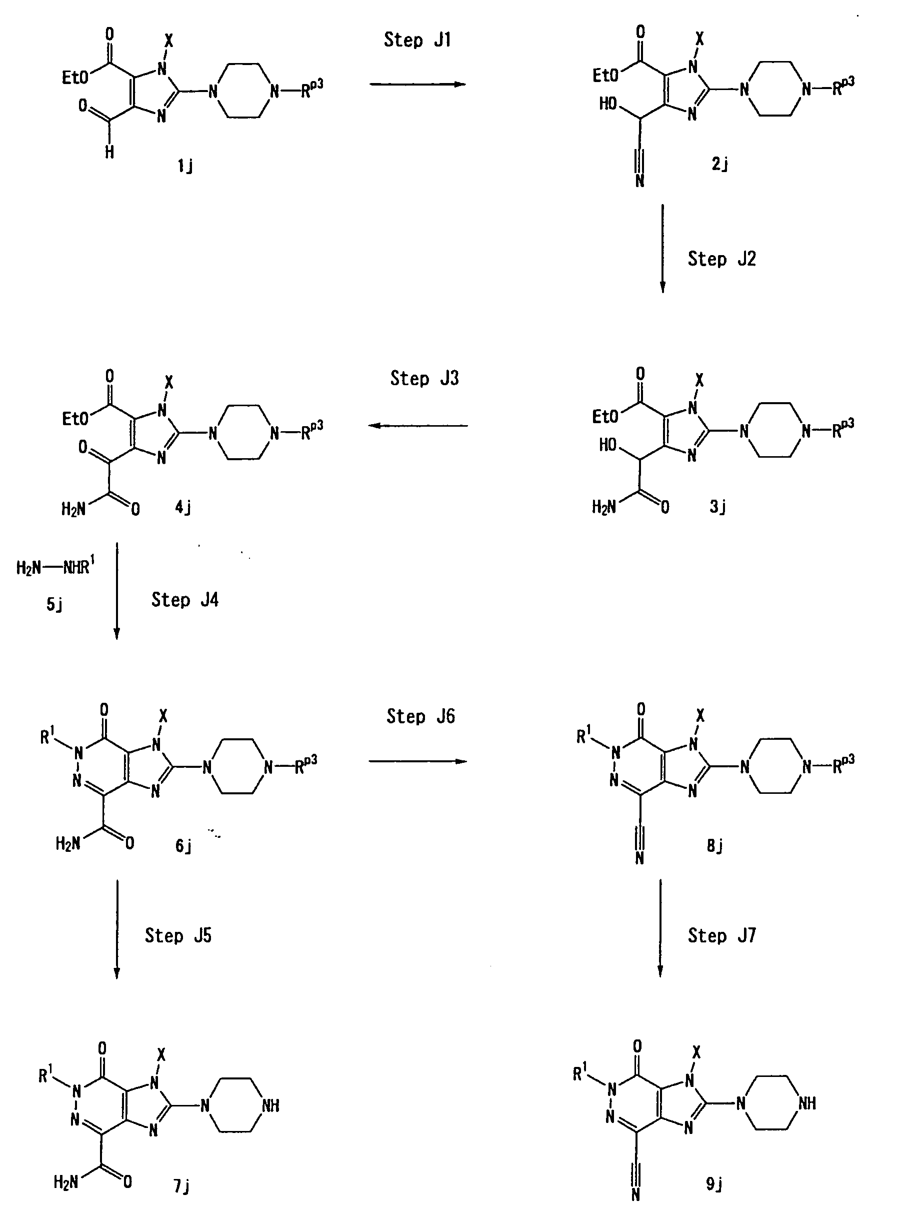

### [Step J1]

In this step, compound (1j) is reacted with a cyanidation agent in the presence of a catalyst to give compound (2j).

Cyanidation agents include sodium cyanide, and potassium cyanide. Catalysts include acetic acid. Solvents include, for example, acetonitrile. The reaction can be conducted at a temperature ranging from 0°C to 100°C.

### [Step J2]

In this step, the nitrile group of compound (2j) is hydrolyzed to give compound (3j). The reaction can be conducted under the same conditions as used in [Step H1] of production method H.

### [Step J3]

In this step, the hydroxyl group of compound (3j) is oxidized to give compound (4j). The reaction can be conducted under the same conditions as used in [Step I2] of production method I.

### [Step J4]

In this step, compound (4j) is reacted with compound (5j) to give compound (6j). The reaction can be conducted under the same conditions as used in [Step C11] of production method C.

### [Step J5]

In this step, R^{p3} of compound (6j) is removed to give compound (7j). The reaction can be conducted under the same conditions as used in [Step A13] of production method A.

### [Step J6]

In this step, the carbamoyl group of compound (6j) is dehydrated in the presence of a base to give compound (8j).

Dehydrating agents include, for example, phosphorus oxychloride. Bases include alkyl amines such as triethylamine. Solvents include dichloromethane, and chloroform. Alternatively, the reaction can be carried out in the absence of solvent. The reaction can be conducted at a temperature ranging from 0°C to 100°C.

### [Step J7]

In this step, R^{p3} of compound (8j) is removed to give compound (9j). The reaction can be conducted under the same conditions as used in [Step A13] of production method A.

### Production method K

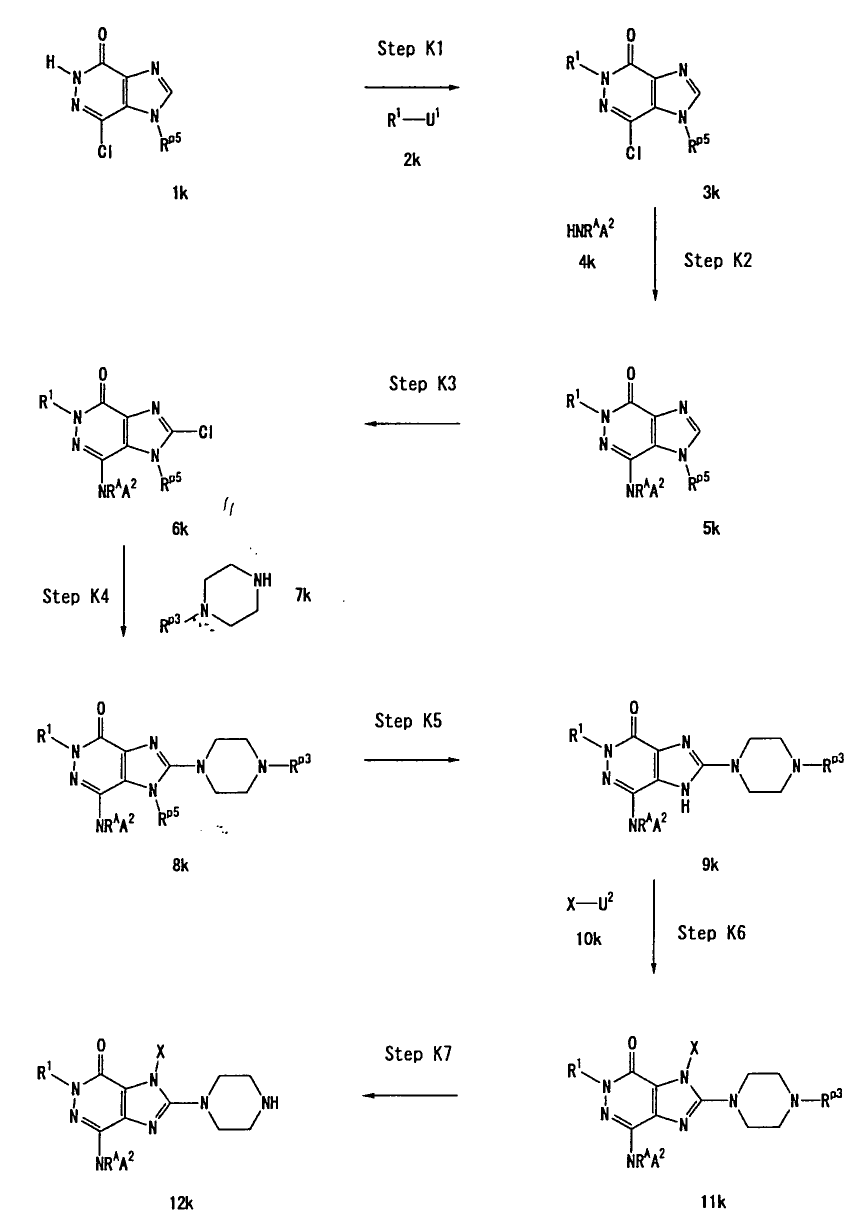

### [Step K1]

In this step, a substitution reaction using compound (1k) and compound (2k) is carried out to give compound (3k). The reaction can be conducted under the same conditions as used in [Step A2] of production method A.

### [Step K2]

In this step, a substitution reaction using compound (3k) and compound (4k) is carried out to give compound (5k).

Compound (5k) can be obtained, for example, by reacting a mixture of compounds (3k) and (4k) in a solvent such as methanol, ethanol, 1-methyl-2-pyrrolidone, 1,4-dioxane, tetrahydrofuran, or dimethoxyethane, or in the absence of solvent at a temperature ranging from 20°C to 200°C. However, the reaction conditions are not limited thereto.

### [Step K3]

In this step, compound (5k) is chlorinated to give compound (6k). The reaction can be conducted under the same conditions as used in [Step D7] of production method D.

### [Step K4]

In this step, compound (6k) is reacted with compound (7k) to give compound (8k). The reaction can be conducted under the same conditions as used in [Step A6] of production method A.

### [Step K5]

In this step, R^{p5} of compound (8k) is removed to give compound (9k).

The deprotection reaction for R^{p5} can be carried out under standard reaction conditions for removing an -NH-protecting group.

For example, when R^{p5} is a benzyl group, the reaction can be achieved using a metal such as lithium or sodium in liquid ammonia at a temperature within the range of -78°C to -30°C.

### [Step K6]

In this step, a substitution reaction using compound (9k) and compound (10k) is carried out to give compound (11k). The reaction can be conducted under the same conditions as used in [Step A4] of production method A.

### [Step K7]

In this step, R^{p3} of compound (11k) is removed to give compound (12k). The reaction can be conducted under the same conditions as used in [Step A13] of production method A.

### Production method L

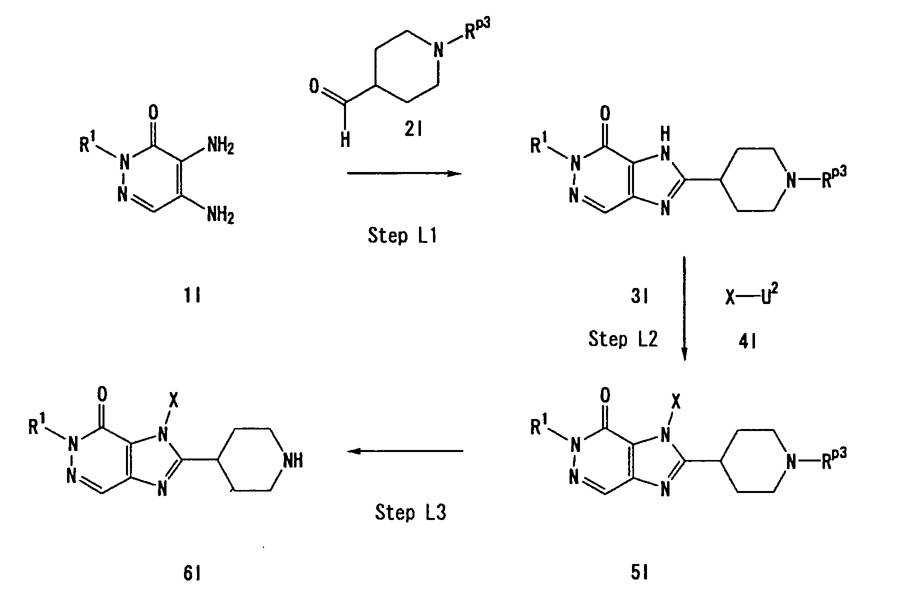

### [Step L1]

In this step, compound (11) is reacted with compound (21) in the presence of an oxidant to give compound (31).

Oxidants include salts such as iron (III) chloride. Solvents include methanol, ethanol, and water. The reaction can be conducted at a temperature ranging from 20°C to 100°C.

When such a reaction results in removal of -R^{p3}, -NH- is re-protected through a protection reaction. Specifically, for example, when R^{p3} is a *t*-butoxycarbonyl group, the reaction can be carried out using a reagent such as di-*t*-butyl dicarbonate, in a solvent such as dichloromethane, chloroform, N,N-dimethylformamide, or tetrahydrofuran, in the presence of a base such as pyridine, 4-aminopyridine, triethylamine, or N,N-diisopropylethylamine, at a temperature ranging from 0°C to 80°C. However, the reaction is not limited thereto.

### [Step L2]

In this step, compound (31) is reacted with compound (41) to give compound (51). The reaction can be conducted under the same conditions as used in [Step A4] of production method A.

### [Step L3]

In this step, R^{p3} of compound (51) is removed to give compound (61). The reaction can be conducted under the same conditions as used in [Step A13] of production method A.

### Production method M

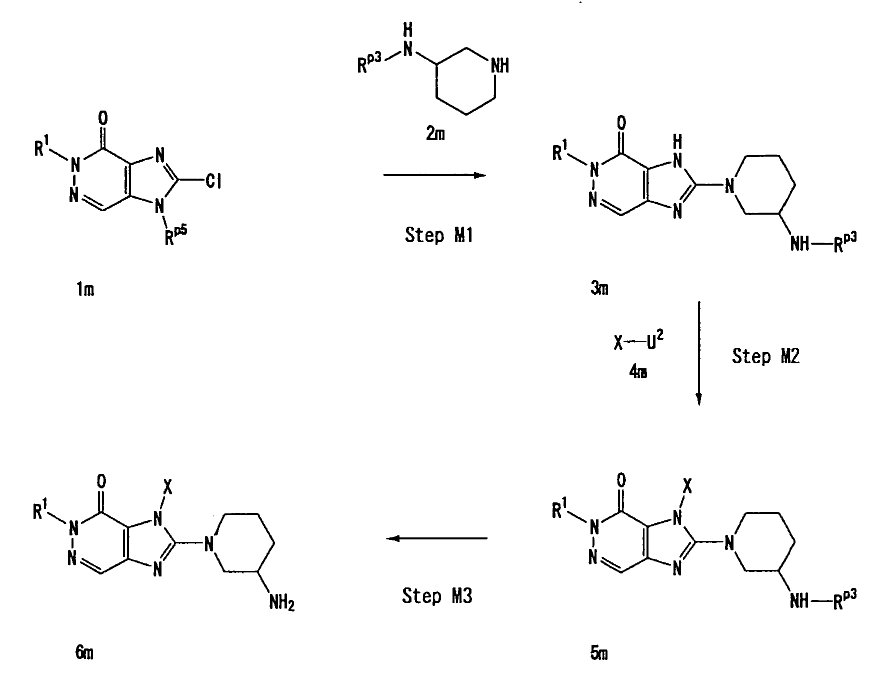

### [Step M1]

In this step, compound (1m) is reacted with compound (2m) to give compound (3m). The reaction can be conducted under the same conditions as used in [Step A6] of production method A.

### [Step M2]

In this step, compound (3m) is reacted with compound (4m) to give compound (5m). The reaction can be conducted under the same conditions as used in [Step A4] of production method A.

### [Step M3]

In this step, R^{p3} of compound (5m) is removed to give compound (6m). The reaction can be conducted under the same conditions as used in [Step A13] of production method A. Production method N

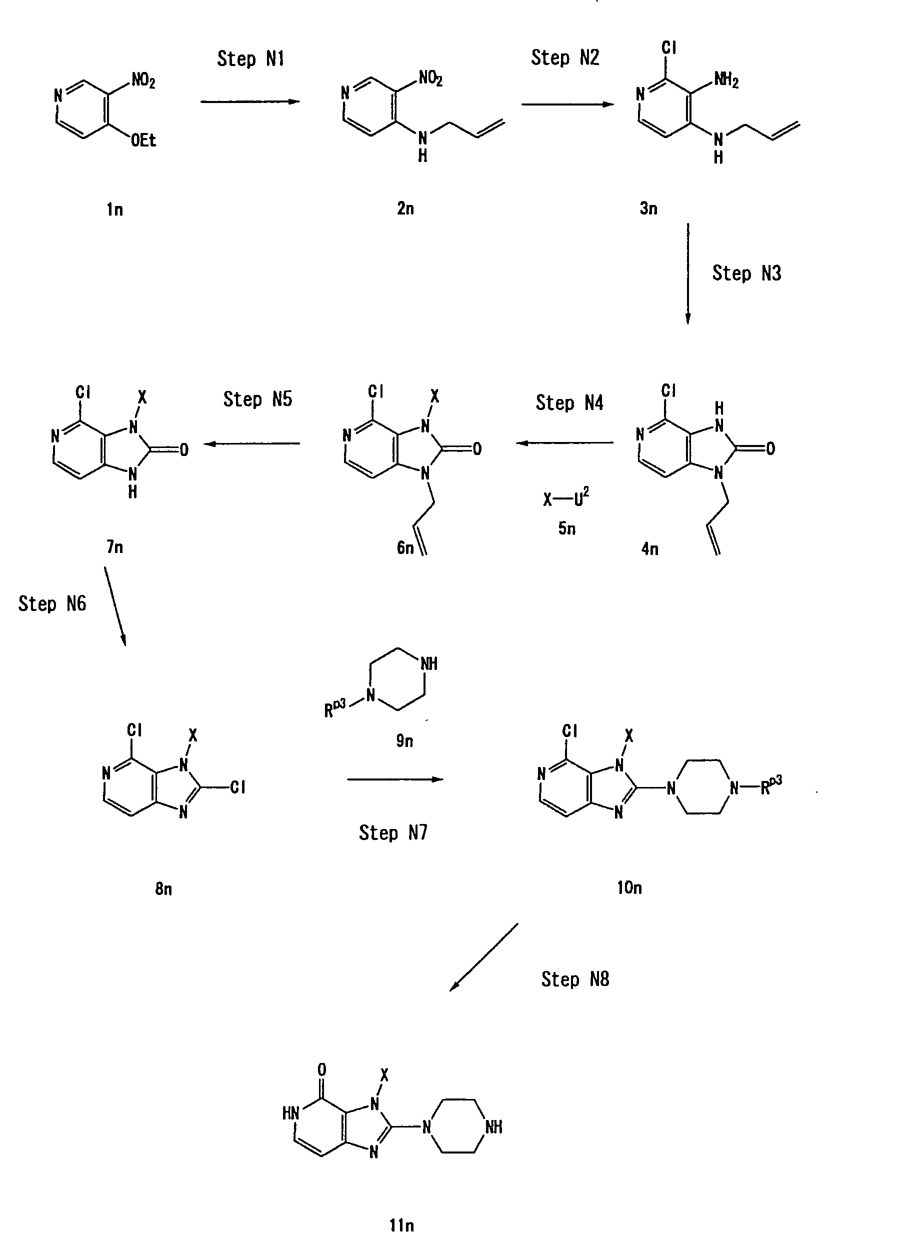

### [Step N1]

In this step, compound (1n) is reacted with allylamine to give compound (2n).

The reaction can be conducted at a temperature ranging from 20°C to 150°C. Solvents for the reaction include methanol, ethanol, water, and a mixed solvent thereof.

### [Step N2]

In this step, compound (2n) is reduced while being chlorinated to give compound (3n).

Reducing agents include tin salts such as tin chloride. Solvents include concentrated hydrochloric acid. The reaction can be conducted at a temperature ranging from 20°C to 150°C.

### [Step N3]

In this step, compound (3n) is reacted with N,N'-disuccinimidyl carbonate to give compound (4n).

The reaction can be achieved using a solvent such as acetonitrile or tetrahydrofuran. The reaction can be conducted at a temperature ranging from 20°C to 100°C.

### [Step N4]

In this step, compound (4n) is reacted with compound (5n) to give compound (6n). The reaction can be conducted under the same conditions as used in [Step A4] of production method A.

### [Step N5]

In this step, the allyl group is removed from compound (6n) to give compound (7n).

Compound (7n) can be obtained, for example, by reacting compound (6n) with osmic acid and sodium periodate in a solvent such as tetrahydrofuran, 1,4-dioxane, 1,2-dimethoxyethane, or water at a temperature ranging from 20°C to 100°C. However, the reaction conditions are not limited to this example.

### [Step N6]

In this step, compound (7n) is chlorinated to give compound (8n).

There are no particular limitations on the reaction conditions. The reaction can be conducted under standard reaction conditions to be used for chlorination. Compound (8n) can be obtained, for example, by using a reagent such as phosphorus pentachloride in a solvent such as phosphorus oxychloride, at a temperature of 0°C to 150°C.

### [Step N7]

In this step, compound (8n) is reacted with compound (9n) to give compound (10n). The reaction can be conducted under the same conditions as used in [Step A6] of production method A.

### [Step N8]

In this step, R^{p3} of compound (10n) is removed to give compound (11n). The reaction can be conducted under the same conditions as used in [Step A13] of production method A.

### Production method O

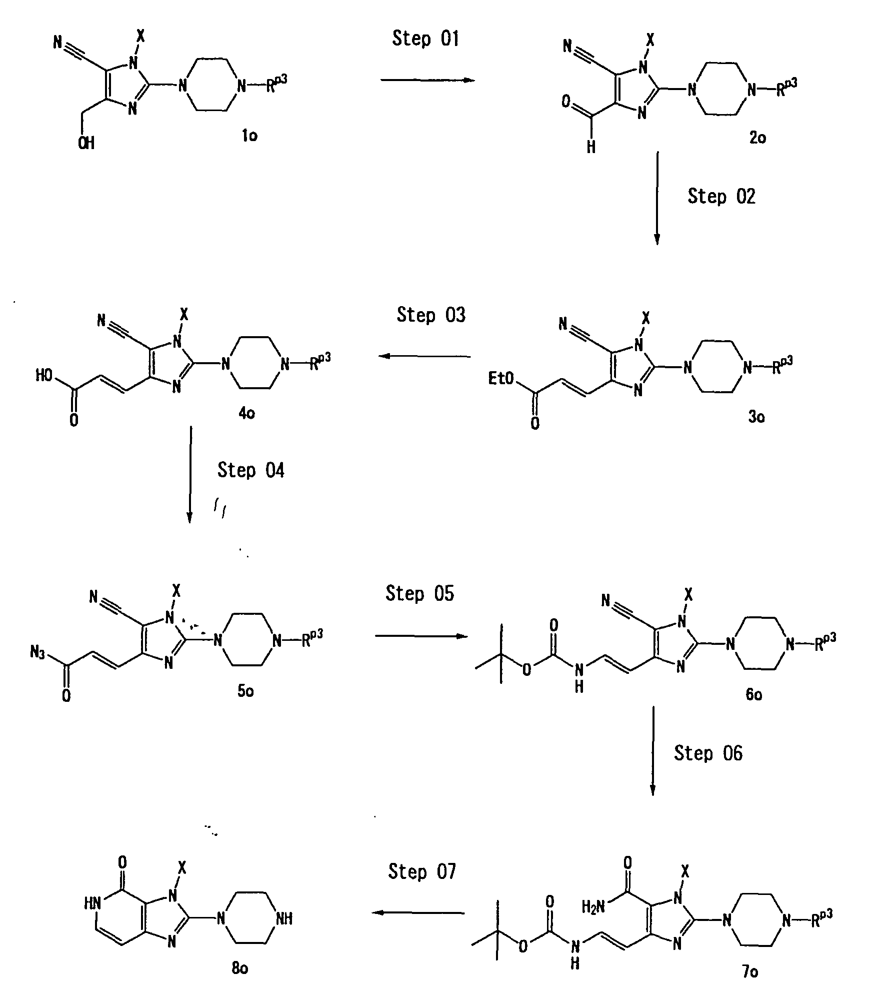

### [Step O1]

In this step, the hydroxyl group of compound (1o) is oxidized to give compound (2o). The reaction can be conducted under the same conditions as used in [Step I2] of production method I.

### [Step O2]

In this step, compound (2o) is reacted with ethyl diethylphosphonoacetate in the presence of a base to give compound (3o).

Bases include sodium hydride and lithium diisopropylamide. Solvents include, for example, tetrahydrofuran and N,N-diformamide. The reaction can be conducted at a temperature ranging from 0°C to 100°C.

### [Step O3]

In this step, the ester of compound (3o) is hydrolyzed to give compound (4o). The reaction can be conducted under the same condition as used in [Step C16] of production method C.

### [Step O4]

In this step, compound (4o) is reacted with diphenylphosphoryl azide in the presence of a base to give compound (5o).

Solvents for the reaction include toluene, *t*-butanol, tetrahydrofuran, and dichloromethane. Bases include tertiary amines such as triethylamine and diisopropylethylamine. The reaction can be conducted at a temperature ranging from -50°C to 50°C.

### [Step O5]

In this step, compound (5o) is rearranged to give compound (6o).

The reaction can be achieved in *t*-butanol at a temperature ranging from 50°C to 100°C.

### [Step O6]

In this stop, the nitrile group of compound (6o) is hydrolyzed to give compound (7o). The reaction can be conducted under the same conditions as used in [Step H1] of production method H.

### [Step O7]

In this step, compound (7o) is reacted with an acid to give compound (8o).

Acids include hydrochloric acid, sulfuric acid, and trifluoroacetic acid. Solvents include methanol, ethanol, 1,4-dioxane, water, and mixtures thereof. The reaction can be conducted at a temperature ranging from 0°C to 50°C.

### Production method P

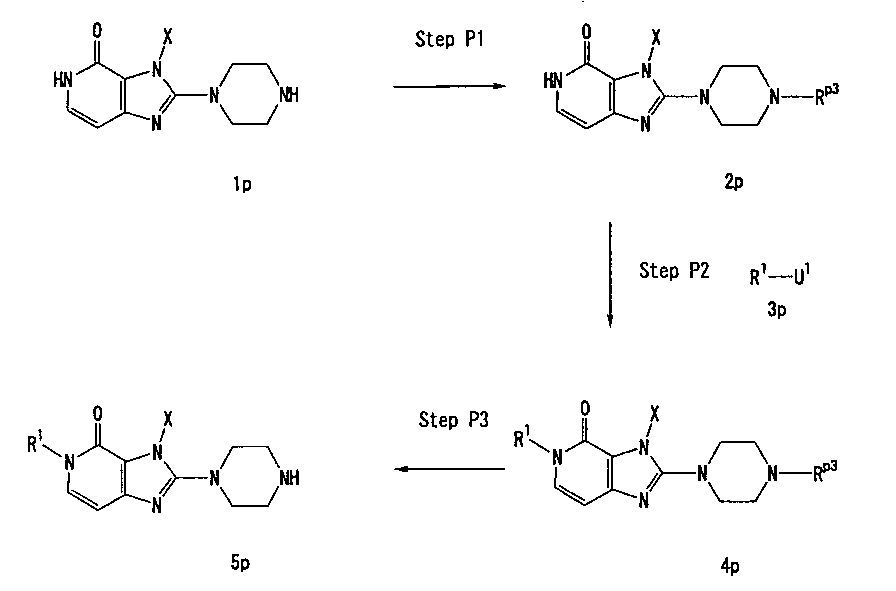

### [Step P1]

In this step, compound (1p) is protected to give compound (2p).

A typical NH group-protecting reagent that is generally used in protecting NH groups can be used as an NH group-protecting reagent. For example, when R^{p3} is a *t*-butoxycarbonyl group, the reaction can be achieved at a temperature ranging from 0°C to 80°C using a reagent such as di-*t*-butyl dicarbonate, in a solvent such as dichloromethane, chloroform, N,N-dimethylformamide, and tetrahydrofuran, in the presence of a base such as pyridine, 4-aminopyridine, triethylamine, and N,N-diisopropylethylamine.

### [Step P2]

In this step, compound (2p) is reacted with compound (3p) to give compound (4p). The reaction can be conducted under the same conditions as used in [Step A2] of production method A.

### [Step P3]

In this step, R^{p3} of compound (4p) is removed to give compound (5p). The reaction can be conducted under the same conditions as used in [Step A13] of production method A.

### Production method Q

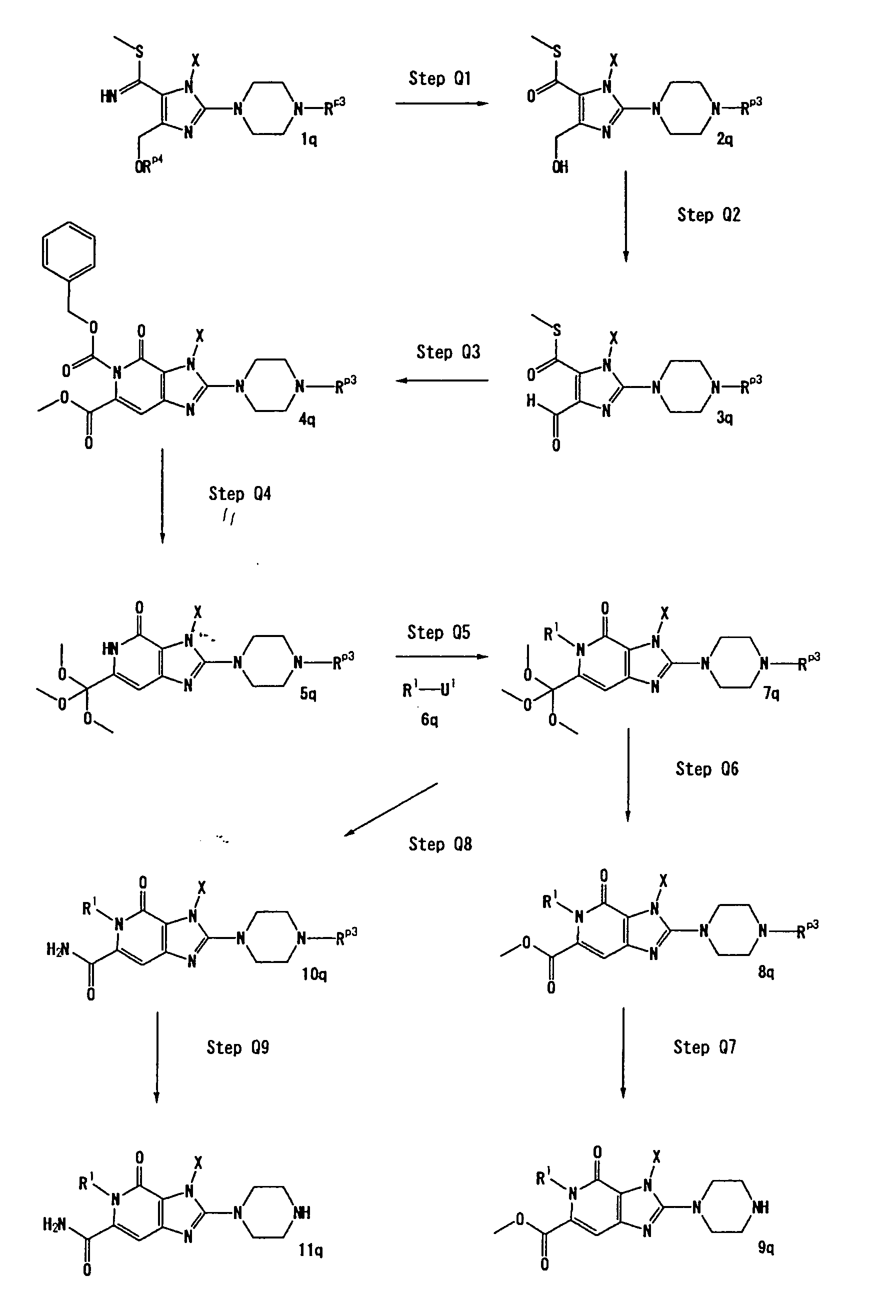

### [Step Q1]

In this step, compound (1q) is hydrolyzed to give compound (2q).

Reaction solvents include tetrahydrofuran, methanol, and ethanol. Acids include inorganic acids such as hydrochloric acid and sulfuric acid. The reaction can be conducted at a temperature ranging from 0°C to 100°C.

### [Step Q2]

In this step, the hydroxyl group of compound (2q) is oxidized to give compound (3q). The reaction can be conducted under the same conditions as used in [Step I2] of production method I.

### [Step Q3]

In this step, compound (3q) is reacted with methyl benzyloxycarbonylamino(dimethoxyphosphoryl)acetate in the presence of a base to give compound (4q).

Bases include sodium hydride, potassium *t*-butoxide, and 8-diazabicyclo[5.4.0]-7-undecene. Solvents include dichloromethane, tetrahydrofuran, and N,N-dimethylformamide. The reaction can be conducted at a temperature ranging from 0°C to 100°C.

### [Step Q4]

In this step, compound (4q) is reacted with sodium methoxide to give compound (5q).

Methanol can be used as solvent. The reaction can be conducted at a temperature ranging from 0°C to 80°C.

### [Step Q5]

In this step, compound (5q) is reacted with compound (6q) to give compound (7q). The reaction can be conducted under the same conditions as used in [Step A2] of production method A.

### [Step Q6]

In this step, compound (7q) is reacted with an acid to give compound (8q). The reaction can be conducted under the same conditions as used in [Step O7] of production method O.

### [Step Q7]

In this step, R^{p3} of compound (8q) is removed to give compound (9q). The reaction can be conducted under the same conditions as used in [Step A13] of production method A.

### [Step Q8]

In this step, compound (7q) is reacted with ammonia to give compound (10q).

Reaction solvents include methanol, ethanol, and water. The reaction can be conducted at a temperature ranging from 20°C to 150°C.

### [Step Q9]

In this step, R^{p3} of compound (10q) is removed to give compound (11q). The reaction can be conducted under the same conditions as used in [Step A13] of production method A.

### Production method R

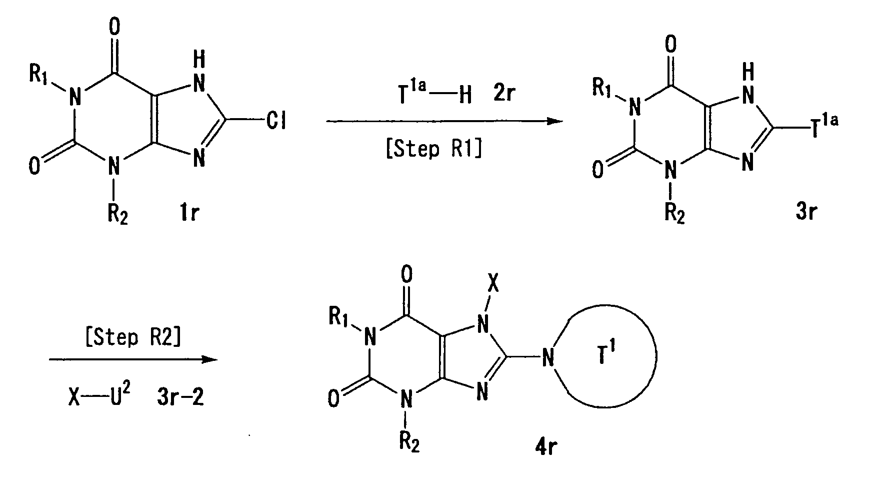

### [Step R1]

In this step, compound (1r) is reacted with compound (2r), to give compound (3r). The reaction is conducted under the same conditions as used in [Step A6] of production method A.

### [Step R2]

In this step, a substituent is introduced into the amino group at the 7-position of compound (3r), through a substitution reaction between compound (3r) and compound (3r-2), and R^{p3} is then removed to give compound (4r).

The substitution reaction is conducted under the same conditions as used in [Step A4] of production method A.

The deprotection reaction for R^{p3} is carried out under the same conditions as used in [Step A13] of production method A.

### Production method S

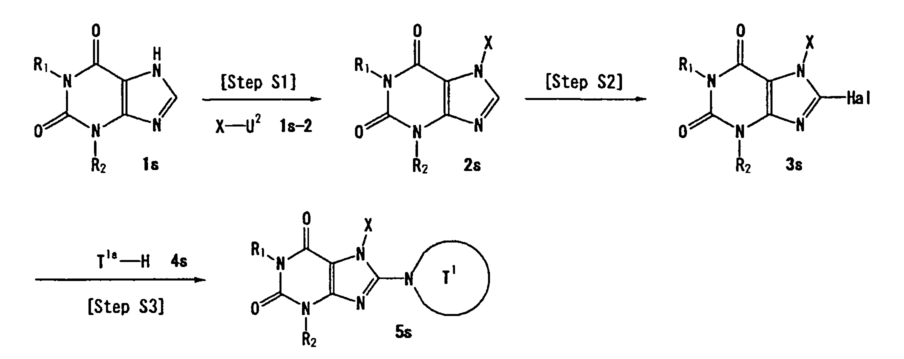

### [Step S1]

In this step, a substituent is introduced into the amino group at the 7-position of compound (1s), through a substitution reaction between compound (1s) and compound (1s-2), to give compound (2s).

The substitution reaction is conducted under the same conditions as used in [step A4] of production method A.

### [Step S2]

In this step, compound (2s) is reacted with a halogenating agent, to give compound (3s).

The halogenation reaction is conducted under the same conditions as used in [Step A5] of production method A.

### [Step S3]

In this step, compound (3s) is reacted with compound (4s), and then R^{p3} is removed to give compound (5s).

The coupling reaction is conducted under the same conditions as used in [Step A6] of production method A.

The deprotection reaction for R^{p3} can be carried out under the same conditions as used in [Step A13] of production method A.

### Production method T

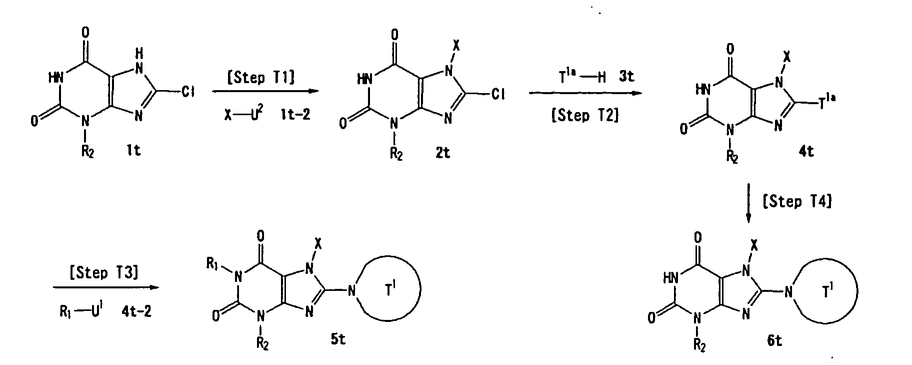

### [Step T1]

In this step, a substituent is introduced into the amino group at the 7-position of compound (1t), through a substitution reaction between compound (1t) and compound (1t-2), to give compound (2t).

The substitution reaction is conducted under the same conditions as used in [step A4] of production method A.

### [Step T2]

In this step, compound (2t) is reacted with compound (3t) to give compound (4t).

The reaction is conducted under the same conditions as used in [Step A6] of production method A.

### [Step T3]

In this step, compound (4t) is alkylated at the 1-position, and then R^{p3} is removed to give compound (5t).

The alkylation reaction is conducted under the same conditions as used in [Step A2] of production method A.

The deprotection reaction for R^{p3} is conducted under the same conditions as used in [Step A13] of production method A.

### [Step T4]

In this step, R^{p3} is removed from compound (4t), to give compound (6t).

The deprotection reaction for R^{p3} is conducted under the same conditions as used in [Step A13] of production method A.

### Production method U

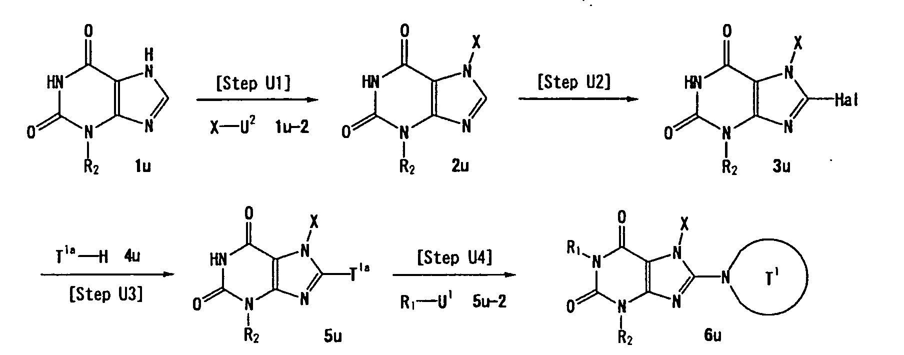

### [Step U1]

In this step, a substituent is introduced into the amino group at the 7-position of compound (1u), through a substitution reaction between compound (1u) and compound (1u-2), to give compound (2u).

The substitution reaction is conducted under the same conditions as used in [Step A4] of production method A.

### [Step U2]

In this step, compound (2u) is reacted with a halogenating agent, to give compound (3u).

The halogenation reaction is conducted under the same conditions as used in [Step A5] of production method A.

### [Step U3]

In this step, compound (3u) is reacted with compound (4u), to give compound (5u).

The reaction is conducted under the same conditions as used in [Step A6] of production method A.

### [Step U4]

In this step, compound (5u) is alkylated at the 1-position, and then R^{p3} is removed to give compound (6u).

The alkylation reaction is conducted under the same conditions as used in [Step A2] of production method A.

The deprotection reaction for R^{p3} is conducted under the same conditions as used in [Step A13] of production method A.

### Production method V

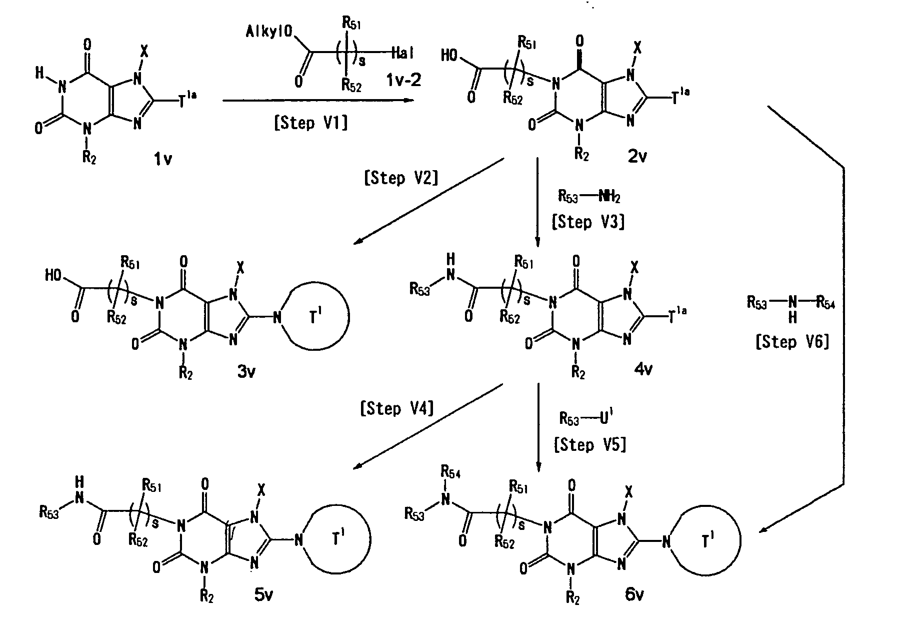

(where each symbol is as defined above; and "Alkyl" represents a C₁₋₆ alkyl group.)

### [Step V1]

In this step, compound (1v) is alkylated at the 1-position, and is then hydrolyzed to give compound (2v).

There are no particular limitations on the reaction conditions for the alkylation. For example, the alkylated compound can be obtained by incubating a compound represented by formula (1v-2), such as methyl bromoacetate or ethyl bromoacetate; in the presence of a base, such as lithium hydroxide, sodium hydroxide, potassium hydroxide, lithium carbonate, sodium carbonate, potassium carbonate, cesium carbonate, lithium hydride, sodium hydride, potassium hydride, butyl lithium, methyl lithium, lithium bis-trimethylsilylamide, sodium bis-trimethylsilylamide, or potassium bis-trimethylsilylamide; in a solvent, such as dimethylsulfoxide, N,N-dimethylformamide, N-methylpyrrolidone, dioxane, tetrahydrofuran, or toluene; at a temperature ranging from 0°C to 150°C

There are no particular limitations on the reaction conditions for the hydrolysis. For example, the reaction can be carried out using an aqueous solution lithium hydroxide, sodium hydroxide, or potassium hydroxide; in a solvent, such as methanol, ethanol, propanol, dimethylsulfoxide, N,N-dimethylformamide, N-methylpyrrolidone, dioxane, or tetrahydrofuran; at a temperature ranging from 0°C to 150°C.

### [Step V2]

In this step, R^{p3} is removed from compound (2v), to give compound (3v).

The deprotection reaction for R^{p3} is conducted under the same conditions as used in [Step A13] of production method A.

### [Step V3]

In this step, compound (2v) is amidated to give compound (4v).

There are no particular limitations on the reaction conditions for the amidation. For example, the reaction can be carried out using an acylating agent such as ethyl chloroformate or isobutylchloroformate; in the presence of an organic base such as triethylamine or N,N-diisopropylethylamine; in a solvent such as acetonitrile, N,N-dimethylformamide, N-methylpyrrolidone, 1,4-dioxane, tetrahydrofuran, or dimethoxyethane; with a corresponding amine at a temperature ranging from 0°C to 150°C.

### [Step V4]

In this step, R^{p3} is removed from compound (4v), to give compound (5v).

The deprotection reaction for R^{p3} is conducted under the same conditions as used in [Step A13] of production method A.

### [Step V5]

In this step, compound (5v) is alkylated, and then R^{p3} is removed, to give compound (6v).

The alkylation reaction is conducted under the same conditions as used in [Step A2] of production method A.

The deprotection reaction for R^{p3} is conducted under the same conditions as used in [Step A13] of production method A.

### [Step V6]

In this step, compound (2v) is amidated, and then R^{p3} is removed, to give compound (6v).

There are no particular limitations on the reaction conditions for the amidation. For example, the amidation can be conducted using a condensation agent such as 1,1'-carbonyldiimidazole or diethyl cyanophosphonate; in a solvent such as dimethylsulfoxide, N,N-dimethylformamide, or tetrahydrofuran. If required, it is possible to add an organic base, such as triethylamine, to the reaction. The reaction can be carried out at a temperature ranging from about an ice-cooling temperature to room temperature.

The deprotection reaction for R^{p3} is conducted under the same conditions as used in [Step A13] of production method A.

### Production method W

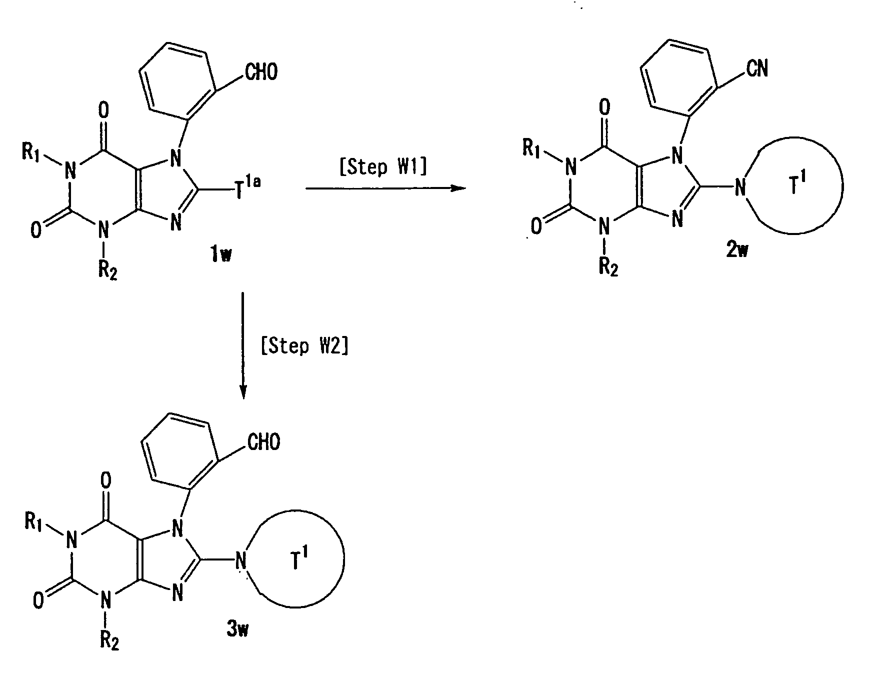

### [Step W1]

In this step, compound (1w) is hydroxy-iminated and the generated hydroxyl group is treated by sulfonylation, followed by removal of R^{p3}, to give compound (2w).

There are no particular limitations on the reaction conditions for the hydroxy imination. For example, the hydroxy imination reaction can be carried out using a reagent such as hydroxylamine hydrochloride; in the presence of a base such as potassium acetate or sodium acetate; in a solvent such as water, methanol, ethanol, propanol, dimethylsulfoxide, N,N-dimethylformamide, N-methylpyrrolidone, dioxane, tetrahydrofuran, or toluene.

There are no particular limitations on the reaction conditions for the sulfonylation. For example, the sulfonylation can be conducted using methane sulfonyl chloride, tosyl chloride, 4-nitrobenzensulfonyl chloride, or similar; in the presence of a base such as triethylamine, diisopropylethylamine, pyridine, or N,N-dimethylaminopyridine; in a solvent such as dichloromethane, chloroform, dioxane, tetrahydrofuran, toluene, or pyridine; at a temperature ranging from 0°C to 150°C.

The deprotection reaction for R^{p3} is conducted under the same conditions as used in [Step A13] of production method A.

### [Step W2]

In this step, R^{p3} is removed from compound (1w), to give compound (3w).

The deprotection reaction for R^{p3} is conducted under the same conditions as used in [Step A13] of production method A.

### Production method X

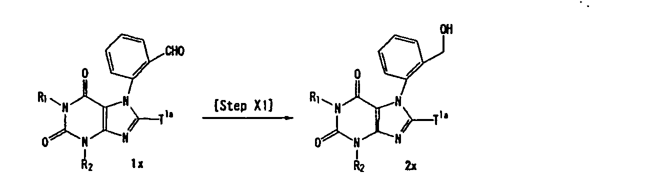

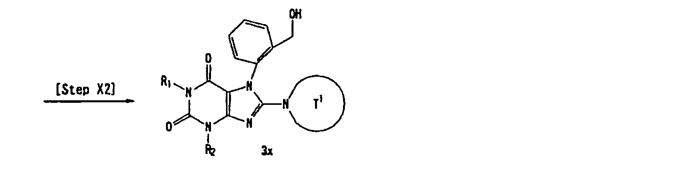

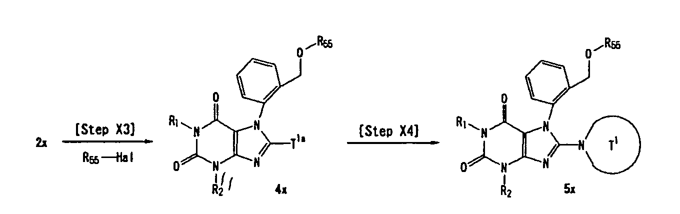

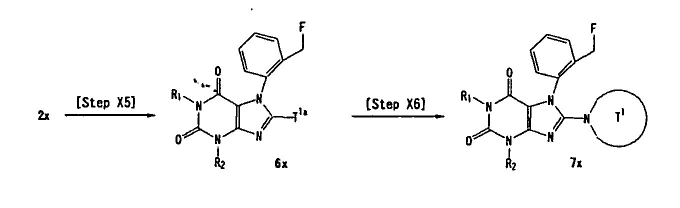

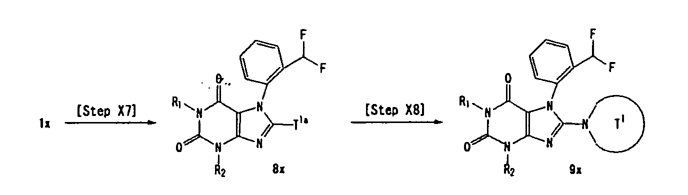

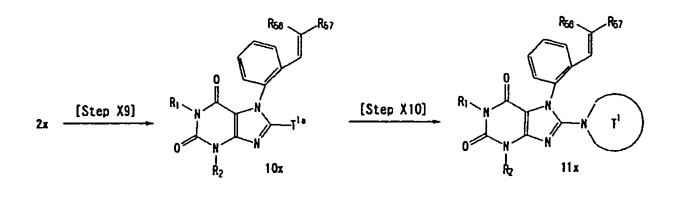

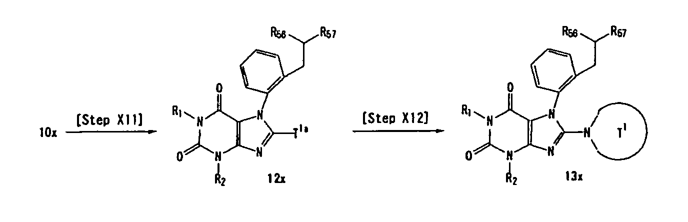

### [Step X1]

In this step, compound (1x) is reduced to give compound (2x).

There are no particular limitations on the reaction conditions. For example, the reaction can be conducted using a reducing agent such as lithium borohydride, sodium borohydride, or potassium borohydride; in a solvent such as methanol, ethanol, acetonitrile, N,N-dimethylformamide, N-methylpyrrolidone, 1,4-dioxane, tetrahydrofuran, dimethoxyethane, or in a mixed solution of these solvents; at a temperature ranging from 0°C to 150°C.

### [Step X3]

In this step, compound (2x) is alkylated to give compound (4x).

There are no particular limitations on the reaction conditions for the alkylation. For example, the alkylation reaction can be carried out using a halogenated alkyl; in the presence of a base such as lithium hydride, sodium hydride, potassium hydride, lithium hydroxide, sodium hydroxide, or potassium hydroxide; in a solvent such as methanol, ethanol, acetonitrile, N,N-dimethylformamide, N-methylpyrrolidone, 1,4-dioxane, tetrahydrofuran, or dimethoxyethane.

### [Step X5]

In this step, compound (2x) is fluorinated to give compound (6x).

There are no particular limitations on the reaction conditions. For example, the reaction can be carried out using a fluorinating agent such as Tris dimethylaminosulfate trifluoride; in a solvent such as dichloromethane, 1,2-dichloroethane, acetonitrile, N,N-dimethylformamide, N-methylpyrrolidone, 1,4-dioxane, tetrahydrofuran, or dimethoxyethane; at a temperature ranging from -78°C to 150°C.

### [Step X7]

In this step, compound (1 x) is fluorinated to give compound (8 x).

There are no particular limitations on the reaction conditions. For example, the reaction can be carried out using a fluorinating agent such as Tris dimethylaminosulfate trifluoride; in a solvent such as dichloromethane, 1,2-dichloroethane, acetonitrile, N,N-dimethylformamide, N-methylpyrrolidone, 1,4-dioxane, tetrahydrofuran, or dimethoxyethane; at a temperature ranging from -78°C to 150°C.

### [Step X9]

In this step, compound (2x) is subjected to the Wittig-Horner-Emmons reaction, to give compound (10x).

There are no particular limitations on the reaction conditions. For example, the reaction can be carried out using a reagent such as a phosphonium salt or phosphonate ester; in the presence of a base such as lithium hydride, sodium hydride, potassium hydride, potassium t-butoxide, or butyl lithium; in a solvent such as dichloromethane, 1,2-dichloroethane, acetonitrile, N,N-dimethylformamide, N-methylpyrrolidone, 1,4-dioxane, tetrahydrofuran, or dimethoxyethane; at a temperature ranging from -78°C to 150°C.

### [Step X11]

In this step, compound (10x) is reduced to give compound (12x).

There are no particular limitations on the reaction conditions for the reduction. For example, the reduction can be conducted in the presence of a metal catalyst, such as palladium carbon, platinum oxide, or Raney nickel; in a solvent, such as methanol, ethanol, propanol, dimethylsulfoxide, N,N-dimethylformamide, N-methylpyrrolidone, dioxane, tetrahydrofuran, or toluene; in a hydrogen atmosphere at a temperature ranging from 0°C to 150°C.

### [Step X2], [Step X4], [Step X6], [Step X8], [Step X10], and [Step X12]

R^{p3} is removed from compounds (2x), (4x), (6x), (8x), (10x), and (12x) to give compounds (3x), (5x), (7x), (9x), (11x), and (13x), respectively.

The deprotection reaction for R^{p3} is conducted under the same conditions as used in [Step A13] of production method A.

### Production method Y

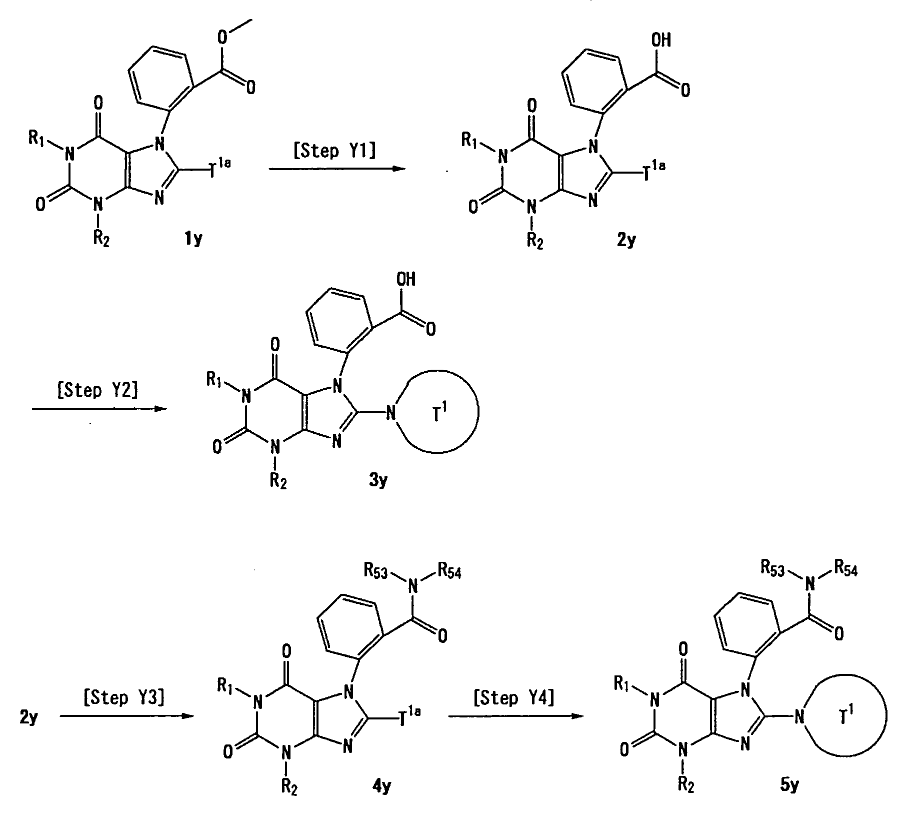

### [Step Y1]

In this step, compound (1y) is hydrolyzed to give compound (2y).

There are no particular limitations on the reaction conditions for the hydrolysis. For example, the hydrolysis can be conducted using an aqueous solution such as lithium hydroxide, sodium hydroxide, potassium hydroxide; in a solvent, such as methanol, ethanol, acetonitrile, N,N-dimethylformamide, N-methylpyrrolidone, 1,4-dioxane, tetrahydrofuran, or dimethoxyethane; at a temperature ranging from 0°C to 150°C.

### [Step Y3]

In this step, compound (2y) is amidated to give compound (4y).

The amidation reaction is conducted under the same conditions as used in [Step V6] of production method V.

### [Step Y2] and [Step Y4]

In this step, R^{p3} is removed from compounds (2y) and (4y), to give compounds (3y) and (5y), respectively.

The deprotection reaction for R^{p3} is conducted under the same conditions as used in [Step A13] of production method A.

### Production method Z

This is an alternative to the method of producing compound (2u) described in Production method U.

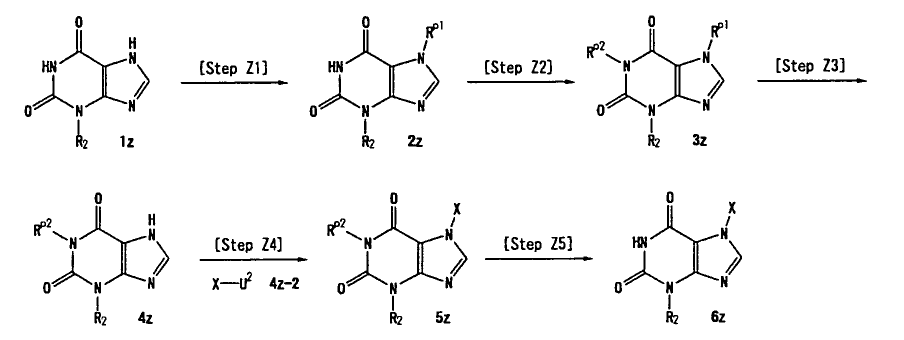

### [Step Z1]

In this step, compound (1z) is protected at the amino group of the 7-position, to give compound (2z).

There are no particular limitations on the types of groups to be used for protecting the amino group, the reaction conditions, and other variables. For example, when the protecting group is a benzyl group, the reaction can be conducted using an alkylating agent such as benzyl bromide; in the presence of a base such as cesium carbonate, lithium carbonate, sodium carbonate, or potassium carbonate; in a solvent such as acetonitrile, N,N-dimethylformamide, N-methylpyrrolidone, 1,4-dioxane, tetrahydrofuran, or dimethoxyethane; at a temperature ranging from 0°C to 150°C.

### [Step Z2]

In this step, compound (2z) is protected at the 1-position, to give compound (3z).

There are no particular limitations on the types of groups to be used for protecting the amino group, the reaction conditions, and other variables. For example, when the protecting group is a pivalyloxymethyl group, the reaction can be conducted using an alkylating agent such as chloromethylpivalate; in the presence of a base such as cesium carbonate, lithium carbonate, sodium carbonate, or potassium carbonate; in a solvent such as acetonitrile, N,N-dimethylformamide, N-methylpyrrolidone, 1,4-dioxane, tetrahydrofuran, or dimethoxyethane; at a temperature ranging from 0°C to 150°C.

### [Step Z3]

In this step, compound (3z) is deprotected at the amino group of the 7-position, to give compound (4z).

The reaction conditions vary depending on the types of protecting groups to be used. For example, when the protecting group is a benzyl group, the reaction can be conducted in the presence of a metal catalyst, such as palladium carbon, platinum oxide, or Raney nickel; in a solvent such as methanol, ethanol, propanol, dimethylsulfoxide, N,N-dimethylformamide, N-methylpyrrolidone, dioxane, tetrahydrofuran, or toluene; in a hydrogen atmosphere at a temperature ranging from 0°C to 150°C.

### [Step Z4]

In this step, a substituent is introduced into the amino group at the 7-position of compound (4z), through a substitution reaction between compound (4z) and compound (4z-2), to give compound (5z).

The substitution reaction is conducted under the same conditions as used in [Step A4] of production method A.

### [Step Z5]

In this step, the protecting group at the 1-position is removed from compound (5z), to give compound (6z)(=2u).

The reaction conditions vary depending on the types of protecting groups to be used. For example, when the protecting group is a pivalyloxymethyl group, the reaction can use a base such as sodium methoxide, sodium hydride, or diazabicyclo undec-7-ene; a solvent such as methanol or a mixed solvent of methanol and tetrahydrofuran; at a temperature ranging from 0°C to 150°C.

### Production method AA

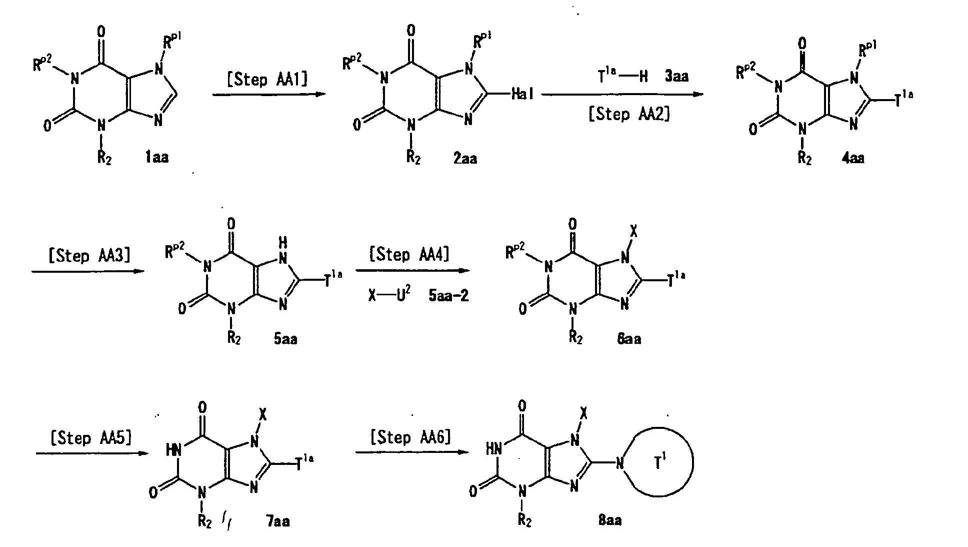

### [Step AA1]

In this step, compound (1aa) is reacted with a halogenating agent, to give compound (2aa).

The halogenation reaction is conducted under the same conditions as used in [Step A5] of production method A.

### [Step AA2]

In this step, compound (2aa) is reacted with compound (3aa) to give compound (4aa).

The reaction is conducted under the same conditions as used in [Step A6] of production method A.

### [Step AA3]

In this step, the protecting group at the amino group of the 7-position is removed from compound (4aa), to give compound (5aa).

The deprotection reaction is conducted under the same conditions as used in [Step Z3] of production method Z.

### [Step AA4]

In this step, a substituent is introduced into the amino group at the 7-position of compound (5aa), through substitution reaction between compound (5aa) and compound (5aa-2), to give compound (6aa).

The substitution reaction is conducted under the same conditions as used in [Step A4] of production method A.

### [Step AA5]

In this step, the protecting group at the 1-position is removed from compound (6aa), to give compound (7aa).

The deprotection reaction is conducted under the same conditions as used in [Step Z5] of production method Z.

### [Step AA6]

In this step, R^{p3} is removed from compound (7aa) to give compound (8aa).

The deprotection reaction for R^{p3} is conducted under the same conditions as used in [Step A13] of production method A.

### Production method BB

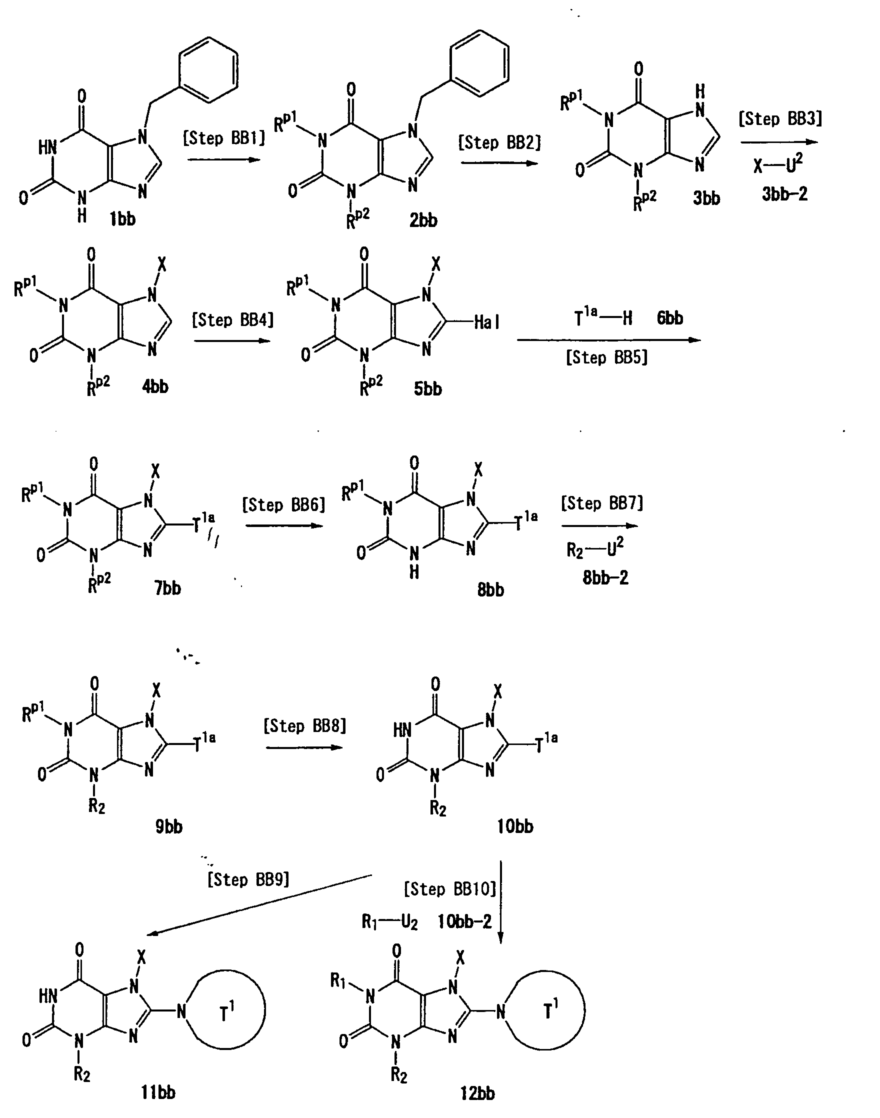

### [Step BB1]

In this step, compound (1bb) is protected at the groups at the 1- and 3-positions, to give compound (2bb).

The reaction is conducted under the same conditions as used in [Step Z2] of production method Z.

### [Step BB2]

In this step, the protecting group of the amino group at the 7-position is removed from compound (2bb), to give compound (3bb).

The deprotection reaction is conducted under the same conditions as used in [Step Z3] of production method Z.

### [Step BB3]

In this step, a substituent is introduced into the amino group at the 7-position of compound (3bb), through a substitution reaction between compound (3bb) and compound (3bb-2), to give compound (4bb).

The substitution reaction is conducted under the same conditions as used in [Step A4] of production method A.

### [Step BB4]

In this step, compound (4bb) is reacted with a halogenating agent, to give compound (5bb).

The halogenation reaction is conducted under the same conditions as used in [Step A5] of production method A.

### [Step BB5]

In this step, compound (5bb) is reacted with compound (6bb), to give compound (7bb).

The reaction is conducted under the same conditions as used in [Step A6] of production method A.

### [Step BB6]

In this step, the protecting group at the 3-position is removed from compound (7bb), to give compound (8bb).

The deprotection reaction is conducted under the same conditions as used in [Step Z5] of production method Z.

### [Step BB7]

In this step, a substituent is introduced into the group at the 3-position of compound (8bb), through substitution reaction between compound (8bb) and compound (8bb-2), to give compound (9bb).

The substitution reaction is conducted under the same conditions as used in [Step A4] of production method A.

### [Step BB8]

In this step, the protecting group at the 1-position is removed from compound (9bb), to give compound (10bb).

The deprotection reaction is conducted under the same conditions as used in [Step Z5] of production method Z.

### [Step BB9]

In this step, R^{p3} is removed from compound (10bb), to give compound (11bb).

The deprotection reaction for R^{p3} is conducted under the same conditions as used in [Step A13] of production method A.

### [Step BB10]

In this step, a substituent is introduced into the group at the 3-position of compound (10bb), through a substitution reaction between compound (10bb) and compound (10bb-2), and then R^{p3} is removed to give compound (12bb).

The substitution reaction is conducted under the same conditions as used in [Step A4] of production method A.

The deprotection reaction for R^{p3} is conducted under the same conditions as used in [Step A13] of production method A.

### Production method CC

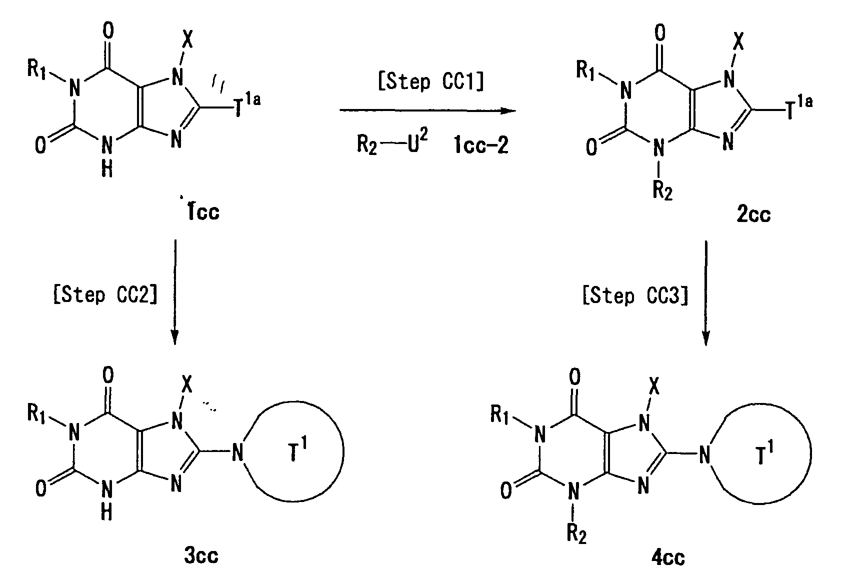

### [Step CC1]

In this step, a substituent is introduced into the group at the 3-position of compound (1cc), through a substitution reaction between compound (1cc) and compound (1cc-2), to give compound (2cc).

The substitution reaction is conducted under the same conditions as used in [Step A4] of production method A.

### [Step CC2][Step CC3]

In these steps, R^{p3} is removed from compounds (1cc) and (2cc), to give compounds (3cc) and (4cc), respectively.

The deprotection reaction for R^{p3} is conducted under the same conditions as used in [Step A13] of production method A.

The methods described above are representative methods for producing compounds (I) and (II) of the present invention. The starting compounds and various reagents to be used in the methods for producing the compounds of the present invention may be salts, hydrates, or solvates, depending on the type of starting materials and solvents to be used, and are not limited providing they do not inhibit the reactions. The types of solvents to be used depend on the types of starting compounds and reagents to be used, and are not limited providing they dissolve starting materials to some extent and do not inhibit the reactions. When compounds (I) and (II) of the present invention are obtained in free forms, such compounds can be converted to salts or hydrates, which are the possible forms of compounds (I) and (II) described above, according to a conventional method.

When compounds (I) and (II) of the present invention are obtained as salts or hydrates, such products can be converted to free forms of compounds (I) and (II), as described above, according to a conventional method.

In addition, various isomers of compounds (I) and (II) of the present invention (for example, geometric isomers, enantiomers on the basis of asymmetric carbon, rotamers, stereoisomers, and tautomers) can be purified and isolated by typical isolation techniques including recrystallization, diastereomer salt method, enzyme-based resolution method, and various chromatographic methods (for example, thin layer chromatography, column chromatography, and gas chromatography).

The pharmaceutical agents of the present invention can be obtained by combining active ingredients, *i*.*e*., a DPPIV inhibitor and a biguanide agent or a pharmaceutical agent that is able to enhance the effects of active circulating GLP-2. The active ingredients described above may be formulated separately or in combination, and they may be mixed with pharmaceutically acceptable carriers, excipients, binders, and similar. The dosage form of the pharmaceutical agents described above includes oral preparations, for example, granules, microgranules, powders, tablets, coated tablets, capsules, and syrups; and non-oral preparations, for example, injections (intravenous injections, subcutaneous injections, intramuscular injections, etc.), suppositories, and external preparations (transdermal therapeutics, ointments, etc.).

Such formulations can be achieved by using typical excipients, binders, disintegrating agents, lubricants, colorants, flavoring agents; and if required, stabilizers, emulsifiers, absorbefacients, detergents, pH adjustors, preservatives, antioxidants, etc., and materials commonly used as ingredients of pharmaceutical preparations according to conventional methods. These materials include, for example, (1) animal and vegetable oils, such as soya bean oil, beef tallow, and synthetic glyceride; (2) hydrocarbons, such as liquid paraffin, squalane, and solid paraffin; (3) ester oils, such as octyldodecyl myristate and isopropyl myristate; (4) higher alcohols, such as cetostearyl alcohol and behenyl alcohol; (5) silicon resins; (6) silicon oils; (7) detergents, such as polyoxyethylene fatty acid ester, sorbitan fatty acid ester, glycerin fatty acid ester, polyoxyethylene sorbitan fatty acid ester, polyoxyethylene hydrogenated castor oil, and polyoxyethylene polyoxypropylene block co-polymer; (8) water-soluble polymers, such as hydroxyethyl cellulose, poly-acrylic acid, carboxyvinyl polymer, polyethylene glycol, polyvinylpyrrolidone, and methyl cellulose; (9) lower alcohols, such as ethanol and isopropanol; (10) polyhydric alcohols such as glycerin, propylene glycol, dipropylene glycol, and sorbitol; (11) sugars such as glucose and sucrose; (12) inorganic powder, such as anhydrous silicic acid, magnesium aluminum silicate, and aluminum silicate; and (13) pure water.

The excipients include, for example, lactose, corn starch, white sugar, glucose, mannitol, sorbitol, crystal cellulose, and silicon dioxide. The binders include, for example, polyvinyl alcohol, polyvinyl ether, methyl cellulose, ethyl cellulose, arabic gum, tragacanth, gelatin, shellac, hydroxypropyl cellulose, hydroxypropyl methyl cellulose, polyvinylpyrrolidone, polypropylene glycol-polyoxyethylene block co-polymer, meglumine, calcium citrate, dextrin, and pectin. The disintegrating agents include, for example, starch, agar, gelatin powder, crystalline cellulose, calcium carbonate, sodium bicarbonate, calcium citrate, dextrin, pectin, and calcium carboxymethyl cellulose. The lubricants include, for example, magnesium stearate, talc, polyethylene glycol, silica, and hydrogenated vegetable oil. The colorants include those that are pharmaceutically acceptable. The flavoring agents include cocoa powder, peppermint camphor, aromatic powder, peppermint oil, Borneo camphor, and cinnamon powder. The antioxidants include those that are pharmaceutically acceptable, such as ascorbic acid and α-tocopherol.

The oral preparation can be produced by combining the active ingredients with an excipient; and if required, a binder, a disintegrating agent, a lubricant, a colorant, a flavoring agent, or such; and formulating the mixture into powders, microgranules, granules, tablets, coated tablets, capsules, or such; according to conventional methods. Tablets and granules may be coated with sugar or gelatin, or if required, any other appropriate coatings. Solutions, such as syrups or injectable preparations to be administered, can be formulated by combining a compound of the present invention with a pH adjustor, a solubilizing agent, an isotonizing agent, or such; and if required, with an auxiliary solubilizing agent, a stabilizer, a buffer, a suspending agent, an antioxidant, or the like; according to conventional methods. The solution may be freeze-dried. Examples of preferred suspending agents are: methylcellulose, Polysorbate 80, hydroxyethyl cellulose, arabic gum, powdered tragacanth, sodium carboxymethylcellulose, and polyoxyethylenesorbitan mono-laurate. Examples of preferred auxiliary solubilizing agents are: polyoxyethylene hydrogenated castor oil, Polysorbate 80, nicotinamide, and polyoxyethylenesorbitan mono-laurate. Examples of preferred stabilizers are: sodium sulfite, sodium metasulfite, and ether. Examples of preferred preservatives are: methyl para-oxybenzoate, ethyl para-oxybenzoate, sorbic acid, phenol, cresol, and chlorocresol. There are no limitations on the types of methods for producing an external preparation, and such preparations can be produced by conventional methods. Various materials commonly used for producing pharmaceuticals, quasi drugs, cosmetics, and others, including animal and vegetable oils, mineral oils, ester oils, wax, higher alcohols, fatty acids, silicone oil, detergents, phospholipids, alcohols, polyhydric alcohols, water-soluble polymers, clay minerals, and pure water can be included as base materials. Furthermore, external preparations of the present invention can contain pH adjustors, antioxidants, chelating agents, antibacterial/antifungal agents, colorants, and flavoring agents, as required. Additionally, external preparations of the present invention can also contain agents that induce differentiation, promote blood flow, activate cells, and antimicrobials, anti-inflammatories, vitamins, amino acids, humectants, keratolytics, and others, if required.

There are no particular limitations on the types of administration methods for the pharmaceutical agents, according to the present invention. A DPPIV inhibitor and either a biguanide agent or a pharmaceutical agent that enhances the effects of active circulating GLP-2, may be used in combination at the time of administration. For example, administration methods may include (1) the administration of a preparation formulated by conjugating a DPPIV inhibitor and either a biguanide agent or a pharmaceutical agent that enhances the effects of active circulating GLP-2; (2) the simultaneous administration of two types of preparations, which are obtained by separately formulating a DPPIV inhibitor and either a biguanide agent or a pharmaceutical agent which enhances the effects of active circulating GLP-2; and (3) the separate administration of two types of preparations, which are obtained by separately formulating a DPPIV inhibitor and either a biguanide agent or a pharmaceutical agent that enhances the effects of active circulating GLP-2 at different times (for example, administering them in the order of the DPPIV inhibitor and then either a biguanide agent or a pharmaceutical agent which enhances the effects of active circulating GLP-2, or in reverse order).

The dose of the pharmaceutical agents according to the present invention can be selected based on the standard dose of each agent. The dose can be appropriately selected based on patient's age, weight, sex, severity of symptoms, dosage form, and disease type. When the DPPIV inhibitor to be administered orally or parenterally is (S)-1-((3-hydroxy-1-adamantyl)amino)acetyl-2-cyanopyrrolidine or (S)-1-(2-((5-cyanopyridin-2-yl)amino)ethyl-aminoacetyl)-2-cyanopyrrolidine, the dose can typically be selected from a range of 0.1 to 250 mg/adult/day, preferably 1 to 100 mg/adult/day. When the DPPIV inhibitor to be administered orally or parenterally is isoleucine thiazolidide, isoleucine pyrrolidide, or valine pyrrolidide, the dose can typically be selected from a range of 0.01 to 2.0 mg/kg/day, preferably 0.01 to 1.0 mg/kg/day. When the DPPIV inhibitor is a compound represented by formula (I) or (II), or a salt or hydrate thereof, and it is to be administered orally to an adult, the dose can typically be selected from a range of 0.03 to 1000 mg/day, preferably 0.1 to 500 mg/day, more preferably 0.1 to 100 mg/day. When the DPPIV inhibitor is a compound represented by formula (I) or (II), or a salt or hydrate thereof, and it is to be administered parenterally to an adult, the dose can typically be selected from a range of about 1 to 3000 µg/kg/day, preferably about 3 to 1000 µg/kg/day. When the DPPIV inhibitor is to be used in combination with another agent, for example, a biguanide agent, the dose typically ranges from 10 to 2500 mg/adult/day, and preferably ranges from 100 to 1000 mg/adult/day.

In the present invention, both the DPPIV inhibitor and the biguanide agent can be administered once or several times at the daily dose described above.

The dose ratio between the respective agents in the pharmaceutical agents according to the present invention can be selected appropriately, based on patient's age, weight, sex, severity of symptoms, dosage form, and disease type. For example, the weight:weight dose ratio between the DPPIV inhibitor and the biguanide agent may typically fall within a range of 1:1 to 1:2500, preferably 1:10 to 1:250.

(S)-1-((3-hydroxy-1-adamantyl)amino)acetyl- indicated herein can be administered at a dose selected from the range of 3 to 1000 µg/kg. When a DPPIV inhibitor is used in combination with another agent, for example, a biguanide agent, the dose typically ranges from 10 to 2500 mg/adult/day, and preferably ranges from 100 to 1000 mg/adult/day.

Compounds of the present invention represented by formulae (I) and (II) indicated above, can be produced by the methods described below in Examples. However, the compounds of the present invention are under no circumstances to be construed as being limited to the specific examples described below.

### [Production Examples]

### Production Example 1

### t-Butyl 4-[1-(2-butynyl)-6-methyl-7-oxo-6,7-dihydro-1H-imidazo[4,5-d]pyridazin-2-yl]piperazin-1-carb oxylate

### (a) t-Butyl 5-methyl-4-oxo-4,5-dihydroimidazo[4,5-d]pyridazine-1-carboxylate

A mixture consisting of 1.0 g of 5-methyl-3,5-dihydroimidazo[4,5-d]pyridazin-4-one, 16 mg of 4-dimethylaminopyridine, 1.6 g of di-t-butyl dicarbonate, and 5 ml of tetrahydrofuran was stirred at room temperature overnight. Then, a 0.5-ml tetrahydrofuran solution containing 300 mg of di-t-butyl dicarbonate was added to the solution, and the resulting mixture was stirred at room temperature for three hours. 5 ml of t-butyl methyl ether was added to the reaction mixture, and the mixture was cooled with ice. The resulting crystals were collected by filtration to give 1.63 g of the title compound.
¹H-NMR(CDCl₃)
δ 1.72 (s, 9H) 3.93 (s, 3H) 8.38 (s, 1H) 8.54 (s, 1H)

### (b) 2-Chloro-5-methyl-1,5-dihydroimidazo[4,5-d]pyridazin-4-one

8.4 ml of lithium hexamethyldisilazide (1.0 M tetrahydrofuran solution) was added dropwise over one hour to a 300-ml tetrahydrofuran solution containing 1.68 g of t-butyl 5-methyl-4-oxo-4,5-dihydroimidazo[4,5-d]pyridazine-1-carboxylate and 4.15 g of hexachloroethane under a nitrogen atmosphere at 0°C. The resulting mixture was stirred for 30 minutes. 2N ammonia water was added to the solut and the mixture was stirred for three hours. Then, the reaction solution was concentrated to 50 ml, and washed with 20 ml of t-butyl methyl ether. The solution was acidified with concentrated hydrochloric acid. The resulting precipitate was collected by filtration, and washed successively with 10 ml of water and 10 ml of t-butyl methyl ether. Thus, 1.03 g of the title compound was obtained.
¹H-NMR(DMSO-d6)
δ 1.45 (s, 9H) 3.72 (s, 3H) 8.33 (s, 1H)

### (c) 3-(2-Butynyl)-2-chloro-5-methyl-3,5-dihydroimidazo[4,5-d]pyridazin-4-one

7.72 g of 2-chloro-5 methyl-1,5-dihydroimidazo[4,5-d]- pyridazin-4-one was suspended in 400 ml of tetrahydrofuran under a nitrogen atmosphere, and 14.22 g of triphenylphosphine and 3.85 g of 2-butyn-1-ol were added thereto. The resulting mixture was cooled to 0°C. A 100-ml tetrahydrofuran solution containing 12.55 g of azodicarboxylic acid di-t-butyl ester was added dropwise, and the reaction mixture was stirred for three hours. The reaction mixture was concentrated under reduced pressure. 50 ml of dichloromethane and 50 ml of trifluoroacetic acid were added to the residue, and the mixture was stirred for 15 hours. The reaction mixture was concentrated under reduced pressure. The resulting residue was dissolved in 400 ml of ethyl acetate, and washed with a 200 ml of a 5N aqueous sodium hydroxide solution. The aqueous layer was extracted with 100 ml of ethyl acetate. The organic layers were combined together, dried over magnesium sulfate, and concentrated under reduced pressure. The resulting residue was purified by silica gel column chromatography. Thus, 8.78 g of the title compound was obtained from the fraction eluted with hexane-ethyl acetate (4:1).
¹H-NMR(CDCl₃)
δ 1.82 (t, J= 2.3Hz, 3H) 3.87 (s, 3H) 5.32 (q, J=2.3Hz, 2H) 8.19 (s, 1H)

### (d) t-Butyl 4-[1-(2-butynyl)-6-methyl-7-oxo-6,7-dihydro-1H-imidazo[4,5-d]pyridazin-2-yl]piperazine-1-car boxylate

5 ml of 1-methyl-2-pyrrolidone was added to a mixture consisting of 1.183 g of 3-(2-butynyl)-2-chloro-5-methyl-3,5-dihydroimidazo [4,5-d]pyridazin-4-one, 0.829 g of potassium carbonate, and 1.395 g of t-butyl piperazine-1-carboxylate under a nitrogen atmosphere. The resulting mixture was heated at 130°C for 6 hours. The reaction mixture was cooled, and 50 ml of water was added thereto. Then, the mixture was extracted with 100 ml of ethyl acetate. The organic layer was washed twice with 50 ml of water and then with 50 ml of an aqueous solution saturated with sodium chloride. The organic layer was dried over magnesium sulfate, and concentrated under reduced pressure. The resulting residue was purified by silica gel column chromatography. Thus, 1.916 g of the title compound was obtained from the fraction eluted with hexane-ethyl acetate (1:4).
¹H-NMR(CDCl₃)
δ 1.52 (s, 9H) 1.83 (t, J=2.3Hz, 3H) 3.38-3.42 (m, 4H) 3.61-3.64 (m, 4H) 3.85 (s, 3H) 5.09 (q, J=2.3Hz, 2H) 8.13 (s, 1H)

### Production Example 2

### t-Butyl 4-[7-(2-butynyl)-2,6-dichloro-7H-purin-8-yl]piperazine-1-carboxylate

### (a) 7-(2-Butynyl)-3-methyl-3,7-dihydropurine-2,6-dione

55.3 ml of 1-bromo-2-butyne and 84.9 g of anhydrous potassium carbonate were added to a mixture of 100 g of 3-methyl xanthine [CAS No. 1076-22-8] and 1000 ml of N,N-dimethylformamide. The resulting mixture was stirred at room temperature for 18 hours. 1000 ml of water was added to the reaction solution, and the mixture was stirred at room temperature for 1 hour. The resulting white precipitate was collected by filtration. The white solid was washed with water and then t-butyl methyl ether. Thus, 112 g of the title compound was obtained.
¹H-NMR(DMSO-d6)
δ 1.82 (t, J=2.2Hz,3H) 3.34 (s, 3H) 5.06 (q, J=2.2Hz, 2H) 8.12 (s, 1H) 11.16 (br.s, 1H)

### (b) 7-(2-Butynyl)-8-chloro-3-methyl-3,7-dihydropurine-2,6-dione

112 g of 7-(2-butynyl)-3-methyl-3,7-dihydropurine-2,6-dione was dissolved in 2200 ml of N,N-dimethylformamide, and 75.3 g ofN-chlorosuccinimide was added thereto. The resulting mixture was stirred at room temperature for five hours. 2200 ml of water was added to the reaction solution, and the mixture was stirred at room temperature for 1.5 hour. The white precipitate was collected by filtration, and the white solid was washed with water and, with t-butyl methyl ether. Thus, 117 g of the title compound was obtained.
¹H-NMR(DMSO-d6)
δ 1.78 (t, J=2.0Hz,3H) 3.30 (s, 3H) 5.06 (q, J=2.0Hz, 2H) 11.34 (br.s, 1H)

### (c) 7-(2-Butynyl)-2,6,8-trichloro-7H-purine

A mixture of 2.52 g of 7-(2-butynyl)-8-chloro-3-methyl-3,7-dihydropurine-2,6-dione and 100 ml of phosphorus oxychloride was stirred at 120°C for 14 hours. After the reaction mixture had been cooled, 4.15 g of phosphorus pentachloride was added to the solution. The resulting mixture was stirred at 120°C for 24 hours. After the reaction solution had been cooled to room temperature, the solvent was evaporated under reduced pressure. The residue was dissolved in tetrahydrofuran. The solution was poured into a saturated sodium bicarbonate solution, and the mixture was extracted with ethyl acetate. The resulting organic layer was washed with water, then saturated brine, and was then concentrated under reduced pressure. The residue was purified by silica gel column chromatography (ethyl acetate: hexane = 1:3) to give 2.40 g of the title compound.
¹H-NMR(CDCl₃)
δ 1.82 (t, J=2.4Hz,3H) 5.21 (q, J=2.4Hz, 2H)

### (d) t-Butyl 4-[7-(2-butynyl)-2,6-dichloro-7H-purin-8-yl]piperazine-1-carboxylate

A mixture of 2.4 g of 7-(2-butynyl)-2,6,8-trichloro-7H-purine, 1.46 g of sodium bicarbonate, 2.43 g of t-butyl piperazine-1-carboxylate, and 45 ml of acetonitrile was stirred at room temperature for 2 hours and 20 minutes. Then, 0.73 g of sodium bicarbonate and 1.21 g of t-butyl piperazine-1-carboxylate were added, and the resulting mixture was stirred at room temperature for 1 hour. The reaction mixture was extracted with ethyl acetate-water, and the organic layer was washed with 1N hydrochloric acid, dried over anhydrous magnesium sulfate, and then concentrated under reduced pressure. The residue was triturated with diethyl ether. The crystals were collected by filtration, and washed with diethyl ether. Thus, 3.0 g of the title compound was obtained as a white solid.
¹H-NMR(DMSO-d6)
δ 1.42 (s, 9H) 1.83 (t, J=2Hz, 3H) 3.48-3.55 (m, 4H) 3.57-3.63 (m, 4H) 4.89 (q, J=2Hz, 2H)

### [Examples]

### Example 1

### Ethyl [7-(2-chlorophenyl)-1-methyl-6-oxo-8-(piperazin-1-yl)-6,7-dihydro-1H-purin-2-yloxy]acetate trifluoroacetate

### (a) [7-Benzyl-2,6-dioxo-1,2,6,7-tetrahydropurin-3-yl]methyl 2,2-dimethylpropionate

8.66 g of 7-benzylxanthine was dissolved in 300 ml of N,N-dimethylformamide, and 1.57 g of sodium hydride and 7.7 ml of chloromethyl pivalate were added thereto. The resulting mixture was stirred at room temperature overnight. The reaction solution was diluted with ethyl acetate, and washed with water and 1N hydrochloric acid. The organic layer was dried over anhydrous magnesium sulfate, then filtered. The solvent was evaporated under reduced pressure. The residue was purified by silica gel column chromatography. Thus, 2.66 g of the title compound was obtained from the fraction eluted with hexane-ethyl acetate (1:1).
¹H-NMR(CDCl₃)
δ 1.18 (s, 9H) 5.45 (s, 2H) 6.06 (s, 2H) 7.34-7.39 (m, 5H) 7.58 (s, 1H) 8.18 (s, 1H).

### (b) [7-Benzyl-1-methyl-2,6-dioxo-1,2,6,7-tetrahydropurin-3-yl]methyl 2,2-dimethylpropionate

2.66 g of [7-benzyl-2,6-dioxo-1,2,6,7-tetrahydropurin-3-yl]methyl 2,2-dimethylpropionate was dissolved in 30 ml of N,N-dimethylformamide, and 1.6 g of potassium carbonate and 1 ml of methyl iodide were added thereto. The mixture was stirred at room temperature overnight. The reaction mixture was diluted with ethyl acetate, and washed with water and 1N hydrochloric acid. The organic layer was dried over anhydrous magnesium sulfate, then filtered. The solvent was evaporated under reduced pressure. The residue was triturated with toluene. Thus, 2.16 g of the title compound was obtained.
¹H-NMR(CDCl₃)
δ 1.18 (s, 9H) 3.41 (s, 3H) 5.49 (s, 2H) 6.11 (s, 2H) 7.26-7.39 (m, 5H) 7.57 (s, 1H).

### (c) [1-Methyl-2,6-dioxo-1,2,6,7-tetrahydropurin-3-yl]methyl 2,2-dimethylpropionate

2.349 g of [7-benzyl-1-methyl-2,6-dioxo-1,2,6,7-tetrahydropurin-3-yl]methyl 2,2-dimethylpropionate was dissolved in 100 ml of acetic acid, and 1 g of 10% palladium carbon was added thereto. The mixture was stirred under a hydrogen atmosphere at room temperature overnight. The reaction mixture was filtered and concentrated to give 1.871 g of the title compound.
¹H-NMR(CDCl₃)
δ 1.19 (s, 9H) 3.48 (s, 3H) 6.17 (s, 2H) 7.83 (s, 1H).

### (d) [7-(2-Chlorophenyl)-1-methyl-2,6-dioxo-1,2,6,7-tetrahydropurin-3-yl]methyl 2,2-dimethylopropionate

1.60 g of [1-methyl-2,6-dioxo-1,2,6,7-tetrahydropurin-3-yl]methyl 2,2-dimethylpropionate, 1.83 g of 2-chlorophenylboronic acid, and 1.5 g of copper (II) acetate were suspended in 30 ml of N,N-dimethylformamide, and 3 ml of pyridine was added thereto. The mixture was stirred at room temperature for 3 days. The reaction mixture was filtered through a short column filled with silica gel, and the filtrate was diluted with ethyl acetate. The organic layer was washed with 1N hydrochloric acid, water, and saturated saline, and dried over anhydrous magnesium sulfate, then filtered. The filtrate was concentrated. The residue was suspended in ether, and the suspension was filtered. The filtrate was purified by silica gel column chromatography. Thus, 724 mg of the title compound was obtained from the fraction eluted with hexane-ethyl acetate (3:2).

### (e) t-Butyl 4-[7-(2-chlorophenyl)-3-(2,2-dimethylpropionyloxymethyl)-1-methyl-2,6-dioxo-2,3,6,7-tetrahyd ro-1H-purin-8-yl]piperazine-1-carboxylate

724 mg of [7-(2-chlorophenyl)-1-methyl-2,6-dioxo-1,2,6,7-tetrahydropurin-3-yl]methyl 2,2-dimethylpropionate was suspended in 15 ml of N,N-dimethylformamide, and 760 mg of N-chlorosuccinimide was added thereto. The reaction solution was stirred overnight, and then diluted with ethyl acetate. The solution was washed with water and 1N hydrochloric acid, and dried over anhydrous magnesium sulfate, then filtered. The filtrate was concentrated. Thus, 764 mg of [8-chloro-7-(2-chlorophenyl)-1-methyl-2,6-dioxo-1,2,6,7-tetrahydropurin-3-yl]methyl 2,2-dimethylpropionate was obtained. This compound was mixed with 4 g of t-butyl piperazine-1-carboxylate. The mixture was heated at 150°C, and stirred for three hours. Ethyl acetate and water were added to the reaction mixture, and the mixture was separated. The organic layer was washed with 1N hydrochloric acid, and dried over anhydrous magnesium sulfate, then filtered. The filtrate was concentrated. The residue was purified by silica gel column chromatography. Thus, 724 mg of the title compound was obtained from the fraction eluted with hexane-ethyl acetate (3:2).

### (f) t-Butyl 4-[7-(2-chlorophenyl)-1-methyl-2,6-dioxo-2,3,6,7-tetrahydro-1H-purin-8-yl]piperazine-1-carbox ylate

t-Butyl 4-[7-(2-chlorophenyl)-3-(2,2-dimethylpropionyloxy methyl)-1-methyl-2,6-dioxo-2,3,6,7-tetrahydro-1H-purin-8-yl]piperazine-1-carboxylate was dissolved in a mixture of 10 ml of methanol and 20 ml of tetrahydrofuran, and 200 mg of sodium hydride was added thereto. The resulting mixture was stirred at room temperature overnight. 1N hydrochloric acid was added to the reaction solution, and the mixture was extracted with ethyl acetate. The organic layer was dried over anhydrous magnesium sulfate, then filtered. The filtrate was concentrated. The residue was suspended in ether and the mixture was filtered. Thus, 450 mg of the title compound was obtained.
¹H-NMR(DMSO-d⁶)
δ 1.35 (s, 9H) 3.04 (s, 3H) 3.06-3.12 (m, 4H) 3.17-3.22 (m, 4H) 7.48 (dt, J=1.6, 7.6Hz, 1H) 7.53 (dt, J=2.0, 7.6Hz, 1H) 7.63 (dd, J=2.0, 8.0Hz, 1H) 7.65 (dd, J=1.6, 8.0Hz, 1H).

### (g) t-Butyl 4-[2-chloro-7-(2-chlorophenyl)-1-methyl-6-oxo-6,7-dihydro-1H-purin-8-yl]piperazine-1-carboxy late (g-1), and t-butyl 4-[2,6-dichloro-7-(2-chlorophenyl)-7H-purin-8-yl]piperazine-1-carboxylate (g-2)

78 mg of t-butyl 4-[7-(2-chlorophenyl)-1-methyl-2,6-dioxo-2,3,6,7-tetrahydro-1H-purin-8-yl]piperazine-1-carbox ylate was dissolved in 3 ml of phosphorus oxychloride, and the mixture was stirred at 120°C overnight. The reaction solution was concentrated, and the residue was dissolved in 1 ml of tetrahydrofuran. This solution was poured into a suspension consisting of 50 mg of di-t-butyl dicarbonate, 1 ml of tetrahydrofuran, and 0.5 ml of water containing 100 mg of sodium bicarbonate. The resulting mixture was stirred at room temperature for three hours. The reaction mixture was diluted with ethyl acetate and washed with water. The organic layer was dried over anhydrous magnesium sulfate, then filtered. The filtrate was concentrated, and the residue was purified by silica gel column chromatography. Thus, 16 mg of t-butyl 4-[2,6-dichloro-7-(2-chlorophenyl)-7H-purin-8-yl]piperazine-1-carboxylate was obtained from the fraction eluted with hexane-ethyl acetate (3:2), and
10 mg of t-butyl
4-[2-chloro-7-(2-chlorophenyl)-1-methyl-6-oxo-6,7-dihydro-1H-purin-8-yl] piperazine-1-carboxylate was obtained from the fraction eluted with hexane-ethyl acetate (1:9).

### (h) Ethyl [7-(2-chlorophenyl)-1-methyl-6-oxo-8-(piperazin-1-yl)-6,7-dihydro-1H-purin-2-yloxy]acetate trifluoroacetate

10 mg of t-butyl 4-[2-chloro-7-(2-chlorophenyl)-1-methyl-6-oxo-6,7-dihydro-1H-purin-8-yl]piperazine-1-carboxy late and 10 mg of ethyl glycolate were dissolved in 0.2 ml ofN-methylpyrrolidone, and 10 mg of sodium hydride was added thereto. The mixture was stirred at room temperature for 2 hours. The reaction solution was dissolved in ethyl acetate, and the mixture was washed with 1N hydrochloric acid. Thus, 24 mg of t-butyl 4-[7-(2-chlorophenyl)-2-ethoxycarbonylmethoxy-1-methyl-6-oxo-6,7-dihydro-1H-purin-8-yl]pip erazine-1-carboxylate was obtained. 8 mg of this compound was dissolved in trifluoroacetic acid, and the mixture was concentrated. The residue was purified by reverse-phase high performance liquid chromatography (using an acetonitrile-water mobile phase (containing 0.1% trifluoroacetic acid)) to give 2.11 mg of the title compound.
MS *m*/*e* (ESI) 447(MH⁺-CF₃COOH)

### Example 4

### Methyl 2-[7-(2-butynyl)-1-methyl-6-oxo-8-(piperazin-1-yl)-6,7-dihydro-1H-purin-2-yloxy]phenylacetate trifluoroacetate

### (a) [7-(2-Butynyl)-1-methyl-2,6-dioxo-1,2,6,7-tetrahydropurin-3-yl]methyl 2,2-dimethylpropionate

1.871 g of [1-methyl-2,6-dioxo-1,2,6,7-tetrahydropurin-3-yl]methyl 2,2-dimethylpropionate was dissolved in 30 ml of N,N-dimethylformamide, and 1.5 g of potassium carbonate and 0.7 ml of 2-butynyl bromide were added thereto. The mixture was stirred at room temperature overnight. The reaction mixture was diluted with ethyl acetate, and washed with water and 1N hydrochloric acid. The organic layer was dried over anhydrous magnesium sulfate, then filtered. The solvent was evaporated under reduced pressure, and the residue was purified by silica gel column chromatography. Thus, 2.12 g of the title compound was obtained from the fraction eluted with hexane-ethyl acetate (3:2).

### (b) 7-(2-Butynyl)-1-methyl-3,7-dihydropurine-2,6-dione

The title compound was obtained by treating [7-(2-butynyl)-1-methyl-2,6-dioxo-1,2,6,7-tetrahydropurin-3-yl]methyl 2,2-dimethylpropionate by the same method as used in Example (1f).
¹H-NMR(CDCl₃)
δ 1.91 (t, J=2.4Hz, 3H) 3.39 (s, 3H) 5.10 (s, 2H) 7.93 (s, 1H) 10.62 (s, 1H).

### (c) t-Butyl 4-[7-(2-butynyl)-1-methyl-2,6-dioxo-2,3,6,7-tetrahydro-1H-purin-8-yl]piperazine-1-carboxylate

The title compound was obtained by treating 7-(2-butynyl)-1-methyl-3,7-dihydropurine-2,6-dione by the same method as used in Example (1e).
¹H-NMR(CDCl₃)
δ 1.48 (s, 9H) 1.83 (t, J=2.4Hz, 3H) 3.37 (s, 3H) 3.37-3.39 (m, 4H) 3.58-3.60 (m, 4H) 4.87 (s, 2H) 9.68 (s, 1H).

### (d) Methyl 2-[7-(2-butynyl)-1-methyl-6-oxo-8-(piperazin-1-yl)-6,7-dihydro-1H-purin-2-yloxy]phenylacetate trifluoroacetate

8 mg of t-butyl 4-[7-(2-butynyl)-1-methyl-2,6-dioxo-2,3,6,7-tetrahydro-1H-purin-8-yl]piperazine-1-carboxylate and 10 mg of methyl 2-bromophenylacetate were dissolved in 0.2 ml of N,N-dimethylformamide, and 10 mg of potassium carbonate was added thereto. The mixture was stirred at 50°C overnight. Ethyl acetate was added to the reaction solution, and the mixture was washed with water and 1N hydrochloric acid. The organic layer was concentrated. The residue was dissolved in trifluoroacetic acid, and the mixture was concentrated. The residue was purified by reverse-phase high performance liquid chromatography (using an acetonitrile-water mobile phase (containing 0.1% trifluoroacetic acid)) to give 1.07 mg of the title compound.
MS *m*/*e* (ESI) 451(MH⁺-CF₃COOH)

### Example 7

### 7-(2-Butynyl)-2-cyclopentyloxy-1-methyl-8-(piperazin-1-yl)-1,7-dihydropurin-6-one trifluoroacetate

Using bromocyclopentane instead of methyl 2-bromophenylacetate in Example (4d), the title compound was obtained by the same method as used in Example 4.
MS *m*/*e* (ESI) 371(MH⁺-CF₃COOH)

### Example 9

### Ethyl 2-[7-(2-butynyl)-1-methyl-6-oxo-8-(piperazin-1-yl)-6,7-dihydro-1H-purin-2-yloxy]propionate

Using ethyl 2-bromopropionate instead of methyl 2-bromophenylacetate in Example (4d), trifluoroacetate of the title compound was obtained by the same method as used in Example 4. The compound was purified by chromatography using NH-silica gel (silica gel whose surface had been modified with amino groups: Fuji Silysia Chemical Ltd. NH-DM 2035). Thus, the title compound was obtained from the fraction eluted with ethyl acetate-methanol (20:1).
MS *m*/*e* (ESI) 404(MH⁺)

### Example 11

### 7-(2-Butynyl)-2-methoxy-1-methyl-8-(piperazin-1-yl)-1,7-dihydropurin-6-one trifluoroacetate

### (a) t-Butyl 4-[7-(2-butynyl)-2-chloro-1-methyl-6-oxo-6,7-dihydro-1H-purin-8-yl]piperazine-1-carboxylate(a -1), and t-butyl 4-[7-(2-butynyl)-2,6-dichloro-7H-purin-8-yl]piperazine-1-carboxylate (a-2)

5.127 g of t-butyl 4-[7-(2-butynyl)-1-methyl-2,6-dioxo-2,3,6,7-tetrahydro-1H-purin-8-yl]piperazine-1-carboxylate was dissolved in 75 ml of phosphorus oxychloride, and then the mixture was stirred at 120°C overnight. The reaction solution was concentrated, and the residue was dissolved in 50 ml of tetrahydrofuran. This solution was poured into a suspension consisting of 7 g of di-t-butyl dicarbonate, 50 ml of tetrahydrofuran, 100 g of sodium bicarbonate, and 200 ml of water, and the mixture was stirred at room temperature for one hour. The reaction mixture was diluted with ethyl acetate, and the mixture was washed with water. The organic layer was dried over anhydrous magnesium sulfate, then filtered. The filtrate was concentrated, and the residue was purified by silica gel column chromatography. Thus, 1.348 g of t-butyl 4-[7-(2-butynyl)-2,6-dichloro-7H-purin-8-yl]piperazine-1-carboxylate [¹H-NMR(CDCl₃) δ 1.50 (s, 9H) 1.87 (t, J=2.4Hz, 3H) 3.64 (m, 8H) 4.81 (q, J=2.4Hz, 2H)] was obtained from the fraction eluted with hexane-ethyl acetate (1:1), and 1.238 g of t-butyl 4-[7-(2-butynyl)-2-chloro-1-methyl -6-oxo-6,7-dihydro-1H-purin-8-yl]piperazine-1-carboxylate [¹H-NMR(CDCl₃) δ 1.49 (s, 9H) 1.83 (t, J=2.4Hz, 3H) 3.42-3.44 (m, 4H) 3.59-3.62 (m, 4H) 3.73 (s, 3H) 4.93 (q, J=2.4Hz, 2H)] was obtained from the fraction eluted with hexane-ethyl acetate (1:9).

### (b) 7-(2-Butynyl)-2-methoxy-1-methyl-8-(piperazin-1-yl)-1,7-dihydropurin-6-one trifluoroacetate

8 mg of t-butyl 4-[7-(2-butynyl)-2-chloro-1-methyl-6-oxo-6,7-dihydro-1H-purin-8-yl]piperazine-1-carboxylate was dissolved in 0.2 ml of methanol, and 10 mg of sodium hydride was added thereto. The mixture was stirred at room temperature for one hour. 1N hydrochloric acid was added to the reaction solution, and the mixture was extracted with ethyl acetate. The organic layer was concentrated, and the residue was dissolved in trifluoroacetic acid. The mixture was concentrated, and the residue was purified by reverse-phase high performance liquid chromatography (using an acetonitrile-water mobile phase (containing 0.1% trifluoroacetic acid)) to give 1.72 mg of the title compound.
MS *m*/*e* (ESI) 317(MH⁺-CF₃COOH)

### Example 12

### 7-(2-Butynyl)-2-ethoxy-1-methyl-8-(piperazin-1-yl)-1,7-dihydropurin-6-one

Using ethanol instead of methanol in Example (11b), the trifluoroacetate of the title compound was obtained by the same method as used in Example 11. This compound was purified by chromatography using NH-silica gel. Thus, the title compound was obtained from the fraction eluted with ethyl acetate-methanol (20:1).
¹H-NMR(CDCl₃)
δ 1.42 (t, J=7.2Hz, 3H) 1.82 (t, J=2.4Hz, 3H) 3.02-3.06 (m, 4H) 3.40-3.42 (m, 4H) 3.46 (s, 3H) 4.51 (q, J=7.2Hz, 2H) 4.90 (q, J=2.4Hz, 2H).
MS *m*/*e* (ESI) 331 (MH⁺)

### Example 13

### Ethyl [7-(2-butynyl)-1-methyl-6-oxo-8-(piperazin-1-yl)-6,7-dihydro-1H-purin-2-yloxy]acetate

### Example 14

### [7-(2-Butynyl)-1-methyl-6-oxo-8-(piperazin-1-yl)-6,7-dihydro-1H-purin-2-yloxy]acetic acid

Ethyl [7-(2-butynyl)-1-methyl-6-oxo-8-(piperazin-1-yl)-6,7-dihydro-1H-purin-2-yloxy]acetate trifluoroacetate and [7-(2-butynyl)-1-methyl-6-oxo-8-(piperazin-1-yl)-6,7-dihydro-1H-purin-2-yloxy]acetic acid trifluoroacetate [MS *m*/*e* (ESI) 361(MH⁺-CF₃COOH)] were obtained by treating t-butyl 4-[7-(2-butynyl)-2-chloro-1-methyl-6-oxo-6,7-dihydro-1H-purin-8-yl]piperazine-1-carboxylate using ethyl 2-hydroxyacetate, instead of ethanol, by the same method as used in Example 11. Ethyl [7-(2-butynyl)-1-methyl-6-oxo-8-(piperazin-1-yl)-6,7-dihydro-1H-purin-2-yloxy] acetate trifluoroacetate was purified by chromatography using NH-silica gel. Thus, ethyl [7-(2-butynyl)-1-methyl-6-oxo-8-(piperazin-1-yl)-6,7-dihydro-1H-purin-2-yloxy]acetate [¹H-NMR(CDCl₃) δ 1.29 (t, J=7.2Hz, 3H) 1.83 (t, J=2.4Hz, 3H) 3.02-3.06 (m, 4H) 3.38-3.41 (m, 4H) 3.55 (s, 3H) 4.22 (q, J=7.2Hz, 2H) 4.90 (q, J=2.4Hz, 2H) 5.03 (s, 2H) ; MS *m*/*e* (ESI) 389(MH⁺)] was obtained from the fraction eluted with ethyl acetate-methanol (20:1)

### Example 16

### Ethyl 1-[7-(2-butynyl)-1-methyl-6-oxo-8-(piperazin-1-yl)-6,7-dihydro-1H-purin-2-yloxy]cyclopropane carboxylate

Using ethyl 1-hydroxycyclopropanecarboxylate instead of ethyl 2-hydroxyacetate in Example 13, the trifluoroacetate of the title compound was obtained by the same method as used in Example 13. The compound was purified by chromatography using NH-silica gel. Thus, the title compound was obtained from the fraction eluted with ethyl acetate-methanol (20:1).
1H-NMR(CDCl₃)
δ 1.19 (t, J=7.2Hz, 3H) 1.39-1.42 (m, 2H) 1.67-1.71 (m, 2H) 1.83 (t, J=2.4Hz, 3H) 3.02-3.05 (m, 4H) 3.37-3.40 (m, 4H) 3.49 (s, 3H) 4.14 (q, J=7.2Hz, 2H) 4.90 (q, J=2.4Hz, 2H)
MS *m*/*e* (ESI) 415(MH⁺)

### Example 20

### 7-(2-Butynyl)-1-methyl-2-phenoxy-8-(piperazin-1-yl)-1,7-dihydropurin-6-one trifluoroacetate

Using phenol instead of ethyl 2-hydroxyacetate in Example 13, the title compound was obtained by the same method as used in Example 13.
MS *m*/*e* (ESI) 379(MH⁺-CF₃COOH)

### Example 22

### 7-(2-Butynyl)-1,2-dimethyl-8-(piperazin-1-yl)-1,7-dihydropurin-6-one trifluoroacetate

8 mg of t-butyl 4-[7-(2-butynyl)-2-chloro-1-methyl-6-oxo-6,7-dihydro-1H-purin-8-yl]piperazine-1-carboxylate and 2 mg of tetrakis(triphenylphosphine)palladium were dissolved in 0.2 ml of dioxane, and 0.2 ml of methylzinc chloride (1.5 M tetrahydrofuran solution) was added thereto. The mixture was stirred at 50°C for 0.5 hour. The reaction solution was concentrated, and the residue was dissolved in trifluoroacetic acid. The mixture was concentrated, and the residue was purified by reverse-phase high performance liquid chromatography (using an acetonitrile-water mobile phase (containing 0.1% trifluoroacetic acid)) to give 4.56 mg of the title compound.
MS *m*/*e* (ESI) 301(MH⁺-CF₃COOH)

### Example 29

### 7-(2-Butynyl)-1-methyl-2-dimethylamino-8-(piperazin-1-yl)-1,7-dihydropurin-6-one trifluoroacetate

8 mg of t-butyl 4-[7-(2-butynyl)-2-chloro-1-methyl-6-oxo-6,7-dihydro-1H-purin-8-yl] piperazine-1-carboxylate was dissolved in 0.2 ml of an aqueous solution of 40% dimethylamine, and the mixture was stirred at 80°C for 5 hours. The reaction solution was concentrated, and the residue was dissolved in trifluoroacetic acid. The mixture was concentrated, and the residue was purified by reverse-phase high performance liquid chromatography (using an acetonitrile-water mobile phase (containing 0.1% trifluoroacetic acid)) to give 6.95 mg of the title compound.
¹H-NMR(CDCl₃)
δ 1.82 (t, J=2.4Hz, 3H) 2.83 (s, 6H) 3.02-3.05 (m, 4H) 3.39-3.42 (m, 4H) 3.56 (s, 3H) 4.90 (d, J=2.4Hz, 2H)
MS *m*/*e* (ESI) 330(MH⁺-CF₃COOH)

### Example 41

### 7-(2-Butynyl)-2-(2-ethoxyethylamino)-1-methyl-8-(piperazin-1-yl)-1,7-dihydro-purin-6-one trifluoroacetate

10 mg of t-butyl 4-[7-(2-butynyl)-2-chloro-1-methyl-6-oxo-6,7-dihydro-1H-purin-8-yl] piperazine-1-carboxylate was dissolved in 0.15 ml of 1-methyl-2-pyrrolidone, and 20 µl of 2-ethoxyethylamine was added thereto. After the mixture had been stirred at 80°C for 12 hours, the reaction solution was concentrated by flushing with nitrogen. The resulting residue was dissolved in 0.40 ml of trifluoroacetic acid, and the mixture was concentrated by flushing with nitrogen gas. The residue was purified by reverse-phase high performance liquid chromatography (using an acetonitrile-water mobile phase (containing 0.1% trifluoroacetic acid)) to give 6.95 mg of the title compound.
MS *m*/*e* (ESI) 374(MH⁺-CF₃COOH)

### Example 53

### (S)-1-[7-(2-Butynyl)-1-methyl-6-oxo-8-(piperazin-1-yl)-6,7-dihydro-1H-purin-2-yl]pyrrolidine-2 -carboxylic acid trifluoroacetate

Using L-proline t-butyl ester instead of 2-ethoxyethylamine in Example 41, 4.07 mg of the title compound was obtained by the same method as used in Example 41.
MS *m*/*e* (ESI) 400(MH⁺-CF₃COOH)

### Example 63

### (R)-1-[7-(2-Butynyl)-1-methyl-6-oxo-8-(piperazin-1-yl)-6,7-dihydro-1H-purin-2-yl]pyrrolidine-2-carboxylic acid trifluoroacetate

6 mg of t-butyl 4-[7-(2-butynyl)-2-chloro-1-methyl-6-oxo-6,7-dihydro-1H-purin-8-yl]piperazine-1-carboxylate was dissolved in 0.15 ml of 1-methyl-2-pyrrolidone, and 15 mg of D-proline methyl ester hydrochloride and 50 µl of triethylamine were added thereto. After the resulting mixture had been stirred at 80°C for 12 hours, the reaction solution was concentrated by flushing with nitrogen gas. The residue was dissolved in a solution consisting of 0.20 ml of ethanol and 0.20 ml of a 5N aqueous sodium hydroxide solution. The mixture was stirred at room temperature for five hours, and then concentrated by flushing with nitrogen gas. The residue was dissolved in 0.40 ml of trifluoroacetic acid, and the mixture was concentrated by flushing with nitrogen gas. The residue was purified by reverse-phase high performance liquid chromatography (using an acetonitrile-water mobile phase (containing 0.1% trifluoroacetic acid)) to give 3.42 mg of the title compound.
MS *m*/*e* (ESI) 400(MH⁺-CF₃COOH)

### Example 64

### 2-[7-(2-Butynyl)-1-methyl-6-oxo-8-(piperazin-1-yl)-6,7-dihydro-1H-purin-2-ylamino]propionic acid trifluoroacetate

Using DL-alanine methyl ester hydrochloride instead of D-proline methyl ester hydrochloride in Example 63, 1.12 mg of the title compound was obtained by the same method as used in Example 63.
MS *m*/*e* (ESI) 374(MH⁺-CF₃COOH)

### Example 68

### Methyl [7-(2-butynyl)-1-methyl-6-oxo-8-(piperazin-1-yl)-6,7-dihydro-1H-purin-2-ylsulfanyl]acetate trifluoroacetate

6 mg of t-butyl 4-[7-(2-butynyl)-2-chloro-1-methyl-6-oxo-6,7-dihydro-1H-purin-8-yl]piperazine-1-carboxylate was dissolved in 0.15 ml of 1-methyl-2-pyrrolidone, and 20 µl of methyl mercaptoacetate and 6 mg of potassium carbonate were added thereto. The mixture was stirred at room temperature for five hours. An aqueous solution saturated with ammonium chloride was added to the reaction solution, and the mixture was extracted with ethyl acetate. The organic layer was concentrated, and the residue was dissolved in 0.40 ml of trifluoroacetic acid. The solution was concentrated by flushing with nitrogen gas. The residue was purified by reverse-phase high performance liquid chromatography (using an acetonitrile-water mobile phase (containing 0.1% trifluoroacetic acid)) to give 4.83 mg of the title compound.
MS *m*/*e* (ESI) 391 (MH⁺-CF₃COOH)

### Example 73

### 7-(2-Butynyl)-1-methyl-8-(piperazin-1-yl)-2-(pyridin-2-ylsulfanyl)-1,7-dihydropurin-6-one trifluoroacetate

Using 2-mercaptopyridine instead of methyl mercaptoacetate in Example 68, 4.66 mg of the title compound was obtained by the same method as used in Example 68.
MS *m*/*e* (ESI) 396(MH⁺-CF₃COOH)

### Example 76

### 7-(2-Butynyl)-2-isopropylsulfanyl-1-methyl-8-(piperazin-1-yl)-1,7-dihydropurin-6-one trifluoroacetate

6 mg oft-butyl 4-[7-(2-butynyl)-2-chloro-1-methyl-6-oxo-6,7-dihydro-1H-purin-8-yl]piperazine-1-carboxylate was dissolved in 0.15 ml of 1-methyl-2-pyrrolidone, and 15 mg of the sodium salt of propane-2-thiol was added thereto. The mixture was stirred at room temperature for five hours. A saturated ammonium chloride solution was added to the reaction solution, and the mixture was extracted with ethyl acetate. The organic layer was concentrated, and the residue was dissolved in 0.40 ml of trifluoroacetic acid. The solution was concentrated by flushing with nitrogen gas. The residue was purified by reverse-phase high performance liquid chromatography (using an acetonitrile-water mobile phase (containing 0.1% trifluoroacetic acid)) to give 4.56 mg of the title compound.
MS *m*/*e* (ESI) 361(MH⁺-CF₃COOH)

### Example 79

### [7-(2-Butynyl)-1-methyl-6-oxo-8-(piperazin-1-yl)-6,7-dihydro-1H-purin-2-ylsulfanyl]acetic acid trifluoroacetate

6 mg of t-butyl 4-[7-(2-butynyl)-2-chloro-1-methyl-6-oxo-6,7-dihydro-1H-purin-8-yl]piperazine-1-carboxylate was dissolved in 0.15 ml of N-methylpyrrolidone, and 20 µl of methyl mercaptoacetate and 6 mg of potassium carbonate were added thereto. After the mixture had been stirred at room temperature for five hours, an aqueous solution saturated with ammonium chloride was added to the reaction solution. The mixture was extracted with ethyl acetate. The organic layer was concentrated. The resulting residue was dissolved in a solution consisting of 0.20 ml of ethanol and 0.20 ml of a 5N aqueous sodium hydroxide solution. The mixture was stirred at room temperature overnight, and then concentrated by flushing with nitrogen gas. The residue was dissolved in 0.40 ml of trifluoroacetic acid, and the solution was concentrated by flushing with nitrogen gas. The residue was purified by reverse-phase high performance liquid chromatography (using an acetonitrile-water mobile phase (containing 0.1% trifluoroacetic acid)) to give 0.96 mg of 7-(2-butynyl)-2-mercapto-1-methyl-8-(piperazin-1-yl)-1,7-dihydropurin-6-one trifluoroacetate [MS *m*/*e* (ESI)319(MH⁺-CF₃COOH)] and 0.61 mg of [7-(2-butynyl)-1-methyl-6-oxo-8-(piperazin-1-yl)-6,7-dihydro-1H-purin-2-ylsulfanyl]acetic acid trifluoroacetate [MS *m*/*e* (ESI)377(MH⁺-CF₃COOH)].

### Example 82

### 7-(2-Butynyl)-2-cyano-1-methyl-8-(piperazin-1-yl)-1,7-dihydropurin-6-one trifluoroacetate

8 mg of t-butyl 4-[7-(2-butynyl)-2-chloro-1-methyl-6-oxo-6,7-dihydro-1H-purin-8-yl]piperazine-1-carboxylate was dissolved in 0.2 ml of N-methylpyrrolidone, and 10 mg of sodium cyanide was added thereto. The mixture was stirred at 50°C for 1 hour. Water was added to the reaction mixture, and the mixture was extracted with ethyl acetate. The organic layer was concentrated to give 14 mg of t-butyl 4-[7-(2-butynyl)-2-cyano-1-methyl-6-oxo-6,7-dihydro-1H-purin-8-yl]piperazine-1-carboxylate. 5 mg of this compound was dissolved in trifluoroacetic acid, and the solution was concentrated. The residue was purified by reverse-phase high performance liquid chromatography (using an acetonitrile-water mobile phase (containing 0.1% trifluoroacetic acid)) to give 4.12 mg of the title compound.
MS *m*/*e* (ESI) 312(MH⁺-CF₃COOH)

### Example 83

### 7-(2-Butynyl)-1-methyl-6-oxo-8-(piperazin-1-yl)-6,7-dihydro-1H-purine-2-carboxamide

### (a) t-Butyl 4-[7-(2-butynyl)-2-carbamoyl-1-methyl-6-oxo-6,7-dihydro-1H-purin-8-yl]piperazine-1-carboxyl ate

176 mg of t-butyl 4-[7-(2-butynyl)-2-chloro-1-methyl-6-oxo-6,7-dihydro-1H-purin-8-yl]piperazine-1-carboxylate was dissolved in 2 ml of N-methylpyrrolidone, and 100 mg of sodium cyanide was added thereto. The mixture was stirred at 50°C for 0.5 hour. Water was added to the reaction mixture, and the mixture was extracted with ethyl acetate. The organic layer was concentrated to give 170 mg of t-butyl 4-[7-(2-butynyl)-2-cyano-1-methyl-6-oxo-6,7-dihydro-1H-purin-8-yl]piperazine-1-carboxylate. 98 mg of this compound was dissolved in a mixture of 3 ml of tetrahydrofuran and 2 ml of methanol, and 0.5 ml of an aqueous solution of 20% ammonia and 0.5 ml of an aqueous solution of 30% hydrogen peroxide were added thereto. The mixture was stirred at room temperature overnight. Ethyl acetate was added to the reaction solution, and the mixture was washed with water. The organic layer was dried over anhydrous magnesium sulfate, then filtered. The solvent was evaporated under reduced pressure. The residue was purified by silica gel column chromatography. Thus, 77 mg of the title compound was obtained from the fraction eluted with ethyl acetate-methanol.
¹H-NMR(CDCl₃)
δ 1.49 (s, 9H) 1.83 (t, J=1.2Hz, 3H) 3.42-3.49 (m, 4H) 3.58-3.65 (m, 4H) 3.95 (s, 3H) 5.01 (d, J=2.4Hz, 2H) 5.54 (br, 1H) 7.61 (br, 1H)

### (b) 7-(2-Butynyl)-1-methyl-6-oxo-8-(piperazin-1-yl)-6,7-dihydro-1H-purine-2-carboxamide

77 mg of t-butyl 4-[7-(2-butynyl)-2-carbamoyl-1-methyl-6-oxo-6,7-dihydro-1H-purin-8-yl]piperazine-1-carboxyl ate was dissolved in 1 ml of trifluoroacetic acid, and the solution was concentrated. The residue was purified by chromatography using NH-silica gel. Thus, 49 mg of the title compound was obtained from the fraction eluted with ethyl acetate-methanol (5:1).
¹H-NMR(CDCl₃)
δ 1.83 (t, J=2.4Hz, 3H) 3.05-3.07 (m, 4H) 3.45-3.48 (m, 4H) 3.94 (s, 3H) 4.98 (s, 2H) 5.57 (br, 1H) 7.65 (br, 1H)

### Example 86

### 7-(2-Butynyl)-2-methoxy-1-(2-phenylethyl)-8-(piperazin-1-yl)-1,7-dihydropurin-6-one hydrochloride

### (a)[7-Benzyl-2,6-dioxo-1-(2-phenylethyl)-1,2,6,7-tetrahydropurin-3-yl]methyl 2,2-dimethylpropionate

A mixture consisting of 500 mg of [7-benzyl-2,6-dioxo-1,2,6,7-tetrahydropurin-3-yl]methyl 2,2-dimethylpropionate, 0.38 ml of 2-bromoethyl benzene, 390 mg of anhydrous potassium carbonate, and 5 ml of N,N-dimethylformamide was stirred in an oil bath at 50°C for two hours. The reaction mixture was extracted with ethyl acetate and water, and the organic layer was washed with water and then with saturated saline. The organic liquid was dried over anhydrous magnesium sulfate, and then concentrated under reduced pressure. The residue was crystallized with ethyl acetate-hexane to give 540 mg of the title compound.
¹H-NMR(CDCl₃)
δ 1.19 (s, 9H) 2.92-2.98 (m, 2H) 4.19-4.25 (m, 2H) 5.48 (s, 2H) 6.11 (s, 2H) 7.17-7.40 (m, 10H) 7.54 (s, 1H)

### (b)[7-(2-Butynyl)-8-chloro-2,6-dioxo-1-(2-phenylethyl)-1,2,6,7-tetrahydropurin-3-yl]methyl 2,2-dimethyl propionate

A mixture consisting of 540 mg of [7-benzyl-2,6-dioxo-1-(2-phenylethyl)-1,2,6,7-tetrahydropurin-3-yl]methyl 2,2-dimethylpropionate, 50 mg of 10% palladium carbon, and 8 ml of acetic acid was stirred under a hydrogen atmosphere at room temperature overnight. The reaction mixture was filtered and then concentrated under reduced pressure to give 410 mg of residue.

The entire residue was combined with 0.15 ml of 1-bromo-2-butyne, 300 mg of anhydrous potassium carbonate, and 5 ml of N,N-dimethylformamide. The mixture was stirred at room temperature for 2 hours. The reaction solution was extracted with ethyl acetate and water. The organic layer was washed with water and then with saturated brine. The organic liquid was dried over anhydrous magnesium sulfate and concentrated under reduced pressure to give 470 mg of residue.

The entire residue was combined with 180 mg of N-chlorosuccinimide and 5 ml of N,N-dimethylformamide. The mixture was stirred at room temperature for 2 hours. After 0.5 ml of an aqueous solution of 1M sodium thiosulfate had been added to the reaction solution, the mixture was extracted with ethyl acetate and water. The organic layer was washed with water and then with saturated brine. The organic liquid was dried over anhydrous magnesium sulfate, and then concentrated under reduced pressure. 380 mg of the title compound was obtained by crystallization using ethyl acetate-hexane.
¹H-NMR(CDCl₃)
δ 1.21 (s, 9H) 1.83 (t, J=2Hz, 3H) 2.92-2.98 (m, 2H) 4.19-4.25 (m, 2H) 5.11 (q, J=2Hz, 2H) 6.05 (s, 2H) 7.18-7.32 (m, 5H)

### (c) t-Butyl 4-[7-(2-butynyl)-2,6-dioxo-1-(2-phenylethyl)-2,3,6,7-tetrahydro-1H-purin-8-yl]piperazine-1-car boxylate

A mixture consisting of 3 80 mg of[7-(2-butynyl)-8-chloro-2,6-dioxo-1-(2-phenylethyl)-1,2,6,7-tetrahydropurin-3-yl]methyl 2,2-dimethyl propionate, 460 mg of t-butyl piperazine-1-carboxylate, and 0.5 ml of N-methylpyrrolidone was stirred in an oil bath at 150°C for 15 minutes. The reaction mixture was extracted with ethyl acetate and water, and the organic layer was washed with water and then with saturated brine. The organic layer was dried over anhydrous magnesium sulfate, and then concentrated under reduced pressure. The residue was dissolved in ethyl acetate/hexane (1/1). The solution was filtered through a small amount of silica gel, and then washed with ethyl acetate/hexane (1/1). The filtrate was combined with the washing solution. The mixed solution was concentrated under reduced pressure to give 570 mg of residue.

The entire residue was combined with 5 ml of tetrahydrofuran and 2.5 ml of methanol. 33 mg of sodium hydride was added to the mixture, and the resulting mixture was stirred at room temperature for 30 minutes. 1 ml of 1 N hydrochloric acid was added to the reaction solution, and then the mixture was extracted with ethyl acetate and water, then was washed with water and then with saturated brine. The organic liquid was dried over anhydrous magnesium sulfate, and concentrated under reduced pressure to give 350 mg of the title compound.
¹H-NMR(CDCl₃)
δ 1.50 (s, 9H) 1.85 (t, J=2Hz, 3H) 2.91-2.98 (m, 2H) 3.37 (br.s, 4H) 3.56-3.62 (m, 4H) 4.15-4.22 (m, 2H) 4.87 (q, J=2Hz, 2H) 7.18-7.35 (m, 5H)

### (d) t-Butyl 4-[7-(2-butynyl)-2-chloro-6-oxo-1-(2-phenylethyl)-6,7-dihydro-1H-purin-8-yl]piperazine-1-carb oxylate

A mixture consisting of 290 mg of t-butyl 4-[7-(2-butynyl)-2,6-dioxo-1-(2-phenylethyl)-2,3,6,7-tetrahydro-1H-purin-8-yl]piperazine-1-car boxylate and 4 ml of phosphorus oxychloride was heated and stirred in an oil bath at 120°C for 8 hours. The reaction solution was concentrated under reduced pressure, and the residue was dissolved in 5 ml of tetrahydrofuran. This solution was added dropwise to a mixture consisting of 250 mg of di-t-butyl dicarbonate, 10 ml of a saturated sodium bicarbonate solution, and 10 ml of tetrahydrofuran while the mixture was being stirred and cooled with ice. The mixture was incubated at room temperature for 4 hours, and then extracted with ethyl acetate. The organic layer was washed with water then with saturated brine, dried over anhydrous magnesium sulfate, and then concentrated under reduce pressure. The residue was purified by silica gel column chromatography using 30 to 50% ethyl acetate/hexane. Then, the material was further purified by reverse-phase column chromatography using 50 to 100% methanol/water to give 60 mg of the title compound.
¹H-NMR(CDCl₃)
δ 1.49 (s, 9H) 1.84 (t, J=2Hz, 3H) 3.10-3.16 (m, 2H) 3.40-3.46 (m, 2H) 3.57-3.63 (m, 4H) 4.42-4.49 (m, 4H) 4.94 (q, J=2Hz, 2H) 7.21-7.34 (m, 5H)

### (e) 7-(2-Butynyl)-2-methoxy-1-(2-phenylethyl)-8-(piperazin-1-yl)-1,7-dihydropurin-6-one hydrochloride

10 mg of sodium hydride (60%; oily) was added to a mixture consisting of 7 mg of t-butyl 4-[7-(2-butynyl)-2-chloro-6-oxo-1-(2-phenylethyl)-6,7-dihydro-1H-purin-8-yl]piperazine-1-carb oxylate and 0.5 ml of methanol. The mixture was stirred at room temperature for 20 minutes. Water was added to the reaction solution. The mixture was extracted with ethyl acetate. The organic layer was washed with water and then with saturated brine, and concentrated. 0.5 ml of trifluoroacetic acid was added to the residue. The mixture was stirred at room temperature for 30 minutes, and then concentrated. The residue was purified by reverse-phase column chromatography using 20 to 80% methanol/water (containing 0.1% concentrated hydrochloric acid) to give 4.3 mg of the title compound.
¹H-NMR(DMSO-d6)
δ 1.80 (br.s, 3H) 2.85 (t, J=7Hz, 2H) 3.28 (br.s, 4H) 3.48-3.54 (m, 4H) 3.83 (s, 3H) 4.15 (t, J=7Hz, 2H) 4.97 (br.s, 2H) 7.16-7.24 (m, 3H) 7.29 (t, J=8Hz, 2H) 9.08 (br.s, 2H)

### Example 88

### Methyl [7-(2-butynyl)-6-oxo-1-(2-phenylethyl)-8-(piperazin-1-yl)-6,7-dihydro-1H-purin-2-ylsulfanyl]ac etate hydrochloride

Using methyl thioglycolate instead of methanol and using potassium carbonate as a base in Example 86(e), the title compound was synthesized by the same method as used in Example 86.
¹H-NMR(DMSO-d6)
δ 1.80 (s, 3H) 2.96 (t, J=8Hz, 2H) 3.29 (br.s, 4H) 3.50-3.56 (m, 4H) 3.68 (s, 3H) 4.16 (s, 2H) 4.23 (t, J=8Hz, 2H) 4.99 (s, 2H) 7.24-7.38 (m, 5H) 8.96 (br.s, 2H)

### Example 95

### 7-(2-Butynyl)-2-chloro-8-(piperazin-1-yl)-1,7-dihydropurin-6-one trifluoroacetate

### (a) t-Butyl 4-[7-(2-butynyl)-2-chloro-6-oxo-6,7-dihydro-1H-purin-8-yl]piperazine-1-carboxylate

A mixture consisting of 1.0 g of t-butyl 4-[7-(2-butynyl) -2,6-dichloro-7H-purin-8-yl]piperazine-1-carboxylate, 580 mg of sodium acetate, and 10 ml of dimethyl sulfoxide was stirred in an oil bath at 80°C for 24 hours. The reaction solution was extracted with ethyl acetate and water. The organic layer was washed with water and then with saturated brine, then was dried over anhydrous magnesium sulfate, and concentrated under reduced pressure. The residue was purified by silica gel column chromatography using 50 to 70% ethyl acetate/hexane and crystallized with ethyl acetate-hexane to give 800 mg of the title compound.
¹H-NMR(CDCl₃)
δ 1.49 (s, 9H) 1.83 (t, J=2Hz, 3H) 3.44 (br.s, 4H) 3.56-3.63 (m, 4H) 4.94 (q, J=2Hz, 2H)

### (b) 7-(2-Butynyl)-2-chloro-8-(piperazin-1-yl)-1,7-dihydropurin-6-one trifluoroacetate

8 mg of t-butyl 4-[7-(2-butynyl)-2-chloro-6-oxo-6,7-dihydro-1H-purin-8-yl]piperazine-1-carboxylate was dissolved in trifluoroacetic acid, and the solution was concentrated. The residue was purified by reverse-phase high performance liquid chromatography (using an acetonitrile-water mobile phase (containing 0.1% trifluoroacetic acid)) to give 3.45 mg of the title compound.
MS *m*/*e* (ESI) 307(MH⁺-CF₃COOH)

### Example 96

### 2-[7-(2-Butynyl)-2-dimethylamino-6-oxo-8-(piperazin-1-yl)-6,7-dihydropurin-1-ylmethyl]benzo nitrile hydrochloride

### (a) t-Butyl 4-[7-(2-butynyl)-2-chloro-1-(2-cyanobenzyl)-6-oxo-6,7-dihydro-1H-purin-8-yl]piperazine-1-car boxylate

A mixture consisting of 100 mg of t-butyl 4-[7-(2-butynyl)-2-chloro-6-oxo-6,7-dihydro-1H-purin-8-yl]piperazine-1-carboxylate, 60 mg of 2-cyanobenzyl bromide, 68 mg of anhydrous potassium carbonate, and 1 ml of N,N-dimethylformamide was stirred at room temperature for 4 hours. Ethyl acetate/hexane (1/1) and water were added to the reaction solution. The insoluble material was removed by filtration. The filtrate was extracted with ethyl acetate. The organic layer was washed with water and then with saturated brine, dried over anhydrous magnesium sulfate, and then concentrated under reduced pressure. The residue was purified by silica gel column chromatography using 30 to 50% ethyl acetate/hexane to give 50 mg of the title compound.
¹H-NMR(CDCl₃)
δ 1.49 (s, 9H) 1.83 (t, J=2Hz, 3H) 3.43-3.49 (m, 4H) 3.58-3.64 (m, 4H) 4.95 (q, J=2Hz, 2H) 5.72 (s, 2H) 7.06 (d, J=8Hz, 1H) 7.39 (t, J=8Hz, 1H) 7.51 (t, J=8Hz, 1H) 7.71 (d, J=8Hz, 1H)

### (b) t-Butyl

### 4-[7-(2-butynyl)-1-(2-cyanobenzyl)-2-dimethylamino-6-oxo-6,7-dihydro-1H-purin-8-yl]piperazi ne-1-carboxylate

A mixture consisting of 8 mg of t-butyl 4-[7-(2-butynyl)-2-chloro-1-(2-cyano benzyl)-6-oxo-6,7-dihydro-1H-purin-8-yl] piperazine-1-carboxylate, 20 µl of an aqueous solution of 50% dimethylamine, and 0.2 ml of N,N-dimethylformamide was stirred at room temperature for 2 hours. The reaction solution was extracted with ethyl acetate and water. The organic layer was washed with water and with saturated brine, and concentrated. The residue was separated by silica gel thin-layer chromatography using 70% ethyl acetate/hexane to give 6.5 mg of the title compound.
¹H-NMR(CDCl₃)
δ 1.50 (s, 9H) 1.81 (t, J=2Hz, 3H) 2.73 (s, 6H) 3.38-3.45 (m, 4H) 3.56-3.64 (m, 4H) 4.91, (q, J=2Hz, 2H) 5.55 (s, 2H) 7.07 (d, J=8Hz, 1H) 7.32 (t, J=8Hz, 1H) 7.46, (t, J=8Hz, 1H) 7.65 (d, J=8Hz, 1H)

### (c) 2-[7-(2-Butynyl)-2-dimethylamino-6-oxo-8-(piperazin-1-yl)-6,7-dihydropurin-1-ylmethyl]benzo nitrile hydrochloride

6.5 mg of t-butyl 4-[7-(2-butynyl)-1-(2-cyanobenzyl)-2-dimethylamino-6-oxo-6,7-dihydro-1H-purin-8-yl] piperazine-1-carboxylate was dissolved in 0.5 ml of trifluoroacetic acid, and the mixture was allowed to stand at room temperature for 20 minutes. The reaction solution was concentrated, and the residue was purified by reverse-phase column chromatography using 20 to 80% methanol/water (containing 0.1% concentrated hydrochloric acid) to give 6.4 mg of the title compound.
¹H-NMR(DMSO-d6)
δ 1.76 (s, 3H) 2.69 (s, 6H) 3.28 (br.s, 4H) 3.51 (br.s, 4H) 4.91 (s, 2H) 5.40 (s, 2H) 7.04 (d, J=8Hz, 1H) 7.43 (t, J=8Hz, 1H) 7.60 (t, J=8Hz, 1H) 7.83 (d, J=8Hz, 1H) 8.90 (br.s, 2H)

### Example 98

### 2-[7-(2-Butynyl)-2-methoxy-6-oxo-8-(piperazin-1-yl)-6,7-dihydropurin-1-ylmethyl]benzonitrile hydrochloride

Using methanol instead of dimethylamine and using anhydrous potassium carbonate as a base in Example 96(b), the title compound was synthesized by the same method as used in Example 96.
¹H-NMR(DMSO-d6)
δ 1.79 (s, 3H) 3.28 (br.s, 4H) 3.48-3.56 (m, 4H) 3.91 (s, 3H) 4.97 (s, 2H) 5.32 (s, 2H) 7.19 (d, J=8Hz, 1H) 7.48 (t, J=8Hz, 1H) 7.63 (t, J=8Hz, 1H) 7.87 (d, J=8Hz, 1H) 9.05 (br.s, 2H)

### Example 109

### 7-Benzyl-1-methyl-8-(piperazin-1-yl)-1,7-dihydropurin-6-one trifluoroacetate

### (a) 7-Benzyl-1,7-dihydropurin-6-one

18.23 g of inosine was dissolved in 90 ml of dimethyl sulfoxide, and 16 ml of benzyl bromide was added thereto. The mixture was stirred at room temperature overnight. The reaction solution was poured into 3 L of ethyl acetate. The resulting supernatant was removed and the precipitated oil was dissolved in 10% hydrochloric acid (135 ml). The solution was heated at 70°C with stirring for 4 hours. The solution was cooled to room temperature, and then neutralized to pH 7 using a 5N aqueous sodium hydroxide solution. The precipitated solid was collected by filtration, and dried to give 12.748 g of the title compound.

### (b) t-Butyl 4-(7-benzyl-6-oxo-6,7-dihydro-1H-purin-8-yl)piperazine-1-carboxylate

12.748 g of 7-benzyl-1,7-dihydropurin-6-one was dissolved in 150 ml of N,N-dimethylformamide, and 7.9 g of N-chlorosuccinimide was added thereto. The reaction solution was stirred overnight, and then diluted with ethyl acetate. The solution was washed with water and 1N hydrochloric acid, and dried over anhydrous magnesium sulfate. The solution was filtered, and the filtrate was concentrated to give 6.103 g of 7-benzyl-8-chloro-1,7-dihydropurin-6-one. This compound was combined with 20 g of t-butyl piperazine-1-carboxylate, and the mixture was heated at 150°C. After being stirred for one hour, the reaction mixture was combined with ethyl acetate and water, and partitioned. The organic layer was washed with 1N hydrochloric acid, and dried over anhydrous magnesium sulfate. After filtration, the filtrate was concentrated. The residue was purified by silica gel column chromatography. Thus, 1.539 g of the title compound was obtained from the fraction eluted with ethyl acetate-methanol (10:1).
¹H-NMR(CDCl₃)
δ 1.39 (s, 9H) 3.07-3.10 (m, 4H) 3.35-3.39 (m, 4H) 5.44 (s, 2H) 7.16-7.18 (m, 2H) 7.22-7.32 (m, 3H) 7.91 (s, 1H) 12.18 (s, 1H)

### (c) 7-Benzyl-1-methyl-8-(piperazin-1-yl)-1,7-dihydropurin-6-one trifluoroacetate

15 mg of t-butyl 4-(7-benzyl-6-oxo-6,7-dihydro-1H-purin-8-yl)piperazine-1-carboxylate was dissolved in 1 ml of N,N-dimethylformamide, and 10 mg of sodium hydride and 10 µl of methyl iodide were added thereto. The mixture was stirred at room temperature for 3 days, then ethyl acetate and water were added and the layers separated. The organic layer was concentrated, and the residue was dissolved in trifluoroacetic acid. The solution was concentrated. The residue was purified by reverse-phase high performance liquid chromatography (using an acetonitrile-water mobile phase (containing 0.1% trifluoroacetic acid)) to give 4.31 mg of the title compound.
MS *m*/*e* (ESI) 325(MH⁺-CF₃COOH)

### Example 115

### 3-(2-Butynyl)-5-methyl-2-(piperazin-1-yl)-3,5-dihydroimidazo[4,5-d]pyridazin-4-one trifluoroacetate

### (a) Ethyl 2-bromo-3-(2-butynyl)-5-cyano-3H-imidazole-4-carboxylate

4.56 ml of sulfuric acid was added to 170 ml of ethanol containing 16.80 g of 2-bromo-1H-imidazole-4,5-dicarbonitrile [CAS No. 50847-09-1], and the mixture was heated under reflux for 48 hours. The solution was cooled, and then 500 ml of ethyl acetate and 200 ml of water were added thereto. The organic layer was dried over anhydrous magnesium sulfate, filtered,and concentrated under reduced pressure. The residue was dissolved in N,N-dimethylformamide, and 14.1 g of potassium carbonate and 8.6 ml of 2-butynyl bromide were added thereto. The mixture was stirred at room temperature for 18 hours. 500 ml of ethyl acetate was added to the solution, and the mixture was washed three times with 300 ml of water, and then with 300 ml of a saturated sodium chloride solution. Then, the solution was dried over anhydrous magnesium sulfate, and filtered. The filtrate was concentrated under reduced pressure. The residue was purified by silica gel column chromatography. Thus, 4.09 g of the title compound was obtained from the fraction eluted with hexane-ethyl acetate (9:1).
¹H-NMR(CDCl₃)
δ 1.43 (t, J=7.2Hz, 3H) 1.81 (s, 3H) 4.47 (q, J=7.2Hz, 2H) 5.16 (s, 2H)

### (b) t-Butyl 4-[1-(2-butynyl)-4-cyano-5-ethoxycarboxyl-1H-imidazol-2-yl]piperazine-1-carboxylate

4.09 g of ethyl 2-bromo-3-(2-butynyl)-5-cyano-3H-imidazole-4-carboxylate was combined with 7.70 g of t-butyl piperazine-1-carboxylate, and the mixture was heated to 150°C with stirring for 50 minutes. The reaction mixture was dissolved in toluene. The mixture was purified by silica gel column chromatography. Thus, 4.47 g of the title compound was obtained from the fraction eluted with hexane-ethyl acetate (2:1).
¹H-NMR(CDCl₃)
δ 1.43 (t, J=7.2Hz, 3H) 1.47 (s, 9H) 1.82 (t, J=2.3Hz, 3H) 3.08-3.13 (m, 4H) 3.57-3.61 (m, 4H) 4.44 (q, J=7.2Hz, 2H) 4.89 (q, J=2.3Hz, 2H)

### (c) t-Butyl 4-[1-(2-butynyl)-5-ethoxycarbonyl-4-thiocarbamoyl-1H-imidazol-2-yl]piperazine-1-carboxylate

5 ml of an aqueous solution of 50% ammonium sulfide was added to a 20-ml ethanol solution containing 0.80 g of t-butyl 4-[1-(2-butynyl)-4-cyano-5-ethoxycarbonyl-1H-imidazol-2-yl] piperazine-1-carboxylate, and the mixture was heated at 60°C for 14 hours. 100 ml of ethyl acetate and 50 ml of water were added to the mixture, and the organic layer was washed successively with 50 ml of water and 50 ml of a saturated sodium chloride solution. The reaction solution was dried over anhydrous magnesium sulfate, then filtered. The filtrate was concentrated under reduced pressure. The residue was purified by silica gel column chromatography. Thus, 0.58 g of the title compound was obtained from the fraction eluted with hexane-ethyl acetate (3:2).
¹H-NMR(CDCl₃)
δ 1.43 (t, J=7.2Hz, 3H) 1.48 (s, 9H) 1.82 (t, J=2.3Hz, 3H) 3.12-3.16 (m, 4H) 3.54-3.59 (m, 4H) 4.44 (q, J=7.2Hz, 2H) 4.89 (q, J=2.3Hz, 2H) 7.41 (br.s, 1H) 8.88 (br.s, 1H)

### (d) t-Butyl 4-[1-(2-butynyl)-5-ethoxycarbonyl-4-methylsulfanylcarbonimidoyl-1H-imidazol-2-yl]piperazine -1-carboxylate

0.235 of trimethyl oxonium tetrafluoroborate was added to a 20-ml dichloromethane solution of 0.58 g of t-butyl 4-[1-(2-butynyl)-5-ethoxycarbonyl-4-thiocarbamoyl-1H-imidazol-2-yl]piperazine-1-carboxylate, and the mixture was stirred at room temperature for 18 hours. 50 ml of dichloromethane was added to the solution, and the mixture was washed with 20 ml of a saturated sodium bicarbonate solution. The mixture was dried over anhydrous magnesium sulfate, and concentrated under reduced pressure to give 0.55 g of the title compound.
¹H-NMR(CDCl₃)
δ 1.41 (t, J=7.2Hz, 3H) 1.47 (s, 9H) 1.81 (t, J=2.3Hz, 3H) 2.39 (s, 3H) 3.12-3.16 (m, 4H) 3.56-3.59 (m, 4H) 4.42 (q, J=7.2Hz, 2H) 4.80 (q, J=2.3Hz, 2H)

### (e) t-Butyl 4-[1-(2-butynyl)-5-ethoxycarbonyl-4-methylsulfanylcarbonyl-1H-imidazol-2-yl]piperazine-1-car boxylate

5 ml of a 2N aqueous solution of hydrochloric acid was added to a 30-ml ethanol solution of 0.55 g of t-butyl 4-[1-(2-butynyl)-5-ethoxycarbonyl-4-methyl sulfanylcarbonimidoyl-1H-imidazol-2-yl] piperazine-1-carboxylate, and the mixture was heated at 60°C for 5 hours. After the reaction solution had been concentrated under reduced pressure, 25 ml of ethyl acetate and 1N sodium hydroxide solution were added thereto. The aqueous layer was extracted with 25 ml of ethyl acetate, and the organic layers were combined together. The mixture was washed with 10 ml of a saturated sodium chloride solution containing 1 ml of 1N sodium hydroxide solution, and dried over anhydrous magnesium sulfate. The solution was filtered, and the filtrate was concentrated under reduced pressure. The residue was dissolved in 10 ml of dichloromethane, and 0.10 ml of triethylamine and 0.256 g of di-t-butyl dicarbonate were added thereto. The mixture was stirred at room temperature for 15 hours, and then 25 ml of ethyl acetate was added thereto. The mixture was washed successively with 10 ml of 0.1N hydrochloric acid, 10 ml of a saturated sodium bicarbonate solution, and 10 ml of a saturated sodium chloride solution, and then dried over anhydrous magnesium sulfate. The solution was concentrated under reduced pressure. The residue was purified by silica gel column chromatography. Thus, 0.15 g of the title compound was obtained from the fraction eluted with hexane-ethyl acetate (4:1).
¹H-NMR(CDCl₃)
δ 1.43 (t, J=7.1Hz, 3H) 1.48 (s, 9H) 1.81 (t, J=2.3Hz, 3H) 2.40 (s, 3H) 3.16-3.20 (m, 4H) 3.55-3.59 (m, 4H) 4.35 (q, J=7.1Hz, 2H) 4.80 (q, J=2.3Hz, 2H)

### (f) t-Butyl 4-[1-(2-butynyl)-5-ethoxycarbonyl-4-hydroxymethyl-1H-imidazol-2-yl]piperazine-1-carboxylate

0.187 g of mercury (II) acetate and 0.090 of sodium borohydride were added to 8 ml of an ethanol solution containing 0.265 g of t-butyl 4-[1-(2-butynyl)-5-ethoxycarbonyl-4-methylsulfanyl carbonyl-1H-imidazol-2-yl]piperazine-1-carboxylate at 0°C, and the mixture was stirred at room temperature for 4 hours. After 0.187 g of mercury (II) acetate and 0.090 of sodium borohydride had been added to the solution, the mixture was stirred at room temperature for 15 hours. 100 ml of ethyl acetate and 50 ml of 0.5N hydrochloric acid were added to the solution, and the organic layer was washed successively with 50 ml of water and 50 ml of a saturated sodium chloride solution. The mixture was dried over anhydrous magnesium sulfate, and concentrated under reduced pressure. The residue was purified by silica gel column chromatography. 0.172 g of the starting material was collected from the fraction eluted with hexane-ethyl acetate (4:1). Then, 0.061 g of the title compound was obtained from the fraction eluted with hexane-ethyl acetate (1:4).
¹H-NMR(CDCl₃)
δ 1.42 (t, J=7.1Hz, 3H) 1.48 (s, 9H) 1.81 (t, J=2.3Hz, 3H) 3.17-3.21 (m, 4H) 3.41 (t, J=4.8Hz, 1H) 3.56-3.60 (m, 4H) 4.36 (q, J=7.1Hz, 2H) 4.75 (d, J=4.8Hz, 2H) 4.81 (q, J=2.3Hz, 2H)

### (g) t-Butyl

### 4-[1-(2-butynyl)-5-ethoxycarbonyl-4-formyl-1H-imidazol-2-yl]piperazine-1-carboxylate

0.120 g of manganese dioxide was added to a 2-ml dichloromethane solution of 0.061 g of t-butyl 4-[1-(2-butynyl)-5-ethoxycarbonyl-4-hydroxymethyl-1H-imidazol-2-yl]piperazine-1-carboxylate, and the mixture was stirred at room temperature for 15 hours. The reaction solution was filtered through celite, and the filtrate was concentrated under reduced pressure. The residue was purified by silica gel column chromatography. Thus, 0.055 g of the title compound was obtained from the fraction eluted with hexane-ethyl acetate (7:3).
¹H-NMR(CDCl₃)
δ 1.42 (t, J=7.1Hz, 3H) 1.48 (s, 9H) 1.82 (t, J=2.3Hz, 3H) 3.23-3.26 (m, 4H) 3.55-3.59 (m, 4H) 4.45 (q, J=7.1Hz, 2H) 4.89 (q, J=2.3Hz, 2H) 10.36 (s, 1H)

### (h) t-Butyl

### 4-[1-(2-butynyl)-6-methyl-7-oxo-6,7-dihydro-1H-imidazo[4,5-d]pyridazin-2-yl]piperazine-1-car boxylate

0.05 ml of methylhydrazine was added to a 2.5-ml ethanol solution of 0.055 g of t-butyl 4-[1-(2-butynyl)-5-ethoxycarbonyl-4-formyl-1H-imidazol-2-yl] piperazine-1-carboxylate. The mixture was stirred at 80°C for 15 hours, and then heated at 130°C for 14 hours. The reaction solution was concentrated under reduced pressure. Then, the residue was purified by silica gel column chromatography. Thus, 0.035 g of the title compound was obtained from the fraction eluted with hexane-ethyl acetate (1:1).
¹H-NMR(CDCl₃)
δ 1.52 (s, 9H) 1.83 (t, J=2.3Hz, 3H) 3.38-3.42 (m, 4H) 3.61-3.64 (m, 4H) 3.85 (s, 3H) 5.09 (q, J=2.3Hz, 2H) 8.13 (s, 1H)
MS *m*/*e* (ESI) 387.4(MH⁺)

### (i) 3-(2-Butynyl)-5-methyl-2-(piperazin-1-yl)-3,5-dihydroimidazo[4,5-d]pyridazin-4-one trifluoroacetate

0.4 ml of trifluoroacetic acid was added to a 0.4-ml dichloromethane solution of 0.0351 g of t-butyl 4-[1-(2-butynyl)-6-methyl-7-oxo-6,7-dihydro-1H-imidazo[4,5-d]pyridazin-2-yl]piperazine-1-car boxylate, and the mixture was stirred at room temperature for one hour. The solvent was concentrated. The residue was purified by reverse-phase high performance liquid chromatography (using an acetonitrile-water mobile phase (containing 0.1% trifluoroacetic acid)) to give 0.0295 g of the title compound.
¹H-NMR(CD₃OD)
δ 1.83 (t, J=2.3Hz, 3H) 3.45-3.49 (m, 4H) 3.65-3.69 (m, 4H) 3.83 (s, 3H) 5.15 (q, J=2.3Hz, 2H) 8.20 (s, 1H)
MS *m*/*e* (ESI) 287.09(MH⁺-CF₃COOH)

### Example 116

### 5-Benzyloxymethyl-3-(2-butynyl)-2-(piperazin-1-yl)-3,5-dihydro-imidazo[4,5-d]pyridazin-4-one trifluoroacetate

### (a) 5-Benzyloxymethyl-4-oxo-4,5-dihydroimidazo[4,5-d]pyridazine-1-sulfonic acid dimethylamide

2.08 g of triethylamine, 2.80 g of N,N-dimethyl sulfamoyl chloride, and 0.22 g of 4-dimethylaminopyridine were added to 50 ml of a dichloromethane solution of 3.04 g of 5-benzyloxy methylimmidazo[4,5-d]pyridazin-4-one [CAS NO. 82137-50-6] (R. Paul Gagnier, Michael J. Halat, and Brian A. Otter Journal of Heterocyclic Chemistry, 21, p481, 1984), and the mixture was heated under reflux for 4 hours. 250 ml of ethyl acetate was added to the solution, and the mixture was washed successively with 50 ml of an aqueous solution of 1N hydrochloric acid, 50 ml of a saturated sodium bicarbonate solution, and 50 ml of a saturated sodium chloride solution. The mixture was dried over anhydrous magnesium sulfate, and concentrated under reduced pressure. The residue was purified by silica gel column chromatography. Thus, 2.86 g of the title compound was obtained from the fraction eluted with hexane-ethyl acetate (2:3).
¹H-NMR(CDCl₃)
δ 2.98 (s, 6H) 4.77 (s, 2H) 5.74 (s, 2H) 7.30-7.39 (m, 5H) 8.21 (s, 1H) 8.46 (s, 1H)

### (b) 5-Benzyloxymethyl-2-chloro-4-oxo-4,5-dihydroimidazo[4,5-d]pyridazine-1-sulfonic acid dimethylamide

5.3 ml of n-butyl lithium (2.0 M cyclohexane solution) was added to a 150-ml tetrahydrofuran solution of 3.34 g of 5-benzyloxymethyl-4-oxo-4,5-dihydroimidazo[4,5-d]pyridazine-1-sulfonic acid dimethylamide under a nitrogen atmosphere at -78°C, and the mixture was stirred at -78°C for one hour. Then, 20 ml of a tetrahydrofuran solution of 3.26 g of hexachloroethane was added to this solution. The mixture was allowed to warm to room temperature. 25 ml of a 5% aqueous solution of ammonium chloride was added to the solution, and the mixture was extracted with 50 ml of ethyl acetate. The organic layer was washed successively with 25 ml of water and 25 ml of a saturated sodium chloride solution, and then dried over anhydrous magnesium sulfate. The organic liquid was concentrated under reduced pressure. The residue was purified by silica gel column chromatography. Thus, 2.31 g of the title compound was obtained from the fraction eluted with hexane-ethyl acetate (2:3).
¹H-NMR(CDCl₃)
δ 3.12 (s, 6H) 4.77 (s, 2H) 5.70 (s, 2H) 7.30-7.39 (m, 5H) 8.48 (s, 1H)

### (c) t-Butyl 4-(6-benzyloxymethyl-7-oxo-6,7-dihydro-1H-imidazo[4,5-d]pyridazin-2-yl)piperazine-1-carbox ylate

A mixture consisting of 2.31 g of 5-benzyloxymethyl-2-chloro -4-oxo-4,5-dihydroimidazo[4,5-d]pyridazine-1-sulfonic acid dimethylamide and 4.49 g of t-butyl piperazine-1-carboxylate was heated at 150°C under nitrogen atmosphere for 2.5 hours. The residue was purified by silica gel column chromatography. Thus, 1.94 g of the title compound was obtained from the fraction eluted with ethyl acetate.
¹H-NMR(CDCl₃)
δ 3.54-3.58 (m, 4H) 3.71-3.75 (m, 4H) 4.68 (s, 2H) 5.65 (s, 2H) 7.25-7.35 (m, 5H) 8.21 (s, 1H) 12.58 (br.s, 1H)

### (d) t-Butyl 4-[6-benzyloxymethyl-1-(2-butynyl)-7-oxo-6,7-dihydro-1H-imidazo[4,5-d] pyridazin-2-yl]piperazine-1-carboxylate

0.74 g of potassium carbonate and 0.078 g of 2-butynyl bromide were added to a 20-ml N,N-dimethylformamide solution of 0.216 g of t-butyl 4-(6-benzyloxymethyl-7-oxo-6,7-dihydro-1H-imidazo [4,5-d]pyridazin-2-yl)piperazine-1-carboxylate, and the mixture was stirred at room temperature for 16 hours. Then, 50 ml of ethyl acetate was added to the solution. The organic layer was washed three times with 20 ml of water, and then with 10 ml of a saturated sodium chloride solution. The solution was dried over anhydrous magnesium sulfate, and then concentrated under reduced pressure. The residue was purified by silica gel column chromatography. Thus, 0.139 g of the title compound was obtained from the fraction eluted with hexane-ethyl acetate (3:2).
¹H-NMR(CDCl₃)
δ 1.50 (s, 9H) 1.86 (t, J=2.3Hz, 3H) 3.38-3.44 (m, 4H) 3.61-3.66 (m, 4H) 4.72 (s, 2H) 5.10 (q, J=2.3Hz, 2H) 5.65 (s, 2H) 7.25-7.38 (m, 5H) 8.18 (s, 1H)

### (e) 5-Benzyloxymethyl-3-(2-butynyl)-2-(piperazin-1-yl)-3,5-dihydroimidazo[4,5-d]pyridazin-4-one trifluoroacetate

0.0043 g of the title compound was obtained by treating 0.0073 g of t-butyl 4-[6-benzyloxymethyl-1-(2-butynyl)-7-oxo-6,7-dihydro-1H-imidazo[4,5-d]pyridazin-2-yl]pipera zine-1-carboxylate and purifying the product by the same method as used in Example 115(i).
¹H-NMR(CD₃OD)
δ 1.83 (t, J=2.3Hz, 2H) 3.45-3.49 (m, 4H) 3.65-3.69 (m, 4H) 4.69 (s, 2H) 5.15 (q, J=2.3Hz, 2H) 5.64 (s, 2H) 7.17-7.32 (m, 5H) 8.20 (s, 1H)
MS *m*/*e* (ESI) 393.28(MH⁺-CF₃COOH)

### Example 117

### 3-(2-Butynyl)-2-(piperazin-1-yl)-3,5-dihydroimidazo[4,5-d]pyridazin-4-one trifluoroacetate

8 ml of a dichloromethane solution of 0.123 g of t-butyl 4-[6-benzyloxymethyl-1-(2-butynyl)-7-oxo-6,7-dihydro-1H-imidazo[4,5-d]pyridazin-2-yl]pipera zine-1-carboxylate was cooled to -78°C under a nitrogen atmosphere, and 1.9 ml of boron trichloride (1.0 M dichloromethane solution) was added thereto. The mixture was stirred at -78°C for five hours, and 10 ml of a 1:1 mixed solvent of dichloromethane-methanol was added thereto. The mixture was stirred at -78°C for two hours, and then allowed to warm to room temperature. The solvent was concentrated under reduced pressure, and 10 ml of methanol was added thereto. Then, the solution was again concentrated under reduced pressure. The residue was dissolved in 3 ml of pyridine, and the mixture was heated under reflux for two hours. 0.3 ml of this solution was concentrated under reduced pressure. The residue was purified by reverse-phase high performance liquid chromatography (using an acetonitrile-water mobile phase (containing 0.1% trifluoroacetic acid)) to give 0.005 g of the title compound.
¹H-NMR(CD₃OD)
δ 1.83 (t, J=2.3Hz, 3H) 3.45-3.49 (m, 4H) 3.65-3.69 (m, 4H) 5.16 (q, J=2.3Hz, 2H) 8.21 (s, 1H)
MS *mle* (ESI) 273.16 (MH⁺-CF₃COOH)

### Example 118

### 2-[7-(2-Butynyl)-1-methyl-6-oxo-8-(piperazin-1-yl)-6,7-dihydro-1H-purin-2-yloxy]benzamide hydrochloride

### (a) t-Butyl 4-[7-(2-butynyl)-2-(2-carbamoylphenoxy)-1-methyl-6-oxo-6,7-dihydro-1H-purin-8-yl]piperazine -1-carboxylate

200 mg of t-butyl 4-[7-(2-butynyl)-2-chloro-1-methyl-6-oxo-6,7-dihydro-1H-purin-8-yl]piperazine-1-carboxylate was dissolved in 2.0 ml of 1-methyl-2-pyrrolidone, and 85 mg of salicylamide and 129 mg of potassium carbonate were added thereto. The mixture was stirred at 100°C for 2 hours. After the reaction mixture had been cooled to room temperature, 5.0 ml of water was added thereto. After the mixture had been stirred at room temperature for 1 hour, the white precipitate was collected by filtration. The resulting white solid was washed with water and ether to give of 221 mg of the title compound (89%).
¹H-NMR(DMSO-d6)
δ 1.43 (s, 9H) 1.79 (t, J=2.5Hz, 3H) 3.23-3.27 (m, 4H) 3.36 (s, 3H) 3.48-3.52 (m, 4H) 4.95 (q, 2.5Hz, 2H) 6.59 (td, J=8.0, 1.0Hz, 1H) 6.63 (dd, J=8.0, 1.0Hz, 1H) 7.14 (ddd, J=8.0, 7.5, 2.0Hz, 1H) 7.80 (dd, J=7.5, 2.0Hz, 1H)
MS *m*/*e* (ESI) 522(MH⁺)

### (b) 2-[7-(2-Butynyl)-1-methyl-6-oxo-8-(piperazin-1-yl)-6,7-dihydro-1H-purin-2-yloxy]benzamide hydrochloride

210 mg of t-butyl 4-[7-(2-butynyl)-2-(2-carbamoylphenoxy)-1-methyl-6-oxo-6,7-dihydro-1H-purin-8-yl]piperazine -1-carboxylate was combined with 3.5 ml of methanol and 2.1 ml of 4N hydrochloric acid-ethyl acetate solution. After the mixture had been stirred at room temperature for 4 hours, the reaction solution was concentrated by flushing with nitrogen gas. The resulting residue was washed with ethanol and ethyl acetate to give 177 mg of the title compound (96%).
¹H-NMR(DMSO-d6)
δ 1.82 (t, J=2.3Hz, 3H) 3.28-3.32 (m, 4H) 3.48 (s, 3H) 3.54-3.58 (m, 4H) 5.04 (q, 2.3Hz, 2H) 6.96 (br.t, J=7.0Hz, 1H) 6.99 (br.d, J=8.0Hz, 1H) 7.46 (ddd, J=8.0, 7.0, 1.5Hz, 1H) 7.93 (br.d, J=8.0Hz, 1H)
MS *m*/*e* (ESI) 422(MH⁺-HCl)

### Example 119

### 3-(2-Butynyl)-5-methyl-2-(piperazin-1-yl)-3,5-dihydroimidazo[4,5-d]pyridazin-4-one

### (a) 5-Methyl-1-trityl-1,5-dihydroimidazo[4,5-d]pyridazin-4-one

78.8 g of 5-methyl-1,5-dihydroimidazo [4,5-d] pyridazin-4-one [CAS No. 76756-58-6] (Shih-Fong Chen and Raymond P. Panzica, Journal of Organic Chemistry 46, p2467, 1981) was suspended in 2.5 L of dichloromethane at room temperature, and 78.8 of triethylamine was added thereto. 176 g of trityl chloride was added to the mixture, which was then stirred for three hours. 7.5 L of ethyl acetate was added to the mixture. After being washed successively with 3 L of water and 3 L of a saturated sodium chloride solution, the mixture was dried over anhydrous magnesium sulfate and concentrated under reduced pressure. The residue was purified by silica gel column chromatography. Thus, 136.5 g of the title compound was obtained from the fraction eluted with hexane-ethyl acetate (20:80 to 0:100).
¹H-NMR(CDCl₃)
δ 3.79 (s, 3H) 6.92 (s, 1H) 7.07-7.13 (m, 6H) 7.32-7.40 (m, 9H) 7.87 (s, 1H)

### (b) 2-Chloro-5-methyl-1-trityl-1,5-dihydroimidazo[4,5-d]pyridazin-4-one

220 ml of lithium hexamethyldisilazide (1.0 M tetrahydrofuran solution) was added to a 4-L tetrahydrofuran solution of 68.3 g of 5-methyl-1-trityl-1,5-dihydroimidazo[4,5-d]pyridazin-4-one at -75°C under a nitrogen atmosphere, and the mixture was stirred at -75°C for 1 hour. Then, 200 ml of a tetrahydrofuran solution of 82.3 g of hexachloroethane was added to the solution. The mixture was allowed to warm to -20°C. 5 L of 5% aqueous ammonium chloride was added, and the mixture was extracted with 4 L of ethyl acetate. The organic layer was washed successively with 5 L of water and 5 L of a saturated sodium chloride solution. The solution was dried over anhydrous magnesium sulfate, and concentrated under reduced pressure. The residue was suspended in 150 ml of *t*-butyl methyl ether, and then collected by filtration. The solid was washed twice with 100 ml of *t*-butyl methyl ether to give 69.7 g of the title compound.
¹H-NMR(CDCl₃)
δ 3.78 (s, 3H) 5.81 (s, 1H) 7.25-7.27 (m, 6H) 7.28-7.38 (m, 9H)

### (c) t-Butyl 4-(6-methyl-7-oxo-6,7-dihydro-1H-imidazo[4,5-d]pyridazin-2-yl)piperazine-1-carboxylate

69.7 g of 2-chloro-5-methyl-1-trityl-1,5-dihydroimidazo [4,5-d] pyridazin-4-one was combined with 153.4 g of t-butyl piperazine-1-carboxylate, and the mixture was stirred and heated to 100°C under nitrogen atmosphere. When the reaction mixture became easily stirrable, the temperature was raised to 150°C. The mixture was kept at this temperature for one hour. The reaction solution allowed to cool and then suspended in 250 ml of *t*-butyl methyl ether. The suspended material was collected by filtration. The solid was washed twice with 200 ml of *t*-butyl methyl ether and three times with 200 ml of water. The solid was again washed twice with 200 ml of *t*-butyl methyl ether, and dried to give 50.3 g of the title compound.
¹H-NMR(CDCl₃)
δ 1.50 (s, 9H) 3.56-3.62 (m, 4H) 3.73-3.80 (m, 4H) 3.87 (s, 3H) 8.16 (s, 1H) 12.65 (br.s, 1H)

### (d) t-Butyl 4-[1-(2-butynyl)-6-methyl-7-oxo-6,7-dihydro-1H-imidazo[4,5-d]pyridazin-2-yl]piperazine-1-car boxylate

43.9 g of potassium carbonate and 27.8 ml of 2-butynyl bromide were successively added to a 5.5-L N,N-dimethylformamide solution of 88.4 g of t-butyl 4-(6-methyl-7-oxo-6,7-dihydro-1H-imidazo[4,5-d] pyridazin-2-yl)piperazine-1-carboxylate at 15°C under a nitrogen atmosphere. The reaction solution was stirred at room temperature for 22 hours, and then poured into 10 L of water. The mixture was extracted with 5 L of ethyl acetate. The organic layer was successively washed twice with 5 L of water, and with 5 L of a saturated sodium chloride solution. The aqueous layer was extracted twice with 3 L of ethyl acetate. The organic layers were combined together, and then dried over anhydrous magnesium sulfate. The organic layer was concentrated under reduced pressure. The residue was purified by silica gel column chromatography. Thus, 54.3 g of the title compound was obtained from the fraction eluted with hexane-ethyl acetate (3:2 to 3:7).
¹H-NMR(CDCl₃)
δ 1.52 (s, 9H) 1.83 (t, J=2.3Hz, 3H) 3.38-3.42 (m, 4H) 3.61-3.64 (m, 4H) 3.85 (s, 3H) 5.09 (q, J=2.3Hz, 2H) 8.13 (s, 1H)

### (e) 3-(2-Butynyl)-5-methyl-2-(piperazin-1-yl)-3,5-dihydroimidazo[4,5-d]pyridazin-4-one

200 ml of trifluoroacetic acid was added to 200 ml of a dichloromethane solution containing 54.3 g of t-butyl 4-[1-(2-butynyl)-6-methyl-7-oxo-6,7-dihydro-1H-imidazo[4,5-d]pyridazin-2-yl]piperazine-1-car boxylate, and the mixture was stirred at room temperature for 1 hour. The mixture was concentrated under reduced pressure, the residue was dissolved in 500 ml of ethyl acetate. 1 L of 10% aqueous sodium bicarbonate solution was gradually added. Then, 1 L of ethyl acetate and 500 ml of a 5N aqueous sodium hydroxide solution were added to the solution. The organic layer was separated. Then, the aqueous layer was extracted five times with 1 L of dichloromethane. The organic layers were combined together, washed with 500 ml of an aqueous solution of 2N sodium hydroxide, dried over anhydrous magnesium sulfate, and concentrated under reduced pressure. The residue was recrystallized from ethyl acetate to give 30.5g of the crystalline title compound.
¹H-NMR(CDCl₃)
δ 1.84 (t, J=2.3Hz, 3H) 3.05-3.09 (m, 4H) 3.38-3.44 (m, 4H) 3.85 (s, 3H) 5.06 (q, J=2.3Hz, 2H) 8.13 (s, 3H)

### Example 119-2

### 3-(2-Butynyl)-5-methyl-2-(piperazin-1-yl)-3,5-dihydroimidazo[4,5-d]pyridazin-4-one toluene-4-sulfonate

98.7 mg of 3-(2-butynyl)-5-methyl-2-(piperazin-1-yl)-3,5-dihydroimidazo[4,5-d]pyridazin-4-one was dissolved in 1 ml of ethanol, and then 1 ml of an ethanol solution of 101 mg of p-toluenesulfonic acid monohydrate was added thereto while the solution was being stirred. The mixture was cooled with ice for two hours while being stirred. The precipitate was collected by filtration, and then dried under reduced pressure at 50°C for one hour to give 153.2 mg pf the title compound.
¹H-NMR (DMSO-d6)
δ 1.79 (t, *J* = 2 Hz, 3H) 2.27 (s, 3H) 3.25-3.35 (m, 4H) 3.50-3.54(m, 4H) 3.70 (s, 3H) 5.13 (d, *J* = 2 Hz, 2H) 7.10 (d, *J* = 8 Hz, 2H) 7.47 (d, *J* = 8 Hz, 2H) 8.25 (s, 1H) 8.79 (br.s, 2H)

Furthermore, 107.95 mg of the title compound was recrystallized from acetone, yielding 84.9 mg of crystalline product.

### Example 120

### 2-(3-Aminopiperidin-1-yl)-3-(2-butynyl)-5-methyl-3,5-dihydroimidazo[4,5-d]pyridazin-4-one trifluoroacetate

### (a) 9H-fluoren-9-ylmethyl 3-t-butoxycarbonylaminopiperidine-1-carboxylate

1.84 g of diisopropylethylamine and 4.71 g of diphenylphosphorylazide were added to 10 ml of a t-butanol solution of 5.01 g of 9H-fluoren-9-ylmethyl 3-carboxypiperidine-1-carboxylate, and the mixture was heated at 60°C under a nitrogen atmosphere for 18 hours. The reaction solution was cooled, and 150 ml of ethyl acetate was added thereto. The organic layer was washed successively with 100 ml of 5% aqueous sulfuric acid, 100 ml of 5% aqueous sodium bicarbonate solution, 100 ml of water, and 100 ml of a saturated sodium chloride solution, and then dried over anhydrous magnesium sulfate. The organic layer was concentrated under reduced pressure. The residue was purified by silica gel column chromatography. Thus, 1.88 g of the title compound was obtained from the fraction eluted with hexane-ethyl acetate (4:1).
¹H-NMR(CDCl₃)
δ 1.45 (s, 9H) 1.45-1.72 (m, 3H) 1.82-1.87 (br.s, 1H) 3.09-3.30 (br.s, 2H) 3.58 (br.s, 2H) 3.82-3.98 (br.s, 1H) 4.24 (t, J=7.2 Hz, 1H) 4.27-4.48 (br.s, 2H) 4.52-4.59 (br.s, 1H) 7.32 (dd, J=10.3, 10.0 Hz, 2H) 7.39 (t, J=10.0 Hz, 2H) 7.59 (d, J=10.0 Hz, 2H) 7.75 (d, J=10.3 Hz, 2H)

### (b) t-Butyl piperidin-3-ylcarbamate

25 ml of diethylamine was added to 250 ml of an ethanol solution of 1.88 g of 9H-fluoren-9-ylmethyl 3-t-butoxycarbonylaminopiperidine-1-carboxylate, and the mixture was stirred at room temperature for 18 hours. After the solution had been concentrated under reduced pressure, the residue was dissolved in a mixture consisting of 150 ml of toluene and 100 ml of 10% aqueous citric acid solution. The aqueous layer was made alkaline with a 5N aqueous sodium hydroxide solution, and then extracted twice with 100 ml of dichloromethane. The organic layers were combined together, dried over anhydrous magnesium sulfate, and concentrated under reduced pressure to give 0.79 g of the title compound.
¹H-NMR(CDCl₃)
δ 1.45 (s, 9H) 1.41-1.53 (m, 2H) 1.65-1.72 (m, 1H) 1.79-1.86 (m, 1H) 2.48-2.56 (m, 1H) 2.64-2.70 (m, 1H) 2.78-2.86 (m, 1H) 3.06 (dd, J=12.0,4.0 Hz, 1H) 3.48-3.62 (br.s, 1H) 4.71-4.88 (br.s, 1H)

### (c) 2-(3-Aminopiperidin-1-yl)-3-(2-butynyl)-5-methyl-3,5-dihydroimidazo[4,5-d]pyridazin-4-one trifluoroacetate

0.020 g of 2-chloro-5-methyl-1-trityl-1,5-dihydroimidazo [4,5-d]pyridazine-4-one and 0.040 g of t-butyl piperidin-3-ylcarbamate were combined together, and the mixture was heated under a nitrogen atmosphere at 150°C for 1 hour. The reaction mixture was purified by silica gel column chromatography. Thus, 0.016 g of t-butyl [1-(6-methyl-7-oxo-6,7-dihydro-1H-imidazo[4,5-d]pyridazin-2-yl)piperidin-3-yl]carbamate was obtained from the fraction eluted with ethyl acetate. 0.0080 g of this compound was dissolved in 0.6 ml ofN,N-dimethylformamide, and then 0.0038 g of potassium carbonate and 0.003 ml of 2-butynyl bromide were added thereto. The mixture was stirred at room temperature for 18 hours. The reaction mixture was partitioned between 1 ml of ethyl acetate and 1 ml of water, and the organic layer was concentrated. The residue was dissolved in 0.5 ml of dichloromethane, and then 0.5 ml of trifluoroacetic acid was added thereto. After 1 hour, the reaction solution was concentrated. The residue was purified by reverse-phase high performance liquid chromatography (using an acetonitrile-water mobile phase (containing 0.1% trifluoroacetic acid)) to give 0.0046 g of the title compound.
¹H-NMR(CDCl₃)
δ 1.74-1.80 (br.s, 1H) 1.82 (br.s, 3H) 1.96-2.19 (br.m, 3H) 3.43-3.79 (br.m, 5H) 3.86 (s, 3H) 5.05 (br.d, J=16.0 Hz, 1H) 5.23 (br.d, J=16.0 Hz, 1H) 8.15 (s, 1H)

### Example 122

### 2-[7-(2-Butynyl)-1-methyl-6-oxo-8-(piperazin-1-yl)-6,7-dihydro-1H-purin-2-yloxy]benzamide

53.0 g of t-butyl 4-[7-(2-butynyl)-2-(2-carbamoylphenoxy)-1-methyl-6-oxo-6,7-dihydro-1H-purin-8-yl]piperazine -1-carboxylate was dissolved in 160 ml of trifluoroacetic acid, and the mixture was stirred at room temperature for one hour. 1250 ml of a 2 M aqueous sodium hydroxide solution was added drop wise to the reaction solution, and the mixture was stirred at room temperature for one hour and 50 minutes. The resulting white precipitate was collected by filtration. The white solid was washed with water and then with ethanol, and dried at 60°C overnight to give 42.8 g of the title compound.
¹H-NMR(DMSO-d6)
δ 1.78 (t, J=2.4 Hz, 3H) 2.82-2.86 (m, 4H) 3.18-3.22 (m, 4H) 3.36 (s, 3H) 4.91 (q, 2.4 Hz, 2H) 6.58 (td, J=8.4, 1.2 Hz, 1H) 6.63 (dd, J=8.0, 0.8 Hz, 1H) 7.14 (ddd, J=8.0, 7.2, 2.0 Hz, 1H) 7.80 (dd, J=7.6, 2.0 Hz, 1H)
MS *m*/*e* (ESI) 422(MH⁺)

### Example 126

### 3-[7-(2-Butynyl)-1-methyl-6-oxo-8-(piperazin-1-yl)-6,7-dihydro-1H-purin-2-ylsulfanyl]propioni c acid trifluoroacetate

7 mg of t-butyl 4-[7-(2-butynyl)-2-chloro-1-methyl-6-oxo-6,7-dihydro-1H-purin-8-yl]piperazine-1-carboxylate was dissolved in 0.15 ml of 1-methyl-2-pyrrolidone, and then 20 µl of 3-mercaptopropionic acid and 6 mg of potassium carbonate were added thereto. The mixture was stirred at room temperature for five hours. A saturated ammonium chloride solution was added to the reaction solution, and the mixture was extracted with ethyl acetate. The organic layer was concentrated, and the residue was dissolved in 0.40 ml of trifluoroacetic acid. The solution was concentrated by flushing with nitrogen gas. The residue was purified by reverse-phase high performance liquid chromatography (using an acetonitrile-water mobile phase (containing 0.1% trifluoroacetic acid)) to give 4.60 mg of the title compound.
MS *m*/*e* (ESI) 391(MH⁺-CF₃COOH)

### Example 129

### 7-(2-Butynyl)-1-methyl-8-(piperazin-1-yl)-2-propylsulfanyl-1,7-dihydropurin-6-one trifluoroacetate

4.61 mg of the title compound was obtained by using propane-1-thiol, instead of 3-mercaptopropionic acid, by the same method as used in Example 126.
MS *m*/*e* (ESI) 361(MH⁺-CF₃COOH)

### Example 142

### 7-(2-Butynyl)-1-methyl-8-(piperazin-1-yl)-2-(thiazol-2-ylsulfanyl)-1,7-dihydropurin-6-one trifluoroacetate

3.86 mg of the title compound was obtained by using thiazole-2-thiol, instead of 3-mercaptopropionic acid, by the same method as used in Example 126.
MS *m*/*e* (ESI) 402(MH⁺-CF₃COOH)

### Example 146

### 7-(2-Butynyl)-1-methyl-8-(piperazin-1-yl)-2-[1-(thiophen-2-yl)ethylsulfanyl]-1,7-dihydropurin-6 -one trifluoroacetate

0.51 mg of the title compound was obtained by using 1-(thiophen-2-yl)ethanethiol, instead of 3-mercaptopropionic acid, by the same method as used in Example 126.
MS *m*/*e* (ESI) 429(MH⁺-CF₃COOH)

### Example 147

### 7-(2-Butynyl)-1-methyl-2-(1-methyl-1H-imidazol-2-ylsulfanyl)-8-(biperazin-1-yl)-1,7-dihydrop urin-6-one trifluoroacetate

5 mg of t-butyl 4-[7-(2-butynyl)-2-chloro-1-methyl-6-oxo-6,7-dihydro-1H-purin-8-yl]piperazine-1-carboxylate was dissolved in 0.15 ml of 1-methyl-2-pyrrolidone, and then 10 mg of 1-methyl-1H-imidazole-2-thiol and 8 mg of potassium carbonate were added thereto. The mixture was stirred at room temperature for five hours. A saturated ammonium chloride solution was added to the reaction solution, and the mixture was extracted with ethyl acetate. The organic layer was concentrated, and the residue was dissolved in 0.40 ml of trifluoroacetic acid. The solution was concentrated by flushing with nitrogen gas. The residue was purified by reverse-phase high performance liquid chromatography (using an acetonitrile-water mobile phase (containing 0.1% trifluoroacetic acid)) to give 3.75 mg of the title compound.
MS *m*/*e* (ESI) 399(MH⁺-CF₃COOH)

### Example 159

### 7-(2-Butynyl)-1-methyl-2-(4-methylthiazol-2-ylsulfanyl)-8-(piperazin-1-yl)-1,7-dihydropurin-6-one trifluoroacetate

4.01 mg of the title compound was obtained by using 4-methylthiazol-2-thiol, instead of 1-methyl-1H-imidazole-2-thiol, by the same method as used in Example 147.
MS *m*/*e* (ESI) 416(MH⁺-CF₃COOH)

### Example 229

### 7-(2-Butynyl)-1-(2-cyanobenzyl)-6-oxo-8-(piperazin-1-yl)-6,7-dihydro-1H-purine-2-carbonitrile hydrochloride

### (a) t-Butyl 4-[7-(2-butynyl)-2-cyano-1-(2-cyanobenzyl)-6-oxo-6,7-dihydro-1H-purin-8-yl]piperazine-1-carb oxylate

A mixture consisting of 8 mg of t-butyl 4-[7-(2-butynyl)-2-chloro-1-(2-cyanobenzyl)-6-oxo-6,7-dihydro-1H-purin-8-yl]pipereine-1-car boxylate obtained in Example 96(a), 10 mg of sodium cyanide and 0.3 ml of N,N-dimethylformamide was stirred at room temperature for 4 hours. The reaction mixture was extracted with ethyl acetate-water, and the organic layer was washed with water and then with saturated brine. The organic layer was concentrated. The residue was purified by thin layer chromatography (50% ethyl acetate/hexane) to give 6.1 mg of the title compound.
¹H-NMR(CDCl₃)
δ 1.50 (s, 9H) 1.83 (s, 3H) 3.50 (s, 4H) 3.58-3.64 (m, 4H) 4.99 (s, 2H) 5.74 (s, 2H) 7.02 (d, J=8 Hz, 1H) 7.44 (t, J=8 Hz, 1H) 7.55 (t, J=8 Hz, 1H) 7.74 (d, J=8 Hz, 1H)

### (b) 7-(2-Butynyl)-1-(2-cyanobenzyl)-6-oxo-8-(piperazin-1-yl)-6,7-dihydro-1H-purine-2-carbonitrile hydrochloride

A mixture consisting of 6.1 mg of t-butyl 4-[7-(2-butynyl)-2-cyano-1-(2-cyanobenzyl)-6-oxo-6,7-dihydro-1H-purin-8-yl]piperazine-1-carb oxylate and 0.2 ml of trifluoroacetic acid was stirred at room temperature for 20 minutes. The reaction solution was concentrated, and the residue was purified by reverse-phase column chromatography using a 20% to 60% methanol/water (0.1% concentrated hydrochloric acid) solvent to give 5.0 mg of the title compound.
¹H-NMR(DMSO-d6)
δ 1.80 (s, 3H) 3.30 (s, 4H) 3.60-3.70 (m, 4H) 5.09 (s, 2H) 5.60 (s, 2H) 7.27 (d, J=8 Hz, 1H) 7.54 (t, J=8 Hz, 1H) 7.68 (t, J=8 Hz, 1H) 7.94 (d, J=8 Hz, 1H) 9.36 (br.s, 2H)

### Example 230

### 3-[7-(2-Butynyl)-1-(2-cyanobenzyl)-6-oxo-8-(piperazin-1-yl)-6,7-dihydro-1H-purin-2-yloxy]pyr idine-2-carboxylic amide trifluoroacetate

7 mg of t-butyl 4-[7-(2-butynyl)-2-chloro-1-(2-cyanobenzyl)-6-oxo-6,7 -dihydro-1H-purin-8-yl]piperazine-1-carboxylate was dissolved in 0.2 ml of 1-methyl-2-pyrrolidone, and then 8 mg of 3-hydroxypyridine-2-carboxylic amide and 8 mg of potassium carbonate were added thereto. The mixture was stirred at 100°C for 2 hours. 1N hydrochloric acid was added to the reaction mixture, and the mixture was extracted with ethyl acetate. The organic layer was concentrated, and the residue was dissolved in trifluoroacetic acid. The solution was concentrated, and the residue was purified by reverse-phase high performance liquid chromatography (using an acetonitrile-water mobile phase (containing 0.1% trifluoroacetic acid)) to give 2.93 mg of the title compound.
MS *m*/*e* (ESI) 524(MH⁺-CF₃COOH)

### Example 234

### 2-[7-(2-Butynyl)-1-(2-cyanobenzyl)-6-oxo-8-(piperazin-1-yl)-6,7-dihydro-1H-purin-2-yloxy]ben zamide trifluoroacetate

3.74 mg of the title compound was obtained by using salicylamide, instead of 3-hydroxypyridine-2-carboxylic amide, according to the method described in Example 230.
MS *m*/*e* (ESI) 523(MH⁺-CF₃COOH)

### Example 235

### 2-[7-(2-Butynyl)-1-(4-cyanobenzyl)-6-oxo-8-(piperazin-1-yl)-6,7-dihydro-1H-purin-2-yloxy]ben zamide trifluoroacetate

### (a) t-Butyl 4-[7-(2-Butynyl)-2-chloro-1-(4-cyanobenzyl)-6-oxo-6,7-dihydro-1H-purin-8-yl]piperazine-1-car boxylate

100 mg of t-butyl 4-[7-(2-butynyl)-2-chloro-6-oxo-6,7-dihydro-1H-purin-8-yl]piperazine-1-carboxylate was dissolved in 1.2 ml of N,N-dimethylformamide, and then 97 mg of 4-cyanobenzyl bromide and 68 mg of potassium carbonate were added thereto. The mixture was stirred at room temperature for 4 hours. A saturated aqueous ammonium chloride solution was added to the reaction mixture, and the mixture was extracted with ethyl acetate. The organic layer was concentrated, and the residue was purified by silica gel chromatography to give 71 mg of the title compound.
¹H-NMR(CDCl3)
δ 1.49 (s, 9H) 1.84 (t, J=2.5 Hz, 3H) 3.43-3.47 (m, 4H) 3.59-3.63 (m, 4H) 4.94 (q, 2.5 Hz, 2H) 5.53 (s, 2H) 7.42 (d, J=8.0 Hz, 2H) 7.62 (d, J=8.0 Hz, 2H)

### (b) 2-[7-(2-Butynyl)-1-(4-cyanobenzyl)-6-oxo-8-(piperazin-1-yl)-6,7-dihydro-1H-purin-2-yloxy]ben zamide trifluoroacetate

12 mg of t-butyl 4-[7-(2-butynyl)-2-chloro-1-(4-cyanobenzyl)-6-oxo-6,7 -dihydro-1H-purin-8-yl]piperazine-1-carboxylate was dissolved in 0.3 ml of 1-methyl-2-pyrrolidone, and then 10 mg of salicylamide and 10 mg of potassium carbonate were added thereto. The mixture was stirred at 100°C for 12 hours. 1N hydrochloric acid was added to the reaction solution, and the mixture was extracted with ethyl acetate. The organic layer was concentrated, and the residue was dissolved in trifluoroacetic acid. The solution was concentrated, and the residue was purified by reverse-phase high performance liquid chromatography (using an acetonitrile-water mobile phase (containing 0.1% trifluoroacetic acid)) to give 6.69 mg of the title compound.
MS *m*/*e* (ESI) 523(MH⁺-CF₃COOH)

### Example 238

### 2-[7-(2-Butynyl)-1-(3-cyanobenzyl)-6-oxo-8-(piperazin-1-yl)-6,7-dihydro-1H-purin-2-yloxy]ben zamide trifluoroacetate

### (a) t-Butyl 4-[7-(2-butynyl)-2-chloro-1-(3-cyanobenzyl)-6-oxo-6,7-dihydro-1H-purin-8-yl]piperazine-1-car boxylate

100 mg of t-butyl 4-[7-(2-butynyl)-2-chloro-6-oxo-6,7-dihydro-1H-purin-8-yl]piperazine-1-carboxylate was dissolved in 1.2 ml ofN,N-dimethylformamide, and then 97 mg of 3-cyanobenzyl bromide and 68 mg of potassium carbonate were added thereto. The mixture was stirred at room temperature for 12 hours. Then, a saturated ammonium chloride solution was added to the reaction solution, and the mixture was extracted with ethyl acetate. The organic layer was concentrated, and the residue was purified by silica gel chromatography to give 71 mg of the title compound.
¹H-NMR(CDCl3)
δ 1.49 (s, 9H) 1.84 (t, J=2.5 Hz, 3H) 3.43-3.47 (m, 4H) 3.59-3.63 (m, 4H) 4.94 (q, 2.5 Hz, 2H) 5.53 (s, 2H) 7.42 (d, J=8.0 Hz, 2H) 7.62 (d, J=8.0 Hz, 2H)

### (b) 2-[7-(2-Butynyl)-1-(3-cyanobenzyl)-6-oxo-8-(piperazin-1-yl)-6,7-dihydro-1H-purin-2-yloxy]ben zamide trifluoroacetate

12 mg of t-butyl 4-[7-(2-butynyl)-2-chloro-1-(3-cyanobenzyl)-6-oxo-6,7-dihydro-1H-purin-8-yl]piperazine-1-carboxylate was dissolved in 0.3 ml of 1-methyl-2-pyrrolidone, and then 10 mg of salicylamide and 10 mg of potassium carbonate were added thereto. The mixture was stirred at 100°C for five hours. 1N hydrochloric acid was added to the reaction solution, and the mixture was extracted with ethyl acetate. The organic layer was concentrated, and the residue was dissolved in trifluoroacetic acid. The solution was concentrated, and the residue was purified by reverse-phase high performance liquid chromatography (using an acetonitrile-water mobile phase (containing 0.1% trifluoroacetic acid)) to give 8.76 mg of the title compound.
MS *m*/*e* (ESI) 523(MH⁺-CF₃COOH)

### Example 242

### 8-(3-amino piperidin-1-yl)-7-(2-butynyl)-1-(2-cyanobenzyl)-6-oxo-6,7-dihydro-1H-purine-2-carbonitrile hydrochloride

### (a) Benzyl 3-t-butoxycarbonylaminopiperidine-1-carboxylate

88 g of benzyl chloroformate (30% toluene solution) was added dropwise to a mixture consisting of 24.3 g of ethyl piperidine-3-carboxylate, 26 ml of triethylamine and 300 ml of ethyl acetate over 30 minutes while the mixture was being cooled with ice. The reaction mixture was filtered to remove insoluble material. The filtrate was again filtered through a small amount of silica gel. The filtrate was concentrated.

200 ml of ethanol and 40 ml of a 5 M aqueous sodium hydroxide solution were added to the residue. The mixture was stirred at room temperature overnight. The reaction solution was concentrated, and 200 ml of water was added to the residue. The mixture was extracted with t-butyl methyl ether. 5 M aqueous hydrochloric acid was added to the aqueous layer, and the mixture was extracted with ethyl acetate. The organic layer was washed with water and then with saturated brine. The organic layer was dried over anhydrous magnesium sulfate, and then concentrated to give an oily residue (30.9 g).

A mixture consisting of 30 g of this residue, 24.5 ml of diphenyl phosphoryl azide, 15.9 ml of triethylamine and 250 ml of t-butanol was stirred at room temperature for 1.5 hours. The mixture was further stirred in an oil bath at 100°C for 20 hours. The reaction solution was concentrated, and the residue was extracted with ethyl acetate-water. The organic layer was washed with dilute aqueous sodium bicarbonate solution and then with saturated brine. The organic layer was dried over anhydrous magnesium sulfate, and then concentrated. The residue was purified by silica gel column chromatography using 10% to 20% ethyl acetate/hexane, followed by recrystallization from ethyl acetate-hexane to give 21.4 g of the title compound.
¹H-NMR(CDCl₃)
δ 1.43 (s, 9H) 1.48-1.92 (m, 4H) 3.20-3.80 (m, 5H) 4.58 (br.s, 1H) 5.13 (s, 2H) 7.26-7.40(m, 5H)

### (b) t-Butyl piperidin-3-ylcarbamate

A mixture consisting of 10 g of benzyl 3-t-butoxycarbonylaminopiperidine-1-carboxylate, 500 mg of 10% palladium carbon and 100 ml of ethanol was stirred at room temperature under a hydrogen atmosphere overnight. The catalyst was removed by filtration. The filtrate was concentrated and dried to give 6.0 g of the title compound.
¹H-NMR(CDCl₃)
δ 1.44 (s, 9H) 1.47-1.80 (m, 4H) 2.45-2.60 (m, 1H) 2.60-2.75 (m, 1H) 2.75-2.90 (m, 1H) 3.05 (dd, J=3 Hz, 12 Hz, 1H) 3.57 (br.s, 1H) 4.83 (br.s, 1H)

### (c) t-Butyl [1-[7-(2-butynyl)-2,6-dichloro-7H-purin-8-yl]piperidin-3-yl]carbamate

A mixture consisting of 1.25 g of 7-(2-butynyl)-2,6,8-trichloro-7H-purine, 1.0 g of t-butyl piperidin-3-ylcarbamate and 10 ml of acetonitrile was stirred at room temperature for 10 minutes. 0.63 ml of triethylamine was added dropwise over 10 minutes, and then the mixture was continuously stirred at room temperature for 30 minutes. The reaction solution was partitioned between ethyl acetate and water, and the organic layer was washed with saturated brine. The organic layer was dried over anhydrous magnesium sulfate, and then concentrated. The residue was crystallized with t-butyl methyl ether-hexane to give 1.79 g of the title compound.
¹H-NMR(CDCl₃)
δ 1.43 (s, 9H) 1.60-2.02 (m, 4H) 1.83 (t, J=2 Hz, 3H) 3.32-3.41 (m, 1H) 3.42-3.52 (m, 1H) 3.67-3.76 (m, 1H) 3.80-3.91 (m, 1H) 4.76-4.90 (m, 3H)

### (d) t-Butyl [1-[7-(2-butynyl)-2-chloro-6-oxo-6,7-dihydro-1H-purin-8-yl]piperidin-3-yl]carbamate

A mixture consisting of 1.79 g of t-butyl [1-[7-(2-butynyl)-2,6-dichloro-7H-purin-8-yl]piperidin-3-yl]carbamate, 1.0 g of sodium acetate and 18 ml of dimethyl sulfoxide was stirred in an oil bath at 120°C for three hours. The mixture was removed from the oil bath, and 18 ml of water was added to the reaction solution. The mixture was cooled to room temperature. The crystals were collected by filtration, and washed with water and then with t-butyl methyl ether. The crystals were then dried to give 1.59 g of the title compound.
¹H-NMR(DMSO-d6)
δ 1.39 (s, 9H) 1.34-1.88 (m, 4H) 1.78 (s, 3H) 2.81 (t, J=11 Hz, 1H) 2.95 (t, J=11 Hz, 1H) 3.48-3.60 (m, 2H) 3.64 (d, J=6 Hz, 1H) 4.90 (s, 2H) 6.94 (d, J=8 Hz, 1H)

### (e) t-Butyl [1-[7-(2-butynyl)-2-chloro-1-(2-cyanobenzyl)-6-oxo-6,7-dihydro-1H-purin-8-yl]piperidin-3-yl]c arbamate

A mixture consisting of 100 mg of t-butyl [1-[7-(2-butynyl)-2-chloro-6-oxo-6,7-dihydro-1H-purin-8-yl]piperidin-3-yl]carbamate, 66 mg of anhydrous potassium carbonate, 70 mg of 2-cyanobenzyl bromide and 1 ml of N,N-dimethylformamide was stirred at room temperature for five hours. The reaction solution was partitioned between ethyl acetate and water, and the organic layer was washed with water and then with saturated brine. The organic layer was dried over anhydrous magnesium sulfate, and then concentrated. The residue was purified by silica gel column chromatography using 50% ethyl acetate/hexane to give 44.7 mg of the title compound.
¹H-NMR(CDCl₃)
δ 1.44 (s, 9H) 1.59-1.81 (m, 2H) 1.83 (t, J=2 Hz, 3H) 1.86-1.94 (m, 2H) 3.20-3.50 (m, 3H) 3.66 (d, J=7 Hz, 1H) 3.86 (br.s, 1H) 4.88-5.06 (m, 3H) 5.72 (s, 2H) 7.06 (d, J=8 Hz, 1H) 7.38 (t, J=8 Hz, 1H) 7.51 (t, J=8 Hz, 1H) 7.70 (d, J=8 Hz, 1H)

### (f) t-Butyl [1-[7-(2-butynyl)-2-cyano-1-(2-cyanobenzyl)-6-oxo-6,7-dihydro-1-purin-8-yl]piperidin-3-yl]car bamate

A mixture consisting of 15 mg of t-butyl [1-[7-(2-butynyl)-2-chloro -1-(2-cyanobenzyl)-6-oxo-6,7-dihydro-1H-purin-8-yl]piperidin-3-yl]carbamate, 20 mg of sodium cyanide and 0.2 ml of N,N-dimethylformamide was stirred at room temperature for three hours. The reaction solution was partitioned between ethyl acetate and water, and the organic layer was washed with water and then with saturated brine. Then, the organic layer was concentrated, and the residue was purified by thin layer chromatography using 50% ethyl acetate/hexane solvent (developed three times) to give 10.3 mg of the title compound.
¹H-NMR(CDCl₃)
δ 1.44 (s, 9H), 1.52-1.98 (m, 4H) 1.81 (t, J=2 Hz 3H) 3.24 (dd, J=7 Hz, 12 Hz, 1H) 3.30-3.40 (m, 1H) 3.46-3.56 (m, 1H), 3.72 (d, J=12 Hz, 1H) 3.86 (br.s, 1H) 4.86-5.10 (m, 3H) 5.73 (s, 2H) 7.00 (d, J=8 Hz, 1H) 7.42 (t, J=8 Hz, 1H) 7.54 (dt, J=2 Hz, 8 Hz, 1H) 7.73 (dd, J=2 Hz, 8 Hz, 1H)

### (g) 8-(3-Aminopiperidin-1-yl)-7-(2-butynyl)-1-(2-cyanobenzyl)-6-oxo-6,7-dihydro-1H-purine-2-car bonitrile hydrochloride

A mixture consisting of 10.3 mg of t-butyl [1-[7-(2-butynyl)-2-cyano-1-(2-cyanobenzyl)-6-oxo-6,7-dihydro-1H-purin-8-yl] piperidin-3-yl]carbamate and 0.2 ml of trifluoroacetic acid was stirred for 20 minutes. The reaction solution was concentrated, and the residue was purified by reverse-phase column chromatography using 20% to 80% methanol/water (0.1% concentrated hydrochloric acid) solvent to give 8.0 mg of the title compound.
¹H-NMR(DMSO-d6)
δ 1.60-1.74 (m, 2H) 1.79 (t, J=2 Hz, 3H) 1.88-2.03 (m, 2H) 3.14-3.28 (m, 2H) 3.42 (br.s, 1H) 3.52-3.82 (m, 2H) 4.98-5.12 (m, 2H) 5.58 (s, 2H) 7.26 (d, J=8 Hz, 1H) 7.53 (t, J=8 Hz, 1H) 7.66 (t, J=8 Hz, 1H) 7.93 (d, J=8 Hz, 1H) 8.16 (br.s, 3H)

### Example 243

### 2-[8-(3-Amino piperidin-1-yl)-7-(2-butynyl)-2-methoxy-6-oxo-6,7-dihydropurin-1-ylmethyl]benzonitrile hydrochloride

A mixture consisting of 15 mg of t-butyl [1-[7-(2-butynyl) -2-chloro-1-(2-cyanobenzyl)-6-oxo-6,7-dihydro-1H-purin-8-yl]piperidin-3-yl]carbamate, 20 mg of anhydrous potassium carbonate and 0.2 ml of methanol was stirred for three hours. Subsequent steps were carried out according to the same procedure as used in Examples 242 (f) and (g). Thus, the title compound was synthesized.
¹H-NMR(DMSO-d6)
δ 1.58-1.72 (m, 2H) 1.84-1.94 (m, 1H) 1.96-2.04 (m, 1H) 3.08-3.20 (m, 2H) 3.36-3.70 (m, 3H) 3.90 (s, 3H) 4.90-5.02 (m, 2H) 5.32 (s, 2H) 7.20 (d, J=8 Hz, 1H) 7.47 (t, J=8 Hz, 1H) 7.63 (t, J=8 Hz, 1H) 7.87 (d, J=8 Hz, 1H) 8.12 (br.s, 3H)

### Example 248

### 2-[8-(3-Aminopiperidin-1-yl)-7-(2-butynyl)-1-methyl-6-oxo-6,7-dihydro-1H-purin-2-yloxy]benz amide trifluoroacetate

### (a) t-Butyl [1-[7-(2-butynyl)-2-chloro-1-methyl-6-oxo-6,7-dihydro-1H-purin-8-yl]piperidin-3-yl]carbamate

700 mg of t-butyl [1-[7-(2-butynyl)-2-chloro-6-oxo-6,7-dihydro-1H-purin-8-yl]piperidin-3-yl]carbamate was dissolved in 7.0 ml of dimethyl sulfoxide, and then 114 µl of methyl iodide and 299 mg of potassium carbonate were added thereto. The mixture was stirred at room temperature for 30 minutes, and 40 ml of water was added to the reaction solution. The mixture was stirred at room temperature for 30 minutes, and the white precipitate was collected by filtration. The resulting white solid was washed with water and then with hexane to give 540 mg of the title compound.
¹H-NMR(CDCl3)
δ 1.44 (s, 9H) 1.72-1.94 (m, 4H) 1.81 (t, J=2.4 Hz, 3H) 3.16-3.92 (m, 5H) 3.72 (s, 3H) 4.91 (dd, J= 17.6, 2.4 Hz, 1H) 5.01 (d, J=17.6 Hz, 1H)

### (b)

### 2-[8-(3-Aminopiperidin-1-yl)-7-(2-butynyl)-1-methyl-6-oxo-6,7-dihydro-1H-purin-2-yloxy]benz amide trifluoroacetate

10 mg of t-butyl [1-[7-(2-butynyl)-2-chloro-1-methyl-6-oxo-6,7-dihydro-1H-purin-8-yl]piperidin-3-yl]carbamate was dissolved in 0.3 ml of 1-methyl-2-pyrrolidone, and then 10 mg of salicylamide and 10 mg of potassium carbonate were added thereto. The mixture was stirred at 100°C for 2 hours. 1N hydrochloric acid was added to the reaction solution, and the mixture was extracted with ethyl acetate. The organic layer was concentrated, and the residue was dissolved in trifluoroacetic acid. The solution was concentrated, and the residue was purified by reverse-phase high performance liquid chromatography (using an acetonitrile-water mobile phase (containing 0.1% trifluoroacetic acid)) to give 5.54 mg of the title compound.
MS *m*/*e* (ESI) 436(MH⁺-CF₃COOH)

### Example 258

### 3-(2-Butynyl)-2-(piperazin-1-yl)-5-(2-propynyl)-3,5-dihydroimidazo[4,5-d]pyridazin-4-one trifluoroacetate

### (a) t-Butyl 4-[1-(2-butynyl)-7-oxo-6,7-dihydro-1H-imidazo[4,5-d]pyridazin-2-yl]piperazine-1-carboxylate

0.299 g of triethylamine, 0.023 g of 4-dimethylaminopyridine and 0.645 g of di-t-butyl dicarbonate were added to 20 ml of an N,N-dimethylformamide solution of 0.448 g of 3-(2-butynyl)-2-(piperazin-1-yl)-3,5-dihydroimidazo[4,5-d]pyridazin-4-one trifluoroacetate at room temperature, and the mixture was stirred for five hours. Then, 2 ml of a 5N aqueous sodium hydroxide solution was added to this solution, and the mixture was stirred for one hour. The reaction solution was poured into a mixture of 200 ml of ethyl acetate and 100 ml of a saturated aqueous ammonium chloride solution. The organic layer was washed twice with 100 ml of water and then with 100 ml of a saturated sodium chloride solution. The organic liquid was dried over magnesium sulfate, and concentrated under reduced pressure. The residue was purified by silica gel column chromatography. Thus, 0.298 g of the title compound was obtained from the fraction eluted with ethyl acetate.
¹H-NMR(CDCl₃)
δ 1.50 (s, 9H) 1.84 (t, J=2.3Hz, 3H) 3.41 (m, 4H) 3.63 (m, 4H) 5.06 (q, J=2.3Hz, 2H) 8.17 (s, 1H) 9.92 (br.s, 1H)

### (b) 3-(2-Butynyl)-2-(piperazin-1-yl)-5-(2-propynyl)-3,5-dihydroimidazo[4,5-d]pyridazin-4-one trifluoroacetate

0.005 g of potassium carbonate and 0.003 ml of 3-bromo-1-propyne were added to 0.5 ml of an N,N-dimethylformamide solution of 0.010 g of t-butyl 4-[1-(2-butynyl)-7-oxo-6,7-dihydro-1H-imidazo[4,5-d]pyridazin-2-yl]piperazine-1-carboxylate, and the mixture was stirred at room temperature for 10 hours. 1 ml of ethyl acetate and 1 ml of water were added to the reaction solution, and the layers were separated. The organic layer was concentrated, and the resulting residue was dissolved in a mixture consisting of 0.5 ml of dichloromethane and 0.5 ml of trifluoroacetic acid. The mixture was stirred for 1 hour, and then concentrated. The residue was purified by reverse-phase high performance liquid chromatography (using an acetonitrile-water mobile phase (containing 0.1% trifluoroacetic acid)) to give 0.011 g of the title compound.
MS *m*/*e* (ESI) 311.29(MH⁺-CF₃COOH)

### Example 266

### 3-(2-Butynyl)-5-[2-(3-methoxyphenyl)-2-oxoethyl]-2-(piperazin-1-yl)-3,5-dihydroimidazo[4,5-d ]pyridazin-4-one trifluoroacetate

The title compound was obtained by using t-butyl 4-[1-(2-butynyl)-7-oxo-6,7-dihydro-1H-imidazo[4,5-d]pyridazin-2-yl]piperazine-1-carboxylate and 2-bromo-3'-methoxy acetophenone according to the method described in Example 258(b).
MS *m*/*e* (ESI) 421.33(MH⁺-CF₃COOH)

### Example 267

### 2-[3-(2-Butynyl)-4-oxo-2-(piperazin-1-yl)-3,4-dihydroimidazo[4,5-d]pyridazin-5-ylmethyl]benz onitrile trifluoroacetate

The title compound was obtained by using t-butyl 4-[1-(2-butynyl)-7-oxo-6,7-dihydro-1H-imidazo[4,5-d]pyridazin-2-yl]piperazine-1-carboxylate and 2-bromomethylbenzonitrile according to the method described in Example 258(b).
¹H-NMR(CD₃OD)
δ 1.81 (t, J=2.5Hz, 3H) 3.45-3.49 (m, 4H) 3.66-3.70 (m, 4H) 5.15 (q, J=2.5Hz, 2H) 5.62 (s, 2H) 7.34 (dd, J=7.6,1.5Hz, 1H) 7.45 (td, J=7.6,1.5Hz, 1H) 7.59 (td, J=7.6,1.7Hz, 1H) 7.75 (dd, J=7.6,1.7Hz, 1H) 8.25 (s, 1H)
MS *m*/*e* (ESI) 388.32(MH⁺-CF₃COOH)

### Example 297

### 2-[3-(2-Butynyl)-4-oxo-2-(piperazin-1-yl)-3,4-dihydroimidazo[4,5-d]pyridazin-5-ylmethyl]-3-fl uorobenzonitrile trifluoroacetate

The title compound was obtained by using t-butyl 4-[1-(2-butynyl)-7-oxo-6,7-dihydro-1H-imidazo[4,5-d]pyridazin-2-yl]piperazine-1-carboxylate and 2-bromomethyl-3-fluorobenzonitrile according to the method described in Example 258(b).
MS *m*/*e* (ESI) 406.25(MH⁺-CF₃COOH)

### Example 308

### 3-Benzyl-2-(piperazin-1-yl)-3,5-dihydroimidazo[4,5-d]pyridazin-4-one trifluoroacetate

### (a) t-Butyl 4-(1-benzyl-6-benzyloxymethyl-7-oxo-6,7-dihydro-1H-imidazo[4,5-d]pyridazin-2-yl)piperazine-1-carboxylate

The title compound was obtained by using t-butyl 4-(6-benzyloxymethyl-7-oxo-6,7-dihydro-1H-imidazo[4,5-d]pyridazin-2-yl)piperazine-1-carbox ylate and benzyl bromide according to the method described in Example 116(d).
¹H-NMR(CDCl₃)
δ 1.48 (s, 9H) 3.13-3.18 (m, 4H) 3.50-3.54 (m, 4H) 4.72 (s, 2H) 5.61 (s, 2H) 5.65 (s, 2H) 7.20-7.35(m, 10H) 8.22 (s, 1H)

### (b) 3-Benzyl-2-(pinerazin-1-yl)-3,5-dihydroimidazo[4,5-d]pyridazin-4-one trifluoroacetate

The title compound was obtained by treating t-butyl 4-(1-benzyl-6-benzyloxymethyl-7-oxo-6,7-dihydro-1H-imidazo[4,5-d]pyridazin-2-yl)piperazine-1-carboxylate according to the method described in Example 117.
¹H-NMR(CD₃OD)
δ 3.31-3.37 (m, 4H) 3.40-3.46 (m, 4H) 5.68 (s, 2H) 7.22-7.36(m, 5H) 8.25 (s, 1H)
MS *m*/*e* (ESI) 311.24(MH⁺-CF₃COOH)

### Example 309

### 3-Benzyl-5-methyl-2-(piperazin-1-yl)-3,5-dihydroimidazo[4,5-d]pyridazin-4-one trifluoroacetate

### (a) t-Butyl 4-(1-benzyl-7-oxo-6,7-dihydro-1H-imidazo[4,5-d]pyridazin-2-yl)piperazine-1-carboxylate

The title compound was obtained by using 3-benzyl-2-(piperazin-1-yl)-3,5-dihydroimidazo [4,5-d] pyridazin-4-one trifluoroacetate according to the method described in Example 258(a).
¹H-NMR(CDCl₃)
δ 1.47 (s, 9H) 3.12-3.16 (m, 4H) 3.47-3.52 (m, 4H) 5.58 (s, 2H) 7.20-7.34(m, 5H) 8.20 (s, 1H) 10.04 (br.s, 1H)

### (b) 3-Benzyl-5-methyl-2-(piperazin-1-yl)-3,5-dihydroimidazo[4,5-d]pyridazin-4-one trifluoroacetate

The title compound was obtained by using t-butyl 4-(1-benzyl-7-oxo-6,7-dihydro-1H-imidazo[4,5-d]pyridazin-2-yl)piperazine-1-carboxylate and methyl iodide according to the method described in Example 258(b).
¹H-NMR(CD₃OD)
δ 3.29-3.35 (m, 4H) 3.36-3.41 (m, 4H) 3.83 (s, 3H) 5.68 (s, 2H) 7.21-7.34(m, 5H) 8.20 (s, 1H)
MS *m*/*e* (ESI) 325.01(MH⁺-CF₃COOH)

### Example 311

### 3-Benzyl-5-(2-phenylethyl)-2-(piperazin-1-yl)-3,5-dihydroimidazo[4,5-d]pyridazin-4-one trifluoroacetate

The title compound was obtained by using t-butyl 4-[1-benzyl-7-oxo-6,7-dihydro-1H-imidazo[4,5-d]pyridazin-2-yl] piperazine-1-carboxylate and (2-bromoethyl)benzene according to the method described in Example 258(b).
¹H-NMR(CDCl₃)
δ 3.11 (t, J=8.1Hz,2H) 3.24-3.29 (m, 4H) 3.37-3.42 (m, 4H) 4.46 (t, J=8.1Hz,2H) 5.58 (s, 2H) 7.09-7.34 (m, 10H) 8.20 (s, 1H)
MS *m*/*e* (ESI) 415.54(MH⁺-CF₃COOH)

### Example 332

### 1-(2-Butynyl)-6-methyl-7-oxo-2-(piperazin-1-yl)-6,7-dihydroimidazo [4,5-d] pyridazine-4-carboxamide trifluoroacetate

### (a) t-Butyl 4-[1-(2-butynyl)-4-(cyano-hydroxymethyl)-5-methoxycarbonyl-1H-imidazol-2-yl]piperazine-1-c arboxylate

0.200 g of sodium cyanide and 0.010 ml of acetic acid were added to a 15 ml acetonitrile solution of t-butyl 4-[1-(2-butynyl)-5-methoxycarbonyl-4-formyl-1H-imidazol-2-yl]piperazine-1-carboxylate, and the mixture was stirred at room temperature for 16 hours. 100 ml of ethyl acetate was added to the solution, and the mixture was washed twice with 50 ml of water and then with 50 ml of a saturated sodium chloride solution. The organic layer was dried over magnesium sulfate, and the solvent was concentrated under reduced pressure. The residue was purified by silica gel column chromatography. Thus, 0.274 g of the title compound was obtained from the fraction eluted with ethyl acetate-hexane (2:3).
¹H-NMR(CDCl₃)
δ 1.49 (s, 9H) 1.83 (t, J=2.5Hz, 3H) 3.19-3.23 (m, 4H) 3.56-3.60 (m, 4H) 3.95 (s, 3H) 4.68 (d, J=9.0Hz, 1H) 4.82 (q, J=2.5Hz, 2H) 5.72 (d, J=9.0Hz, 1H)

### (b) t-Butyl

### 4-[1-(2-butynyl)-4-carbamoyl-hydroxymethyl)-5-methoxycarbonyl-1H-imidazol-2-yl]piperazin e-1-carboxylate

3.2 ml of 30% aqueous hydrogen peroxide and 3.2 ml of 28% aqueous ammonia solution were added to an 8 ml methanol solution of 0.274 g of t-butyl 4-[1-(2-butynyl)-4-(cyano-hydroxymethyl)-5-methoxycarbonyl-1H-imidazol-2-yl]piperazine-1-c arboxylate at 5°C, and the mixture was stirred for 15 hours. 100 ml of a saturated sodium hydrogen sulfite solution was added to the solution, and the mixture was extracted twice with 100 ml of ethyl acetate. The organic layers were combined together. The conbined organic layers were dried over magnesium sulfate, and concentrated under reduced pressure. The residue was purified by silica gel column chromatography. Thus, 0.039 g of the title compound was obtained from the fraction eluted with methanol-ethyl acetate (1:9).
¹H-NMR(CDCl₃)
δ 1.48 (s, 9H) 1.83 (t, J=2.5Hz, 3H) 3.13-3.25 (m, 4H) 3.54-3.57 (m, 4H) 3.91 (s, 3H) 4.33-4.37 (br.s, 1H) 4.77 (q, J=2.5Hz, 2H) 5.54 (s, 1H) 5.63 (s, 1H) 6.82 (s, 1H)

### (c) t-Butyl 4-[4-aminooxalyl-1-(2-butynyl)-5-methoxycarbonyl-1H-imidazol-2-yl]piperazine-1-carboxylate

0.051 ml of triethylamine and a 1 ml dimethyl sulfoxide solution of 0.058 g of sulfur trioxide pyridine were added to a 2 ml dichloromethane solution of 0.038 g of t-butyl 4-[1-(2-butynyl)-4-(carbamoyl-hydroxymethyl)-5-methoxycarbonyl-1H-imidazol-2-yl]piperazin e-1-carboxylate at 0°C, and the mixture was stirred at room temperature for 15 hours. Then, 0.102 ml of triethylamine and a 1 ml dimethyl sulfoxide solution of 0.116 g of sulfur trioxide pyridine were added, and the mixture was stirred at room temperature for 8 hours. 50 ml of ethyl acetate was added to the solution, and the organic layer was washed successively with 20 ml of an aqueous solution of 1% sulfuric acid, 20 ml of a saturated sodium bicarbonate solution, and 20 ml of a saturated sodium chloride solution. The organic layer was dried over magnesium sulfate, and concentrated under reduced pressure. The residue was purified by silica gel column chromatography. Thus, 0.021 g of the title compound was obtained from the fraction eluted with ethyl acetate-hexane (2: 1).
¹H-NMR(CDCl₃)
δ 1.48 (s, 9H) 1.82 (t, J=2.5Hz, 3H) 3.19-3.23 (m, 4H) 3.56-3.59 (m, 4H) 3.84 (s, 3H) 4.84 (q, J=2.5Hz, 2H) 5.62 (br.s, 1H) 7.02 (br.s, 1H)

### (d) t-Butyl 4-[1-(2-butynyl)-4-carbamoyl-6-methyl-7-oxo-6,7-dihydro-1H-dihydroimidazo[4,5-d]pyridazin-2-yl]piperazine-1-carboxylate

The title compound was obtained by using t-butyl 4-[4-aminooxalyl-1-(2-butynyl)-5-methoxycarbonyl-1H-imidazol-2-yl]piperazine-1-carboxylate according to the method described in Example 115(h).
¹H-NMR(CDCl₃)
δ 1.50 (s, 9H) 1.84 (t, J=2.3Hz, 3H) 3.46-3.50 (m, 4H) 3.63-3.66 (m, 4H) 3.99 (s, 3H) 5.12 (q, J=2.3Hz, 2H) 6.16 (s, 1H) 8.85 (s, 1H)

### (e) 1-(2-Butynyl)-6-methyl-7-oxo-2-(piperazin-1-yl)-6,7-dihydroimidazo[4,5-d]pyridazine-4-carbox amide trifluoroaceate

The title compound was obtained by using t-butyl 4-[1-(2-butynyl)-4-carbamoyl-6-methyl-7-oxo-6,7-dihydro-1H-dihydroimidazo[4,5-d]pyridazin-2-yl]piperazine-1-carboxylate according to the method described in Example 115(i).
MS *m*/*e* (ESI) 330.18(MH⁺-CF₃COOH)

### Example 338

### 3-(2-Butynyl)-2-(piperazin-1-yl)-3,5-dihydroimidazo[4,5-c]pyridin-4-one trifluoroacetate

### (a) 2-bromo-1-(2-butynyl)-1H-imidazole-4,5-dicarbonitrile

69.8 g of potassium carbonate and 50 ml N,N-dimethylformamide solution of 74 ml of 1-bromo-2-butyne were added to a 520 ml N,N-dimethylformamide solution of 90.6 g of 2-bromo-1H-imidazole-4,5-dicarbonitrile [CAS No 50847-09-1], and the mixture was heated at 50°C for 8 hours. 1 L of ethyl acetate and 500 ml of water were added to the solution, and the organic layer was washed twice with 500 ml of water and then with 500 ml of a saturated sodium chloride solution. The organic layer was dried over magnesium sulfate, and concentrated under reduced pressure. The residue was purified by silica gel column chromatography. Thus, 48.0 g of the title compound was obtained from the fraction eluted with ethyl acetate-hexane (1:4).
¹H-NMR(CDCl₃)
δ 1.87 (t, J=2.3Hz, 3H) 4.85 (q, J=2.3Hz, 2H)

### (b) Ethyl 2-bromo-1-(2-butynyl)-5-cyano-1H-imidazole-4-carboxylate

25 ml of concentrated sulfuric acid was added to a 500 ml ethanol solution of 48.0 g of 2-bromo-1-(2-butynyl)-1H-imidazole-4,5-dicarbonitrile, and the mixture was heated under reflux for 110 hours. The reaction solution was cooled to room temperature, and then concentrated under reduced pressure. The residue was dissolved in a mixture consisting of 500 ml of ethyl acetate and 500 ml of water, and the pH of the solution was adjusted to 8 using potassium hydroxide. The aqueous layer was extracted with 500 ml of ethyl acetate, and the organic layers were combined together. The organic layer was dried over magnesium sulfate, and concentrated under reduced pressure. The residue was purified by silica gel column chromatography. Thus, 21.7 g of the title compound was obtained from the fraction eluted with ethyl acetate-hexane (1:3).
¹H-NMR(CDCl₃)
δ 1.43 (t, J=7.0Hz, 3H) 1.87 (t, J=2.3Hz, 3H) 4.46 (q, J=7.0Hz, 2H) 4.85 (q, J=2.3Hz, 2H)

### (c) t-Butyl 4-[1-(2-butynyl)-5-cyano-4-ethoxycarbonyl-1H-imidazol-2-yl] piperazine-1-carboxylate

25.1 g of the title compound was obtained by using 21.7 g of ethyl 2-bromo-1-(2-butynyl)-5-cyano-1H-imidazole-4-carboxylate according to the method described in Example 115(b).
¹H-NMR(CDCl₃)
δ 1.43 (t, J=7.0Hz, 3H) 1.49 (s, 9H) 1.87 (t, J=2.3Hz, 3H) 3.22-3.26 (m, 4H) 3.56-3.61 (m, 4H) 4.44 (q, J=7.0Hz, 2H) 4.68 (q, J=2.3Hz, 2H)

### (d) t-Butyl 4-[1-(2-butynyl)-4-carboxy-5-cyano-1H-imidazol-2-yl] piperazine-1-carboxylate

16 ml of a 5N aqueous sodium hydroxide solution was added to a 500 ml ethanol solution of 25.1 g of t-butyl 4-[1-(2-butynyl)-5-cyano-4-ethoxycarbonyl-1H-imidazol-2-yl]piperazine-1-carboxylate, and the mixture was stirred at room temperature for two hours. Then, the solvent was concentrated under reduced pressure. The residue was dissolved in a mixture consisting of 1L of ethyl acetate and 500 ml of water. 50 ml of 2N hydrochloric acid was added to the solution. The organic layer was washed with 200 ml of a saturated sodium chloride solution, and dried over magnesium sulfate. The organic liquid was concentrated under reduced pressure to give 23.2 g of the title compound.
¹H-NMR(CDCl₃)
δ 1.49 (s, 9H) 1.87 (t, J=2.3Hz, 3H) 3.22-3.26 (m, 4H) 3.56-3.61 (m, 4H) 4.68 (q, J=2.3Hz, 2H)

### (e) t-Butyl 4-[1-(2-butynyl)-5-cyano-4-hydroxymethyl-1H-imidazol-2-yl] piperazine-1-carboxylate

6.9 g of triethylamine and then 100 ml tetrahydrofuran solution of 10.19 g of isobutyl chloroformate were added dropwise to 600 ml of tetrahydrofuran containing 22.9 g of t-butyl 4-[1-(2-butynyl)-4-carboxy-5-cyano-1H-imidazol-2-yl] piperazine-1-carboxylate at -10°C. After the precipitate had been removed by filtration, the solution was again cooled to -10°C. A 100 ml aqueous solution of 9.45 g of sodium borohydride was added dropwise to the solution. After one hour, 500 ml of ethyl acetate and 500 ml of water were added to the solution. The pH of the solution was adjusted to 5 using 1 N hydrochloric acid, and then adjusted to 10 using a saturated sodium bicarbonate solution. The organic layer was washed successively with 500 ml of water and 500 ml of a saturated sodium chloride solution. The organic layer was dried over magnesium sulfate, and concentrated under reduced pressure. The residue was purified by silica gel column chromatography. Thus, 19.1 g of the title compound was obtained from the fraction eluted with ethyl acetate-hexane (4:1).
¹H-NMR(CDCl₃)
δ 1.48 (s, 9H) 1.84 (t, J=2.3Hz, 3H) 2.26 (t, J=6.3Hz, 1H) 3.13-3.17 (m, 4H) 3.53-3.57 (m, 4H) 4.58 (q, J=2.3Hz, 2H) 4.64 (d, J=6.3Hz, 2H)

### (f) t-Butyl 4-[1-(2-butynyl)-5-cyano-4-formyl-1H-imidazol-2-yl]piperazine-1-carboxylate

3.28 g of manganese dioxide was added to a 5 ml dichloromethane solution of 1.35 g of t-butyl 4-[1-(2-butynyl)-5-cyano-4-hydroxymethyl-1H-imidazol-2-yl]piperazine-1-carboxylate. The reaction solution was stirred at room temperature for 15 hours, then stirred and heated under reflux for five hours. The solution was filtered, and then concentrated under reduced pressure. The residue was purified by silica gel column chromatography. Thus, 1.11 g of the title compound was obtained from the fraction eluted with ethyl acetate-hexane (2:3).
¹H-NMR(CDCl₃)
δ 1.50 (s, 9H) 1.88 (t, J=2.3Hz, 3H) 3.24-3.28 (m, 4H) 3.59-3.63 (m, 4H) 4.70 (q, J=2.3Hz, 2H) 9.87 (s, 1H)

### (g) t-Butyl 4-[1-(2-butynyl)-5-cyano-4-(2-ethoxycarbonylvinyl)-1H-imidazol-2-yl]piperazine-1-carboxylate

0.038 g of sodium hydride was added to a 5 ml tetrahydrofuran solution of 0.243 g of ethyl diethylphosphonoacetate at 5°C under a nitrogen atmosphere. 0.310 g of t-butyl 4-[1-(2-butynyl)-5-cyano-4-formyl-1H-imidazol-2-yl] piperazine-1-carboxylate dissolved in 5 ml of tetrahydrofuran was added, and the mixture was stirred for 30 minutes. 50 ml of ethyl acetate and 25 ml of 0.1N sodium hydroxide were added to the solution. The organic layer was dried over magnesium sulfate, and concentrated under reduced pressure. The residue was purified by silica gel column chromatography. Thus, 0.380 g of the title compound was obtained from the fraction eluted with ethyl acetate-hexane(3:7).
¹H-NMR(CDCl₃)
δ 1.33 (t, J=7.4Hz, 3H) 1.50 (s, 9H) 1.86 (t, J=2.3Hz, 3H) 3.19-3.23 (m, 4H) 3.55-3.59 (m, 4H) 4.25 (q, J=7.4Hz, 2H) 4.59 (q, J=2.3Hz, 2H) 6.70 (d, J=15.8Hz, 1H) 7.50 (d, J=15.8Hz, 1H)

### (h) t-Butyl 4-[1-(2-butynyl)-5-cyano-4-(2-carboxyvinyl)-1H-imidazol-2-yl]piperazine-1-carboxylate

The title compound was obtained by using t-butyl 4-[1-(2-butynyl)-5-cyano-4-(2-ethoxycarbonylvinyl)-1H-imidazol-2-yl]piperazine-1-carboxylate according to the method described in Example 338(d).
¹H-NMR(CDCl₃)
δ 1.50 (s, 9H) 1.86 (t, J=2.3Hz, 3H) 3.19-3.23 (m, 4H) 3.55-3.59 (m, 4H) 4.59 (q, J=2.3Hz, 2H) 6.70 (d, J=15.8Hz, 1H) 7.50 (d, J=15.8Hz, 1H)

### (i) t-Butyl 4-[1-(2-butynyl)-5-cyano-4-(2-azidecarbonylvinyl)-1H-imidazol-2-yl] piperazine-1-carboxylate

A mixture consisting of 0.200 g of t-butyl 4-[1-(2-butynyl)-5-cyano-4-(2-carboxyvinyl)-1H-imidazol-2-yl]piperazine-1-carboxylate, 0.073 ml of triethylamine, and a 2 ml t-butanol solution of 0.108 ml of diphenylphosphoryl azide was heated at 50°C under a nitrogen atmosphere for 4 hours. 50 ml of ethyl acetate was added to the solution, and the mixture was washed with 20 ml of water. The organic layer was dried over magnesium sulfate, and concentrated under reduced pressure. The residue was purified by silica gel column chromatography. Thus, 0.178 g of the title compound was obtained from the fraction eluted with ethyl acetate-hexane (2:3).
¹H-NMR(CDCl₃)
δ 1.48 (s, 9H) 1.86 (t, J=2.2Hz, 3H) 3.19-3.23 (m, 4H) 3.55-3.59 (m, 4H) 4.59 (q, J=2.2Hz, 2H) 6.67 (d, J=15.4Hz, 1H) 7.56 (d, J=15.4Hz, 1H)

### (j) t-Butyl 4-[4-(2-t-butoxycarbonylaminovinyl)-1-(2-butynyl)-5-cyano-1H-imidazol-2-yl] piperazine-1-carboxylate

A 10 ml t-butanol solution of 0.178 g of t-butyl 4-[1-(2-butynyl)-5-cyano-4-(2-azide carbonylvinyl)-1H-imidazol-2-yl] piperazine-1-carboxylate was heated under reflux under a nitrogen atmosphere for 15 hours. The solvent was concentrated under reduced pressure. The residue was purified by silica gel column chromatography. Thus, 0.169 g of the title compound was obtained from the fraction eluted with ethyl acetate-hexane (9: 11).
¹H-NMR(CDCl₃)
δ 1.48 (s, 9H) 1.84 (t, J=2.2Hz, 3H) 3.16-3.19 (m, 4H) 3.54-3.58 (m, 4H) 4.51 (q, J=2.2Hz, 2H) 5.83 (d, J=15.0Hz, 1H) 6.43-6.53 (m, 1H) 7.55-7.66 (m, 1H)

### (k) t-Butyl 4-[4-(2-t-butoxycarbonylaminovinyl)-1-(2-butynyl)-5-carbamoyl-1H-imidazol-2-yl] piperazine-1-carboxylate

The title compound was obtained by using t-butyl 4-[4-(2-t-butoxycarbonylaminovinyl)-1-(2-butynyl)-5-cyano-1H-imidazol-2-yl] piperazine-1-carboxylate according to the method described in Example 332(b).
¹H-NMR(CDCl₃)
δ 1.48 (s, 9H) 1.84 (t, J=2.2Hz, 3H) 3.21-3.25 (m, 4H) 3.54-3.58 (m, 4H) 4.68 (q, J=2.2Hz, 2H) 5.90 (br.s, 1H) 6.36 (br.d, J=14.8Hz, 1H) 6.92 (br.d, J= 8.4Hz, 1H) 7.45 (br.s, 1H) 7.52 (m, 1H)

### (1) 3-(2-Butynyl)-2-(piperazin-1-yl)-3,5-dihydroimidazo[4,5-c]pyridin-4-one trifluoroacetate

0.1 ml of 5N hydrochloric acid was added to a 0.3 ml ethanol solution of 0.0075 g of t-butyl 4-[4-(2-t-butoxycarbonylaminovinyl)-1-(2-butynyl)-5-carbamoyl-1H-imidazol-2-yl]piperazine-1 -carboxylate, and the mixture was stirred at room temperature for 15 hours. The solvent was concentrated under reduced pressure. The residue was purified by reverse-phase high performance liquid chromatography (using an acetonitrile-water mobile phase (containing 0.1% trifluoroacetic acid)) to give 0.0043 g of the title compound.
¹H-NMR(CD₃OD)
δ 1.81 (t, J=2.4Hz, 3H) 3.45-3.48 (m, 4H) 3.62-3.65 (m, 4H) 5.15 (q, J=2.4Hz, 2H) 6.60 (d, J=7.1 Hz, 1H) 7.18 (d, J=7.1Hz, 1H)
MS *m*/*e* (ESI) 272.32(MH⁺-CF₃COOH)

### Example 339

### 3-(2-Butynyl)-5-(2-phenylethyl)-2-(piperazin-1-yl)-3,5-dihydroimidazo[4,5-c]pyridin-4-one trifluoroacetate

### (a) t-Butyl 4-[3-(2-butynyl)-4-oxo-4,5-dihydro-3H-imidazo[4,5-c]pyridin-2-yl]piperazine-1-carboxylate

The title compound was obtained by using 3-(2-butynyl)-2-(piperazin-1-yl)-3,5-dihydroimidazo[4,5-c]pyridin-4-one trifluoroacetate according to the method described in Example 258(a).
¹H-NMR(CDCl₃)
δ 1.49 (s, 9H) 1.83 (t, J=2.3Hz, 3H) 3.35-3.39 (m, 4H) 3.60-3.64 (m, 4H) 5.07 (q, J=2.3Hz, 2H) 6.55 (d, J=7.1Hz, 1H) 6.97 (d, J=7.1Hz, 1H)

### (b) 3-(2-Butynyl)-5-(2-phenylethyl)-2-(piperazin-1-yl)-3,5-dihydroimidazo[4,5-c]pyridin-4-one trifluoroacetate

The title compound was obtained by using t-butyl 4-[3-(2-butynyl)-4-oxo-4,5-dihydro-3H-imidazo[4,5-c]pyridin-2-yl]piperazine-1-carboxylate and (2-bromoethyl)benzene according to the method described in Example 258(b).
¹H-NMR(CD₃OD)
δ 1.83 (t, J=2.4Hz, 3H) 3.05 (t, J=7.3Hz, 2H) 3.45-3.48 (m, 4H) 3.62-3.65 (m, 4H) 4.26 (t, J=7.3Hz, 2H) 5.18 (q, J=2.4Hz, 2H) 6.46 (d, J=7.3Hz, 1H) 7.15 (d, J=7.3Hz, 1H) 7.16-7.30 (m, 5H)
MS *m*/*e* (ESI) 376.36(MH⁺-CF₃COOH)

### Example 340

### 3-(2-Butynyl)-5-(2-phenoxyethyl)-2-(piperazin-1-yl)-3,5-dihydroimidazo[4,5-c]pyridin-4-one trifluoroacetate

The title compound was obtained by using t-butyl 4-[3-(2-butynyl)-4-oxo-4,5-dihydro-3H-imidazo[4,5-c]pyridin-2-yl]piperazine-1-carboxylate and 2-bromoethyl phenyl ether according to the method described in Example 258(b).
¹H-NMR(CD₃OD)
δ 1.80 (t, J=2.4Hz, 3H) 3.45-3.48 (m, 4H) 3.62-3.65 (m, 4H) 4.30 (t, J=5.5Hz, 2H) 4.44 (t, J=5.5Hz, 2H) 5.16 (q, J=2.4Hz, 2H) 6.59 (d, J=6.1Hz, 1H) 6.87-6.91 (m, 3H) 7.20-7.24 (m, 2H) 7.50 (d, J=6.1 Hz, 1H)
MS *m*/*e* (ESI) 392.34(MH⁺-CF₃COOH)

### Example 341

### 3-(2-Butynyl)-5-(2-oxo-2-phenylethyl)-2-(piperazin-1-yl)-3,5-dihydroimidazo[4,5-c]pyridin-4-o ne trifluoroacetate

The title compound was obtained by using t-butyl 4-[3-(2-butynyl)-4-oxo-4,5-dihydro-3H-imidazo[4,5-c]pyridin-2-yl]piperazine-1-carboxylate and 2-bromoacetophenone according to the method described in Example 258(b).
¹H-NMR(CD₃OD)
δ 1.79 (t, J=2.3Hz, 3H) 3.46-3.50 (m, 4H) 3.64-3.68 (m, 4H) 5.16 (q, J=2.3Hz, 2H) 5.61 (s, 2H) 6.65 (d, J=7.3Hz, 1H) 7.37 (d, J=7.3Hz, 1H) 7.57 (t, J=8.0Hz, 2H) 7.69 (t, J=8.0Hz, 1H) 8.10 (d, J=8.0Hz, 2H)
MS *m*/*e* (ESI) 392.34(MH⁺-CF₃COOH)

### Example 353

### 7-(2-Butynyl)-1,3-dimethyl-8-(piperazin-1-yl)-3,7-dihydropurine-2,6-dione

### (a) t-Butyl 4-[7-(2-butynyl)-1,3-dimethyl-2,6-dioxo-2,3,6,7-tetrahydro-1H-purin-8-yl] piperazine-1-carboxylate

4.9 g of 8-chlorotheophylline and 5 g of potassium carbonate were dissolved in 100 mL of N,N-dimethylformamide, and then 2.4 mL of 1-bromo-2-butyne was added. The resulting mixture was stirred at room temperature overnight, and then diluted with ethyl acetate and washed with water. The resulting insoluble white solid was collected by filtration, and washed with ethyl acetate to give 3.8 g of 7-(2-butynyl)-8-chloro-1,3-dimethyl-3,7-dihydropurine-2,6-dione. Then, 1.8 g of the resulting 7-(2-butynyl)-8-chloro-1,3-dimethyl-3,7-dihydropurine-2,6-dione was combined with 3.7 g of t-butyl 1-piperazine-carboxylate, and the mixture was stirred at 150°C for one hour. After being cooled to room temperature, the mixture was extracted with ethyl acetate. The organic layer was washed with water, and then with saturated sodium chloride solution, and dried over anhydrous magnesium sulfate. The solvent was distilled off under reduced pressure. The residue was purified by silica gel column chromatography. Thus, 1.6 g of the title compound was obtained from a fraction eluted with hexane-ethyl acetate (1:4).
¹H-NMR(CDCl₃)
δ: 1.49 (s, 9H) 1.82 (t, J=2.4Hz, 3H) 3.33-3.36 (m, 4H) 3.40 (s, 3H) 3.52 (s, 3H) 3.58-3.61 (m, 4H) 4.88 (q, J=2.4Hz, 2H)

### (b) 7-(2-Butynyl)-1,3-dimethyl-8-(piperazin-1-yl)-3,7-dihydropurine-2,6-dione

2.5 g of t-butyl 4-[7-(2-butynyl)-1,3-dimethyl-2,6-dioxo-2,3,6,7-tetrahydro-1H-purin-8-yl]piperazine-1-carboxyl ate was dissolved in 15 mL of trifluoroacetic acid. The mixture was stirred at room temperature for 30 minutes. The solvent was distilled off under reduced pressure. Then, the residue was purified by column chromatography using NH silica gel (silica gel with a surface that had been modified with amino groups: Fuji Silysia Chemical Ltd. NH-DM 2035). Thus, 1.6 g of the title compound was obtained from a fraction eluted with ethyl acetate.
¹H-NMR(CDCl₃)
δ: 1.82 (t, J=2.4Hz, 3H) 3.13-3.16 (m, 4H) 3.40 (s, 3H) 3.46-3.48 (m, 4H) 3.52 (s, 3H) 4.87 (q, J=2.4Hz, 2H)

### Example 354

### 7-(2-Butynyl)-3-methyl-8-(piperazin-1-yl)-3,7-dihydropurine-2,6-dione

### (a) t-Butyl 4-[7-(2-butynyl)-3-methyl-2,6-dioxo-2,3,6,7-tetrahydro-1H-purin-8-yl] piperazine-1-carboxylate

1.1 g of 3-methylxanthine was dissolved in 15 mL of N,N-dimethylformamide, and then 1.0 g of potassium carbonate and 0.64 mL of 1-bromo-2-butyne were added. The resulting mixture was stirred at room temperature overnight, and then diluted with ethyl acetate and washed with water. The resulting insoluble white solid was collected by filtration, and washed with ethyl acetate to give 1.3 g of 7-(2-butynyl)-3-methyl-3,7-dihydropurine-2,6-dione. Next, 1.3 g of the resulting 7-(2-butynyl)-3-methyl-3,7-dihydropurine-2,6-dione was dissolved in 15 mL of N,N-dimethylformamide, and then 0.89 g of N-chlorosuccinimide was added to the mixture while being cooled on ice. This mixture was stirred at room temperature for 3 hours, and then diluted with ethyl acetate and washed with water. The resulting insoluble white solid was collected by filtration, and washed with ethyl acetate to give 1.1 g of 7-(2-butynyl)-8-chloro-3-methyl-3,7-dihydropurine-2,6-dione. Then, 1.4 g of the resulting 7-(2-butynyl)-8-chloro-3-methyl-3,7-dihydropurine-2,6-dione was combined with 2.8 g of t-butyl 1-piperazine carboxylate, and the mixture was stirred at 150°C for one hour. This mixture was then cooled to room temperature, and extracted with ethyl acetate. The organic layer was washed with water, and then with saturated sodium chloride solution, and dried over anhydrous magnesium sulfate. The solvent was distilled off under reduced pressure. The residue was purified by silica gel column chromatography. Thus, 1.1 g of the title compound was obtained from a fraction eluted with hexane-ethyl acetate (1:4).
¹H-NMR(CDCl₃)
δ: 1.49 (s, 9H) 1.82 (t, J=2.4Hz, 3H) 3.35-3.37 (m, 4H) 3.47 (s, 3H) 3.58-3.61 (m, 4H) 4.85 (q, J=2.4Hz, 2H) 7.73 (s, 1H)

### (b) 7-(2-Butynyl)-3-methyl-8-(piperazin-1-yl)-3,7-dihydropurine-2,6-dione

The title compound was obtained using t-butyl 4-[7-(2-butynyl)-3-methyl-2,6-dioxo-2,3,6,7-tetrahydro-1H-purin-8-yl] piperazine-1-carboxylate by the same procedure described in Example 353-(b).
¹H-NMR(CDCl₃)
δ: 1.82 (t, J=2.4Hz, 3H) 3.02-3.05 (m, 4H) 3.37-3.39 (m, 4H) 3.48 (s, 3H) 4.85 (q, J=2.4Hz, 2H)

### Example 355

### Methyl [7-(2-butynyl)-3-methyl-2,6-dioxo-8-(piperazin-1-yl)-2,3,6,7-tetrahydropurin-1-yl] acetate trifluoroacetate

15 mg of t-butyl 4-[7-(2-butynyl)-3-methyl-2,6-dioxo-2,3,6,7-tetrahydro-1H-purin-8-yl] piperazine-1-carboxylate and 7 mg of potassium carbonate were dissolved in 1 mL of N,N-dimethylformamide, and 10 µL of methyl bromoacetate was then added. The resulting mixture was stirred at room temperature overnight, and then was diluted with ethyl acetate and washed with water. The solvent was distilled off, and then the residue was dissolved in 0.5 mL of trifluoroacetic acid. The mixture was stirred at room temperature for 30 minutes. The solvent was distilled off, and a half aliquot of the residue was purified by HPLC with a reverse-phase column, using water-acetonitrile-trifluoroacetic acid as the solvent for elution. Thus, 6.9 mg of the title compound was obtained.
MS *m*/*e* (ESI) 375(MH⁺-CF₃COOH)

### Example 356

### 7-(2-Butynyl)-1-(2-ethoxyethyl)-3-methyl-8-(piperazin-1-yl)-3,7-dihydropurine-2,6-dione trifluoroacetate

The title compound was obtained using 2-bromoethyl ethyl ether by the same procedure described in Example 355.
MS *m*/*e* (ESI) 375(MH⁺-CF₃COOH)

### Example 357

### 7-(2-Butynyl)-3-methyl-8-(piperazin-1-yl)-1-(2-propynyl)-3,7-dihydropurine-2,6-dione trifluoroacetate

The title compound was obtained using propargyl bromide by the same procedure described in Example 355.
MS *m*/*e* (ESI) 341(MH⁺-CF₃COOH)

### Example 358

### 1,7-bis(2-Butynyl)-3-methyl-8-(piperazin-1-yl)-3,7-dihydropurine-2,6-dione trifluoroacetate

The title compound was obtained using 1-bromo-2-butyne by the same procedure described in Example 355.
MS *m*/*e* (ESI) 355(MH⁺-CF₃COOH)

### Example 359

### [7-(2-Butynyl)-3-methyl-2,6-dioxo-8-(piperazin-1-yl)-2,3,6,7-tetrahydropurin-1-yl] acetonitrile trifluoroacetate

The title compound was obtained using bromoacetonitrile by the same procedure described in Example 355.
MS *m*/*e* (ESI) 342(MH⁺-CF₃COOH)

### Example 360

### 7-(2-Butynyl)-1-ethyl-3-methyl-8-(piperazin-1-yl)-3,7-dihydropurine-2,6-dione trifluoroacetate

The title compound was obtained using ethyl iodide by the same procedure described in Example 355.
MS *m*/*e* (ESI) 331(MH⁺-CF₃COOH)

### Example 361

### 7-(2-Butynyl)-3-methyl-1-[(2-oxo-2-phenyl)ethyl]-8-(piperazin-1-yl)-3,7-dihydropurine-2,6-dio ne trifluoroacetate

The title compound was obtained using 2-bromoacetophenone by the same procedure described in Example 355.
MS *m*/*e* (ESI) 421(MH⁺-CF₃COOH)

### Example 362

### 7-(2-Butynyl)-1-[2-(4-chlorophenyl)-2-oxoethyl]-3-methyl-8-(piperazin-1-yl)-3,7-dihydropurine -2,6-dione trifluoroacetate

The title compound was obtained using 2-bromo-4'-chloroacetophenone by the same procedure described in Example 355.
MS *m*/*e* (ESI) 455(MH⁺-CF₃COOH)

### Example 363

### 7-(2-Butynyl)-3-methyl-1-(2-phenoxyethyl)-8-(piperazin-1-yl)-3,7-dihydropurine-2,6-dione trifluoroacetate

The title compound was obtained using 2-phenoxy ethyl bromide by the same procedure described in Example 355.
MS *m*/*e* (ESI) 423(MH⁺-CF₃COOH)

### Example 364

### 2-[7-(2-Butynyl)-3-methyl-2,6-dioxo-8-(piperazin-1-yl)-2,3,6,7-tetrahydropurin-1-ylmethyl] benzonitrile trifluoroacetate

The title compound was obtained using 2-cyanobenzyl bromide by the same procedure described in Example 355.
MS *m*/*e* (ESI) 418(MH⁺-CF₃COOH)

### Example 365

### Methyl 4-[7-(2-butynyl)-3-methyl-2,6-dioxo-8-(piperazin-1-yl)-2,3,6,7-tetrahydropurin-1-ylmethyl] benzoate trifluoroacetate

The title compound was obtained using methyl 4-(bromomethyl) benzoate by the same procedure described in Example 355.
MS *m*/*e* (ESI) 451(MH⁺-CF₃COOH)

### Example 366

### Methyl 3-[7-(2-butynyl)-3-methyl-2,6-dioxo-8-(piperazin-1-yl)-2,3,6,7-tetrahydropurin-1-ylmethyl] benzoate trifluoroacetate

The title compound was obtained using methyl 3-(bromomethyl) benzoate by the same procedure described in Example 355.
MS *m*/*e* (ESI) 451(MH⁺-CF₃COOH)

### Example 367

### 7-(2-Butynyl)-3-methyl-1-(2-phenylethyl)-8-(piperazin-1-yl)-3,7-dihydropurine-2,6-dione trifluoroacetate

The title compound was obtained using (2-bromoethyl) benzene by the same procedure described in Example 355.
MS *m*/*e* (ESI) 407(MH⁺-CF₃COOH)

### Example 368

### 2-[7-(2-Butynyl)-3-methyl-2,6-dioxo-8-(pinerazin-1-yl)-2,3,6,7-tetrahydropurin-1-yl]-N-phenyla cetamide trifluoroacetate

25 mg of t-butyl 4-[1-carboxymethyl-3-methyl -7-(2-butynyl)-2,6-dioxo-2,3,6,7-tetrahydro-1H-purin-8-yl] piperazine-1-carboxylate was dissolved in 1 mL of tetrahydrofuran. Then, 5 µL of aniline, 9 mg of 1,1-carbonyldiimidazole, and 8 µL of triethylamine were added to the mixture. The resulting mixture was stirred at 60°C for five hours. The solution was diluted with ethyl acetate and washed with water, and dried over anhydrous magnesium sulfate. The solvent was distilled off, and then the residue was dissolved in 0.5 mL of trifluoroacetic acid. The mixture was stirred at room temperature for 30 minutes. The solvent was distilled off, and a half aliquot of the residue was purified by HPLC with a reverse-phase column using water-acetonitrile-trifluoroacetic acid as the solvent for elution. Thus, 2.74 mg of the title compound was obtained.
MS *m*/*e* (ESI) 436(MH⁺-CF₃COOH)

### Example 369

### 7-(2-Methoxyphenyl)-1,3-dimethyl-8-(piperazin-1-yl)-3,7- dihydropurine-2,6-dione trifluoroacetate

### (a) t-Butyl 4-(1,3-dimethyl-2,6-dioxo-2,3,6,7-tetrahydro-1H-purin-8-yl)piperazine-1-carboxylate

3.5 g of 8-chlorotheophylline and 11.69 g of t-butyl piperazine-1-carboxylate were mixed and stirred at 110°C overnight. Then, the mixture was diluted with ethyl acetate and then with water. The resulting insoluble white solid was collected by filtration and washed with ethyl acetate to give 3.65 g of the title compound.
¹H-NMR(CDCl₃)
δ: 1.48 (s, 9H) 3.38 (s, 3H) 3.54-3.57 (m, 7H) 3.66-3.69 (m, 4H) 11.58 (s, 1H)

### (b) 7-(2-Methoxyphenyl)-1,3-dimethyl-8-(piperazin-1-yl)-3,7-dihydropurine-2,6-dione trifluoroacetate

11 mg of t-butyl 4-(1,3-dimethyl-2,6-dioxo-2,3,6,7-tetrahydro-1H-purin-8-yl)piperazine-1-carboxylate, 15 mg of 2-methoxyphenylboronic acid, and 10 mg of copper (II) acetate were suspended in 0.5 mL of anhydrous tetrahydrofuran, and then 0.1 mL of pyridine was added. The resulting mixture was stirred at room temperature for five days. The reaction solution was filtered through a short column filled with NH silica gel, and the filtrate was concentrated. The residue was dissolved in 0.5 mL of trifluoroacetic acid, and the mixture was stirred at room temperature for 30 minutes. After the solvent was concentrated, the resulting residue was purified by reverse phase high performance liquid chromatography. Thus, 3.53 mg of the title compound was obtained.
¹H-NMR(CDCl₃)
δ: 3.05-3.20 (m, 4H) 3.29 (s, 3H) 3.50-3.51 (m, 7H) 3.81 (s, 3H) 7.04-7.07 (m, 2H) 7.26-7.30 (m, 1H) 7.47 (dt, J=2.0, 8.0Hz, 1H)
MS *m*/*e* (ESI) 371(MH⁺-CF₃COOH)

### Example 370

### 7-(2-Cyanophenyl)-1,3-dimethyl-8-(piperazin-1-yl)-3,7- dihydropurine-2,6-dione trifluoroacetate

### (a) t-Butyl 4-[7-(2-formylphenyl)-1,3-dimethyl-2,6-dioxo-2,3,6,7-tetrahydro-1H-purin-8-yl] piperazine-1-carboxylate

226 mg of t-butyl 4-(1,3-dimethyl-2,6-dioxo-2,3,6,7-tetrahydro-1H-purin-8-yl)piperazine-1-carboxylate, 200 mg of 2-formylphenylboronic acid, and 200 mg of copper (II) acetate were suspended in 5 mL of anhydrous tetrahydrofuran, and then 0.2 mL of pyridine was added. The resulting mixture was stirred at room temperature for five days. The reaction solution was filtered through a short column filled with silica gel, and the filtrate was concentrated. The residue was purified by silica gel column chromatography. Thus, 51 mg of the title compound was obtained from a fraction eluted with 1:1 hexane-ethyl acetate.
¹H-NMR(CDCl₃)
δ: 1.42 (s, 9H) 3.10-3.14 (m, 4H) 3.25-3.34 (m, 7H) 3.60 (s, 3H) 7.53 (dd, J=1.2, 8.0Hz, 1H) 7.63-7.67 (m, 1H) 7.73-7.78 (m, 1H) 8.02-8.04 (m, 1H) 9.86 (s, 1H)

### (b) 7-(2-Cyanophenyl)-1,3-dimethyl-8-(piperazin-1-yl)-3,7-dihydropurine-2,6-dione trifluoroacetate

13 mg of t-butyl 4-[7-(2-formylphenyl)-1,3-dimethyl-2,6-dioxo-2,3,6,7-tetrahydro-1H-purin-8-yl] piperazine-1-carboxylate and 10 mg of hydroxylamine hydrochloride were dissolved in a mixture containing 1 mL of ethanol and 0.2 mL of water. Approximately 10 mg of potassium acetate was added to the mixture. The resulting mixture was stirred at room temperature for 30 minutes. The reaction solution was diluted with ethyl acetate, and then washed with an aqueous solution of sodium bicarbonate. The organic layer was dried over anhydrous magnesium sulfate, and then filtered. The filtrate was concentrated under reduced pressure to give t-butyl 4-[7-[2-(hydroxyiminomethyl)phenyl]-1,3-dimethyl-2,6-dioxo-2,3,6,7-tetrahydro-1H-purin-8-yl] piperazine-1-carboxylate. This compound was dissolved in 0.5 mL of dichloromethane, and approximately 0.05 mL of triethylamine and 0.05 mL of methane sulfonyl chloride were then added. The resulting mixture was stirred at room temperature for 0.5 hours. The solvent was distilled off, and the residue was dissolved in trifluoroacetic acid. The solution was concentrated, and the residue was purified by reverse phase high performance liquid chromatography to give 4.14 mg of the title compound.
MS *m*/*e* (ESI) 366(MH⁺-CF₃COOH)

### Example 371

### 7-(2-Vinylphenyl)-1,3-dimethyl-8-(piperazin-1-yl)-3,7-dihydropurine-2,6-dione trifluoroacetate

9 mg of potassium tertiary butoxide was dissolved in 1 mL of tetrahydrofuran, and then 31 mg of methyltriphenylphosphonium bromide was added. The resulting mixture was stirred at room temperature for 30 minutes. 1 mL of tetrahydrofuran solution containing 20 mg of t-butyl 4-[7-(2-formylphenyl)-1,3-dimethyl-2,6-dioxo-2,3,6,7-tetrahydro-1H-purin-8-yl] piperazine-1-carboxylate was added to the mixture, which was then stirred at room temperature for 1 hour. The reaction solution was diluted with ethyl acetate and then with water. The organic layer was dried over anhydrous magnesium sulfate, and then filtered. The filtrate was concentrated under reduced pressure to give 40 mg of t-butyl 4-[7-(2-vinylphenyl)-1,3-dimethyl-2,6-dioxo-2,3,6,7-tetrahydro-1H-purin-8-yl] piperazine-1-carboxylate. 12 mg of this compound was dissolved in trifluoroacetic acid. The solution was concentrated, and the residue was purified by reverse phase high performance liquid chromatography, to give 4.38 mg of the title compound.
MS *m*/*e* (ESI) 367(MH⁺-CF₃COOH)

### Example 372

### 7-(2-Chlorophenyl)-1,3-dimethyl-8-(piperazin-1-yl)-3,7-dihydropurine-2,6-dione

### (a) 7-(2-Chlorophenyl)-1,3-dimethyl-3,7-dihydropurine-2,6-dione

510 mg of theophylline, 1 g of 2-chlorophenylboronic acid, and 220 mg of copper (II) acetate were suspended in 10 mL of N,N-dimethylformamide, and then 1 mL of pyridine was added. The resulting mixture was stirred at room temperature overnight. The reaction solution was diluted with ethyl acetate, and washed with 30% ammonia water. The organic layer was dried over anhydrous magnesium sulfate, and filtered. The filtrate was concentrated under reduced pressure, and the residue was triturated with ether to give 147 mg of the title compound.
¹H-NMR(CDCl₃)
δ: 3.72 (s, 3H) 3.68 (s, 3H) 7.43-7.51 (m, 3H) 7.57-7.60 (m, 1H) 7.68 (s, 1H)

### (b) 8-Chloro-7-(2-chlorophenyl)-1,3-dimethyl-3,7-dihydropurine-2,6-dione

138 mg of 7-(2-chlorophenyl)-1,3-dimethyl-3,7-dihydropurine-2,6-dione and 78 mg of N-chlorosuccinimide were suspended in 1 mL of N,N-dimethylformamide. The resulting mixture was stirred at room temperature for two hours. The reaction solution was diluted with ethyl acetate, and washed with water. The organic layer was dried over anhydrous magnesium sulfate, and then filtered. The filtrate was concentrated under reduced pressure to give 151 mg of the title compound.

### (c) t-Butyl 4-[7-(2-chlorophenyl)-1,3-dimethyl-2,6-dioxo-2,3,6,7-tetrahydro-1H-purin-8-yl] piperazine-1-carboxylate

142 mg of 8-chloro-7-(2-chlorophenyl)-1,3-dimethyl-3,7-dihydropurine-2,6-dione was combined with 500 mg of t-butyl piperazine-1-carboxylate. The mixture was stirred at 150°C for 4 hours, and then diluted with ethyl acetate and washed with water. The organic layer was dried over anhydrous magnesium sulfate and filtered. The filtrate was concentrated under reduced pressure. The residue was purified by silica gel column chromatography. Thus, 143 mg of the title compound was obtained from a fraction eluted with 2:3 hexane-ethyl acetate.
¹H-NMR(CDCl₃)
δ: 1.43 (s, 9H) 3.21-3.23 (m, 4H) 3.30 (s, 3H) 3.31-3.35 (m, 4H) 3.58 (s, 3H) 7.42-7.51 (m, 3H) 7.55-7.57 (m, 1H)

### (d) 7-(2-Chlorophenyl)-1,3-dimethyl-8-(piperazin-1-yl)-3,7-dihydropurine-2,6-dione

102 mg of t-butyl 4-[7-(2-chlorophenyl)-1,3-dimethyl-2,6-dioxo-2,3,6,7-tetrahydro-1H-purin-8-yl] piperazine-1-carboxylate was dissolved in 5 mL of trifluoroacetic acid. The resulting mixture was stirred at room temperature for 30 minutes. The solvent was distilled off, and the residue was purified by column chromatography using NH-silica gel. Thus, 109 mg of the title compound was obtained from a fraction eluted with 9:1 ethyl acetate and methanol.
¹H-NMR(CDCl₃)
δ: 2.77 (dt, J=1.6, 4.8Hz, 4H) 3.24 (t, J=5.2Hz, 4H) 3.30 (s, 3H) 3.58 (s, 3H) 7.41-7.44 (m, 2H) 7.48-7.51 (m, 1H) 7.55-7.56 (m, 1H)

### Example 373

### 7-(2-Chlorophenyl)-3-methyl-8-(piperazin-1-yl)-3,7-dihydropurine-2,6-dione trifluoroacetate

### (a) 7-Benzyl-3-methyl-3,7-dihydropurine-2,6-dione

2.882 g of 3-methylxanthine was suspended in 40 mL of N,N-dimethylformamide, and then 3 g of potassium carbonate and 2.5 mL of benzyl bromide were added. The resulting mixture was stirred at room temperature overnight. The reaction solution was diluted with ethyl acetate, and washed with 1N hydrochloric acid. The precipitated crystals were collected by filtration, and washed with ethyl acetate. Thus, 3.18 g of the title compound was obtained.
¹H-NMR(d⁶-DMSO)
δ: 3.32 (s, 3H) 5.42 (s, 2H) 7.27-7.35 (m, 5H) 8.21 (s, 1H) 11.13 (s; 1H)

### (b) 7-Benzyl-3-methyl-2,6-dioxo-2,3,6,7-tetrahydropurin-1-ylmethyl 2,2-dimethylpropionate

3.18 g of 7-benzyl-3-methyl-3,7-dihydropurine-2,6-dione was suspended in 40 mL of N,N-dimethylformamide. 2.6 g of potassium carbonate and 2.15 mL of chloromethylpivalate were added to the mixture. The resulting mixture was stirred at 40°C overnight. The reaction solution was diluted with ethyl acetate, and washed with 1N hydrochloric acid. The organic layer was dried over anhydrous magnesium sulfate, and filtered. The filtrate was concentrated under reduced pressure. The residue was purified by silica gel column chromatography. Thus, 4.26 g of the title compound was obtained from the fraction eluted with 1:3 hexane and ethyl acetate.
¹H-NMR(CDCl₃)
δ:1.19 (s, 9H) 3.58 (s, 3H) 5.48 (s, 2H) 6.04 (s, 2H) 7.32-7.39 (m, 5H) 7.58 (s, 1H)

### (c) 3-Methyl-2,6-dioxo-2,3,6,7-tetrahydropurin-1-ylmethyl 2,2-dimethylpropionate

4.26 g of 7-benzyl-3-methyl-2,6-dioxo-2,3,6,7-tetrahydropurin-1-ylmethyl 2,2-dimethylpropionate was dissolved in 100 mL of acetic acid, and 1.5 g of 10% palladium carbon was then added. The resulting mixture was stirred under a hydrogen atmosphere at room temperature overnight. The reaction solution was filtered with celite, and the filtrate was concentrated to give 2.98 g of the title compound.
¹H-NMR(CDCl₃)
δ: 1.19 (s, 9H) 3.66 (s, 3H) 6.12 (s, 2H) 7.86 (s, 1H)

### (d) 7-(2-Chlorophenyl)-3-methyl-2,6-dioxo-2,3,6,7-tetrahydropurin-1-ylmethyl 2,2-dimethyl-propionate

The title compound was obtained using 3-methyl-2,6-dioxo-2,3,6,7-tetrahydropurin-1-ylmethyl 2,2-dimethylpropionate by the same procedure described in Example 372-(a).

### (e) 8-Chloro-7-(2-chlorophenyl)-3-methyl-3,7-dihydropurine-2,6-dione

144 mg of 7-(2-chlorophenyl)-3-methyl-2,6-dioxo-2,3,6,7-tetrahydropurin-1-ylmethyl 2,2-dimethyl-propionate was dissolved in a mixture containing 2 mL of methanol and 1 mL of tetrahydrofuran, and 20 mg of sodium hydride was then added. The resulting mixture was stirred at room temperature overnight. The reaction solution was diluted with ethyl acetate, and washed with 1N hydrochloric acid. The organic layer was dried over anhydrous magnesium sulfate, and filtered. The filtrate was concentrated under reduced pressure. The residue was triturated with ethyl acetate-diethyl ether to give 72 mg of 7-(2-chlorophenyl)-3-methyl-3,7-dihydropurine-2,6-dione. This compound was dissolved in 1 mL of N,N-dimethylformamide, and 35 mg of N-chlorosuccinimide was then added. The resulting mixture was stirred at room temperature overnight, and the reaction solution was diluted with ethyl acetate and washed with 1N hydrochloric acid. The organic layer was dried over anhydrous magnesium sulfate and filtered. The filtrate was concentrated under reduced pressure to give 58 mg of the title compound.
¹H-NMR(CDCl₃)
δ: 3.59 (s, 3H) 7.42 (dd, J=1.6, 7.6Hz, 1H) 7.47 (dt, J=1.6, 9.2Hz, 1H) 7.54 (dt, J=1.6, 7.2Hz, 1H) 7.61 (dt, J=1.6, 7.6Hz, 1H) 7.93 (br, 1H)

### (f) t-Buyl 4-[7-(2-chlorophenyl)-3-methyl-2,6-dioxo-2,3,6,7-tetrahydro-1H-purin-8-yl] piperazine-1-carboxylate

58 mg of 8-chloro-7-(2-chlorophenyl)-3-methyl-3,7-dihydropurine-2,6-dione was combined with 150 mg of 1-(tertiary butoxycarbonyl)piperazine, and the mixture was stirred at 150°C for 4 hours. The reaction solution was diluted with ethyl acetate, and washed with water. The organic layer was dried over anhydrous magnesium sulfate, and filtered. The filtrate was concentrated under reduced pressure. The residue was purified by silica gel column chromatography. Thus, 44 mg of the title compound was obtained from a fraction eluted with ethyl acetate.
¹H-NMR(CDCl₃)
δ: 1.41 (s, 9H) 3.17-3.24 (m, 4H) 3.25-3.41 (m, 4H) 3.53 (s, 3H) 7.41-7.51 (m, 3H) 7.55 (dd, J=2.0, 7.6Hz, 1H) 7.66 (br, 1H)

### (g) 7-(2-Chlorophenyl)-3-methyl-8-(piperazin-1-yl)-3,7-dihydropurine-2,6-dione trifluoroacetate

8 mg of t-butyl 4-[7-(2-chlorophenyl)-3-methyl-2,6-dioxo-2,3,6,7-tetrahydro-1H-purin-8-yl] piperazine-1-carboxylate was dissolved in trifluoroacetic acid, and then the solution was concentrated. The residue was purified by reverse phase high performance liquid chromatography, to give 3.86 mg of the title compound.
MS *m*/*e* (ESI) 361(MH⁺-CF₃COOH)
¹H-NMR(CDCl₃)
δ: 2.76 -2.79 (m, 4H) 3.23-3.26 (m, 4H) 3.53 (s, 3H) 7.40-7.43 (m, 2H) 7.48-7.53 (m, 2H)

### Example 374

### Methyl [7-(2-chlorophenyl)-3-methyl-2,6-dioxo-8-(piperazin-1-yl)-2,3,6,7-tetrahydropurin-1-yl] acetate trifluoroacetate

18 mg of t-butyl 4-[7-(2-chlorophenyl)-3-methyl-2,6-dioxo-2,3,6,7-tetrahydro-1H-purin-8-yl] piperazine-1-carboxylate was dissolved in 1 mL of N,N-dimethylformamide, and 0.1 mL of methyl bromoacetate and 10 mg of potassium carbonate were then added. The resulting mixture was stirred at room temperature for 3 days. The reaction solution was diluted with ethyl acetate, and washed with water. The organic layer was dried over anhydrous magnesium sulfate and filtered. The filtrate was concentrated under reduced pressure. The residue was dissolved in trifluoroacetic acid and the solution was concentrated. The residue was purified by reverse phase high performance liquid chromatography, to give 8.79 mg of the title compound.
MS *m*/*e* (ESI) 433(MH⁺-CF₃COOH)

### Example 375

### [7-(2-Chlorophenyl)-3-methyl-2,6-dioxo-8-(piperazin-1-yl)-2,3,6,7-tetrahydropurin-1-yl]acetonit rile trifluoroacetate

### Example 376

### 2-[7-(2-Chlorophenyl)-3-methyl-2,6-dioxo-8-(piperazin-1-yl)-2,3,6,7-tetrahydropurin-1-yl] acetamide trifluoroacetate

18 mg of t-butyl 4-[7-(2-chlorophenyl)-3-methyl-2,6-dioxo-2,3,6,7-tetrahydro-1H-purin-8-yl] piperazine-1-carboxylate was dissolved in 1 mL of N,N-dimethylformamide, and 0.1 mL of bromoacetonitrile and 10 mg of potassium carbonate were then added. The resulting mixture was stirred at room temperature for 3 days. The reaction solution was diluted with ethyl acetate, and washed with water. The organic layer was dried over anhydrous magnesium sulfate and filtered. The filtrate was concentrated under reduced pressure. The residue was dissolved in 1 mL of acetonitrile, and 0.05 mL of trimethylsilyl iodide was then added. The resulting mixture was stirred at room temperature for 1 hour. Then, methanol was added to the mixture. The reaction solution was concentrated. The residue was purified by reverse phase high performance liquid chromatography, to give 7.43 mg of [7-(2-chlorophenyl)-3-methyl-2,6-dioxo-8-(piperazin-1-yl)-2,3,6,7-tetrahydropurin-1-yl]-acetoni trile trifluoroacetate [MS *m*/*e* (ESI) 400 (MH⁺-CF₃COOH)] and 3.71 mg of [7-(2-chlorophenyl)-3-methyl-2,6-dioxo-8-(piperazin-1-yl)-2,3,6,7-tetrahydropurin-1-yl]-acetami de trifluoroacetate [MS *m*/*e* (ESI) 418 (MH⁺-CF₃COOH)].

### Example 377

### 7-(2-Chlorophenyl)-3-methyl-1-(2-phenethyl)-8-(piperazin-1-yl)-3,7-dihydropurine-2,6-dione trifluoroacetate

The title compound was obtained using 2-phenethyl bromide by the same procedure described in Example 374.
MS *m*/*e* (ESI) 465(MH⁺-CF₃COOH)

### Example 378

### 7-(2-Chlorophenyl)-3-methyl-1-(2-oxo-2-phenylethyl)-8-(piperazin-1-yl)-3,7-dihydropurine-2,6-dione trifluoroacetate

The title compound was obtained using phenacyl bromide by the same procedure described in Example 374.
MS *m*/*e* (ESI) 479(MH⁺-CF₃COOH)

### Example 379

### 7-(2-Methoxyphenyl)-3-methyl-8-(piperazin-1-yl)-3,7-dihydropurine-2,6-dione trifluoroacetate

The title compound was obtained using 2-methoxyphenylboronic acid by the same procedure described in Example 373.
MS *m*/*e* (ESI) 476(MH⁺-CF₃COOH)

### Example 380

### [7-(2-Methoxyphenyl)-3-methyl-2,6-dioxo-8-(piperazin-1-yl)-2,3,6,7-tetrahydropurin-1-yl] acetonitrile trifluoroacetate

### Example 381

### 2-[7-(2-Methoxyphenyl)-3-methyl-2,6-dioxo-8-(piperazin-1-yl)-2,3,6,7-tetrahydropurin-1-yl] acetamide trifluoroacetate

[7-(2-methoxyphenyl)-3-methyl-2,6-dioxo-8-(piperazin-1-yl)-2,3,6,7-tetrahydropurin-1-yl] acetonitrile trifluoroacetate [MS *m*/*e* (ESI) 396(MH⁺-CF₃COOH)] and 2-[7-(2-methoxyphenyl)-3-methyl-2,6-dioxo-8-(piperazin-1-yl)-2,3,6,7-tetrahydropurin-1-yl] acetamide trifluoroacetate [MS *m*/*e* (ESI) 414 (MH⁺-CF₃COOH)] were obtained using t-butyl 4-[7-(2-methoxyphenyl)-3-methyl-2,6-dioxo-2,3,6,7-tetrahydro-1H-purin-8-yl] piperazine-1-carboxylate by the same procedures as used in Examples 375 and 376.

### Example 382

### 7-(2-Methoxyphenyl)-3-methyl-1-(2-oxo-2-phenylethyl)-8-(piperazin-1-yl)-3,7-dihydropurine-2, 6-dione trifluoroacetate

The title compound was obtained using t-butyl 4-[7-(2-methoxyphenyl)-3-methyl-2,6-dioxo-2,3,6,7-tetrahydro-1H-purin-8-yl] piperazine-1-carboxylate and 2-bromoacetophenone by the same procedure described in Example 374.
MS *m*/*e* (ESI) 475(MH⁺-CF₃COOH)

### Example 383

### 7-(2-Methoxyphenyl)-3-methyl-1-(2-phenylethyl)-8-(piperazin-1-yl)-3,7-dihydropurine-2,6-dion e trifluoroacetate

The title compound was obtained using t-butyl 4-[7-(2-methoxyphenyl)-3-methyl-2,6-dioxo-2,3,6,7-tetrahydro-1H-purin-8-yl] piperazine-1-carboxylate and (2-bromoethyl) benzene by the same procedure described in Example 374.
MS *m*/*e* (ESI) 461(MH⁺-CF₃COOH)

### Example 384

### 7-(2-Vinylphenyl)-3-methyl-8-(piperazin-1-yl)-3,7-dihydropurine-2,6-dione

### (a) t-Butyl 4-[7-benzyl-1-(2,2-dimethylpropionyloxymethyl)-3-methyl-2,6-dioxo-2,3,6,7-tetrahydro-1H-puri n-8-yl] piperazine-1-carboxylate

The title compound was obtained using 7-benzyl-3-methyl-2,6-dioxo-2,3,6,7-tetrahydropurin-1-ylmethyl 2,2-dimethylpropionate by the same procedure described in Example 373-(e) and (f).

### (b) t-Butyl 4-[1-(2,2-dimethylpropionyloxymethyl)-3-methyl-2,6-dioxo-2,3,6,7-tetrahydro-1H-purin-8-yl] piperazine-1-carboxylate

2.227 g of t-butyl 4-[7-benzyl-1-(2,2-dimethylpropionyloxymethyl)-3-methyl-2,6-dioxo-2,3,6,7-tetrahydro-1H-puri n-8-yl] piperazine-1-carboxylate was dissolved in 100 mL of acetic acid, and 1 g of 10% palladium carbon was then added. The resulting mixture was stirred under a hydrogen atmosphere at room temperature overnight. The reaction solution was filtered. The filtrate was concentrated to give 1.89 g of the title compound.
¹H-NMR(CDCl₃)
δ: 1.09 (s, 9H) 1.41 (s, 9H) 3.36 (s, 3H) 3.37-3.42 (m, 4H) 3.45-3.50 (m, 4H) 5.82 (s, 2H)

### (c) t-Butyl 4-[1-(2,2-dimethylpropionyloxymethyl)-7-(2-vinylphenyl)-3-methyl-2,6-dioxo-2,3,6,7-tetrahydr o-1H-purin-8-yl] piperazine-1-carboxylate

The title compound was obtained using t-butyl 4-[1-(2,2-dimethylpropionyloxymethyl)-3-methyl-2,6-dioxo-2,3,6,7-tetrahydro-1H-purin-8-yl] piperazine-1-carboxylate by the same procedure described in Examples 370 and 371.
¹H-NMR(CDCl₃)
δ: 1.15 (s, 9H) 1.58 (s, 9H) 3.18 (br, 4H) 3.30 (br, 4H) 3.58 (s, 3H) 5.32 (d, J=11.2Hz, 1H) 5.75 (d, J=17.2Hz, 1H) 6.39 (dd, J=10.8, 17.2Hz, 1H) 7.34 (dd, J=1.2, 7.6Hz, 1H) 7.40 (dt, J=1.6, 7.2Hz, 1H) 7.46 (dt, J=1.6, 7.6Hz, 1H) 7.69 (dd, J=1.6, 8.0Hz, 1H)

### (d) 7-(2-Vinylphenyl)-3-methyl-8-(piperazin-1-yl)-3,7-dihydropurine-2,6-dione

187 mg of t-butyl 4-[1-(2,2-dimethylpropionyloxymethyl)-7-(2-vinylphenyl)-3-methyl -2,6-dioxo-2,3,6,7-tetrahydro-1H-purin-8-yl] piperazine-1-carboxylate was dissolved in 3 mL of methanol, and then 14 mg of sodium hydride was added. The resulting mixture was stirred at room temperature overnight. The reaction solution was neutralized with 1N hydrochloric acid, and then extracted with ethyl acetate. The organic layer was dried over anhydrous magnesium sulfate and filtered. The solvent was distilled off. The residue was purified by silica gel column chromatography. Thus, 108 mg of t-butyl 4-[3-methyl-2,6-dioxo-7-(2-vinylphenyl)-2,3,6,7-tetrahydro-1H-purin-8-yl] piperazine-1-carboxylate was obtained from a fraction eluted with 3:2 hexane-ethyl acetate. This compound was dissolved in 2 mL of trifluoroacetic acid and then concentrated. The residue was purified using NH-silica gel. Thus, 84 mg of the title compound was obtained from a fraction eluted with 15:1 ethyl acetate and methanol.
¹H-NMR(CDCl₃)
δ: 2.73 (t, J=5.2Hz, 4H) 3.19 (t, J=5.2Hz, 4H) 3.54 (s, 3H) 5.32 (dd, J=1.2, 10.8Hz, 1H) 5.74 (d, J=0.8, 17.6Hz, 1H) 6.41 (dd, J=10.8, 17.2Hz, 1H) 7.33 (dd, J=1.2, 6.0Hz, 1H) 7.38 (dt, J=1.6, 7.6Hz, 1H) 7.45 (dt, J=1.6, 7.6Hz, 1H) 7.68 (dd, J=1.6, 8.0Hz, 1H)

### Example 385

### 7-(2-Chlorophenyl)-3-ethyl-8-(piperazin-1-yl)-3,7-dihydropurine-2,6-dione trifluoroacetate

### (a) 2-Amino-7-benzyl-1,7-dihydropurin-6-one hydrochloride

100 g of guanosine was suspended in 500 mL of dimethylsulfoxide. 100 mL of benzyl bromide was added dropwise to the suspension at room temperature. The resulting reaction mixture was stirred at room temperature for 4 hours. Then, 250 mL of concentrated hydrochloric acid was added to the reaction, and the resulting mixture was stirred at room temperature for 30 minutes. The reaction mixture was poured into 3 L of methanol, and the mixture was stirred overnight. The precipitated crystals were collected by filtration and then washed with methanol. The crystals were air-dried at 60°C for 24 hours to give 82.5 g of the title compound.
¹H-NMR(d6-DMSO)
δ: 5.23 (s, 2H) 7.32-7.42 (m, 5H) 8.92 (s, 1H)

### (b) 7-Benzyl-3,7-dihydropurine-2,6-dione

A white suspension consisting of 12.88 g of 2-amino-7-benzyl-1,7-dihydropurin-6-one hydrochloride, 320 mL of acetic acid, and 32 mL of water was stirred at 110°C for 10 minutes, and then at 50°C for 10 minutes. Then, 32 mL of an aqueous solution containing 12.88 g of sodium nitrite was slowly added dropwise to the reaction mixture at 50°C. The resulting reaction mixture was stirred at 50°C for 15 hours. The resulting light brown suspension was filtered to give 4.27 g of the title compound.
¹H-NMR(d6-DMSO)
δ: 5.39 (s, 2H) 7.27-7.35 (m, 5H) 8.11(s, 1H) 10.86 (s, 1H) 11.57 (s, 1H)

### (c) [7-Benzyl-3-(2,2-dimethyl-propionyloxy methyl)-2,6-dioxo-2,3,6,7-tetrahydropurin-1-yl] methyl 2,2-dimethyl-propionate

9.54 g of 7-benzyl xanthine was dissolved in 250 mL of N,N-dimethylformamide, and then 17 g of potassium carbonate and 14.2 mL of chloromethylpivalate were added. The resulting mixture was stirred at 50°C overnight. The reaction solution was diluted with ethyl acetate, and washed with water and 1N hydrochloric acid. The organic layer was dried over anhydrous magnesium sulfate and filtered. The solvent was distilled off. The residue was purified by silica gel column chromatography. Thus, 12.8 g of the title compound was obtained from a fraction eluted with 3:2 hexane-ethyl acetate.

### (d) [3-(2,2-Dimethylpropionyloxymethyl)-2,6-dioxo-2,3,6,7-tetrahydropurin-1-yl] methyl 2,2-dimethylpropionate

The title compound was obtained using [7-benzyl-3-(2,2-dimethylpropionyloxymethyl)-2,6-dioxo-2,3,6,7-tetrahydropurin-1-yl] methyl 2,2-dimethylpropionate by the same procedure described in Example 384-(b).

### (e) [7-(2-Chlorophenyl)-3-(2,2-dimethylpropionyloxymethyl)-2,6-dioxo-2,3,6,7-tetrahydropurin-1-y l] methyl 2,2-dimethyl propionate

The title compound was obtained using [3-(2,2-dimethylpropionyloxymethyl)-2,6-dioxo-2,3,6,7-tetrahydropurin-1-yl] methyl 2,2-dimethyl propionate by the same procedure described in Example 373-(d).
¹H-NMR(CDCl₃)
δ: 1.16 (s, 9H) 1.22 (s, 9H) 5.99 (s, 2H) 6.19 (s, 2H) 7.42-7.52 (m, 3H) 7.58-7.61 (m, 1H) 7.73 (s, 1H)

### (f) t-Butyl 4-[7-(2-chlorophenyl)-1,3-bis-(2,2-dimethylpropionyloxymethyl)-2,6-dioxo-2,3,6,7-tetrahydro-1 H-purin-8-yl] piperazine-1-carboxylate

The title compound was obtained using [7-(2-chlorophenyl)-3-(2,2-dimethylpropionyloxymethyl)-2,6-dioxo-2,3,6,7-tetrahydropurin-1-yl ] methyl 2,2-dimethyl propionate by the same procedure described in Example 373-(e) and (f).
¹H-NMR(CDCl₃)
δ: 1.16 (s, 9H) 1.23 (s, 9H) 1.44 (s, 9H) 3.20-3.35 (m, 4H) 3.32-3.37 (m, 4H) 5.92 (s, 2H) 6.09 (s, 2H) 7.41-7.49 (m, 2H) 7.52-7.57 (m, 2H)

### (g) t-Butyl 4-[7-(2-chlorophenyl)-1-(2,2-dimethylpropionyloxymethyl)-2,6-dioxo-2,3,6,7-tetrahydro-1H-pur in-8-yl] piperazine-1-carboxylate

2.227 g of t-butyl 4-[7-(2-chlorophenyl)-1,3-bis-(2,2-dimethylpropionyloxymethyl)-2,6-dioxo-2,3,6,7-tetrahydro-1 H-purin-8-yl] piperazine-1-carboxylate was dissolved in a mixture containing 10 mL of tetrahydrofuran and 20 mL of methanol, and 0.518 mL of 1,8-diazabicyclo[5,4,0] undec-7-ene was then added. The resulting mixture was stirred at room temperature overnight. 1N hydrochloric acid was added to the reaction solution. The resulting precipitated solid was collected by filtration and dried, to give 1.025 g of the title compound.
¹H-NMR(CDCl₃)
δ: 1.16 (s, 9H) 1.44 (s, 9H) 3.22-3.24 (m, 4H) 3.33-3.35 (m, 4H) 5.90 (s, 2H) 7.43-7.47 (m, 2H) 7.51-7.57 (m, 2H) 8.71 (brs, 1H)

### (h) 7-(2-Chlorophenyl)-3-ethyl-8-(piperazin-1-yl)-3,7-dihydropurine-2,6-dione trifluoroacetate

8 mg of t-butyl 4-[7-(2-chlorophenyl)-1-(2,2-dimethylpropionyloxymethyl)-2,6-dioxo-2,3,6,7-tetrahydro-1H-pur in-8-yl] piperazine-1-carboxylate was dissolved in 0.3 mL of N,N-dimethylformamide, and 0.05 mL of iodoethane and 20 mg of potassium carbonate were then added. The resulting mixture was stirred at 50°C overnight. Ethyl acetate was added to the reaction solution, and the mixture was washed with water. The organic layer was concentrated. The residue was dissolved in methanol, and then 5 mg of sodium hydride was added. The mixture was stirred at room temperature for 3 hours. The reaction solution was neutralized with 1N hydrochloric acid, and then extracted with ethyl acetate. The solvent was concentrated. The residue was dissolved in trifluoroacetic acid, and then the solution was concentrated. The residue was purified by reverse phase high performance liquid chromatography to give 4.49 mg of the title compound.
MS *m*/*e* (ESI) 375(MH⁺-CF₃COOH)

### Example 386

### 7-(2-Chlorophenyl)-3-(2-oxo-2-phenethyl)-8-(piperazin-1-yl)-3,7-dihydropurine-2,6-dione trifluoroacetate

The title compound was obtained using phenacyl bromide by the same procedure described in Example 385-(h).
MS *m*/*e* (ESI) 465(MH⁺-CF₃COOH)

### Example 387

### 7-(2-Chlorophenyl)-3-(2-oxotetrahydrofuran-3-yl)-8-(piperazin-1-yl)-3,7-dihydropurine-2,6-dion e trifluoroacetate

### Example 388

### 2-[7-(2-Chlorophenyl)-2,6-dioxo-8-(piperazin-1-yl)-1,2,6,7-tetrahydropurine-3-yl]-4-hydroxybut ylic acid trifluoroacetate

7-(2-chlorophenyl)-3-(2-oxotetrahydrofuran-3-yl)-8-(piperazin-1-yl)-3,7-dihydropurine-2,6-dione trifluoroacetate [MS *m*/*e* (ESI) 431(MH⁺-CF₃COOH)] and 2-[7-(2-chlorophenyl)-2,6-dioxo-8-(piperazin-1-yl)-1,2,6,7-tetrahydropurine-3-yl]-4-hydroxybut ylic acid trifluoroacetate [MS *m*/*e* (ESI) 449(MH⁺-CF₃COOH)] were obtained using α-bromo-γ-butyrolactone by the same procedure described in Example 385-(h).

### Example 389

### 2-[7-(2-chlorophenyl)-2,6-dioxo-8-(piperazin-1-yl)-1,2,6,7-tetrahydropurin-3-yl] acetamide trifluoroacetate

The title compound was obtained using 2-bromoacetamide by the same procedure described in Example 385-(h).
¹H-NMR(d⁶-DMSO)
δ: 2.97-3.04 (m, 4H) 3.22-3.34 (m, 4H) 4.43 (s, 2H) 7.18 (brs, 1H) 7.49-7.59 (m, 2H) 7.62 (s, 1H) 7.66-7.71 (m, 2H) 10.90 (s, 1H)
MS *m*/*e* (ESI) 404(MH⁺-CF₃COOH)

### Example 390

### [7-(2-Chlorophenyl)-1-methyl-2,6-dioxo-8-(piperazin-1-yl)-1,2,6,7-tetrahydropurin-3-yl] acetic acid trifluoroacetate

### (a) t-Butyl 4-[7-(2-chlorophenyl)-3-carboxymethyl-1-methyl-2,6-dioxo-2,3,6,7-tetrahydro-1H-purin-8-yl] piperazine-1-carboxylate

87 mg of t-butyl 4-[7-(2-chlorophenyl)-3-methoxycarbonylmethyl-1-methyl-2,6-dioxo-2,3,6,7-tetrahydro-1H-puri n-8-yl] piperazine-1-carboxylate was dissolved in 2 mL of methanol, and 0.2 mL of an aqueous solution of 5N-sodium hydroxide was then added. The resulting mixture was stirred at room temperature for two hours, and then neutralized with 1N hydrochloric acid. The mixture was extracted with ethyl acetate. The organic layer was dried over anhydrous magnesium sulfate, and filtered. The solvent was distilled off to give the title compound.

### (b) [7-(2-Chlorophenyl)-1-methyl-2,6-dioxo-8-(piperazin-1-yl)-1,2,6,7-tetrahydropurin-3-yl] acetic acid trifluoroacetate

26 mg of t-butyl 4-[7-(2-chlorophenyl)-3-carboxymethyl-1-methyl-2,6-dioxo-2,3,6,7-tetrahydro-1H-purin-8-yl] piperazine-1-carboxylate was dissolved in trifluoroacetic acid, and the mixture was concentrated. The residue was purified by reverse phase high performance liquid chromatography to give 10.73 mg of the title compound.
¹H-NMR(d⁶-DMSO)
δ: 3.15-3.18 (m, 4H) 3.26 (s, 3H) 3.46-3.49 (m, 4H) 4.80 (s, 2H) 7.50-7.59 (m, 2H) 7.63-7.68 (m, 2H)
MS *m*/*e* (ESI) 419(MH⁺-CF₃COOH)

### Example 391

### 2-[7-(2-Chlorophenyl)-1-methyl-2,6-dioxo-8-(piperazin-1-yl)-1,2,6,7-tetrahydropurine-3-yl] acetamide trifluoroacetate

### (a) t-Butyl 4-[7-(2-chlorophenyl)-3-acetamide-1-methyl-2,6-dioxo-2,3,6,7-tetrahydro-1H-purin-8-yl] piperazine-1-carboxylate

53 mg of t-butyl 4-[7-(2-chlorophenyl)-3-carboxymethyl-1-methyl-2,6-dioxo-2,3,6,7-tetrahydro-1H-purin-8-yl] piperazine-1-carboxylate was dissolved in 1 mL of tetrahydrofuran, and then 0.03 mL of triethylamine and 0.015 mL of ethyl chlorocarbonate were added. The resulting mixture was stirred at room temperature for 15 minutes, and 0.1 mL of an aqueous solution of 30% ammonia was then added. The reaction solution was diluted with ethyl acetate, and washed with water and 1N hydrochloric acid. The organic layer was dried over anhydrous magnesium sulfate and filtered. The solvent was distilled off to give 53 mg of the title compound.

### (b) 2-[7-(2-Chlorophenyl)-1-methyl-2,6-dioxo-8-(piperazin-1-yl)-1,2,6,7-tetrahydropurin-3-yl] acetamide trifluoroacetate

53 mg of t-butyl 4-[7-(2-chlorophenyl)-3-acetamide-1-methyl-2,6-dioxo-2,3,6,7-tetrahydro-1H-purin-8-yl] piperazine-1-carboxylate was dissolved in trifluoroacetic acid, and the solution was concentrated. The residue was purified by reverse phase high performance liquid chromatography to give 23.31 mg of the title compound.
¹H-NMR(d⁶-DMSO)
δ: 3.15-3.18 (m, 4H) 3.26 (s, 3H) 3.45-3.48 (m, 4H) 4.76 (s, 2H) 7.50-7.59 (m, 2H) 7.62-7.68 (m, 2H)
MS *m*/*e* (ESI) 418(MH⁺-CF₃COOH)

### Example 392

### [7-(2-Chlorophenyl)-2,6-dioxo-8-(piperazin-1-yl)-1,2,6,7-tetrahydropurine-3-yl] acetic acid trifluoroacetate

The title compound was obtained using t-butyl 4-[7-(2-chlorophenyl)-3-methoxycarbonylmethyl-2,6-dioxo-2,3,6,7-tetrahydro-1H-purin-8-yl] piperazine-1-carboxylate by the same procedure described in Example 390-(a) and (b).
MS *m*/*e* (ESI) 405(MH⁺-CF₃COOH)

### Example 393

### [7-(2-Chlorophenyl)-2,6-dioxo-1-phenethyl-8-(piperazin-1-yl)-1,2,6,7-tetrahydropurin-3-yl] acetic acid trifluoroacetate

The title compound was obtained using t-butyl 4-[7-(2-chlorophenyl)-3-methoxycarbonylmethyl-2,6-dioxo-1-phenethyl-2,3,6,7-tetrahydro-1H-p urin-8-yl] piperazine-1-carboxylate by the same procedure described in Example 390-(a) and (b).
MS *m*/*e* (ESI) 509(MH⁺-CF₃COOH)

### Example 394

### 2-[7-(2-Chlorophenyl)-2,6-dioxo-1-phenethyl-8-(piperazin-1-yl)-1,2,6,7-tetrahydropurin-3-yl] acetamide trifluoroacetate

The title compound was obtained using t-butyl 4-[7-(2-chlorophenyl)-3-carboxymethyl-2,6-dioxo-1-phenethyl-2,3,6,7-tetrahydro-1H-purin-8-yl ] piperazine-1-carboxylate by the same procedure described in Example 391-(a) and (b).
MS *m*/*e* (ESI) 508(MH⁺-CF₃COOH)

### Example 395

### [7-(2-Chlorophenyl)-1-methyl-8-(piperazin-1-yl)]-3,7-dihydropurine-2,6-dione trifluoroacetate

### (a) [7-Benzyl-2,6-dioxo-1,2,6,7-tetrahydropurin-3-yl] methyl 2,2-dimethylpropionate

8.66 g of 7-benzyl xanthine was dissolved in 300 mL of N,N-dimethylformamide, and then 1.57 g of sodium hydride and 7.7 mL of chloromethylpivalate were added. The resulting mixture was stirred at room temperature overnight. The reaction solution was diluted with ethyl acetate, and then washed with water and 1N hydrochloric acid. The organic layer was dried over anhydrous magnesium sulfate, and filtered. The solvent was distilled off. The residue was purified by silica gel column chromatography. Thus, 2.66 g of the title compound was obtained from a fraction eluted with 1:1 hexane-ethyl acetate.
¹H-NMR(CDCl₃)
δ: 1.18 (s, 9H) 5.45 (s, 2H) 6.06 (s, 2H) 7.34-7.39 (m, 5H) 7.58 (s, 1H) 8.18 (s, 1H)

### (b) [7-Benzyl-1-methyl-2,6-dioxo-1,2,6,7-tetrahydropurin-3-yl] methyl 2,2-dimethylpropionate

2.66 g of [7-benzyl-2,6-dioxo-1,2,6,7-tetrahydropurin-3-yl] methyl 2,2-dimethylpropionate was dissolved in 30 mL of N,N-dimethylformamide, and then 1.6 g of potassium carbonate and 1 mL of iodomethane were added. The resulting mixture was stirred at room temperature overnight. The reaction solution was diluted with ethyl acetate, and washed with water and 1N hydrochloric acid. The organic layer was dried over anhydrous magnesium sulfate, and filtered. The solvent was distilled off. The residue was triturated with toluene to give 2.16 g of the title compound.
¹H-NMR(CDCl₃)
δ: 1.18 (s, 9H) 3.41 (s, 3H) 5.49 (s, 2H) 6.11 (s, 2H) 7.26-7.39 (m, 5H) 7.57 (s, 1H)

### (c) [1-Methyl-2,6-dioxo-1,2,6,7-tetrahydropurin-3-yl] methyl 2,2-dimethylpropionate

2.16 g of the title compound was obtained using [7-benzyl-1-methyl-2,6-dioxo-1,2,6,7-tetrahydropurine-3-yl] methyl 2,2-dimethylpropionate by the same procedure described in Example 385-(d).
¹H-NMR(CDCl₃)
δ: 1.19 (s, 9H) 3.48 (s, 3H) 6.17 (s, 2H) 7.83 (s, 1H)

### (d) [7-(2-Chlorophenyl)-1-methyl-2,6-dioxo-1,2,6,7-tetrahydropurine-3-yl] methyl 2,2-dimethylpropionate

The title compound was obtained using [1-methyl-2,6-dioxo-1,2,6,7-tetrahydropurine-3-yl] methyl 2,2-dimethylpropionate by the same procedure described in Example 385-(e).

### (e) t-Butyl 4-[7-(2-chlorophenyl)-3-(2,2-dimethyl-propionyloxymethyl)-1-methyl-2,6-dioxo-2,3,6,7-tetrahy dro-1H-purin-8-yl] piperazine-1-carboxylate

The title compound was obtained using [7-(2-chlorophenyl)-1-methyl-2,6-dioxo-1,2,6,7-tetrahydropurine-3-yl] methyl 2,2-dimethylpropionate by the same procedure described in Example 385-(f).

### (f) t-Butyl 4-[7-(2-chlorophenyl)-1-methyl-2,6-dioxo-2,3,6,7-tetrahydro-1H-purin-8-yl] piperazine-1-carboxylate

The title compound was obtained using t-butyl 4-[7-(2-chlorophenyl)-3-(2,2-dimethyl-propionyloxymethyl)-1-methyl-2,6-dioxo-2,3,6,7-tetrahydro-1H-purin-8-yl] piperazine-1-carboxylate by the same procedure described in Example 373-(e).
¹H-NMR(d⁶-DMSO)
δ: 1.35 (s, 9H) 3.04 (s, 3H) 3.06-3.12 (m, 4H) 3.17-3.22 (m, 4H) 7.48 (dt, J=1.6, 7.6Hz, 1H) 7.53 (dt, J=2.0, 7.6Hz, 1H) 7.63 (dd, J=2.0, 8.0Hz, 1H) 7.65 (dd, J=1.6, 8.0Hz, 1H)

### (g) 7-(2-Chlorophenyl)-1-methyl-8-(piperazin-1-yl)-3,7-dihydropurine-2,6-dione trifluoroacetate

The title compound was obtained using t-butyl 4-[7-(2-chlorophenyl)-1-methyl -2,6-dioxo-2,3,6,7-tetrahydro-1H-purin-8-yl] piperazine-1-carboxylate by the same procedure described in Example 391-(b).
¹H-NMR(d⁶-DMSO)
δ: 2.95-3.03 (m, 4H) 3.14 (s, 3H) 3.23-3.34 (m, 4H) 7.49-7.62 (m, 2H) 7.66-7.71 (m, 2H) 10.90 (s, 1H)
MS *m*/*e* (ESI) 361(MH⁺-CF₃COOH)

### Example 396

### 7-(2-Butynyl)-3-ethyl-1-methyl-8-(piperazin-1-yl)-3,7- dihydropurine-2,6-dione trifluoroacetate

### (a) [7-(2-Butynyl)-1-methyl-2,6-dioxo-1,2,6,7-tetrahydropurin-3-yl] methyl 2,2-dimethylpropionate

1.871 g of [1-methyl-2,6-dioxo-1,2,6,7-tetrahydropurin-3-yl] methyl 2,2-dimethylpropionate was dissolved in 30 mL of N,N-dimethylformamide, and then 1.5 g of potassium carbonate and 0.7 mL of 2-butynyl bromide were added. The resulting mixture was stirred at room temperature overnight. The reaction solution was diluted with ethyl acetate, and washed with water and 1N hydrochloric acid. The organic layer was dried over anhydrous magnesium sulfate, and filtered. The solvent was distilled off. The residue was purified by silica gel column chromatography. Thus, 2.12 g of the title compound was obtained from a fraction eluted with 3:2 hexane-ethyl acetate.

### (b) 7-(2-Butynyl)-1-methyl-3,7-dihydropurine-2,6-dione

The title compound was obtained using [7-(2-butynyl)-1-methyl-2,6-dioxo-1,2,6,7-tetrahydropurine-3-yl] methyl 2,2-dimethylpropionate by the same procedure described in Example 395-(f).
¹H-NMR(CDCl₃)
δ: 1.91 (t, J=2.4Hz, 3H) 3.39 (s, 3H) 5.10 (s, 2H) 7.93 (s, 1H) 10.62 (s, 1H)

### (c) t-Butyl 4-[7-(2-butynyl)-1-methyl-2,6-dioxo-2,3,6,7-tetrahydro-1H-purin-8-yl] piperazine-1-carboxylate

The title compound was obtained using 7-(2-butynyl)-1-methyl-3,7-dihydropurine-2,6-dione by the same procedure described in Example 395-(e).
¹H-NMR(CDCl₃)
δ: 1.48 (s, 9H) 1.83 (t, J=2.4Hz, 3H) 3.37 (s, 3H) 3.37-3.39 (m, 4H) 3.58-3.60 (m, 4H) 4.87 (s, 2H) 9.68 (s, 1H)

### (d) 7-(2-Butynyl)-3-ethyl-1-methyl-8-(piperazin-1-yl-3,7-dihydropurine-2,6-dione trifluoroacetate

The title compound was obtained using t-butyl 4-[7-(2-butynyl)-1-methyl-2,6-dioxo-2,3,6,7-tetrahydro-1H-purin-8-yl] piperazine-1-carboxylate by the same procedure described in Example 385-(h).
MS *m*/*e* (ESI) 331(MH⁺-CF₃COOH)

### Example 397

### 7-(2-Butynyl)-3-benzyl-1-methyl-8-(piperazin-1-yl)-3,7-dihydropurine-2,6-dione trifluoroacetate

The title compound was obtained using benzyl bromide by the same procedure described in Example 396-(d).
¹H-NMR(CDCl₃)
δ: 1.83 (t, J=2.4Hz, 3H) 3.03-3.06 (m, 4H) 3.38 (s, 3H) 3.38-3.41 (m, 4H) 4.84 (q, J=2.4Hz, 2H) 5.21 (s, 2H) 7.26-7.30 (m, 3H) 7.52-7.54 (m, 2H)
MS *m*/*e* (ESI) 393(MH⁺-CF₃COOH)

### Example 398

### Methyl [7-(2-butynyl)-1-methyl-2,6-dioxo-8-(piperazin-1-yl)-1,2,6,7-tetrahydropurin-3-yl] acetate trifluoroacetate

The title compound was obtained using methyl bromoacetate by the same procedure described in Example 396-(d).
¹H-NMR(CDCl₃)
δ: 1.84 (t, J=2.4Hz, 3H) 3.00-3.03 (m, 4H) 3.34-3.36 (m, 4H) 3.40 (s, 3H) 3.79 (s, 3H) 4.78 (s, 2H) 4.84 (q, J=2.4Hz, 2H)
MS *m*/*e* (ESI) 375(MH⁺-CF₃COOH)

### Example 399

### 7-(2-Butynyl)-3-cyclobutyl-1-methyl-8-(piperazin-1-yl)-3,7-dihydropurine-2,6-dione trifluoroacetate

8 mg of t-butyl 4-[7-(2-butynyl)-1-methyl-2,6-dioxo-2,3,6,7-tetrahydro-1H-purin-8-yl] piperazine-1-carboxylate was dissolved in 0.4 mL of N,N-dimethylformamide, and then 10 mg of potassium carbonate and 0.01 mL of cyclobutyl bromide were added. The resulting mixture was stirred at 50°C overnight. The reaction solution was diluted with ethyl acetate. The organic layer was concentrated. The residue was dissolved in trifluoroacetic acid and the solution was concentrated. The residue was purified by reverse phase high performance liquid chromatography, to give 3.72 mg of the title compound.
MS *m*/*e* (ESI) 357(MH⁺-CF₃COOH)

### Example 400

### 7-(2-Butynyl)-3-(2-tetrahydrofuranyl)methyl-1-methyl-8-(piperazin-1-yl)-3,7-dihydropurine-2,6-dione trifluoroacetate

The title compound was obtained using 2-bromomethyl tetrahydrofuran by the same procedure described in Example 399.
¹H-NMR(CDCl₃)
δ: 1.70-1.77 (m, 1H) 1.84 (t, J=2.4Hz, 3H) 1.88-1.93 (m, 1H) 1.97-2.06 (m, 2H) 3.01-3.04 (m, 4H) 3.34-3.36 (m, 4H) 3.39 (s, 3H) 3.77 (dd, J=8.4, 14.0Hz, 1H) 3.92-3.97 (m, 2H) 4.19 (dd, J=8.4, 13.6Hz, 1H) 4.45-4.50 (m, 1H) 4.83 (q, J=2.4Hz, 2H)
MS *m*/*e* (ESI) 387(MH⁺-CF₃COOH)

### Example 401

### 2-[7-(2-Butynyl)-1-methyl-2,6-dioxo-8-(piperazin-1-yl)-1,2,6,7-tetrahydropurin-3-yl] acetamide trifluoroacetate

The title compound was obtained using 2-bromoacetamide by the same procedure described in Example 399.
¹H-NMR(CDCl₃)
δ: 1.68 (t, J=2.4Hz, 3H) 3.15-3.19 (m, 4H) 3.23 (s, 3H) 3.46-3.51 (m, 4H) 4.55 (s, 2H) 4.71 (q, J=2.4Hz, 2H) 6.00 (br, 1H) 6.91 (br, 1H)
MS *m*/*e* (ESI) 360(MH⁺-CF₃COOH)

### Example 402

### Methyl [7-(2-butynyl)-1-methyl-2,6-dioxo-8-(piperazin-1-yl)-1,2,6,7-tetrahydropurin-3-yl] phenylacetate trifluoroacetate

The title compound was obtained using methyl 2-bromophenyl acetate by the same procedure described in Example 399.
¹H-NMR(CDCl₃)
δ: 1.83 (t, J=2.4Hz, 3H) 3.02-3.05 (m, 4H) 3.36-3.38 (m, 4H) 3.37 (s, 3H) 3.80 (s, 3H) 4.82 (q, J=2.4Hz, 2H) 6.50 (s, 1H) 7.30-7.32 (m, 3H) 7.65-7.67 (m, 2H)
MS *m*/*e* (ESI) 451(MH⁺-CF₃COOH)

### Example 403

### 7-(2-Butynyl)-3-propyl-1-methyl-8-(piperazin-1-yl)-3,7-dihydropurine-2,6-dione trifluoroacetate

The title compound was obtained using iodopropane by the same procedure described in Example 399.
MS *m*/*e* (ESI) 345(MH⁺-CF₃COOH)

### Example 404

### 7-(2-Butynyl)-3-(2-oxo-2-phenethyl)-1-methyl-8-(piperazin-1-yl)-3,7-dihydropurine-2,6-dione trifluoroacetate

The title compound was obtained using phenacyl bromide by the same procedure described in Example 399.
¹H-NMR(CDCl₃)
δ: 1.85 (t, J=2.4Hz, 3H) 2.96-2.99 (m, 4H) 3.28-3.31 (m, 4H) 3.41 (s, 3H) 4.85 (q, J=2.4Hz, 2H) 5.48 (s, 2H) 7.50-7.54 (m, 2H) 7.61-7.65 (m, 1H) 8.02-8.05 (m, 2H)
MS *m*/*e* (ESI) 421 (MH⁺-CF₃COOH)

### Example 405

### Ethyl 2-[7-(2-butynyl)-1-methyl-2,6-dioxo-8-(piperazin-1-yl)-1,2,6,7-tetrahydropurin-3-yl] propionate trifluoroacetate

The title compound was obtained using ethyl 2-bromopropionate by the same procedure described in Example 399.
¹H-NMR(CDCl₃)
δ: 1.23 (t, J=7.2Hz, 3H) 1.70 (d, J=7.2Hz, 3H) 1.84 (t, J=2.4Hz, 3H) 3.00-3.03 (m, 4H) 3.33-3.37 (m, 4H) 3.38 (s, 3H) 4.15-4.25 (m, 2H) 4.85 (q, J=2.4Hz, 2H) 5.43 (q, J=7.2Hz, 1H)
MS *m*/*e* (ESI) 403(MH⁺-CF₃COOH)

### Example 406

### 7-(2-Butynyl)-3-(2-oxo-tetrahydrofuran-3-yl)-1-methyl-8-(piperazin-1-yl)-3,7-dihydropurine-2,6 -dione trifluoroacetate

The title compound was obtained using α-bromo-γ-butyrolactone by the same procedure described in Example 399.
¹H-NMR(CDCl₃)
δ: 1.84 (t, J=2.4Hz, 3H) 2.59-2.68 (m, 1H) 2.69-2.91 (m, 1H) 3.01-3.03 (m, 4H) 3.34-3.37 (m, 5H) 3.38 (s, 3H) 4.39-4.45 (m, 1H) 4.68 (dt, J=2.8, 9.2Hz, 2H) 4.84 (br, 2H)
MS *m*/*e* (ESI) 387(MH⁺-CF₃COOH)

### Example 407

### 7-(2-Butynyl)-3-(2-ethoxyethyl)-1-methyl-8-(piperazin-1-yl)-3,7-dihydropurine-2,6-dione trifluoroacetate

The title compound was obtained using 2-ethoxyethyl bromide by the same procedure described in Example 399.
¹H-NMR(CDCl₃)
δ: 1.16 (t, J=7.2Hz, 3H) 1.83 (t, J=2.4Hz, 3H) 3.01-3.06 (m, 4H) 3.33-3.46 (m, 4H) 3.39 (s, 3H) 3.58 (q, J=7.2Hz, 2H) 3.77 (t, J=6.0Hz, 2H) 4.26 (t, J=6.0Hz, 2H) 4.85 (q, J=2.4Hz, 2H)
MS *m*/*e* (ESI) 375(MH⁺-CF₃COOH)

### Example 408

### 7-(2-Butynyl)-3-isopropyl-1-methyl-8-(piperazin-1-yl)-3,7-dihydropurine-2,6-dione trifluoroacetate

The title compound was obtained using 2-iodopropane by the same procedure described in Example 399.
MS *m*/*e* (ESI) 345 (MH⁺-CF₃COOH)

### Example 409

### 7-(2-Butynyl)-3-(3,3-dimethyl-2-oxobutyl)-1-methyl-8-(pinerazin-1-yl)-3,7-dihydropurine-2,6-di one trifluoroacetate

The title compound was obtained using 1-bromopinacolone by the same procedure described in Example 399.
MS *m*/*e* (ESI) 401(MH⁺-CF₃COOH)

### Example 410

### 7-(2-Butynyl)-1-methyl-3-(2-oxopyrrolidin-3-yl)-8-(piperazin-1-yl)-3,7-dihydropurine-2,6-dione hydrochloride

The title compound was obtained using 3-bromo-2-oxopyrrolidine by the same procedure described in Example 399.
¹H-NMR(d6-DMSO)
δ: 1.80 (t, J=2Hz, 3H) 2.32-2.48 (m, 2H) 3.17 (s, 3H) 3.20-3.55 (m, 10H) 4.96 (q, J=2Hz, 2H) 5.14 (t, J=10Hz) 7.94 (brs, 1H) 9.04 (brs, 2H)

### Example 411

### 7-(2-Butynyl)-3-(2-ethoxyethyl)-8-(piperazin-1-yl)-3,7-dihydropurine-2,6-dione trifluoroacetate

### (a) t-Butyl 4-[7-(2-butynyl)-1,3-bis-(2,2-dimethylpropionyloxymethyl)-2,6-dioxo-2,3,6,7-tetrahydro-1H-pur in-8-yl] piperazine-1-carboxylate

A mixture containing 1.0 g of [3-(2,2-dimethylpropionyloxymethyl)-2,6-dioxo-2,3,6,7-tetrahydropurin-1-yl] methyl 2,2-dimethylpropionate, 0.28 mL of 1-bromo-2-butyne, 0.73 g of anhydrous potassium carbonate, and 15 mL of N,N-dimethylformamide was stirred at room temperature for two hours. The reaction solution was extracted with ethyl acetate-water. The organic layer was washed with water and saturated sodium chloride solution, and then dried over anhydrous magnesium sulfate. The liquid was concentrated under reduced pressure. The residue was purified by silica gel column chromatography using 20-30% ethyl acetate/hexane, to give 1.06 g of [7-(2-butynyl)-3-(2,2-dimethylpropionyloxymethyl)-2,6-dioxo-2,3,6,7-tetrahydropurin-1-yl] methyl 2,2-dimethylpropionate.

The whole quantity of the compound was combined with 390 mg of N-chlorosuccinimide and 5 mL of N,N-dimethylformamide. The mixture was stirred at room temperature for one hour. The reaction solution was extracted with ethyl acetate-water. The organic layer was washed with water and saturated sodium chloride solution, and then dried over anhydrous magnesium sulfate. The liquid was concentrated under reduced pressure. The residue was purified by silica gel column chromatography using 20-30% ethyl acetate/hexane to give 1.18 g of [7-(2-butynyl)-8-chloro-3-(2,2-dimethylpropionyloxymethyl)-2,6-dioxo-2,3,6,7-tetrahydropurin-1-yl] methyl 2,2-dimethylpropionate.

The whole quantity of the compound was combined with 1.4 g of t-butyl piperazine-1-carboxylate, and the mixture was stirred at 150°C in an oil bath while being stirred for 30 minutes. The reaction solution was purified by silica gel column chromatography using 20-30% ethyl acetate/hexane to give 1.34 g of the title compound.
¹H-NMR(CDCl₃)
δ: 1.18 (s, 18H) 1.49 (s, 9H) 1.84 (t, J=2Hz, 3H) 3.36 (t, J=5Hz, 4H) 3.58 (t, J=5Hz) 4.86 (q, J=2Hz, 2H) 6.02 (s, 2H), 6.03 (s, 2H)

### (b) t-Butyl 4-[7-(2-butynyl)-1-(2,2-dimethylpropionyloxymethyl)-2,6-dioxo-2,3,6,7-tetrahydro-1H-purin-8-yl] piperazine-1-carboxylate

0.63 g of t-butyl 4-[7-(2-butynyl)-1,3-bis-(2,2-dimethylpropionyloxymethyl)-2,6-dioxo-2,3,6,7-tetrahydro-1H-pur in-8-yl] piperazine-1-carboxylate was dissolved in a mixed solvent of 4 mL of tetrahydrofuran and 2 mL of methanol, and then 0.18 mL of diazabicyclo[5.4.0]undecene was added. The resulting mixture was stirred at room temperature overnight. The reaction solution was concentrated. The residue was purified by silica gel column chromatography. Thus, 0.29 g of the title compound was obtained from a fraction eluted with hexane-ethyl acetate (1:5).
¹H-NMR(CDCl₃)
δ: 1.19 (s, 9H) 1.48 (s, 9H) 1.83 (t, J=2.4Hz, 3H) 3.37-3.39 (m, 4H) 3.58-3.60 (m, 4H) 4.86 (q, J=2.4Hz, 2H) 6.00 (s, 2H) 9.08 (s, 1H)

### (c) 7-(2-Butynyl)-3-(2-ethoxyethyl)-8-(piperazin-1-yl)-3,7-dihydropurine-2,6-dione trifluoroacetate

50 mg oft-butyl 4-[7-(2-butynyl)-1-(2,2-dimethylpropionyloxymethyl)-2,6-dioxo-2,3,6,7-tetrahydro-1H-purin-8-yl] piperazine-1-carboxylate and 15 mg of potassium carbonate were dissolved in 1.2 mL N,N-dimethylformamide, and then 12 µL of 2-bromoethyl ethyl ether was added. The resulting mixture was stirred at 60°C for two hours, and then diluted with ethyl acetate and washed with water. The liquid was dried over anhydrous magnesium sulfate. The organic layer was concentrated. The residue was purified by silica gel column chromatography. Thus, t-butyl 4-[7-(2-butynyl)-1-(2,2-dimethylpropionyloxymethyl)-3-(2-ethoxyethyl)-2,6-dioxo-2,3,6,7-tetrah ydro-1H-purin-8-yl] piperazine-1-carboxylate was obtained from a fraction eluted with hexane-ethyl acetate (2:1). Then, the resulting t-butyl 4-[7-(2-butynyl)-1-(2,2-dimethylpropionyloxymethyl)-3-(2-ethoxyethyl)-2,6-dioxo-2,3,6,7-tetrah ydro-1H-purin-8-yl] piperazine-1-carboxylate was dissolved in a mixed solvent of 1.0 mL of tetrahydrofuran and 0.5 mL of methanol, and then 5 mg of sodium hydride was added. The resulting mixture was stirred at room temperature for 1 hour. The reaction solution was neutralized with 2N hydrochloric acid, and extracted with ethyl acetate. Then, the organic layer was dried over anhydrous magnesium sulfate. The solvent was distilled off to give t-butyl 4-[7-(2-butynyl)-3-(2-ethoxyethyl)-2,6-dioxo-2,3,6,7-tetrahydro-1H-purin-8-yl] piperazine-1-carboxylate. A 1/4 equivalent of the resulting t-butyl 4-[7-(2-butynyl)-3-(2-ethoxyethyl)-2,6-dioxo-2,3,6,7-tetrahydro-1H-purin-8-yl] piperazine-1-carboxylate was dissolved in 0.5 mL of trifluoroacetic acid, and the mixture was stirred at room temperature for 30 minutes. The solvent was distilled off. Then, a half aliquot of the residue was purified by HPLC using a reverse-phase column with water-acetonitrile-trifluoroacetic acid as the solvent for elution, to give 3.2 mg of the title compound.
MS *m*/*e* (ESI) 361(MH⁺-CF₃COOH)

### Example 412

### Methyl [7-(2-butynyl)-3-(2-ethoxyethyl)-2,6-dioxo-8-(piperazin-1-yl)-2,3,6,7-tetrahydropurin-1-yl] acetate trifluoroacetate

A 1/4 equivalent of t-butyl 4-[7-(2-butynyl)-3-(2-ethoxyethyl)-2,6-dioxo-2,3,6,7-tetrahydro-1H-purin-8-yl] piperazine-1-carboxylate obtained in Example 411-(c) and 7 mg of potassium carbonate were dissolved in 0.8 mL of N,N-dimethylformamide, and 10 µL of methyl bromoacetate was then added. The resulting mixture was stirred at room temperature overnight, and then diluted with ethyl acetate and washed with water. The liquid was dried over anhydrous magnesium sulfate. The organic layer was concentrated, and then the residue was dissolved in 0.5 mL of trifluoroacetic acid. The mixture was stirred at room temperature for 30 minutes. The solvent was distilled off, and a half aliquot of the residue was purified by HPLC using a reverse-phase column with water-acetonitrile-trifluoroacetic acid as the elution solvent, to give 3.2 mg of the title compound.
MS *m*/*e* (ESI) 433(MH⁺-CF₃COOH)

### Example 413

### 7-(2-Butynyl)-3-(2-ethoxyethyl)-1-(2-oxo-2-phenylethyl-8-(piperazin-1-yl)-3,7-dihydropurine-2, 6-dione trifluoroacetate

The title compound was obtained using 2-bromoacetophenone by the same procedure described in Example 412.
MS *m*/*e* (ESI) 479(MH⁺-CF₃COOH)

### Example 414

### Methyl [7-(2-butynyl)-1-(2-ethoxyethyl)-2,6-dioxo-8-(piperazin-1-yl)-1,2,6,7-tetrahydropurin-3-yl] acetate trifluoroacetate

### (a) t-butyl 4-[7-(2-butynyl)-3-(2,2-dimethylpropionyloxymethyl)-2,6-dioxo-2,3,6,7-tetrahydro-1H-purin-8-yl] piperazine-1-carboxylate

1.1 g oft-butyl 4-[7-(2-butynyl)-2,6-dioxo-2,3,6,7-tetrahydro-1H-purin-8-yl] piperazine-1-carboxylate and 0.43 g of potassium carbonate were dissolved in 15 mL of N,N-dimethylformamide. Then, 0.60 mL of chloromethylpivalate was added to the mixture on ice. The resulting mixture was stirred at room temperature overnight, and then diluted with ethyl acetate and washed with water. The resulting insoluble white solid was collected by filtration and washed with a mixed solution of hexane and ethyl acetate (1:1), to give 0.57 g of the title compound.
¹H-NMR(CDCl₃)
δ: 1.18 (s, 9H) 1.49 (s, 9H) 1.83 (t, J=2.4Hz, 3H) 3.33-3.36 (m, 4H) 3.57-3.59 (m, 4H) 4.84 (q, J=2.4Hz, 2H) 5.99 (s, 2H) 7.72 (s, 1H)

### (b) Methyl [7-(2-butynyl)-1-(2-ethoxyethyl)-2,6-dioxo-8-(piperazin-1-yl)-1,2,6,7-tetrahydropurine-3-yl] acetate trifluoroacetate

40 mg of t-butyl 4-[7-(2-butynyl)-3-(2,2-dimethylpropionyloxymethyl)-2,6-dioxo-2,3,6,7-tetrahydro-1H-purin-8-yl] piperazine-1-carboxylate and 17 mg of potassium carbonate were dissolved in 1.5 mL of N,N-dimethylformamide, and then 14 µL of 2-bromoethyl ethyl ether was added. The resulting mixture was stirred at 60°C for 5 hours, and then diluted with ethyl acetate and washed with water. The liquid was dried over anhydrous magnesium sulfate. The solvent was distilled off, and the residue was purified by silica gel column chromatography. Thus, t-butyl 4-[7-(2-butynyl)-3-(2,2-dimethylpropionyloxymethyl)-1-(2-ethoxyethyl)-2,6-dioxo-2,3,6,7-tetrah ydro-1H-purin-8-yl] piperazine-1-carboxylate was obtained from a fraction eluted with hexane-ethyl acetate (1:1). Then, the resulting t-butyl 4-[7-(2-butynyl)-3-(2,2-dimethylpropionyloxymethyl)-1-(2-ethoxyethyl)-2,6-dioxo-2,3,6,7-tetrah ydro-1H-purin-8-yl] piperazine-1-carboxylate was dissolved in a mixed solvent of 1.0 mL of tetrahydrofuran and 0.5 mL of methanol, and 5 mg of sodium hydride was then added. The resulting mixture was stirred at room temperature for 1 hour. The reaction solution was neutralized with 2N hydrochloric acid, and extracted with ethyl acetate. Then, the organic layer was dried over anhydrous magnesium sulfate, and the solvent was distilled off. The resulting residue was dissolved in 1 mL of N,N-dimethylformamide, and 10 mg of potassium carbonate and 10 µL of methyl bromoacetate were then added. The resulting mixture was stirred at room temperature for 2 hours, and then diluted with ethyl acetate and washed with water. The organic layer was concentrated, and the residue was dissolved in 0.5 mL of trifluoroacetic acid. The resulting mixture was stirred at room temperature for 30 minutes. The solvent was distilled off, and then a half aliquot of the residue was purified by HPLC using a reverse-phase column with water-acetonitrile-trifluoroacetic acid as the elution solvent, to give 6.2 mg of the title compound.
MS *m*/*e* (ESI) 433(MH⁺-CF₃COOH)

### Example 415

### Methyl [7-(2-butynyl)-2,6-dioxo-1-(2-oxo-2-phenylethyl)-8-(piperazin-1-yl)-1,2,6,7-tetrahydropurin-3-y l] acetate trifluoroacetate

The title compound was obtained using 2-bromoacetophenone by the same procedure described in Example 414.
MS *m*/*e* (ESI) 479(MH⁺-CF₃COOH)

### Example 416

### Ethyl [7-(2-butynyl)-2,6-dioxo-1-(2-phenethyl)-8-(piperazin-1-yl)-1,2,6,7-tetrahydropurin-3-yl] acetate hydrochloride

### (a) Ethyl (7-benzyl-2,6-dioxo-1,2,6,7-tetrahydropurine-3-yl) acetate

A mixture containing 3.0 g of 7-benzyl-3,7-dihydropurine-2,6-dione, 2.0 g of anhydrous potassium carbonate, and 60 mL of N,N-dimethylformamide was stirred at 40°C in an oil bath, and 1.5 g of ethyl bromoacetate was then added. The resulting mixture was stirred for four hours at 40°C. The reaction solution was diluted with ethyl acetate and water, and extracted with ethyl acetate. The organic layer was washed with water and saturated sodium chloride solution, and then dried over anhydrous magnesium sulfate. The liquid was concentrated under reduced pressure. The residue was purified by silica gel column chromatography using 20-40% (20% 2-propanol/ethyl acetate)/hexane to give 1.3 g of the title compound.
¹H-NMR(CDCl₃)
δ: 1.28 (t, J=7Hz, 3H) 4.23 (q, J=7Hz, 2H) 4.78 (s, 2H) 5.04 (s, 2H) 7.31-7.39 (m, 5H) 7.51 (s, 1H) 8.01 (br.s, 1H)

### (b) Ethyl [7-benzyl-1-(2-phenylethyl)-2,6-dioxo-1,2,6,7-tetrahydropurine-3-yl] acetate

A mixture containing 300 mg of ethyl (7-benzyl-2,6-dioxo-1,2,6,7-tetrahydropurine-3-yl) acetate, 250 mg of anhydrous potassium carbonate, 0.25 mL of 2-bromoethyl benzene, and 5 mL of N,N-dimethylformamide was stirred at 50°C in an oil bath for two hours. The reaction solution was diluted with ethyl acetate and water, and extracted with ethyl acetate. The organic layer was washed with water and saturated sodium chloride solution, and then dried over anhydrous magnesium sulfate. The liquid was concentrated under reduced pressure. The residue was purified by silica gel column chromatography using 10-20% (20% 2-propanol/ethyl acetate)/hexane to give 366 mg of the title compound.
¹H-NMR(CDCl₃)
δ: 1.29 (t, J=7Hz, 3H) 2.95 (t, J=8Hz, 2H) 4.22 (t, J=8Hz, 2H) 4.24 (q, J=7Hz, 2H) 4.83 (s, 2H) 5.48 (s, 2H) 7.17-7.39 (m, 10H) 7.49 (s, 1H)

### (c) Ethyl [7-(2-butynyl)-8-chloro-1-(2-phenylethyl)-2,6-dioxo-1,2,6,7-tetrahydropurine-3-yl] acetate

A catalytic amount of 10% palladium carbon was added to a mixture containing 366 mg of ethyl [7-benzyl-1-(2-phenylethyl)-2,6-dioxo-1,2,6,7-tetrahydropurine-3-yl] acetate and 10 mL acetic acid. The resulting mixture was stirred under a hydrogen atmosphere at room temperature overnight. After the catalyst was removed by filtration, the liquid was concentrated under reduced pressure to give 320 mg of residue. The whole quantity of the concentrated residue was combined with 260 mg of anhydrous potassium carbonate, 0.1 mL of 1-bromo-2-butyne, and 5 mL of N,N-dimethylformamide. The resulting mixture was stirred at room temperature for two hours. The reaction solution was diluted with ethyl acetate and water, and extracted with ethyl acetate. The organic layer was washed with water and saturated sodium chloride solution, and then dried over anhydrous magnesium sulfate. The liquid was concentrated under reduced pressure. The residue was purified by silica gel column chromatography using 20-30% ethyl acetate/hexane to give 290 mg of an oily material. The whole quantity of the oily material was combined with 3 mL of N,N-dimethylformamide and 120 mg of N-chlorosuccinimide. The resulting mixture was stirred at room temperature for one hour. The reaction solution was extracted with ethyl acetate and water. The organic layer was washed with water and saturated sodium chloride solution, and then dried over anhydrous magnesium sulfate. The liquid was concentrated under reduced pressure. The residue was purified by silica gel column chromatography using 20-30% ethyl acetate/hexane, to give 273 mg of the title compound.
¹H-NMR(CDCl₃)
δ: 1.31 (t, J=7Hz, 3H) 1.82 (t, J=2Hz, 3H) 2.94 (t, J=8Hz, 2H) 4.21 (t, J=8Hz, 2H) 4.25 (q, J=7Hz, 2H) 4.78 (s, 2H) 5.09 (q, J=2Hz, 2H) 7.19-7.24 (m, 1H), 7.26-7.33 (m, 4H)

### (d) t-Butyl 4-[7-(2-butynyl)-3-ethoxycarbonylmethyl-1-(2-phenylethyl)-2,6-dioxo-1,2,6,7-tetrahydropurin-8 -yl] piperazine-1-carboxylate

A mixture containing 273 mg of ethyl [7-(2-butynyl)-8-chloro-1-(2-phenylethyl)-2,6-dioxo-1,2,6,7-tetrahydropurin-3-yl] acetate and 360 mg of t-butyl piperazine-1-carboxylate was heated at 150°C in an oil bath for 30 minutes. The reaction solution was purified by silica gel column chromatography using 20-30% ethyl acetate/hexane to give 320 mg of the title compound.
¹H-NMR(CDCl₃)
δ: 1.30 (t, J=7Hz, 3H) 1.49 (s, 9H) 1.84 (t, J=2Hz, 3H) 2.93 (t, J=8Hz, 2H) 3.33 (t, J=5Hz, 4H) 3.57 (t, J=5Hz, 4H) 4.19 (t, J=8Hz, 2H) 4.25 (q, J=7Hz, 2H) 4.76 (s, 2H) 4.86 (q, J=2Hz, 2H) 7.19 (t, J=7Hz, 1H) 7.25-7.34 (m, 4H)

### (e) Ethyl [7-(2-butynyl)-2,6-dioxo-1-(2-phenethyl)-8-(piperazin-1-yl)-1,2,6,7-tetrahydropurin-3-yl] acetate hydrochloride

A mixture containing 27 mg of t-butyl 4-[7-(2-butynyl)-3-ethoxycarbonylmethyl-1-(2-phenylethyl)-2,6-dioxo-1,2,6,7-tetrahydropurin-8 -yl] piperazine-1-carboxylate and 0.25 mL of trifluoroacetic acid was stirred at room temperature for 30 minutes. The reaction solution was concentrated, and the residue was purified by reverse-phase column chromatography using 20-80% methanol/water (containing 0.1% concentrated hydrochloric acid), to give 17 mg of the title compound.
¹H-NMR(d6-DMSO)
δ: 1.22 (t, J=7Hz, 3H) 1.82 (t, J=2Hz, 3H) 2.80 (t, J=8Hz, 2H) 3.22-3.28 (m, 4H) 3.46-3.51 (m, 4H) 4.05 (t, J=8Hz, 2H) 4.17 (q, J=7Hz, 2H) 4.69(s, 2H) 4.96(q, J=2Hz, 2H) 7.19-7.24 (m, 3H) 7.30 (t, J=7Hz, 2H)

### Example 417

### [7-(2-Butynyl)-2,6-dioxo-1-(2-phenethyl)-8-(piperazin-1-yl)-1,2,6,7-tetrahydropurin-3-yl] acetic acid hydrochloride

### (f) t-Butyl 4-[7-(2-butynyl)-3-carboxymethyl-1-(2-phenylethyl)-2,6-dioxo-1,2,6,7-tetrahydropurin-8-yl] piperazine-1-carboxylate

A mixture containing 190 mg of t-butyl 4-[7-(2-butynyl)-3-ethoxycarbonylmethyl-1-(2-phenylethyl)-2,6-dioxo-1,2,6,7-tetrahydropurin-8 -yl] piperazine-1-carboxylate, 3 mL of ethanol, and 0.5 mL of 1N-aqueous sodium hydroxide solution was stirred in an oil bath at 50°C for two hours. 0.55 mL of an aqueous solution of 1N hydrochloric acid was added to the reaction solution, and then extracted with ethyl acetate and water. The organic layer was washed with water and saturated sodium chloride solution, and dried over anhydrous magnesium sulfate. The liquid was concentrated under reduced pressure, and ethyl acetate-hexane was added to the liquid for crystallization. Thus, 166 mg of the title compound was obtained.
¹H-NMR(CDCl₃)
δ: 1.49 (s, 9H) 1.84 (t, J=2Hz, 3H) 2.93 (t, J=8Hz, 2H) 3.34 (t, J=5Hz, 4H) 3.58 (t, J=5Hz, 4H) 4.19 (t, J=8Hz, 2H) 4.82 (s, 2H) 4.85 (q, J=2Hz, 2H) 7.19 (t, J=7Hz, 1H) 7.24-7.33 (m, 4H)

### (g) [7-(2-Butynyl)-2,6-dioxo-1-(2-phenethyl)-8-(piperazin-1-yl)-1,2,6,7-tetrahydropurin-3-yl] acetic acid hydrochloride

2.2 mg of the title compound was obtained using 22 mg of t-butyl 4-[7-(2-butynyl)-3-carboxymethyl-1-(2-phenylethyl)-2,6-dioxo-1,2,6,7-tetrahydropurin-8-yl] piperazine-1-carboxylate by the same procedure described in Example 416-(e).
¹H-NMR(d6-DMSO)
δ: 1.82 (t, J=2Hz, 3H) 2.80 (t, J=8Hz, 2H) 3.23-3.28 (m, 4H) 3.46-3.53(m, 4H) 4.05 (t, J=8Hz, 2H) 4.59 (s, 2H) 4.96 (q, J=2Hz, 2H) 7.19-7.25 (m, 3H) 7.30 (t, J=7Hz, 2H)

### Example 418

### 7-(2-Butynyl)-3-[2-oxo-2-(pyrrolidin-1-yl)ethyl]-1-(2-phenethyl)-8-(piperazin-1-yl)-3,7-dihydro purine-2,6-dione hydrochloride

A mixture containing 20 mg of t-butyl 4-[7-(2-butynyl)-3-carboxymethyl-1-(2-phenylethyl)-2,6-dioxo-1,2,6,7-tetrahydropurin-8-yl] piperazine-1-carboxylate, 8 µL of diethyl cyanophosphate, 10 µL of triethylamine, 20 µL of pyrrolidine, and 0.3 mL of N,N-dimethylformamide stood at room temperature for 3 days. The reaction solution was diluted with ethyl acetate and water, and extracted with ethyl acetate. The organic layer was washed with water and saturated sodium chloride solution, and then concentrated. 0.5 mL of trifluoroacetic acid was added to the residue, and the resulting mixture was incubated at room temperature for 30 minutes. The reaction solution was concentrated, and the residue was purified by reverse-phase column chromatography using 20-80% methanol/water (containing 0.1% concentrated hydrochloric acid) to give 3.2 mg of the title compound.
¹H-NMR(d6-DMSO)
δ:1.76-1.84 (m, 5H) 1.95 (quint. J=7Hz, 2H), 2.79 (t, J=8Hz, 2H) 3.22-3.34 (m, 6H) 3.45-3.52 (m, 4H) 3.55 (t, J=7Hz, 2H) 4.03 (t, J=8Hz, 2H) 4.68 (s, 2H) 4.96 (q, J=2Hz, 2H) 7.18-7.26 (m, 3H) 7.31 (t, J=8Hz, 2H)

### Example 419

### 2-[7-(2-Butynyl)-2,6-dioxo-1-(2-phenethyl)-8-(piperazin-1-yl)-1,2,6,7-tetrahydropurin-3-yl]-N-methylacetamide hydrochloride

The title compound was synthesized using an aqueous solution of methylamine by the same procedure described in Example 418.
¹H-NMR(d6-DMSO)
δ:1.82 (t, J=2Hz, 3H) 2.61 (d, J=5Hz, 3H) 2.79 (t, J=8Hz, 2H) 3.20-3.28 (m, 4H) 3.44-3.52 (m, 4H) 4.03 (t, J=8Hz, 2H) 4.48 (s, 2H) 4.96 (q, J=2Hz, 2H) 7.19-7.26 (m, 3H) 7.31 (t, J=7Hz, 2H) 8.09 (brd, J=5Hz, 1H)

### Example 420

### 2-[7-(2-Butynyl)-2,6-dioxo-1-(2-phenethyl)-8-(piperazin-1-yl)-1,2,6,7-tetrahydropurin-3-yl]-N-c yclopropyl acetamide hydrochloride

The title compound was synthesized using cyclopropylamine by the same procedure described in Example 418.
¹H-NMR(d6-DMSO)
δ:0.39-0.44 (m, 2H) 0.60-0.66 (m, 2H) 1.82 (t, J=2Hz, 3H) 2.60-2.68 (m, 1H) 2.79 (t, J=8Hz, 2H) 3.20-3.30 (m, 4H) 3.44-3.54 (m, 4H) 4.03 (t, J=8Hz, 2H) 4.44 (s, 2H) 4.96 (q, J=2Hz, 2H) 7.19-7.27 (m, 3H) 7.31 (t, J=8Hz, 2H) 8.27 (d, J=4Hz, 1H)

### Example 421

### 2-[7-(2-Butynyl)-2,6-dioxo-1-(2-phenethyl)-8-(piperazin-1-yl)-1,2,6,7-tetrahydropurin-3-yl]-N-p henylacetamide hydrochloride

The title compound was synthesized using aniline by the same procedure described in Example 418.
¹H-NMR(d6-DMSO)
δ:1.83 (t, J=2Hz, 3H) 2.81 (t, J=8Hz, 2H) 3.20-3.30 (m, 4H) 3.44-3.54 (m, 4H) 4.05 (t, J=8Hz, 2H) 4.74 (s, 2H), 4.98 (q, J=2Hz, 2H) 7.06 (t, J=8Hz, 1H) 7.18-7.35 (m, 7H) 7.56 (d, J=8Hz, 2H) 9.01 (brs, 2H) 10.39 (s, 1H)

### Example 422

### 2-[7-(2-Butynyl)-2,6-dioxo-1-(2-phenethyl)-8-(piperazin-1-yl)-1,2,6,7-tetrahydropurin-3-yl]-N-( 2-propynyl) acetamide hydrochloride

The title compound was synthesized using propargylamine by the same procedure described in Example 418.
¹H-NMR(d6-DMSO)
δ:1.81 (t, J=3Hz) 2.80 (t, J=8Hz, 2H) 3.18 (t, J=2Hz 1H), 3.22-3.32 (m, 4H) 3.44-3.54 (m, 4H) 3.90 (dd, J=2Hz, 5Hz, 2H) 4.03 (t, J=8Hz, 2H) 4.51 (s, 2H) 4.96 (q, J=2Hz, 2H) 7.16-7.34 (m, 5H) 8.66 (t, J=5Hz, 1H) 8.96 (br.s, 2H)

### Example 423

### Ethyl [7-(2-butynyl)-2,6-dioxo-1-(2-phenoxy ethyl)-8-(piperazin-1-yl)-1,2,6,7-tetrahydropurin-3-yl] acetate hydrochloride

The title compound was synthesized using 2-bromoethyl phenyl ether by the same procedure described in Example 416.
¹H-NMR(d6-DMSO)
δ:1.20 (t, J=7Hz, 3H) 1.81 (s, 3H) 3.22-3.28 (m, 4H) 3.46-3.53 (m, 4H) 4.06-4.19 (m, 4H) 4.25 (t, J=6Hz, 2H) 4.69 (s, 2H) 4.97 (s, 2H) 6.88-6.96 (m, 3H) 7.26 (t, J=7Hz, 2H) 8.96 (brs, 2H)

### Example 424

### Ethyl [1-methyl-2,6-dioxo-8-(piperazin-1-yl)-7-(2-vinylphenyl)-1,2,6,7-tetrahydropurin-3-yl] acetate trifluoroacetate

### (a) [1-(2,2-Dimethylpropionyloxymethyl)-7-(2-formylphenyl)-2,6-dioxo-1,2,6,7-tetrahydropurin-3-y l] methyl 2,2-dimethylpropionate

10.2 g of [3-(2,2-dimethylpropionyloxymethyl)-2,6-dioxo-2,3,6,7-tetrahydropurin-1-yl] methyl 2,2-dimethylpropionate, 8.04 g of 2-formylphenylboronic acid, and 7.30 g of copper (II) acetate were suspended in 50 mL of N,N-dimethylformamide, and then 4.34 mL of pyridine was added. The mixture was stirred at room temperature for 37 hours. The reaction solution was diluted with ethyl acetate, and washed with water. The organic layer was dried over anhydrous magnesium sulfate, and filtered. The filtrate was concentrated under reduced pressure. The residue was purified by silica gel column chromatography. Thus, 4.12 g of the title compound was obtained from a fraction eluted with hexane-ethyl acetate (1:2).
¹H-NMR(CDCl₃)
δ: 1.16 (s, 9H) 1.23 (s, 9H) 5.95 (s, 2H) 6.20 (s, 2H) 7.46-7.48 (m, 1H) 7.42-7.78 (m, 2H) 7.75 (s, 1H) 8.03-8.06 (m, 1H) 9.92 (s, 1H)

### (b)

### [8-Chloro-1-(2,2-dimethylpropionyloxymethyl)-7-(2-formylphenyl)-2,6-dioxo-1,2,6,7-tetrahydro purin-3-yl] methyl 2,2-dimethylpropionate

2.50 g of [1-(2,2-dimethylpropionyloxymethyl)-7-(2-formylphenyl)-2,6-dioxo-1,2,6,7-tetrahydropurin-3-y 1] methyl 2,2-dimethylpropionate and 896 mg of N-chlorosuccinimide were dissolved in 25 mL of N,N-dimethylformamide. The resulting mixture was stirred at room temperature for 8 hours. The reaction solution was diluted with ethyl acetate, and washed with water. The organic layer was dried over anhydrous magnesium sulfate, and filtered. The filtrate was concentrated under reduced pressure, and the residue was purified by silica gel column chromatography. Thus, 2.0 g of the title compound was obtained from a fraction eluted with hexane-ethyl acetate (2:1).
¹H-NMR(CDCl₃)
δ: 1.15 (s, 9H) 1.24 (s, 9H) 5.91 (s, 2H) 6.14 (s, 2H) 7.49-7.51 (m, 1H) 7.81-7.83 (m, 2H) 8.03-8.06 (m, 1H) 9.92 (s, 1H)

### (c) t-Butyl 4-[1,3-bis(2,2-dimethylpropionyloxymethyl)-7-(2-formylphenyl)-2,6-dioxo-2,3,6,7-tetrahydro-1 H-purin-8-yl] piperazine-1-carboxylate

2.0 g of [8-chloro-1-(2,2-dimethyl-propionyloxy methyl)-7-(2-formylphenyl)-2,6-dioxo-1,2,6,7-tetrahydropurin-3-yl] methyl 2,2-dimethylpropionate was combined with 2.15 g of t-butyl piperazine-1-carboxylate. The resulting mixture was stirred at 150°C for 70 minutes. The reaction mixture was diluted with chloroform, and then purified by silica gel column chromatography. Thus, 1.94 g of the title compound was obtained from a fraction eluted with hexane-ethyl acetate (1:1).

### (d) t-Butyl 4-[1,3-bis(2,2-dimethylpropionyloxymethyl)-2,6-dioxo-7-(2-vinylphenyl)-2,3,6,7-tetrahydro-1H-purin-8-yl] piperazine-1-carboxylate

3.52 g of methyl triphenylphosphonium bromide was dissolved in 20 mL of tetrahydrofuran, and then 948 mg of potassium tertiary butoxide was added. The resulting mixture was stirred at room temperature for 1 hour. 20 mL of tetrahydrofuran solution containing 1.94 g of t-butyl 4-[1,3-bis(2,2-dimethylpropionyloxymethyl)-7-(2-formylphenyl)-2,6-dioxo-2,3,6,7-tetrahydro-1 H-purin-8-yl] piperazine-1-carboxylate was added to the reaction mixture at room temperature. The mixture was stirred at room temperature for 3 hours and 50 minutes. The reaction solution was diluted with ethyl acetate, and then washed with water. The organic layer was dried over anhydrous magnesium sulfate, and filtered. The filtrate was concentrated under reduced pressure. The residue was purified by silica gel column chromatography. Thus, 704 mg of the title compound was obtained from a fraction eluted with hexane-ethyl acetate (2:1).

### (e) t-Butyl 4-[1-(2,2-dimethylpropionyloxymethyl)-2,6-dioxo-7-(2-vinylphenyl)-2,3,6,7-tetrahydro-1H-puri n-8-yl] piperazine-1-carboxylate

704 mg of t-butyl 4-[1,3-bis (2,2-dimethylpropionyloxymethyl)-2,6-dioxo-7-(2-vinylphenyl)-2,3,6,7-tetrahydro-1H-purin-8-y l] piperazine-1-carboxylate was dissolved in a mixed solvent of 7 mL of tetrahydrofuran and 14 mL of methanol, and then 51 mg of sodium hydride was added. The resulting mixture was stirred at room temperature for 17 minutes. The reaction solution was diluted with chloroform, and washed with a saturated sodium chloride solution. The organic layer was dried over anhydrous magnesium sulfate, and filtered. The filtrate was concentrated under reduced pressure, and the residue was purified by silica gel column chromatography. Thus, 510 mg of the title compound was obtained from a fraction eluted with hexane-ethyl acetate (2:3).

### (f) t-Butyl 4-[1-(2,2-dimethylpropionyloxymethyl)-3-ethoxycarbonylmethyl-2,6-dioxo-7-(2-vinylphenyl)-2, 3,6,7-tetrahydro-1H-purin-8-yl] piperazine-1-carboxylate

80 mg of t-butyl 4-[1-(2,2-dimethylpropionyloxymethyl)-2,6-dioxo-7-(2-vinylphenyl)-2,3,6,7-tetrahydro-1H-puri n-8-yl] piperazine-1-carboxylate was dissolved in 2 mL of N,N-dimethylformamide, and then 19 µL of ethyl bromoacetate and 22 mg of potassium carbonate were added. The resulting mixture was stirred at room temperature for 14 hours. The reaction solution was diluted with ethyl acetate, and washed with water. The organic layer was dried over anhydrous magnesium sulfate, and filtered. The filtrate was concentrated under reduced pressure to give 89 mg of the title compound.

### (g) t-Butyl 4-[3-ethoxycarbonylmethyl-2,6-dioxo-7-(2-vinylphenyl)-2,3,6,7-tetrahydro-1H-purin-8-yl] piperazine-1-carboxylate

89 mg of t-butyl 4-[1-(2,2-dimethylpropionyloxymethyl)-3-ethoxycarbonylmethyl-2,6-dioxo-7-(2-vinylphenyl)-2, 3,6,7-tetrahydro-1H-purin-8-yl] piperazine-1-carboxylate was dissolved in a mixed solvent containing 1 mL of tetrahydrofuran and 2 mL of methanol, and then 7 mg of sodium hydride was added. The resulting mixture was stirred at room temperature for 3.5 hours. The reaction solution was diluted with ethyl acetate, and washed with water. The organic layer was dried over anhydrous magnesium sulfate, and filtered. The filtrate was concentrated under reduced pressure. The residue was purified by silica gel column chromatography. Thus, 60 mg of the title compound was obtained from a fraction eluted with hexane-ethyl acetate (1:2).

### (h) t-Butyl 4-[3-ethoxycarbonylmethyl-1-methyl-2,6-dioxo-7-(2-vinylphenyl)-2,3,6,7-tetrahydro-1H-purin-8 -yl] piperazine-1-carboxylate

60 mg of t-butyl 4-[3-ethoxycarbonylmethyl-2,6-dioxo-7-(2-vinylphenyl)-2,3,6,7-tetrahydro-1H-purin-8-yl] piperazine-1-carboxylate was dissolved in 2 mL of N,N-dimethylformamide, and then 17 µL of methyl iodide and 17 mg of potassium carbonate were added. The resulting mixture was stirred at room temperature for 13 hours. The reaction solution was diluted with ethyl acetate, and washed with water. The organic layer was dried over anhydrous magnesium sulfate, and filtered. The filtrate was concentrated under reduced pressure to give 48 mg of the title compound.

### (i) Ethyl [1-methyl-2,6-dioxo-8-(piperazin-1-yl)-7-(2-vinylphenyl)-1,2,6,7-tetrahydropurin-3-yl] acetate trifluoroacetate

8 mg of t-butyl 4-[3-ethoxycarbonylmethyl-1-methyl-2,6-dioxo-7-(2-vinylphenyl)-2,3,6,7-tetrahydro-1H-purin-8 -yl] piperazine-1-carboxylate was dissolved in trifluoroacetic acid, and then the solution was concentrated. The residue was purified by reverse phase high performance liquid chromatography to give 2.68 mg of the title compound.
MS m/e (ESI) 439(MH⁺-CF₃COOH)

### Example 425

### [1-Methyl-2,6-dioxo-8-(piperazin-1-yl)-7-(2-vinylphenyl)-1,2,6,7-tetrahydropurin-3-yl] acetic acid trifluoroacetate

40 mg of t-butyl 4-[3-ethoxycarbonylmethyl-1-methyl-2,6-dioxo-7-(2-vinylphenyl)-2,3,6,7-tetrahydro-1H-purin-8 -yl] piperazine-1-carboxylate was dissolved in 4 mL of tetrahydrofuran, and then 1 mL of 2N sodium hydroxide was added. The resulting mixture was stirred at 90°C for 4 hours. The reaction solution was concentrated under reduced pressure, and then treated by azeotropic distillation using toluene. The residue was dissolved in trifluoroacetic acid and the solution was concentrated. The residue was purified by reverse phase high performance liquid chromatography to give 29.5 mg of the title compound.
MS m/e (ESI) 411 (MH⁺-CF₃COOH)

The following formulae represent compounds that were confirmed to be synthesized according to the general synthesis methods described above, and the same methods as described above in Production Examples and Examples.

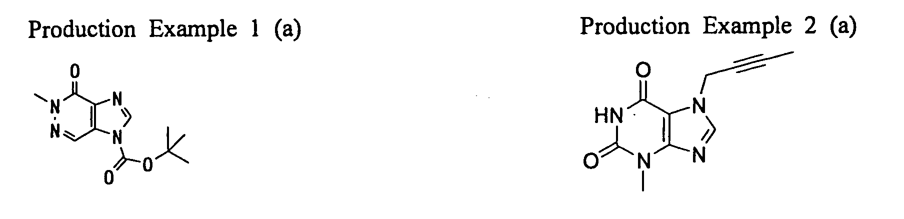

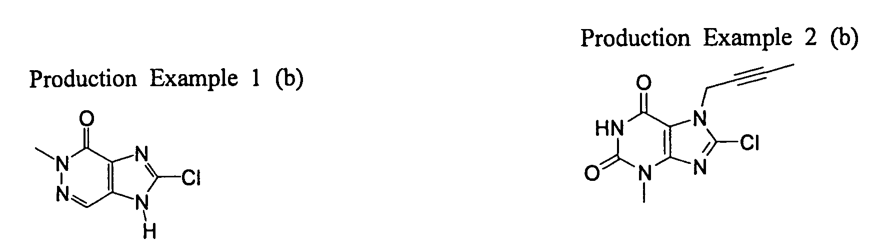

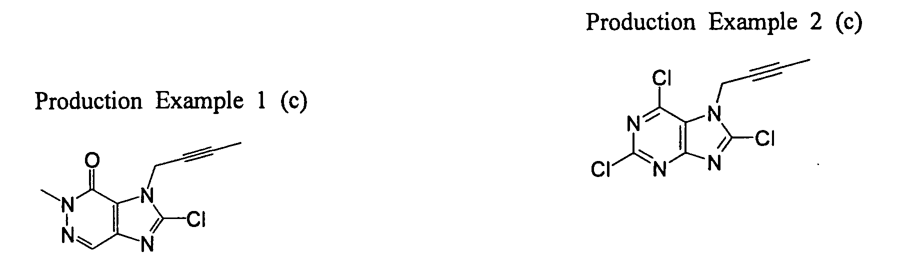

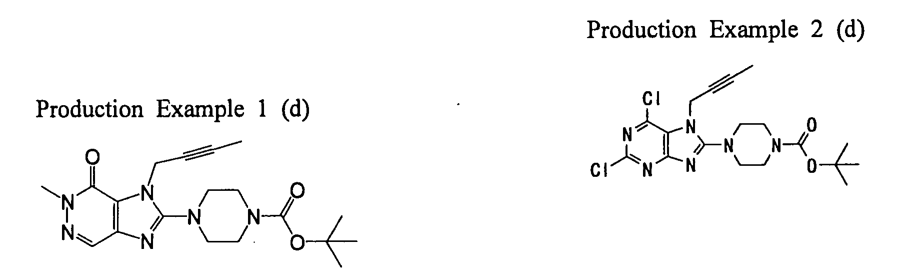

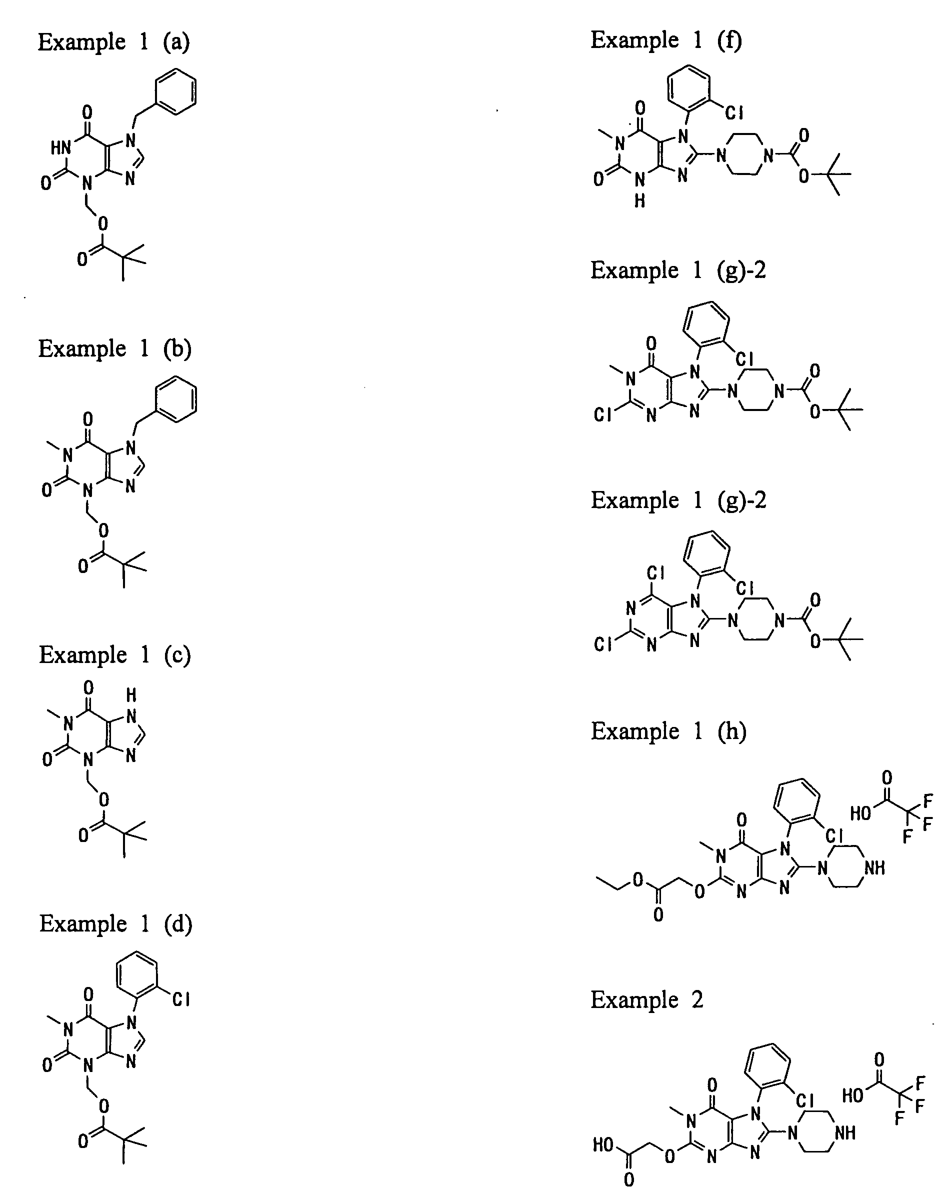

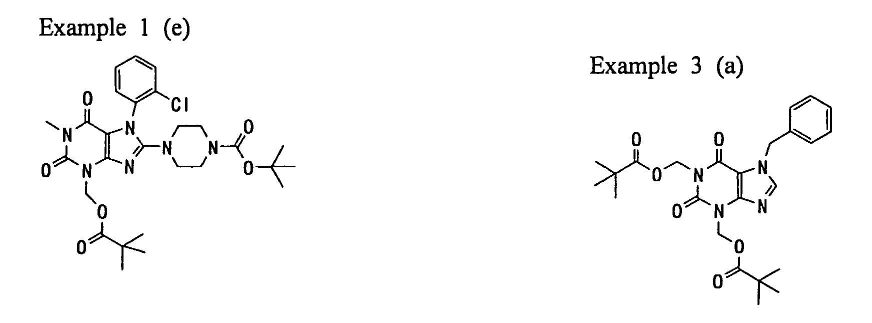

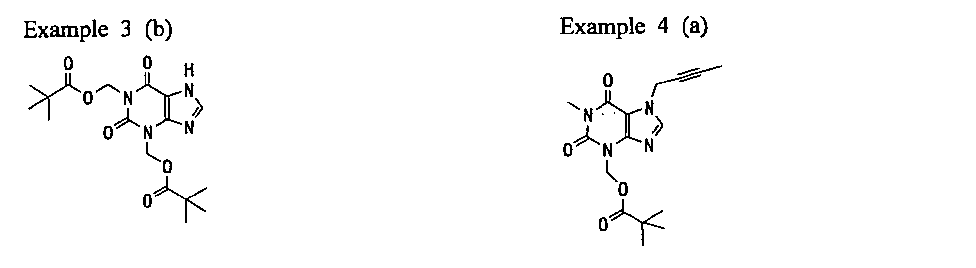

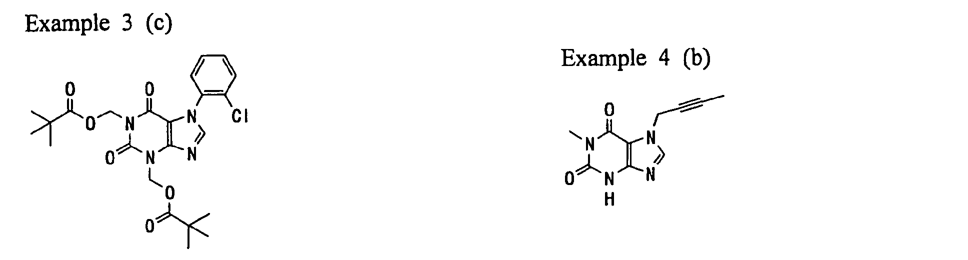

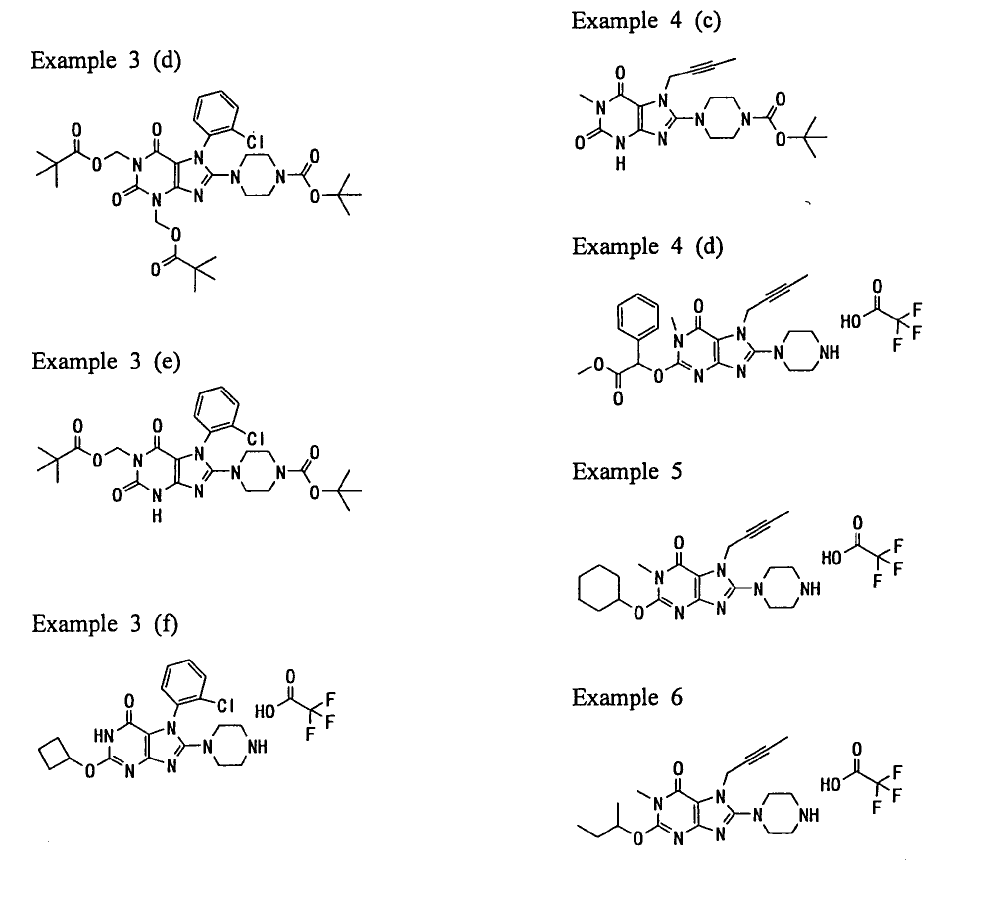

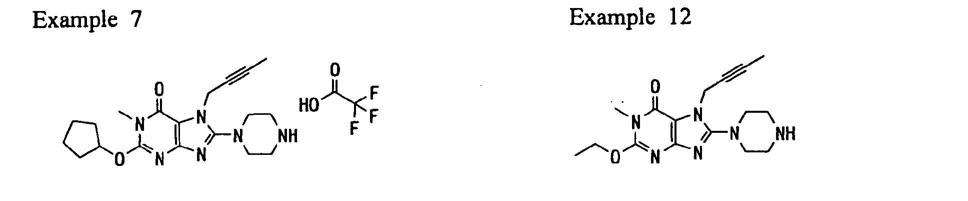

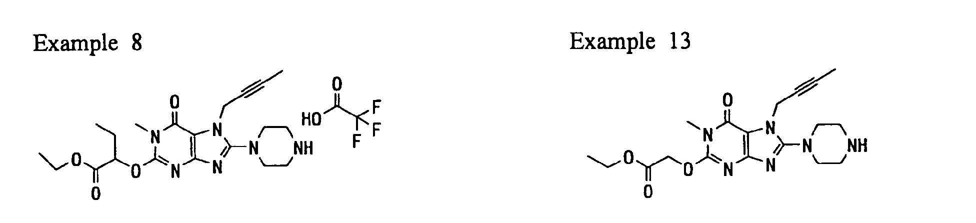

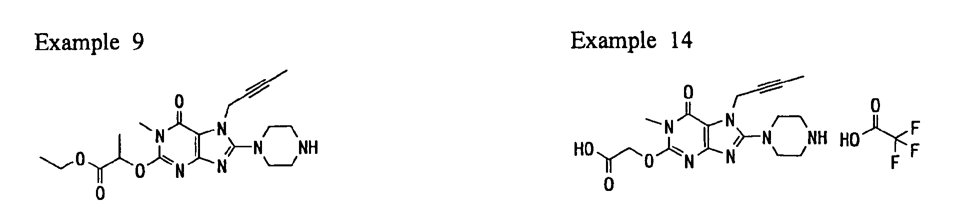

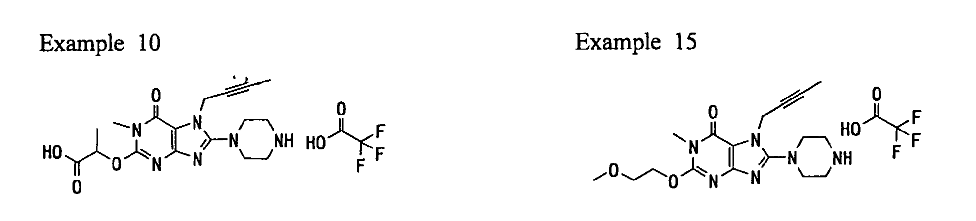

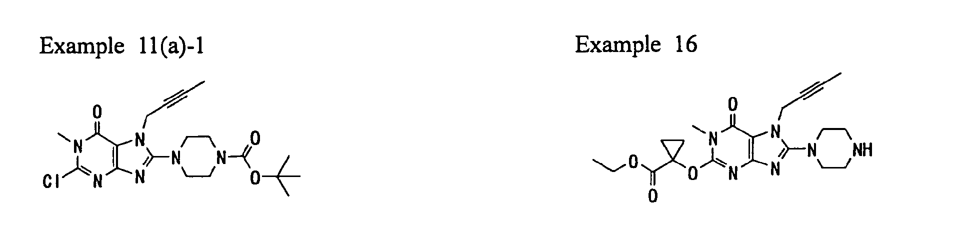

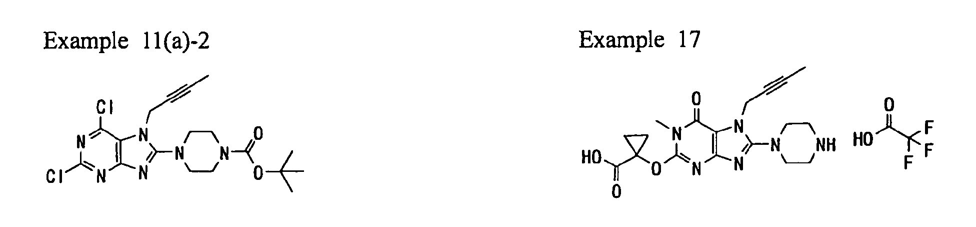

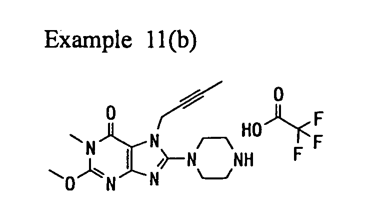

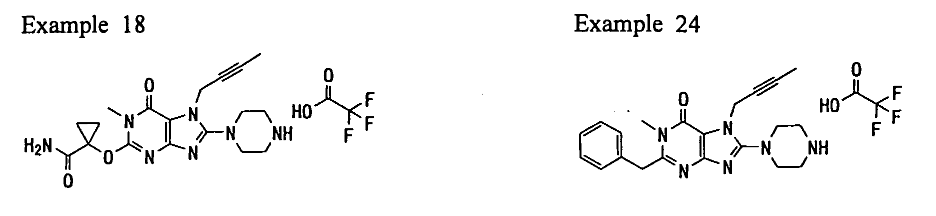

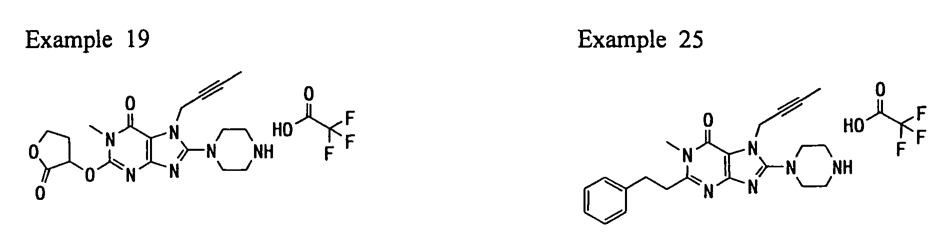

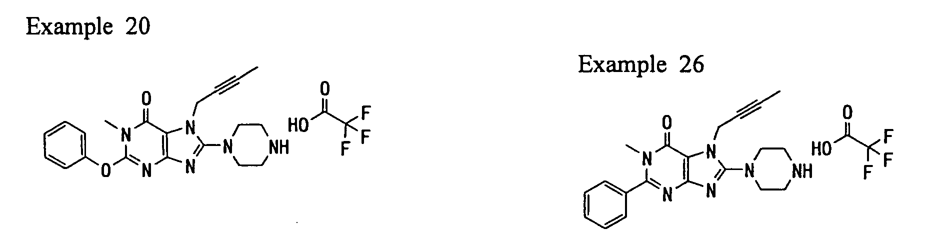

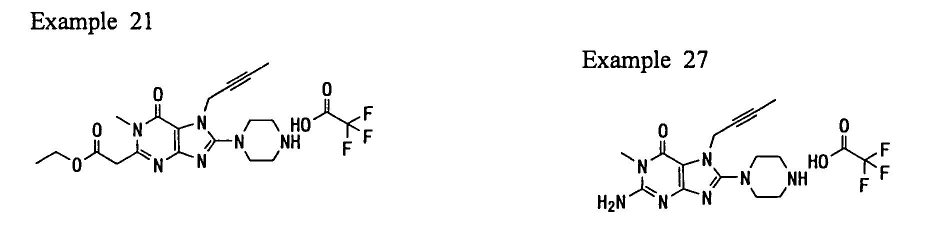

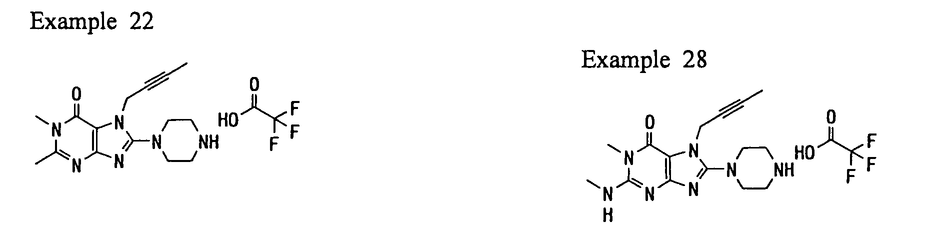

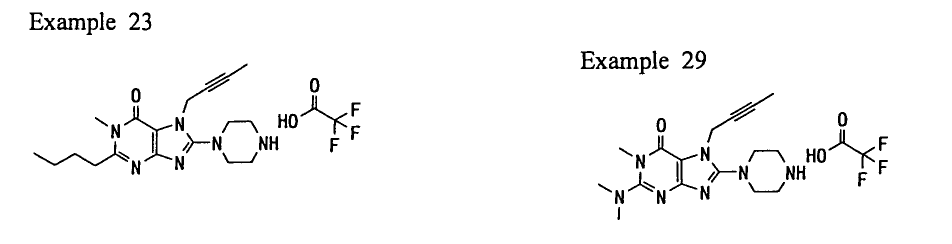

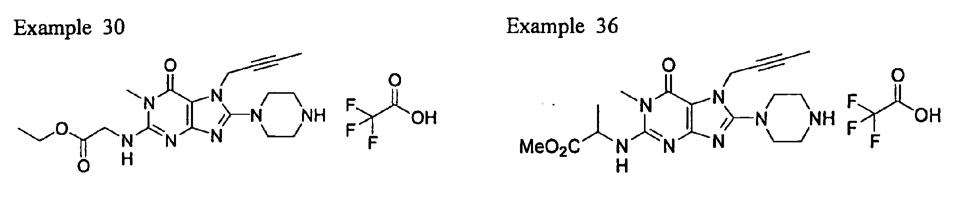

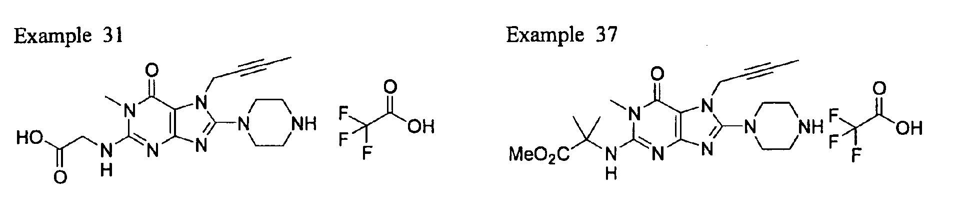

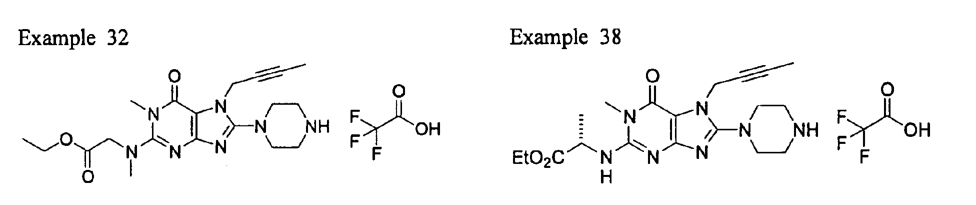

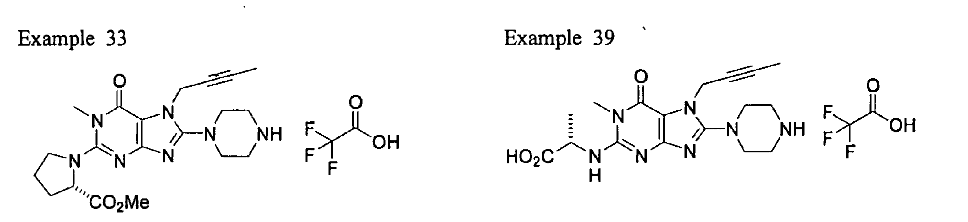

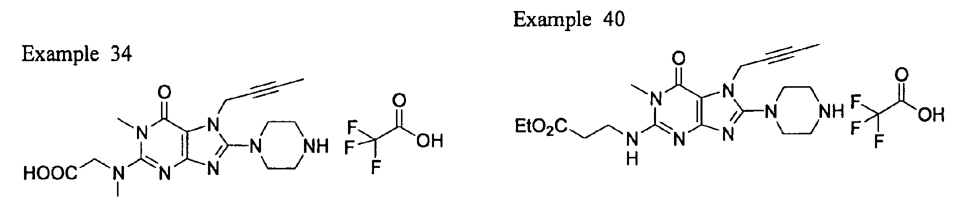

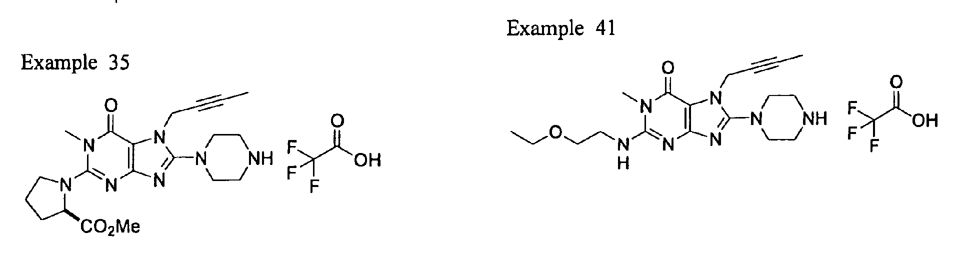

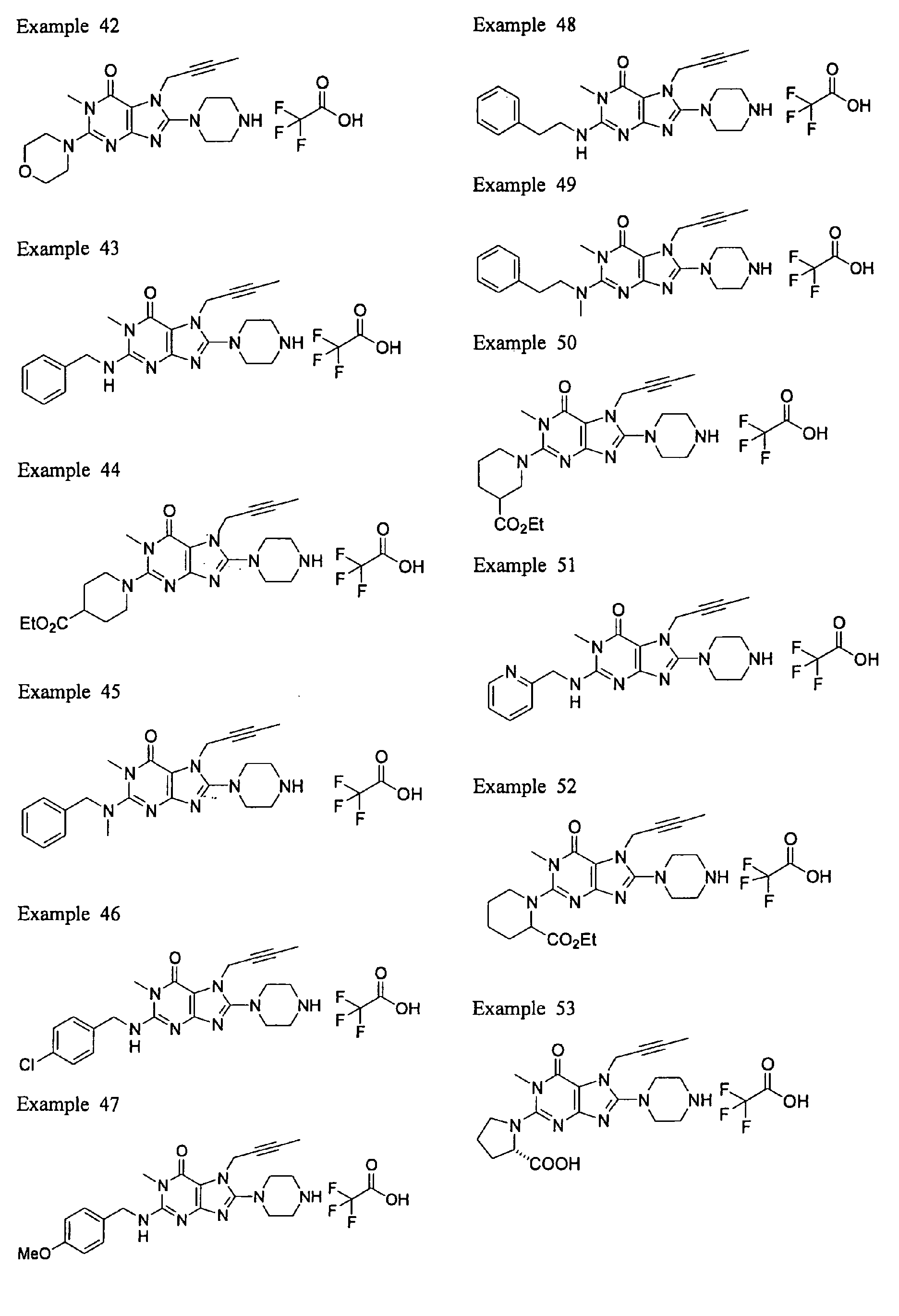

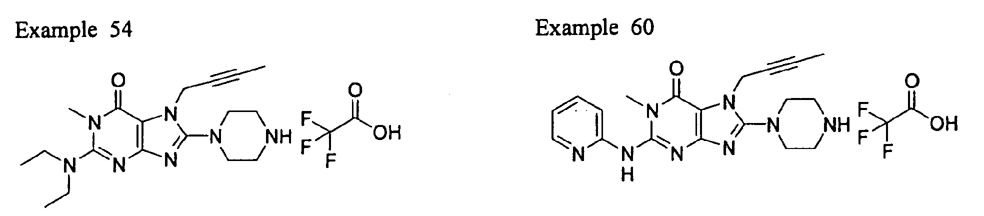

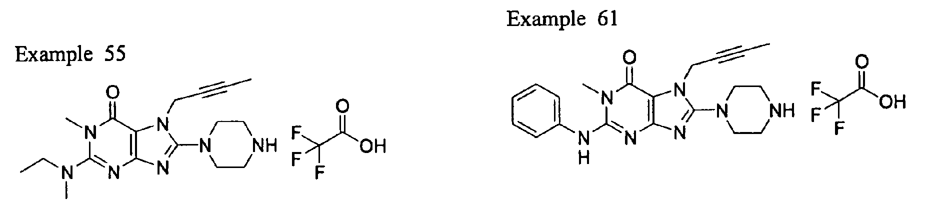

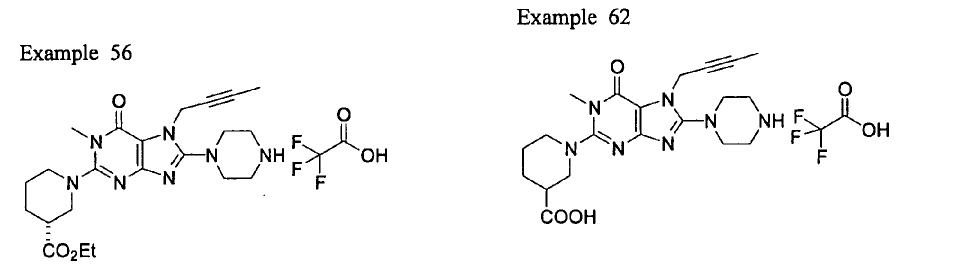

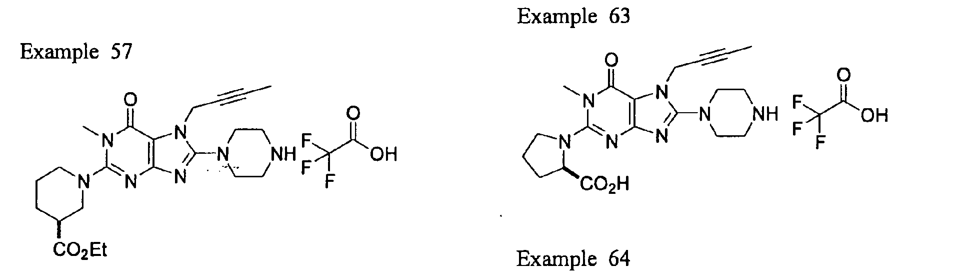

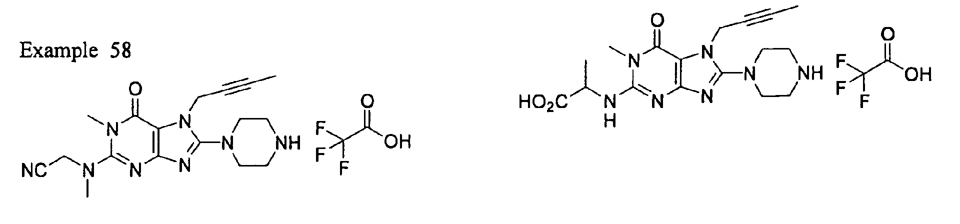

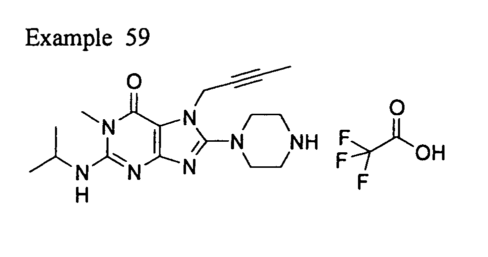

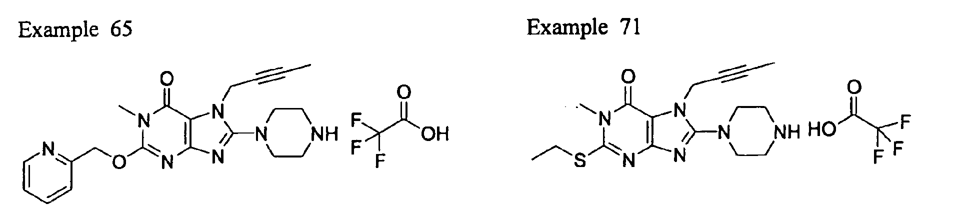

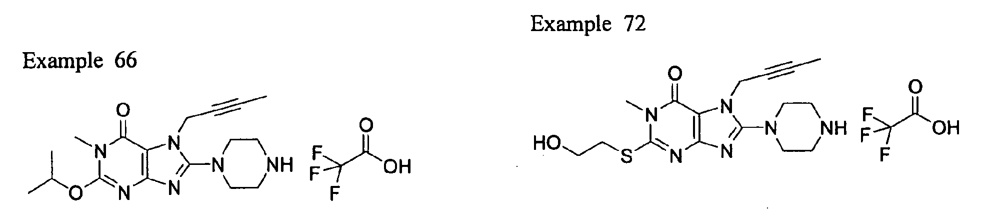

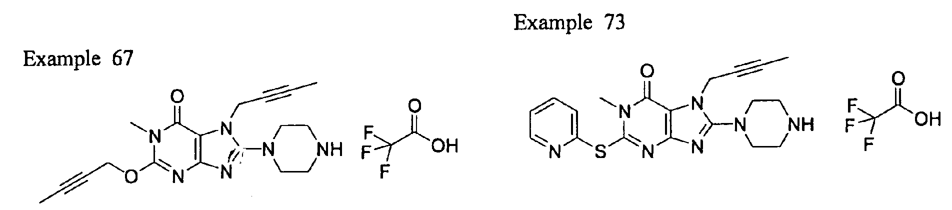

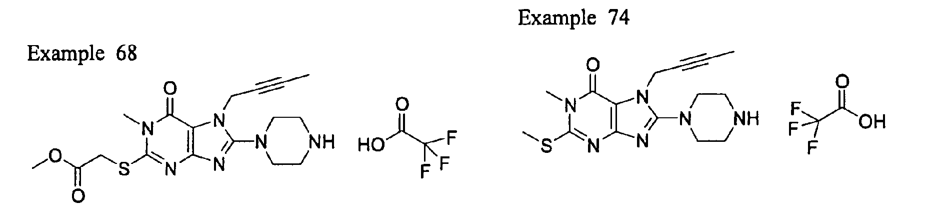

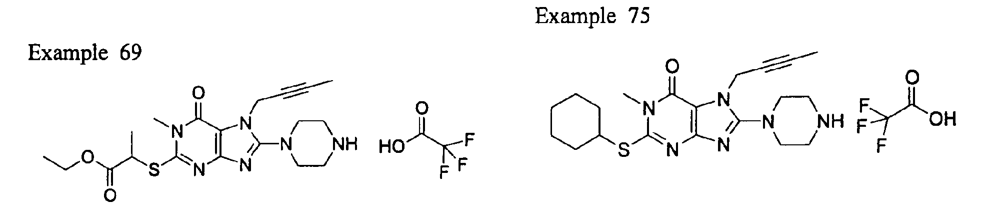

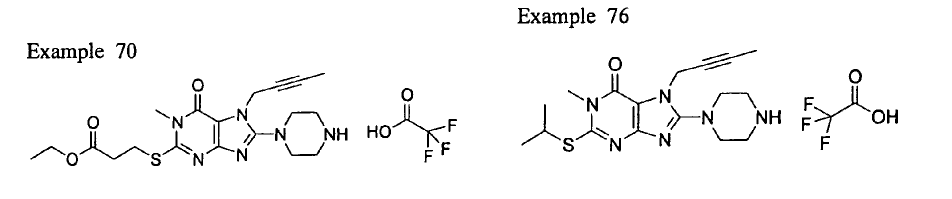

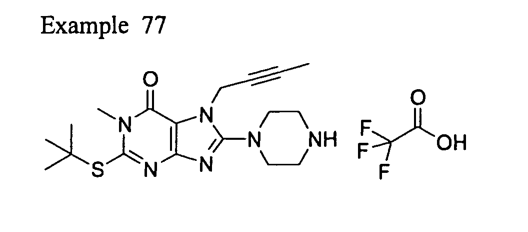

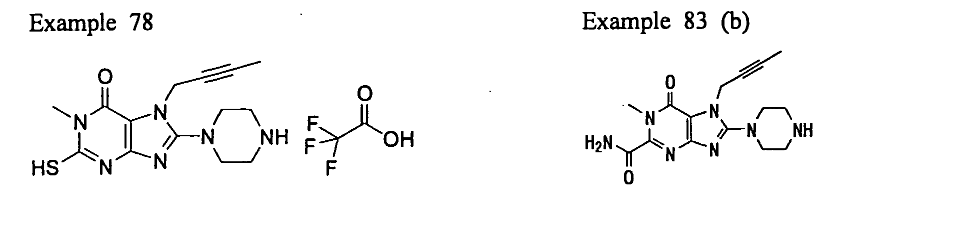

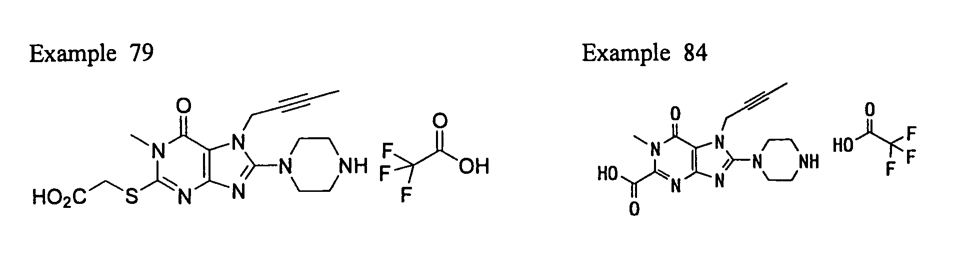

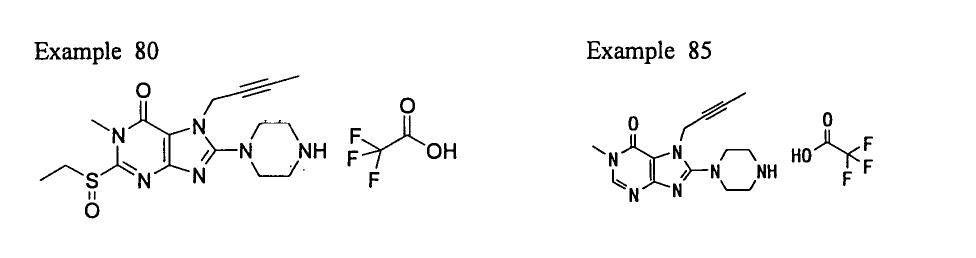

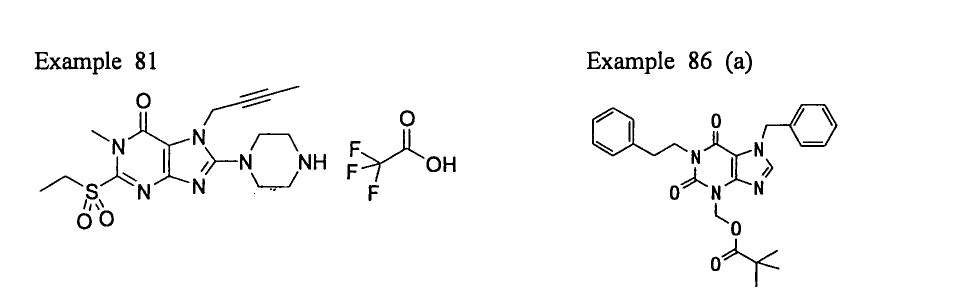

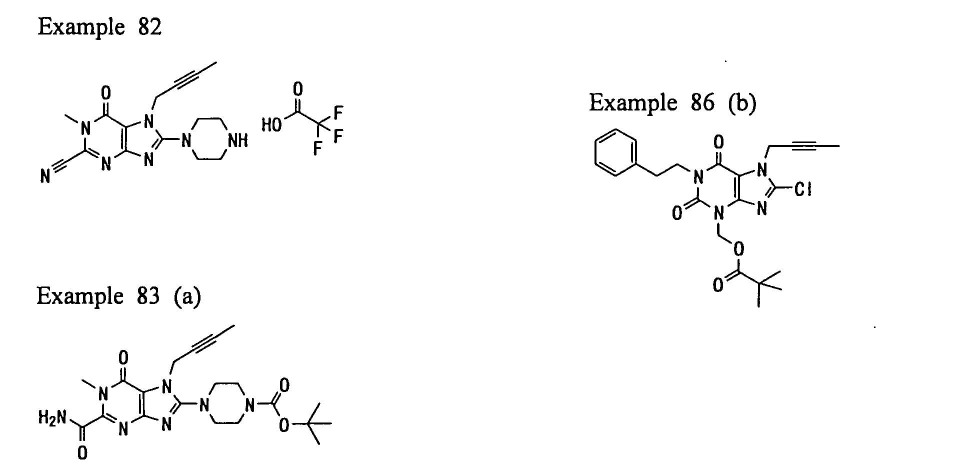

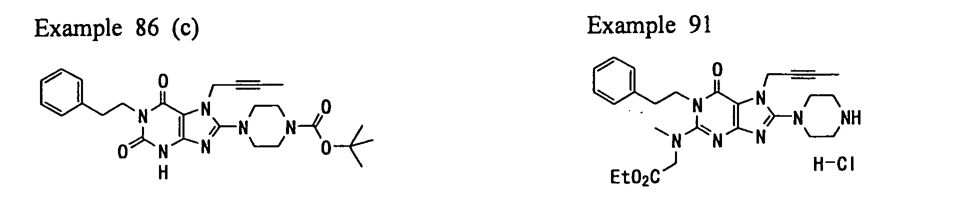

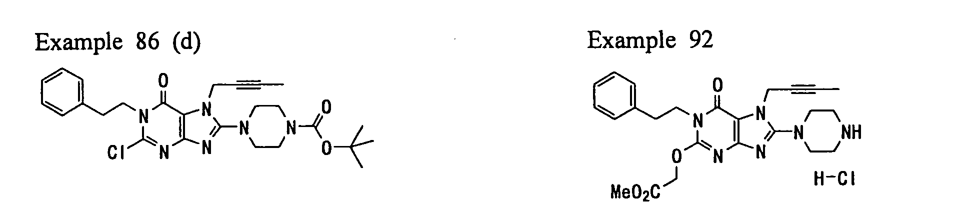

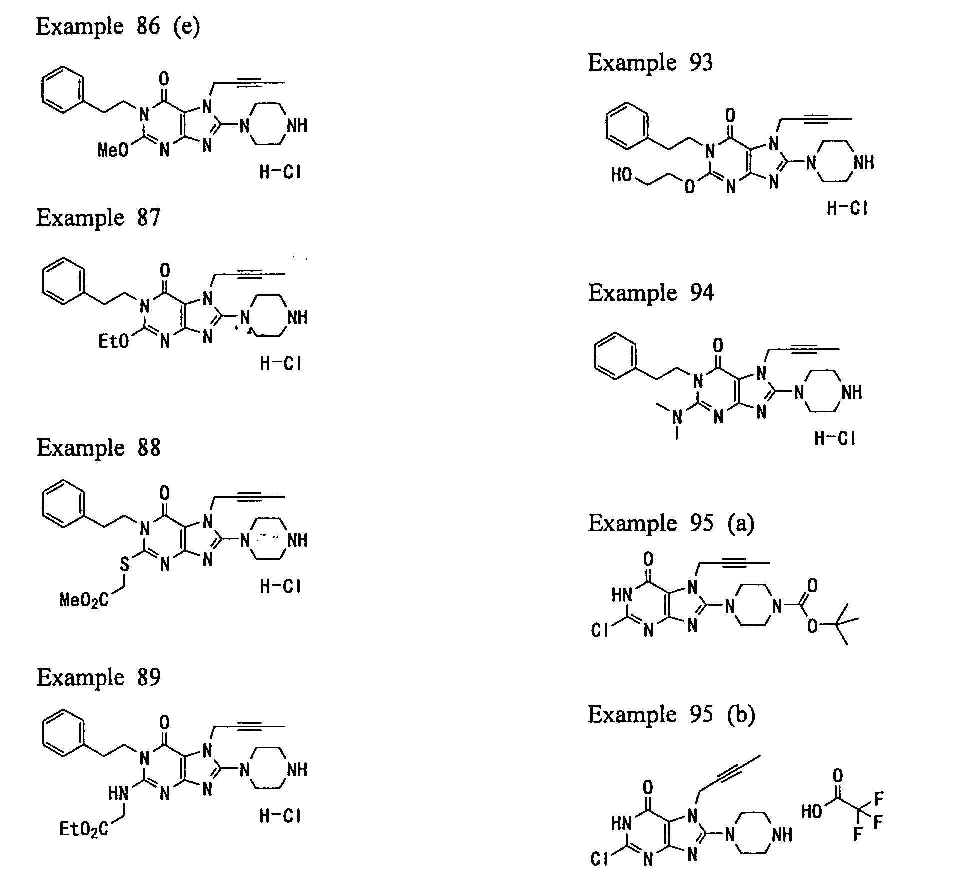

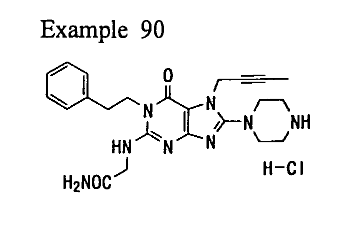

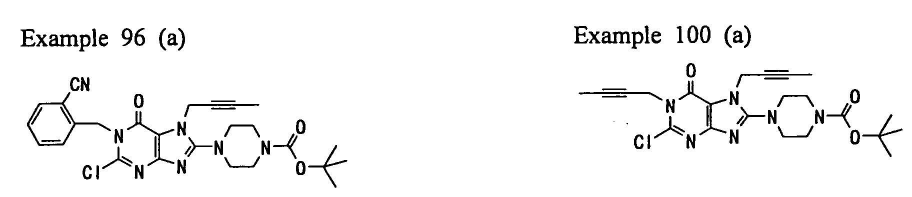

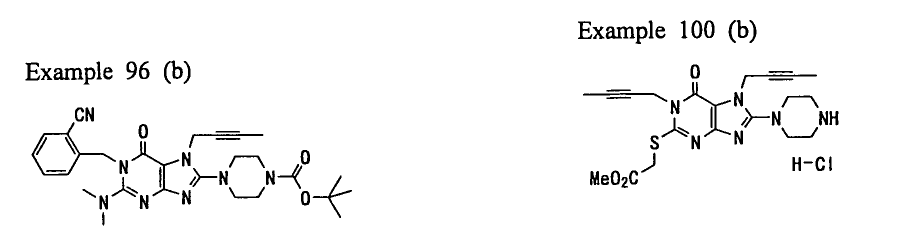

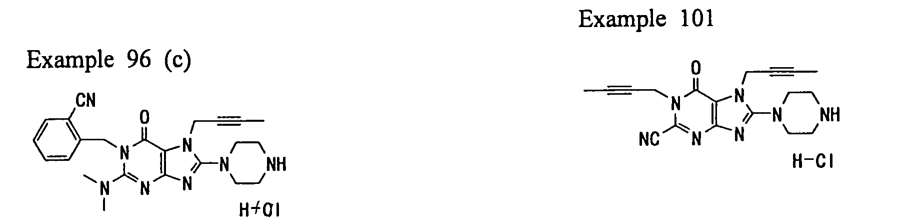

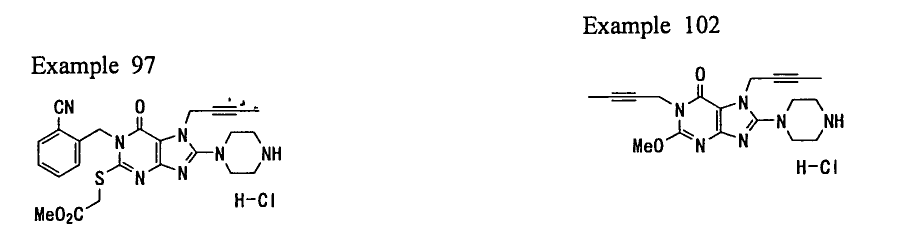

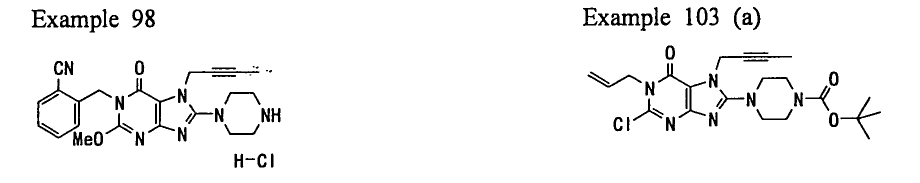

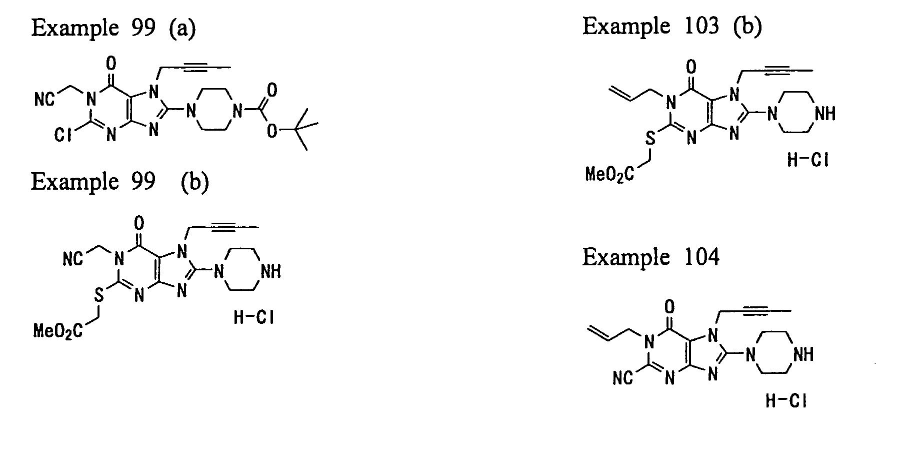

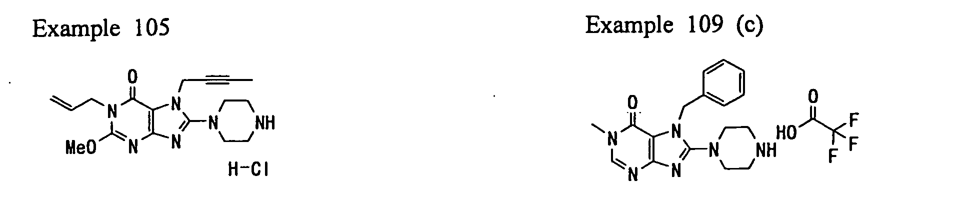

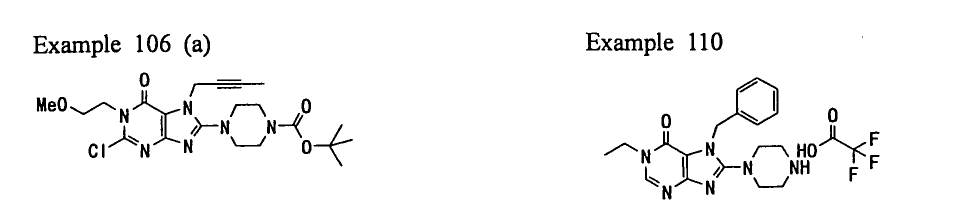

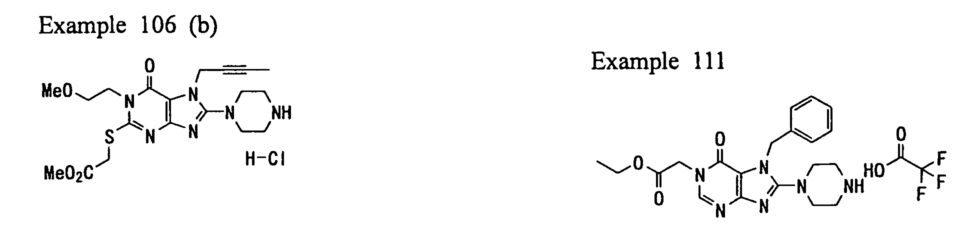

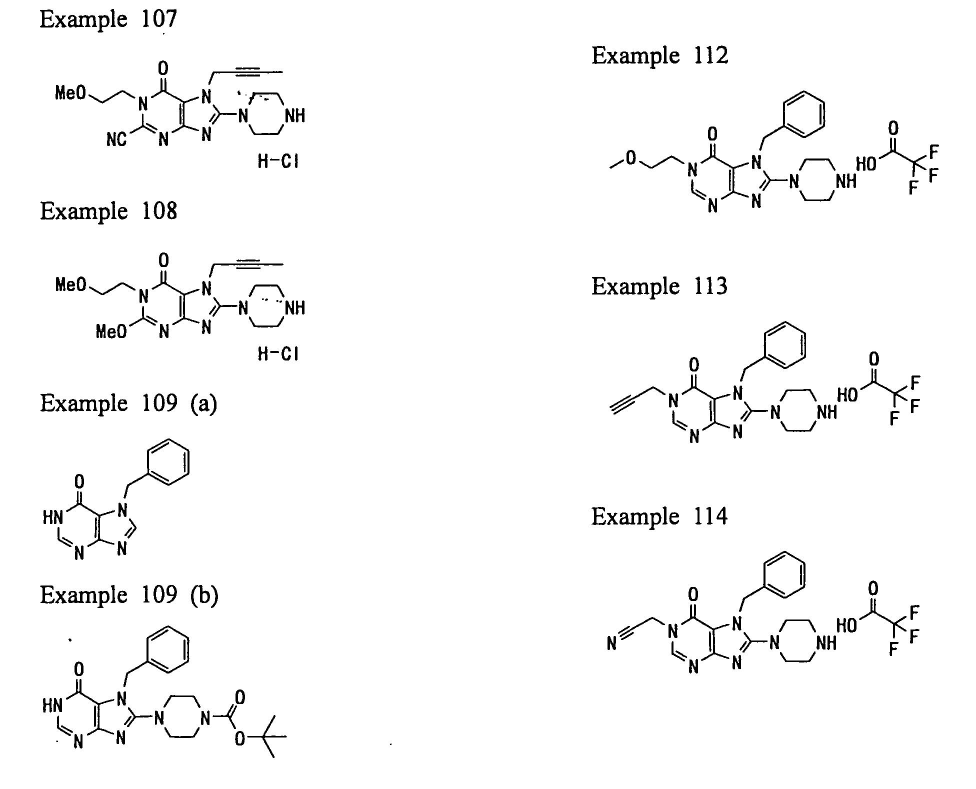

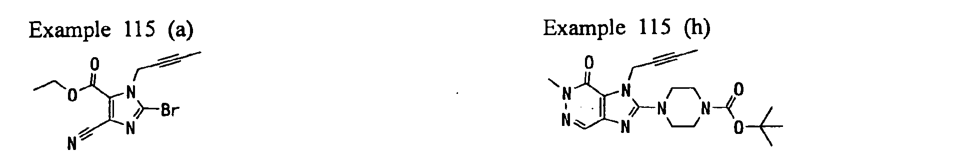

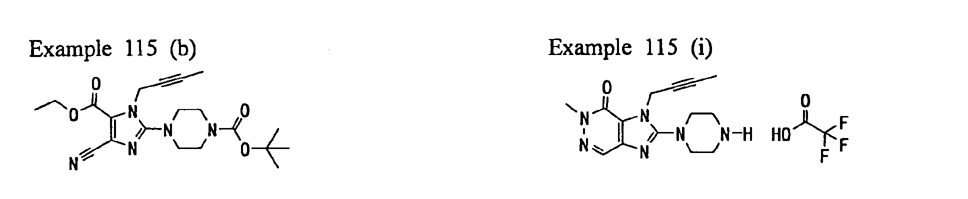

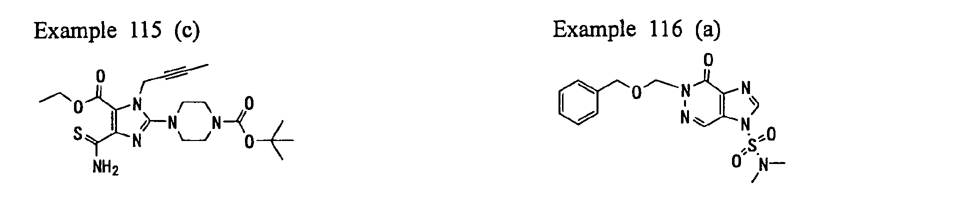

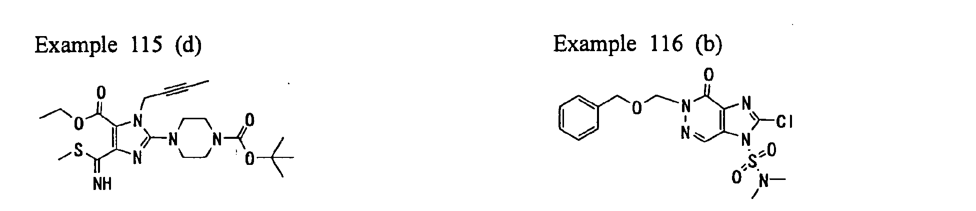

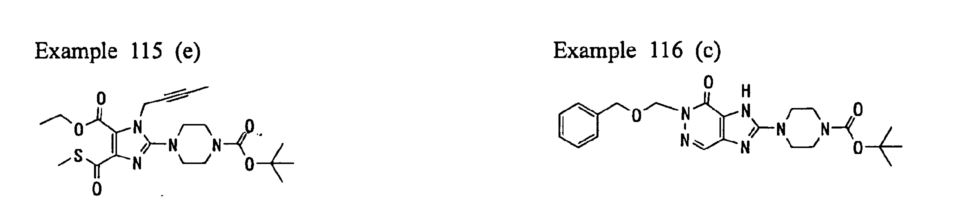

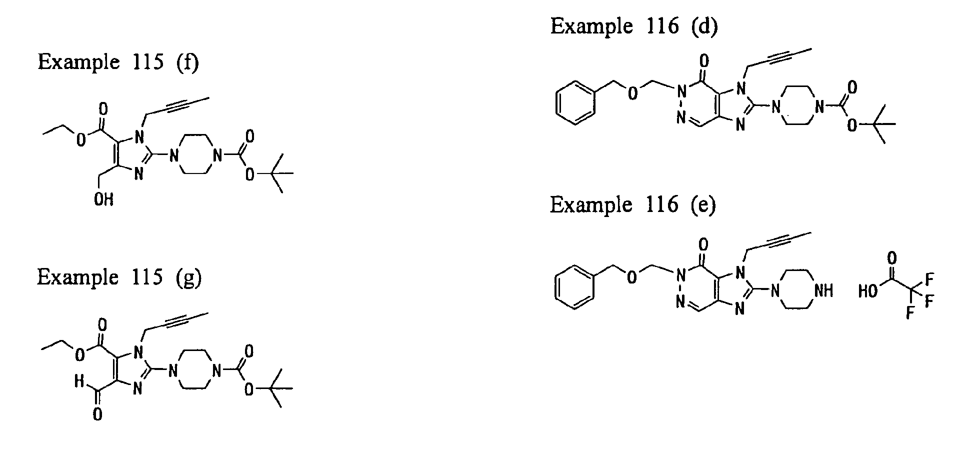

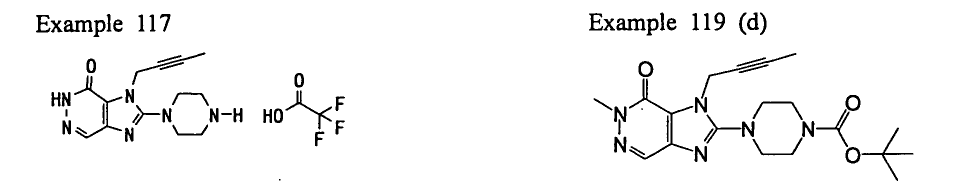

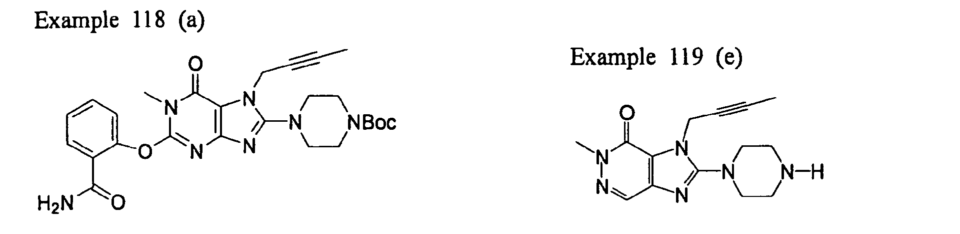

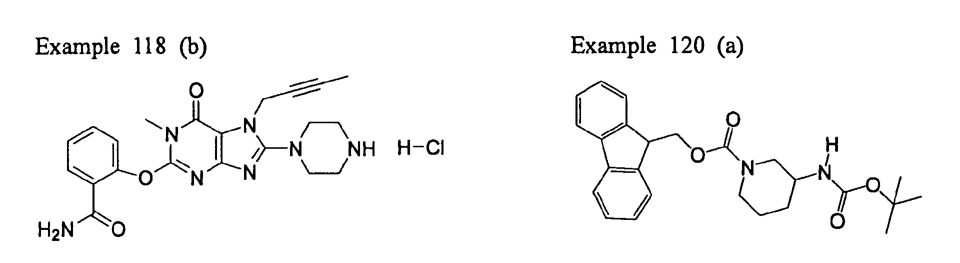

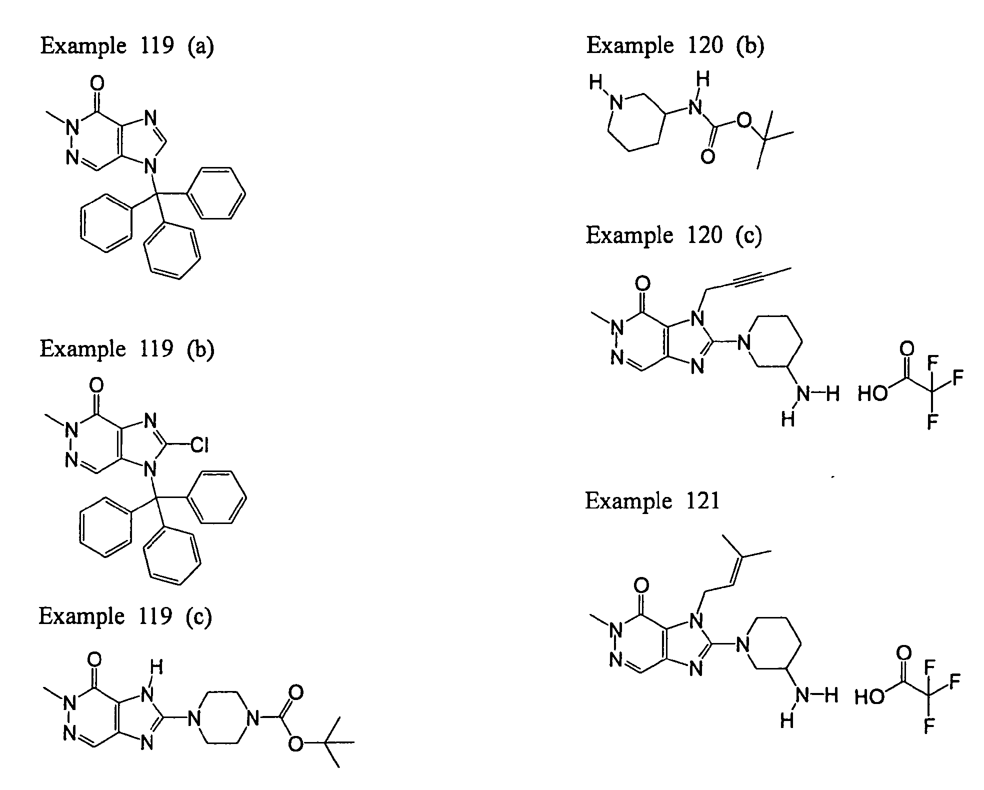

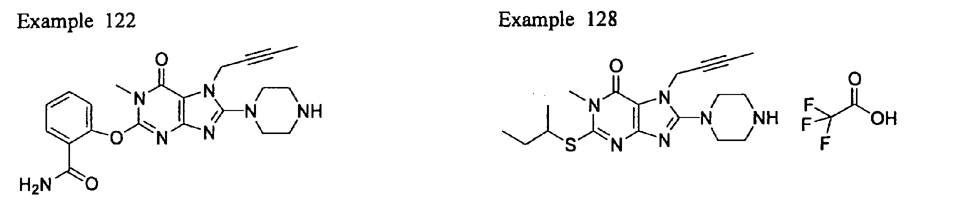

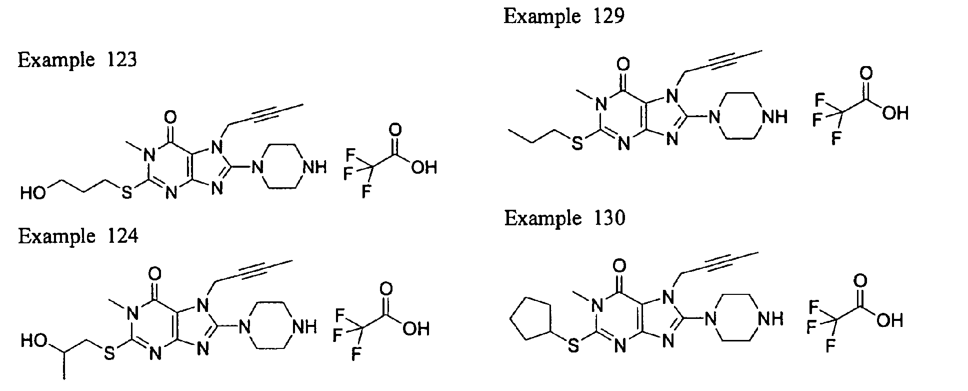

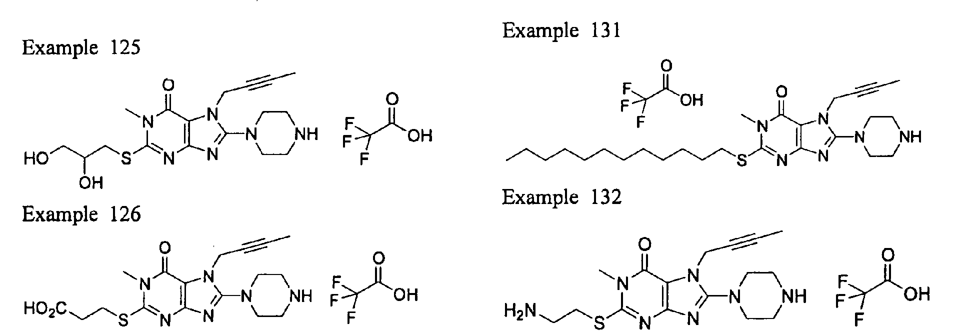

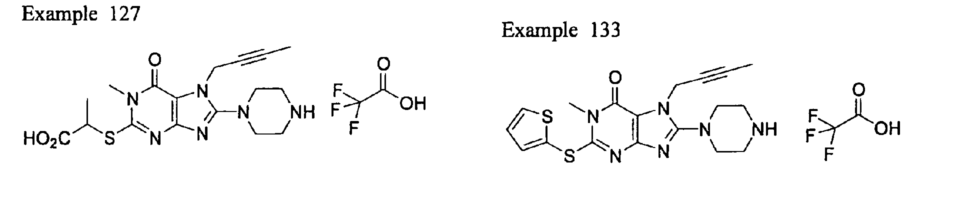

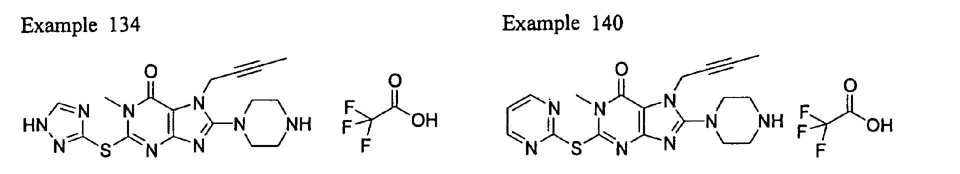

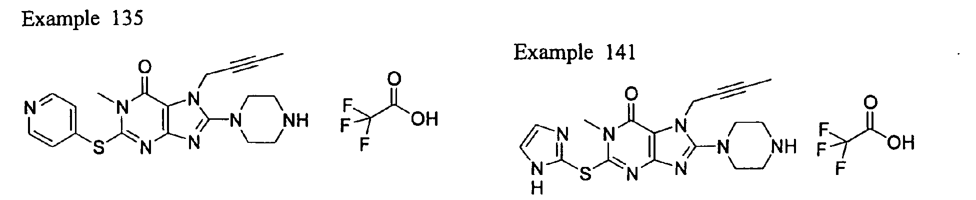

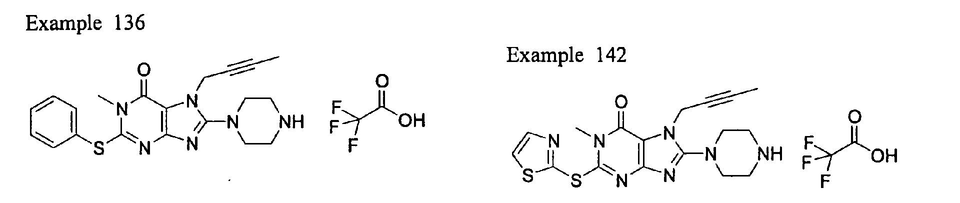

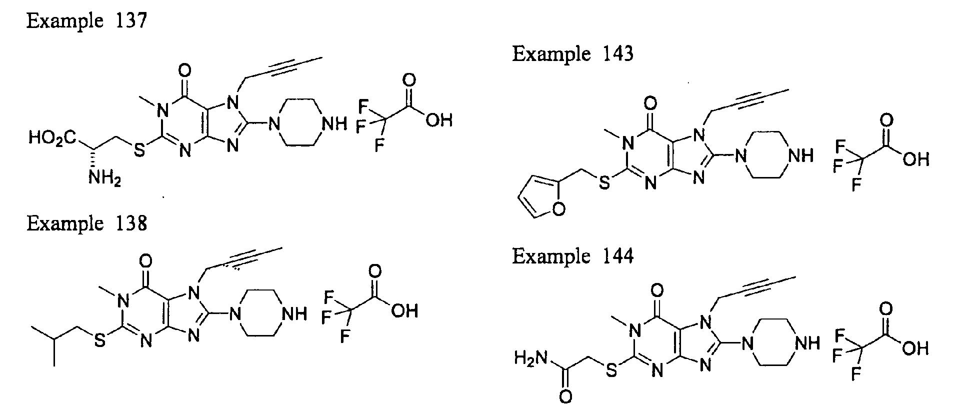

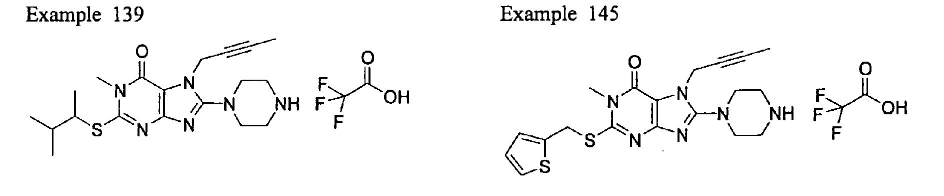

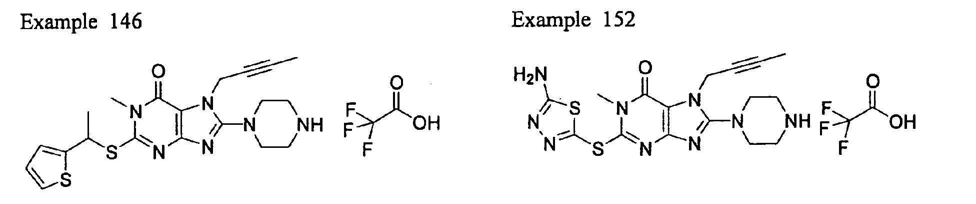

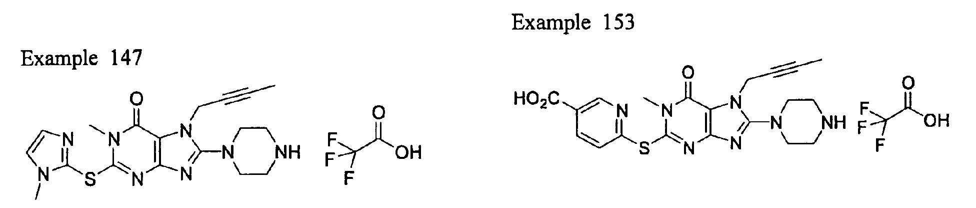

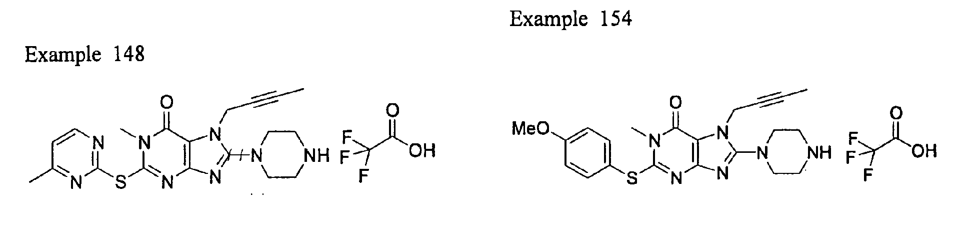

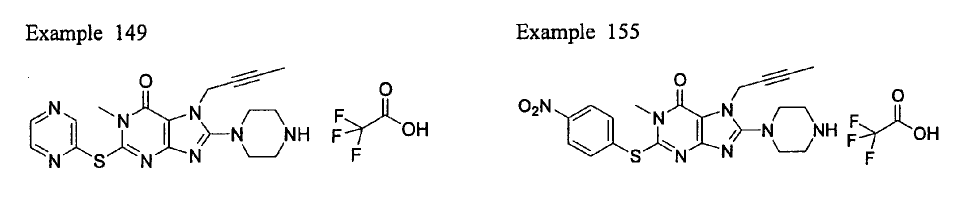

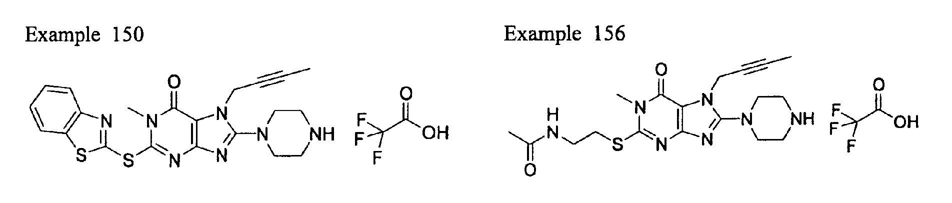

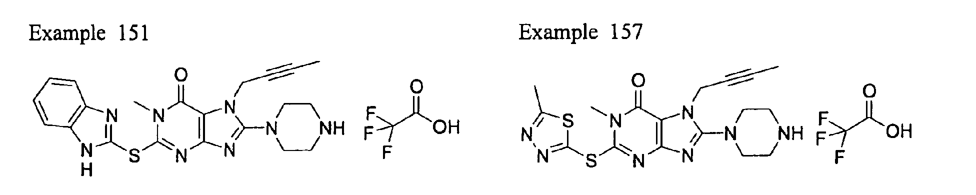

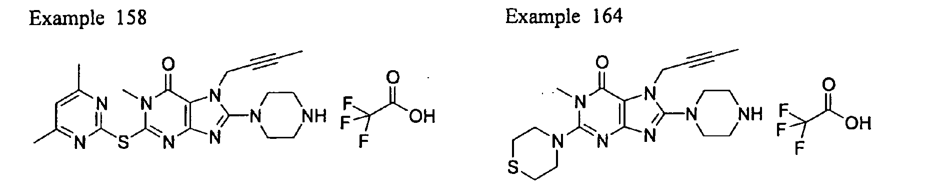

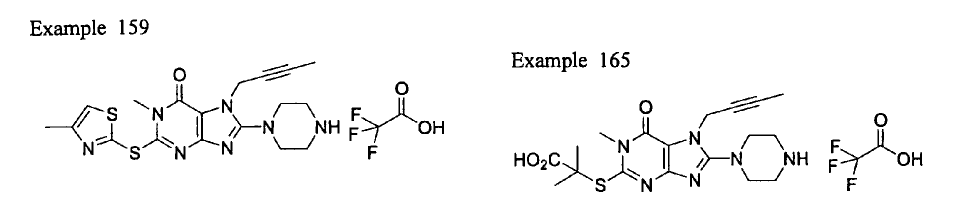

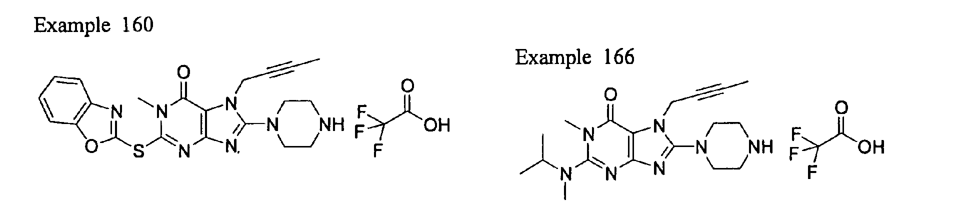

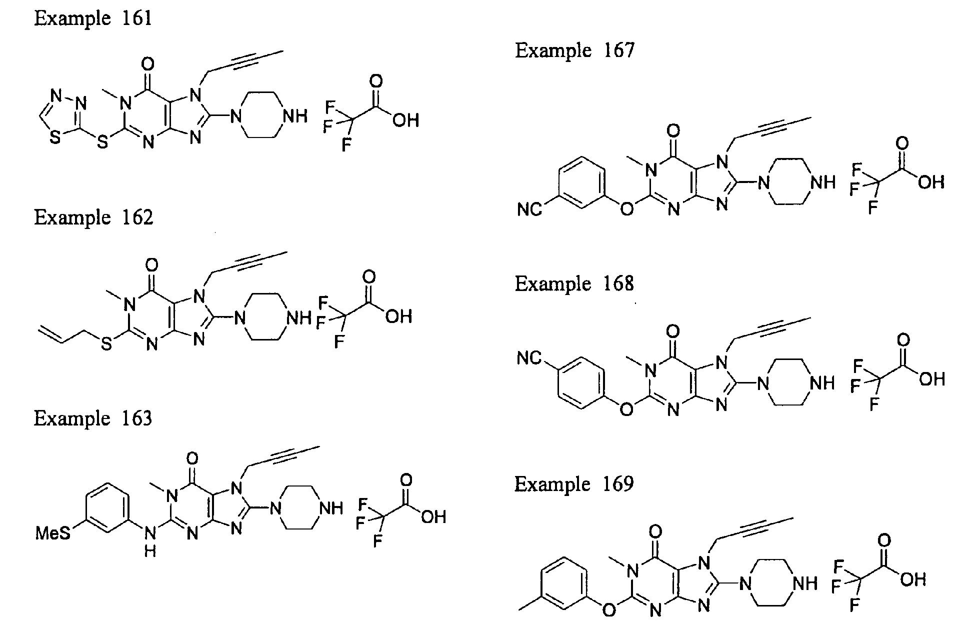

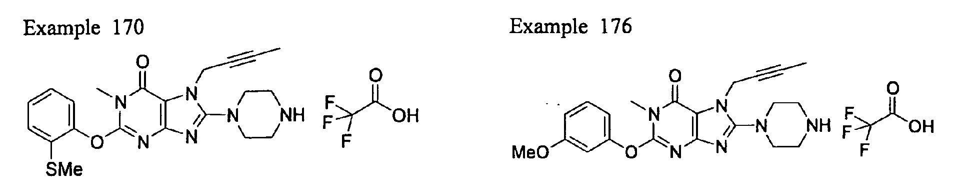

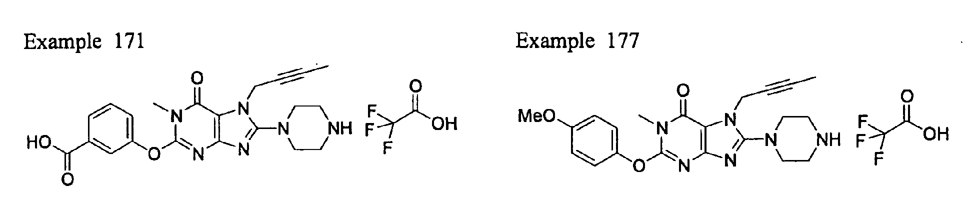

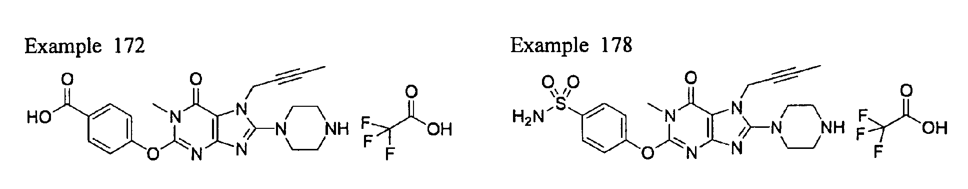

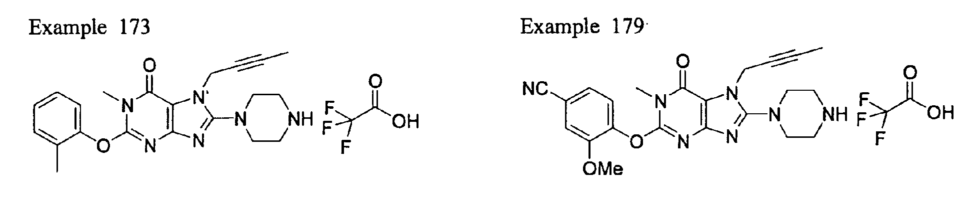

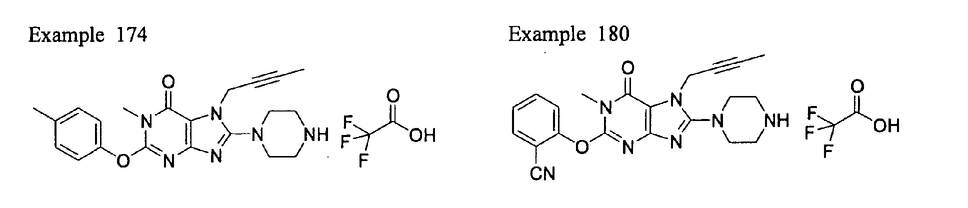

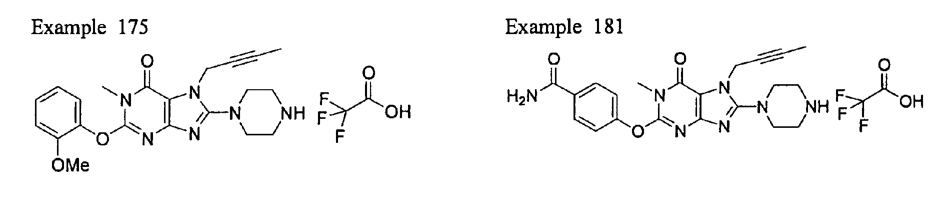

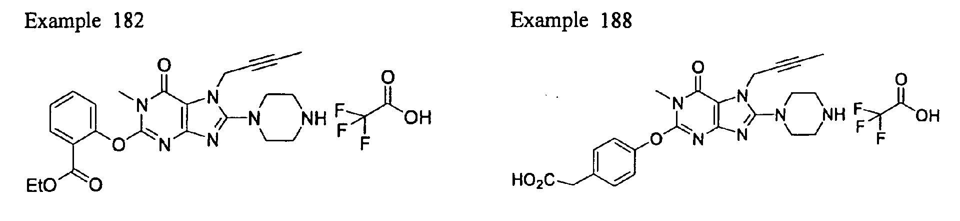

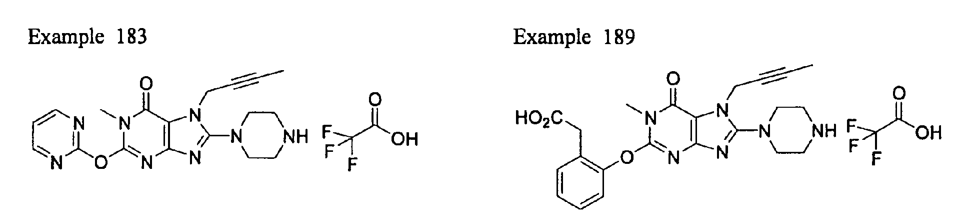

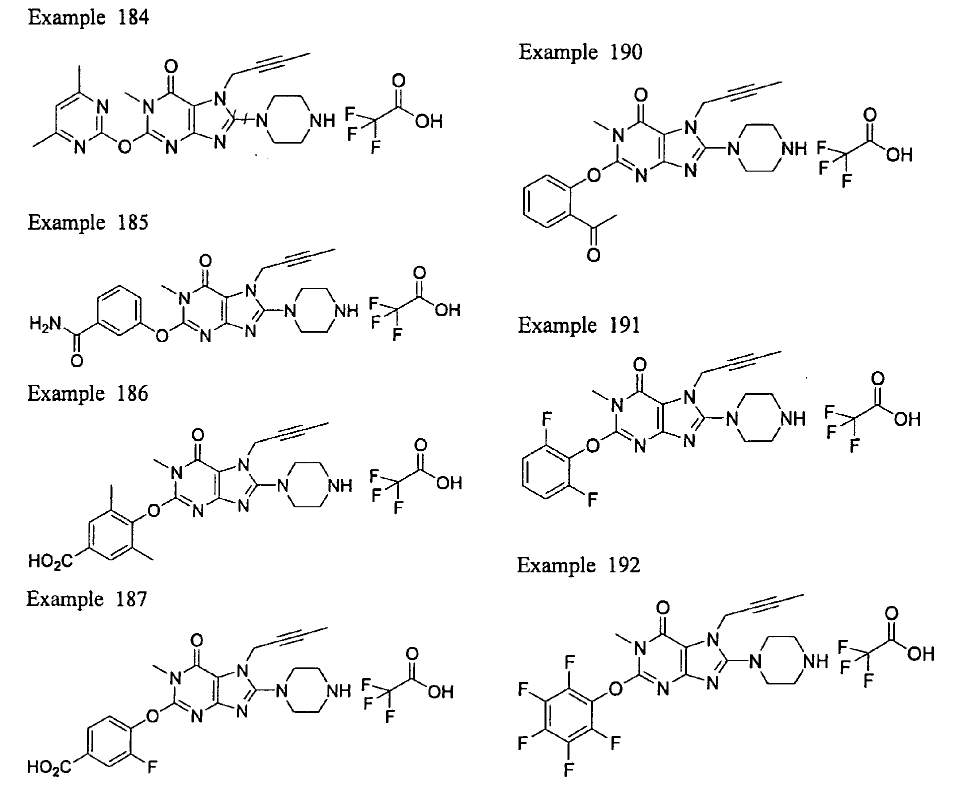

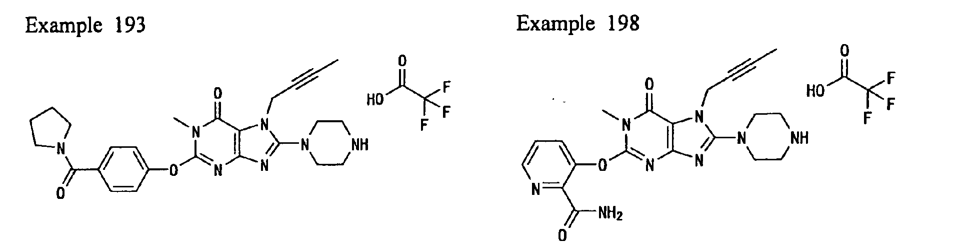

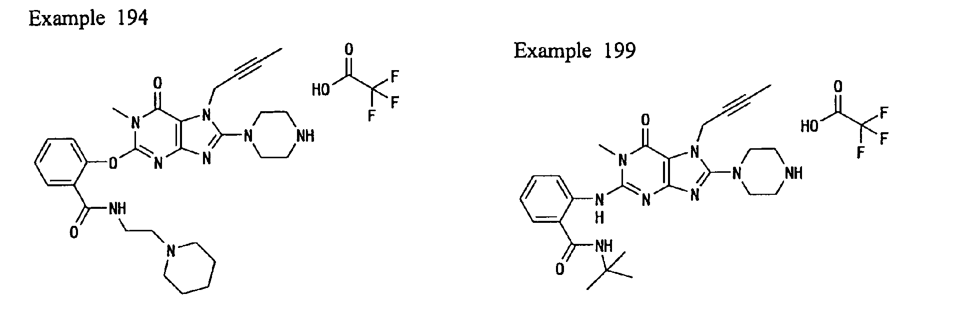

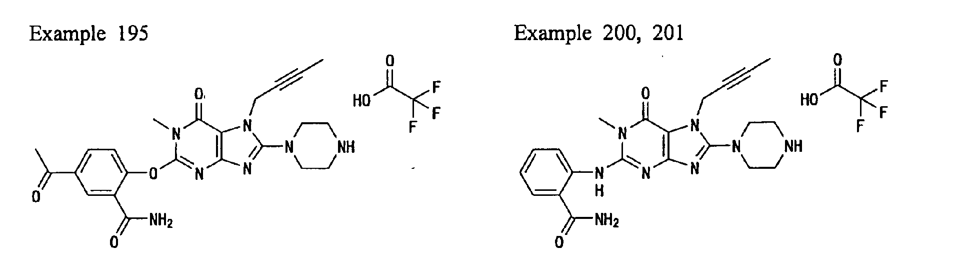

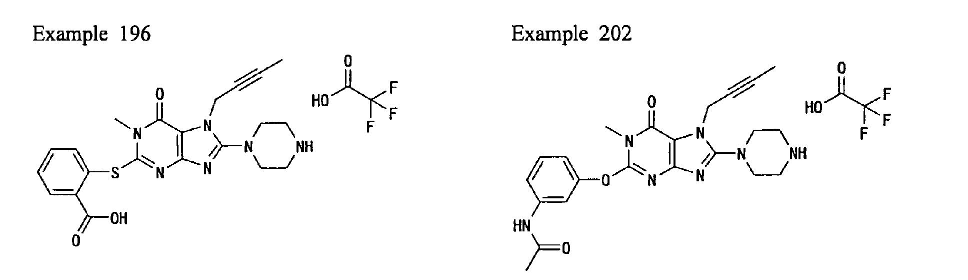

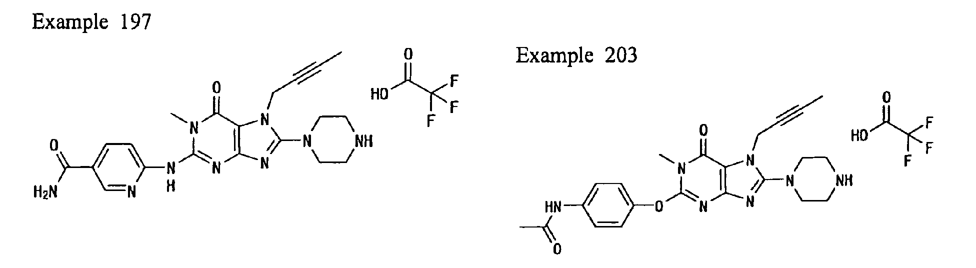

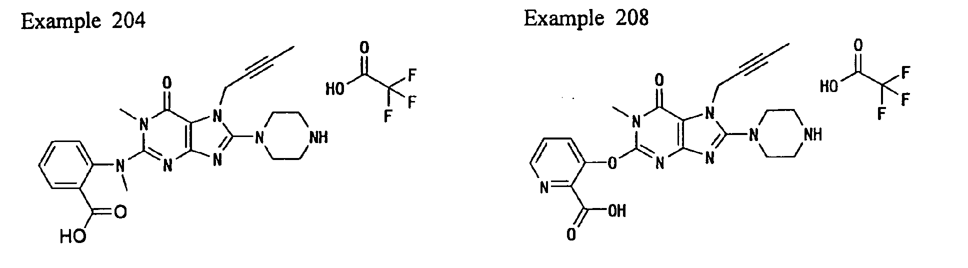

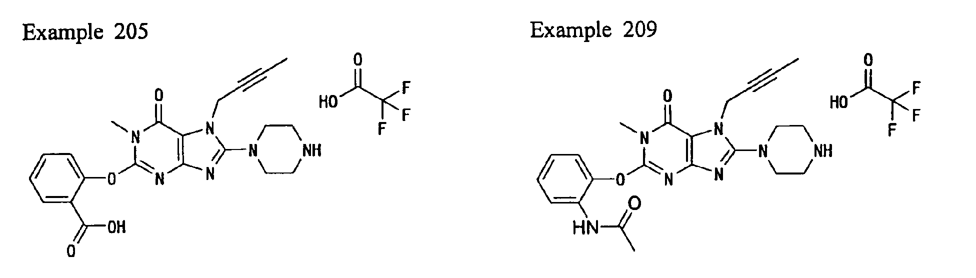

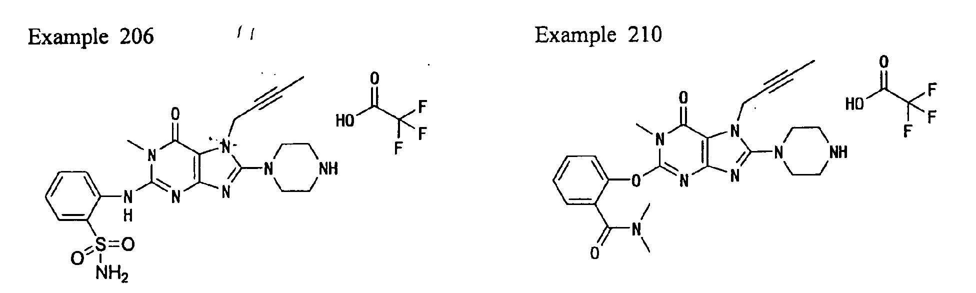

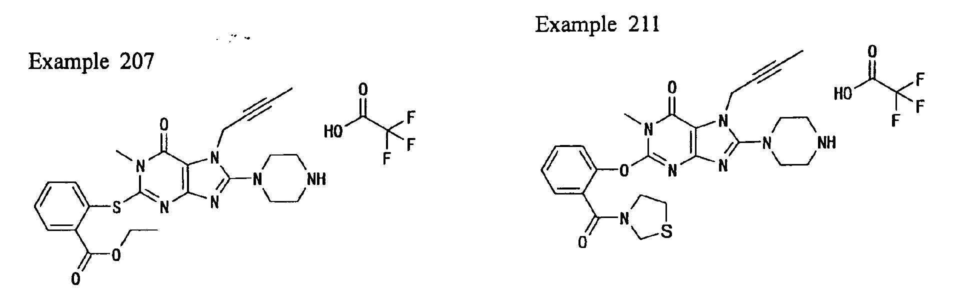

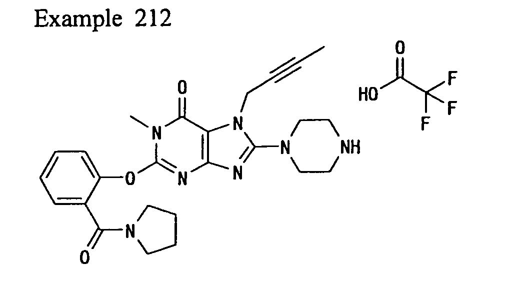

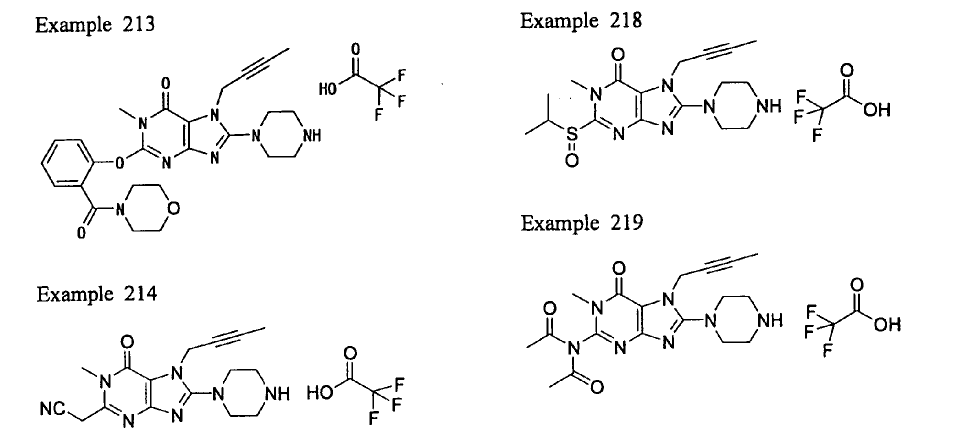

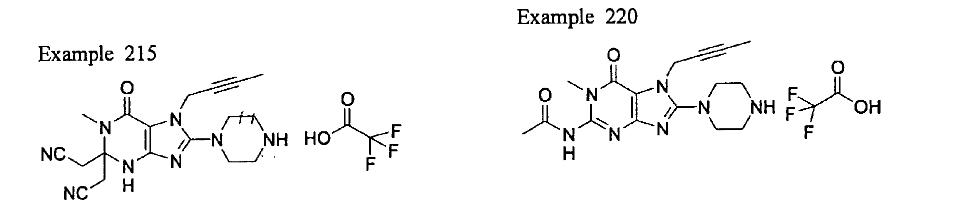

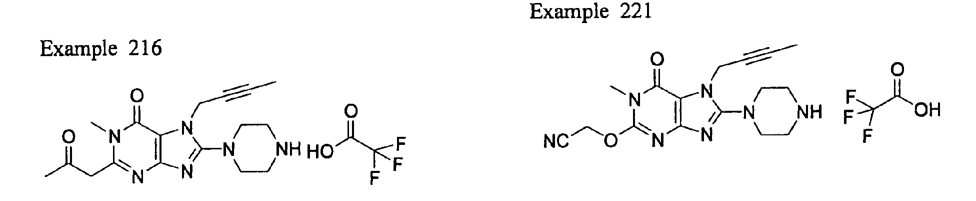

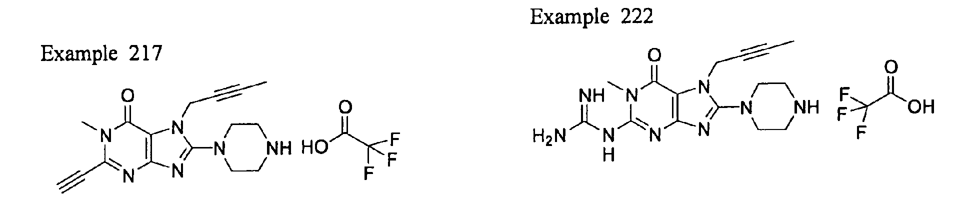

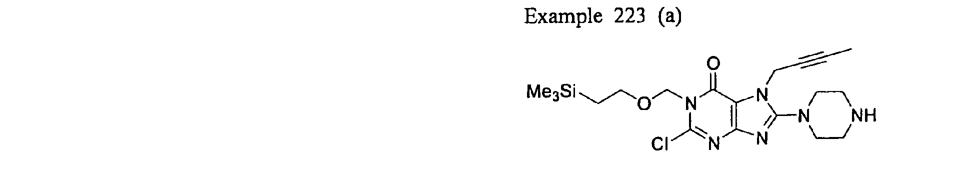

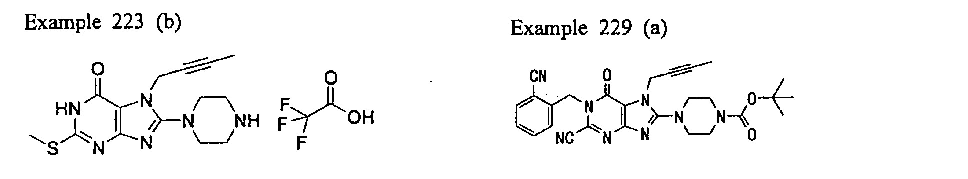

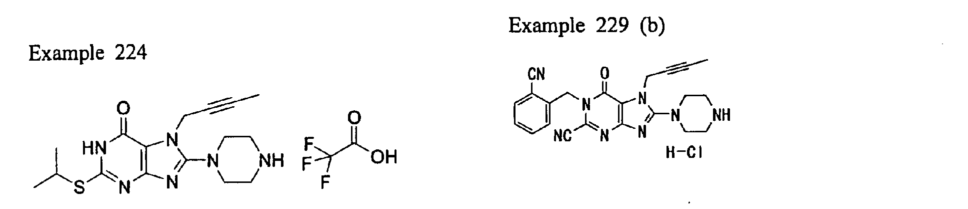

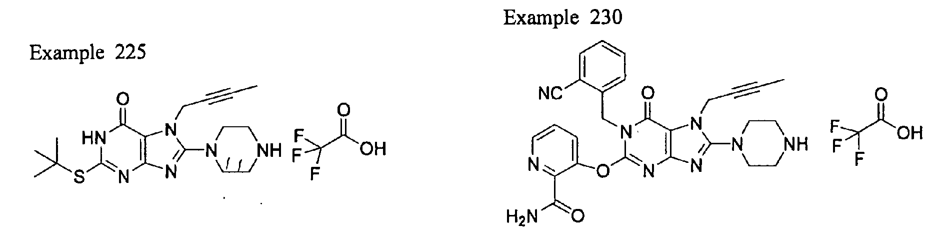

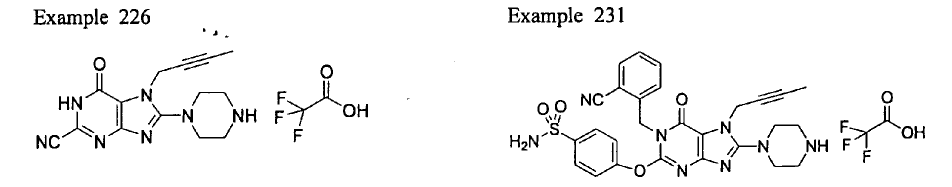

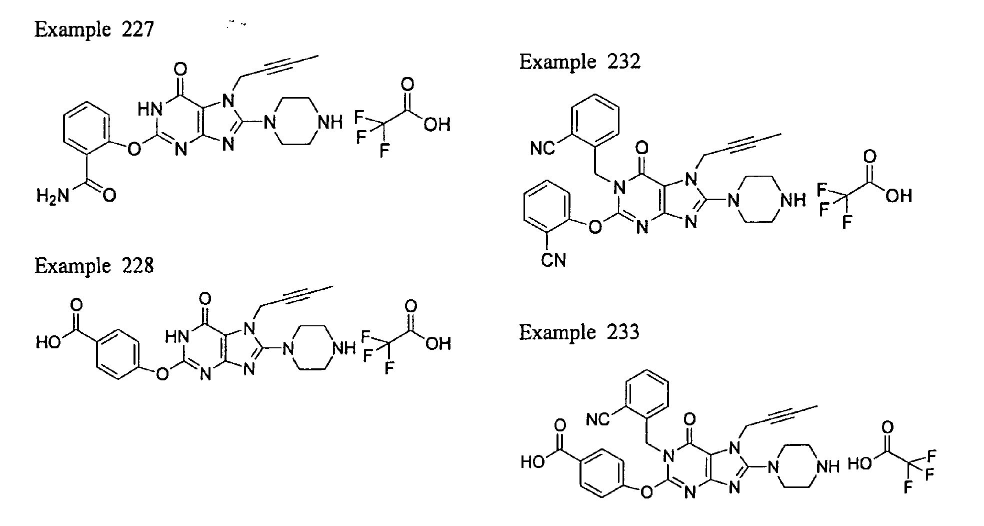

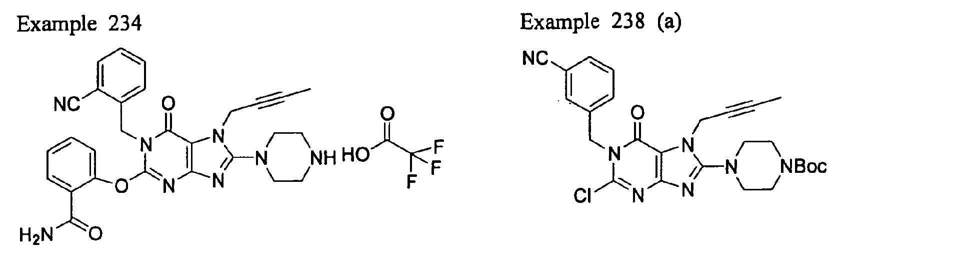

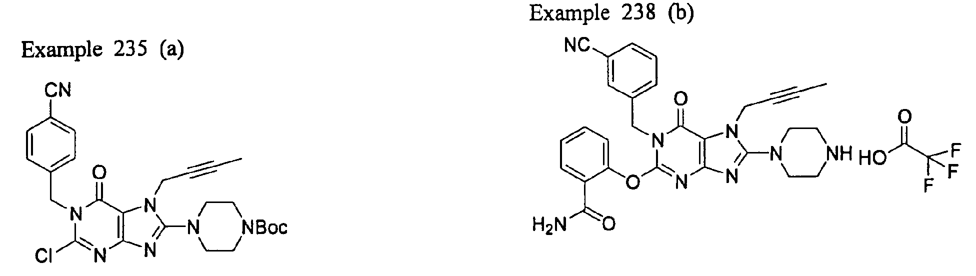

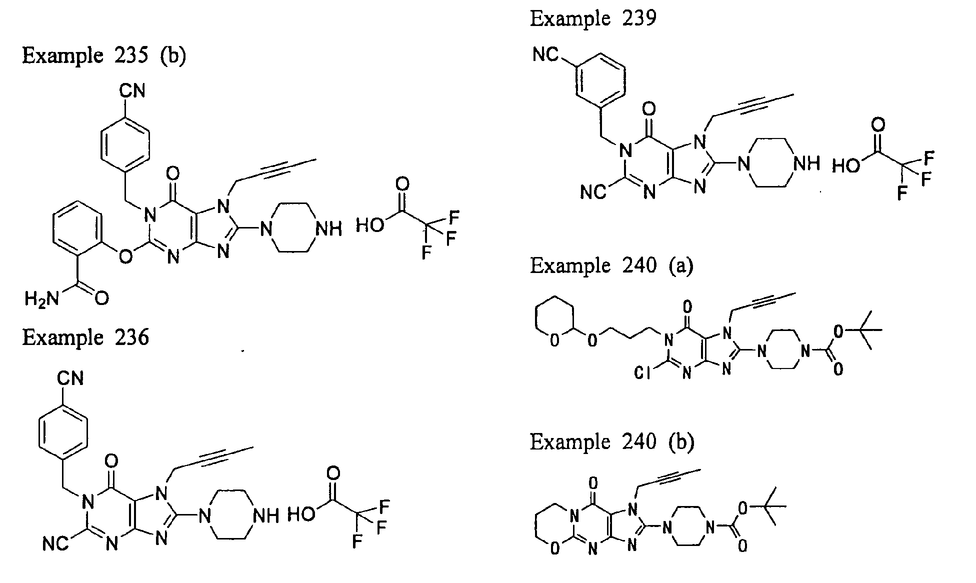

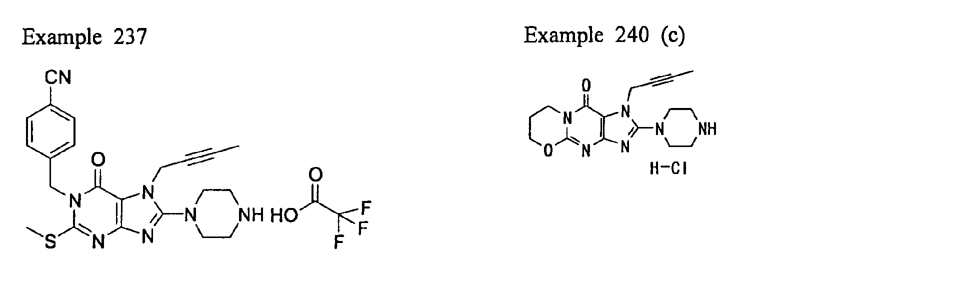

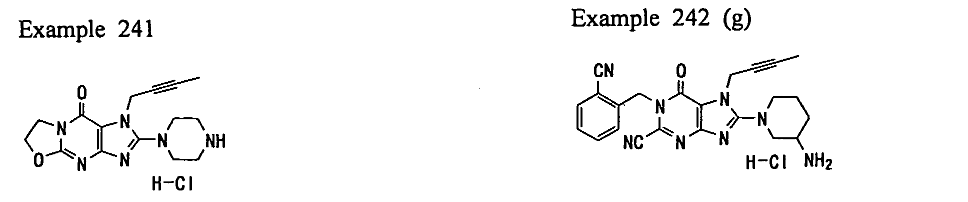

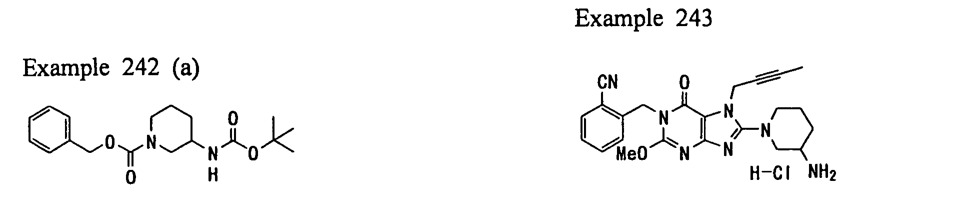

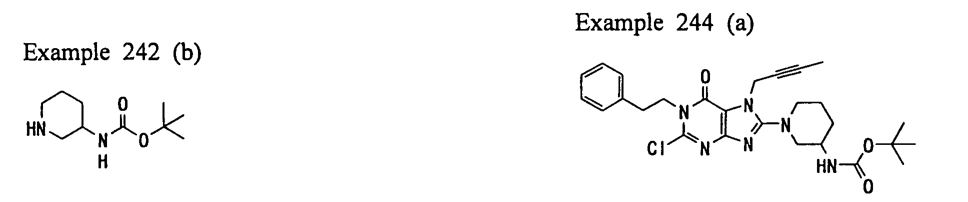

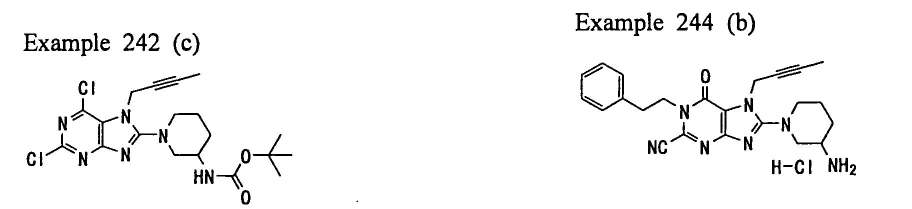

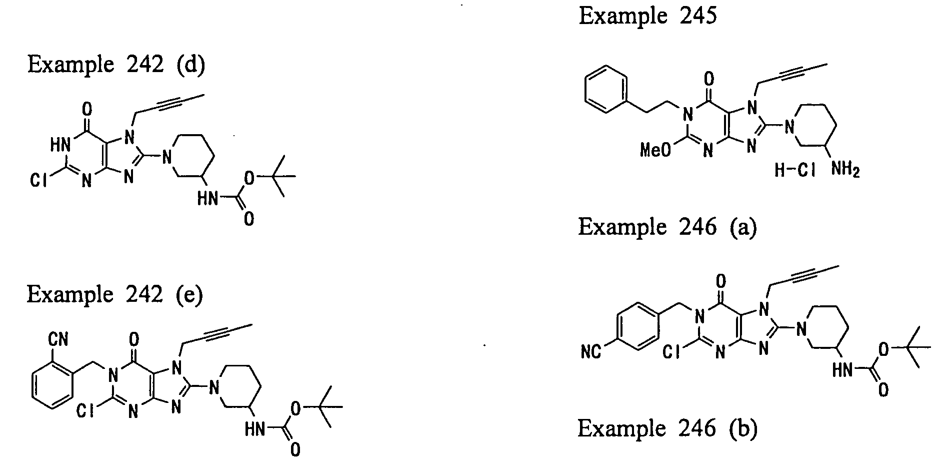

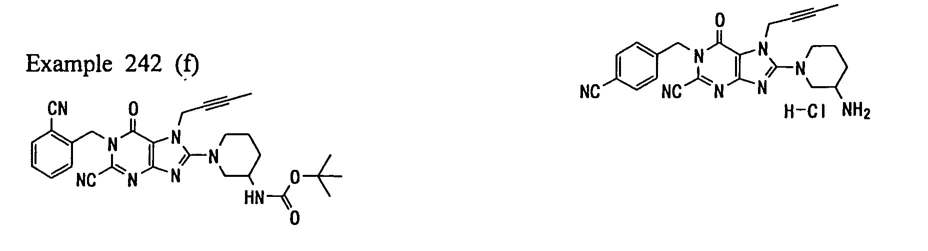

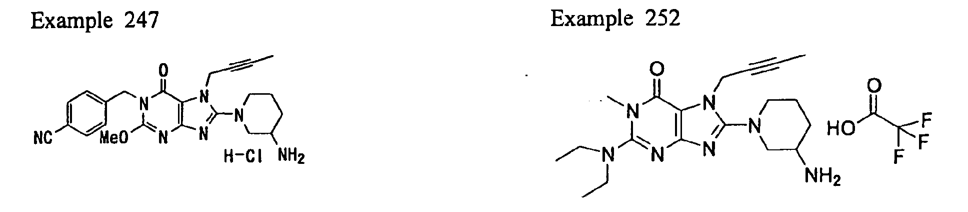

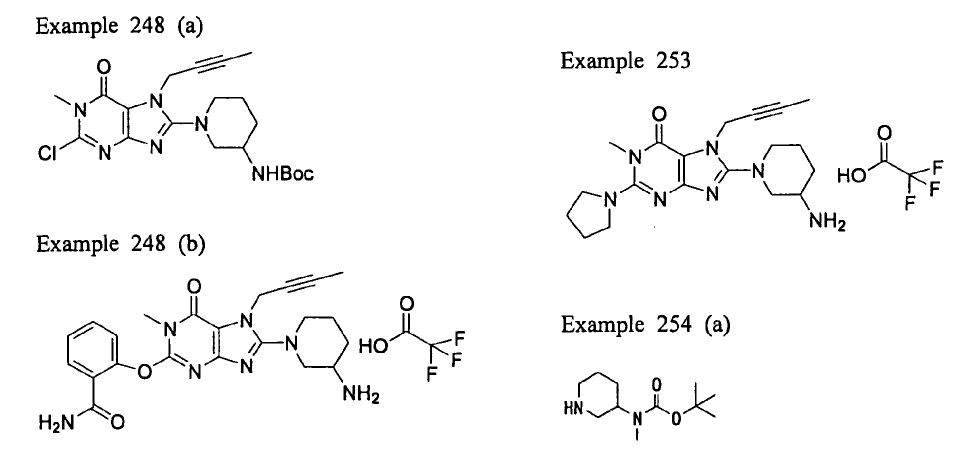

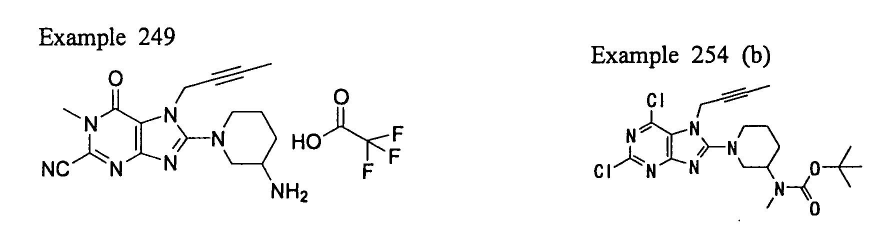

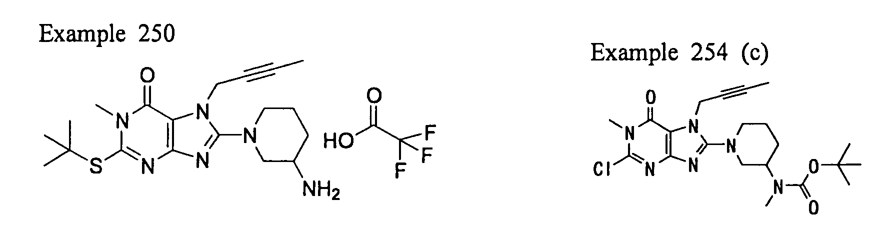

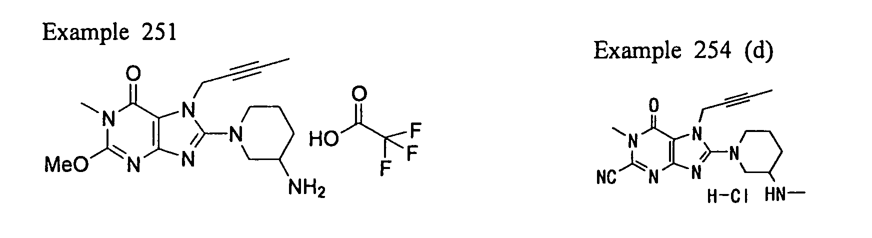

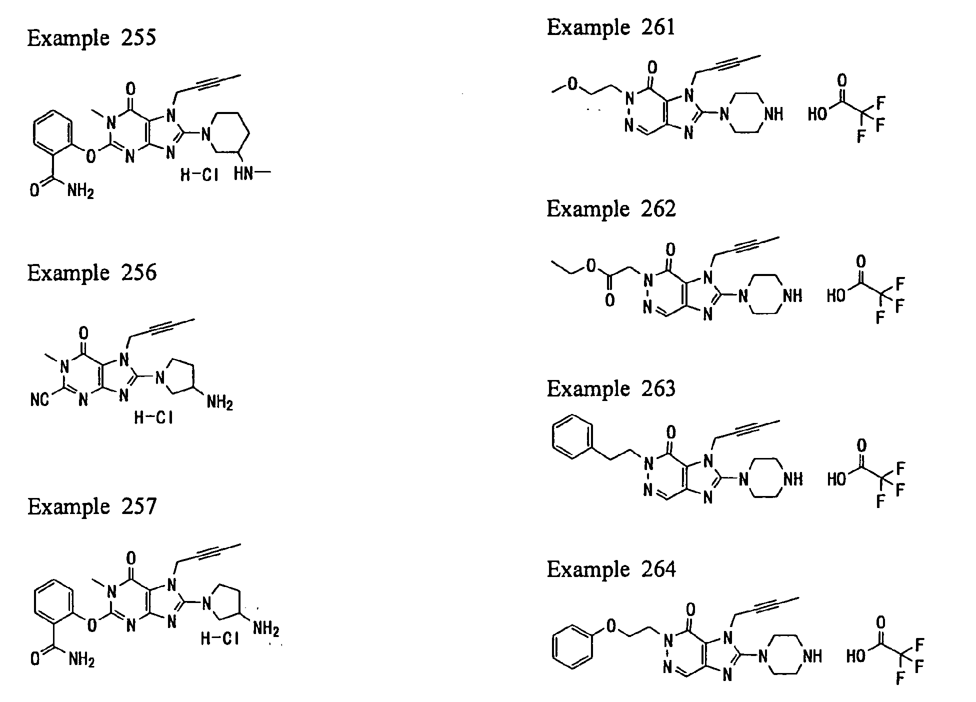

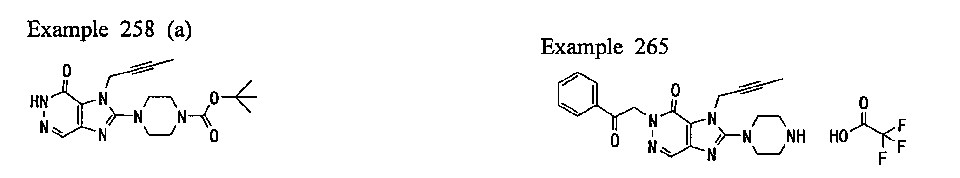

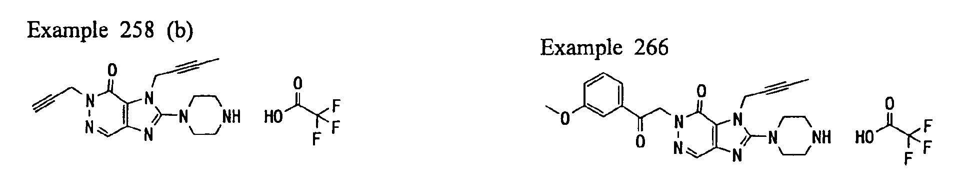

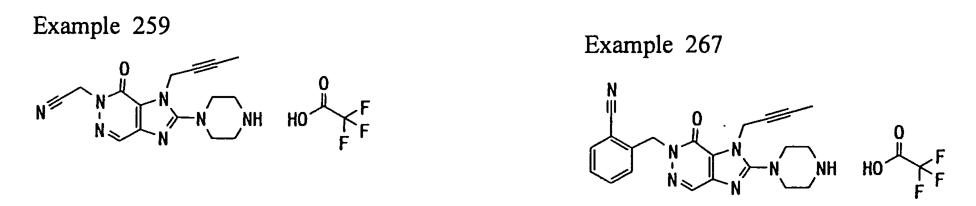

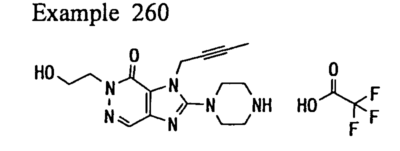

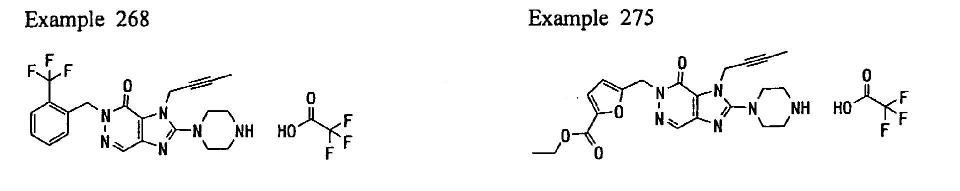

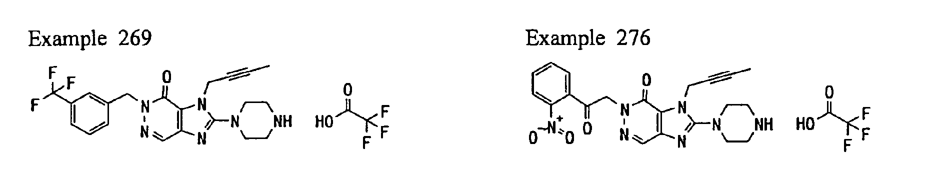

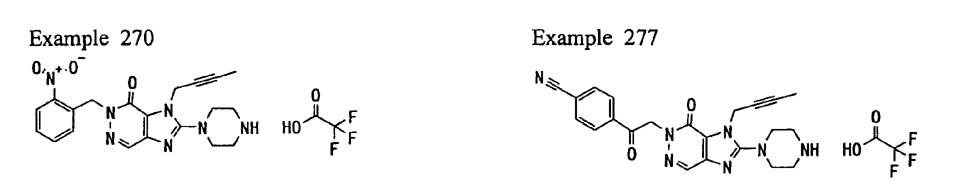

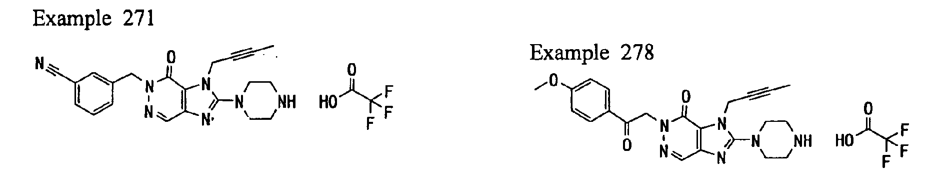

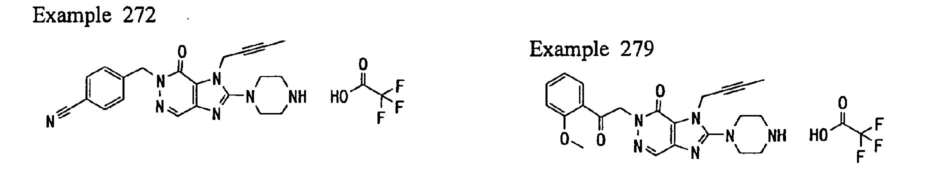

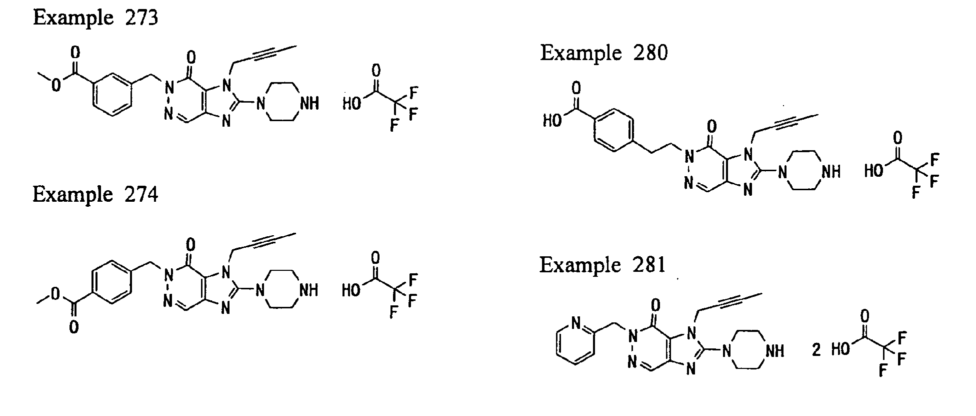

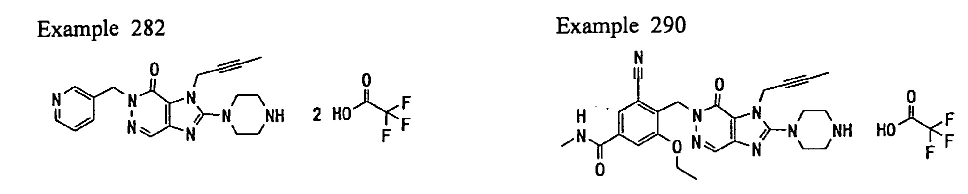

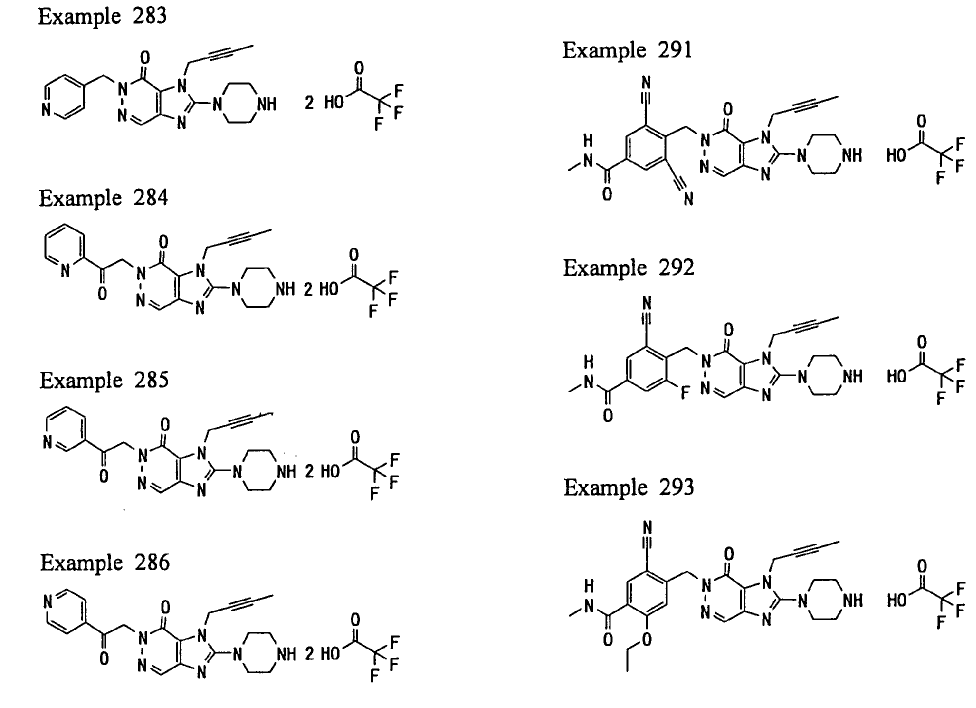

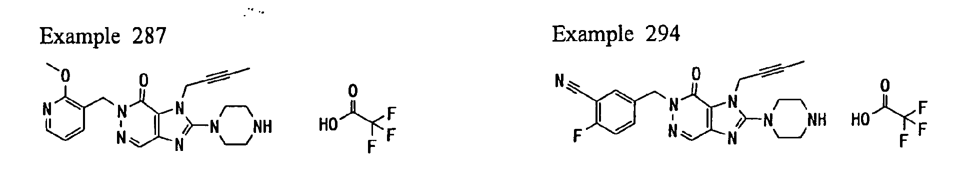

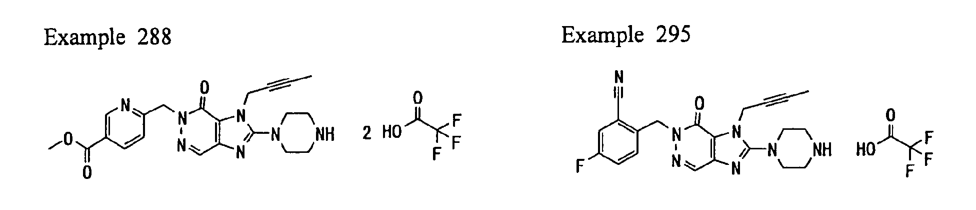

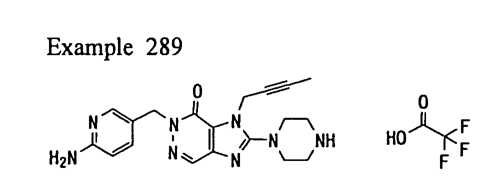

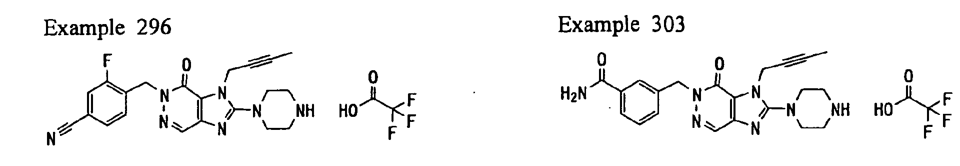

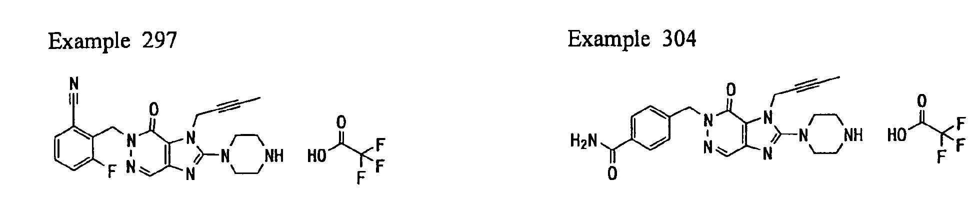

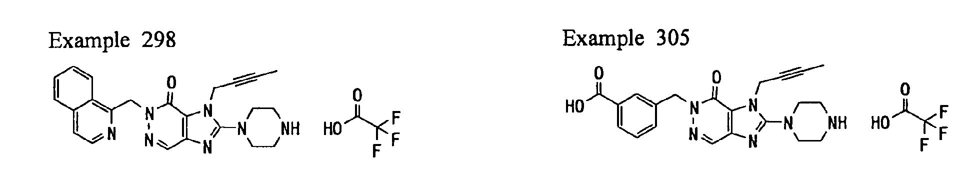

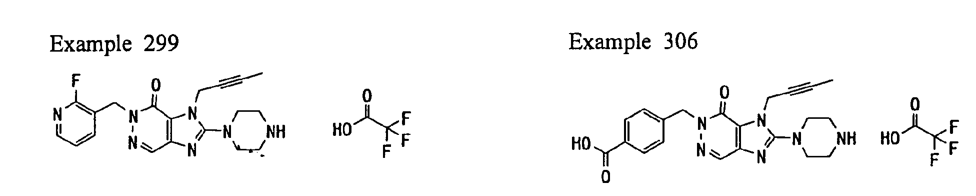

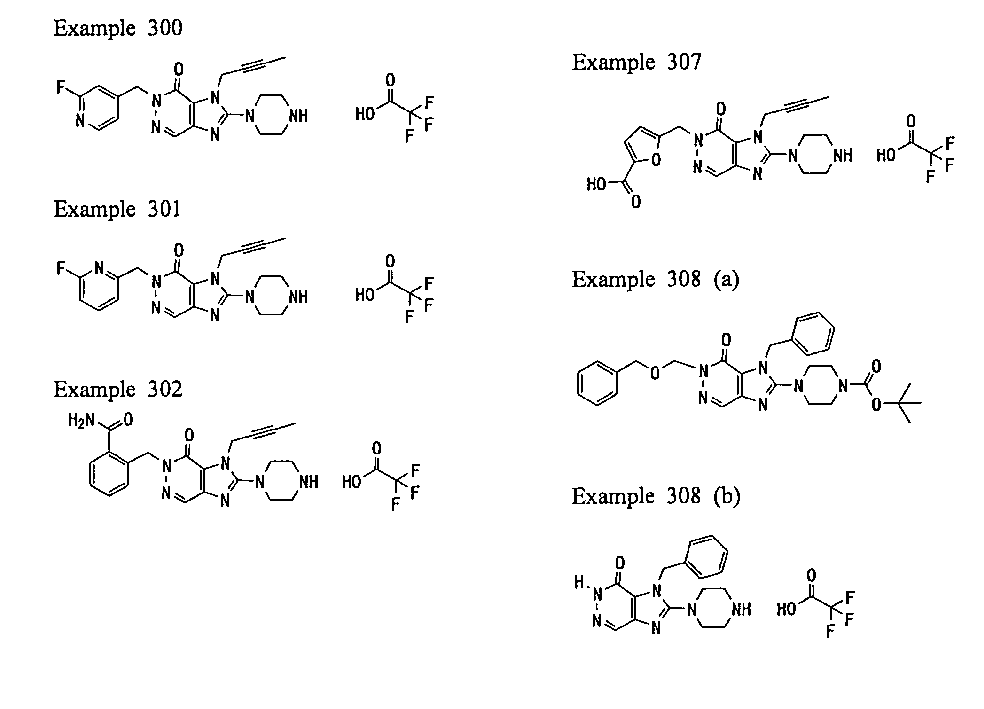

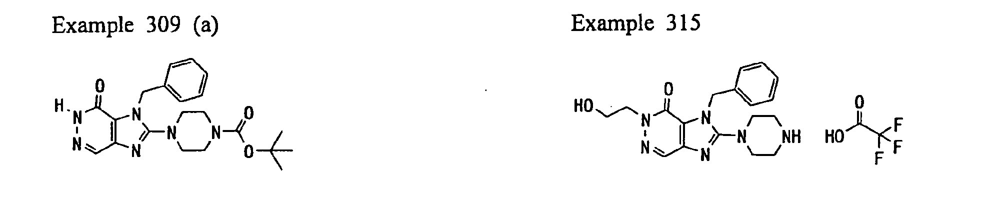

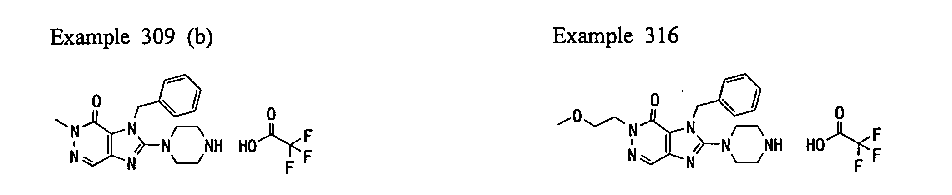

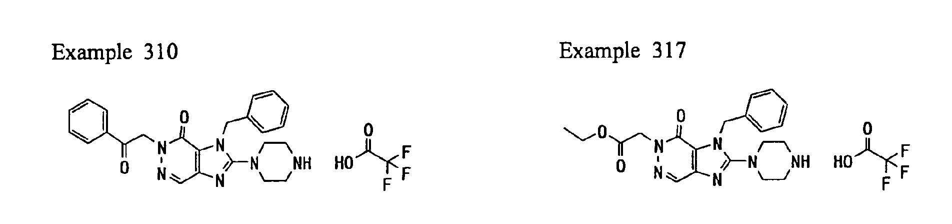

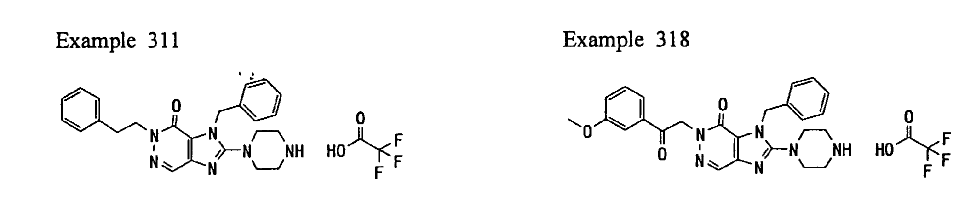

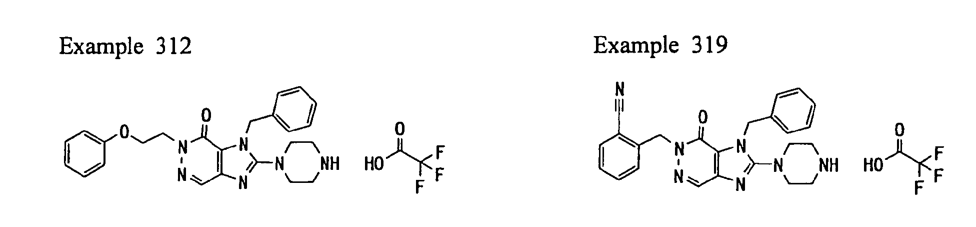

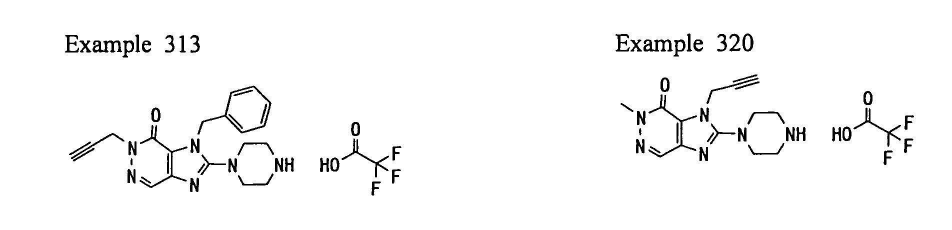

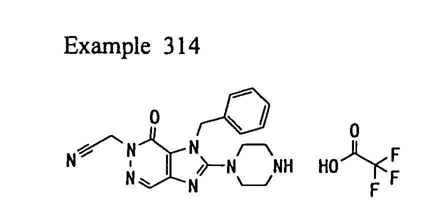

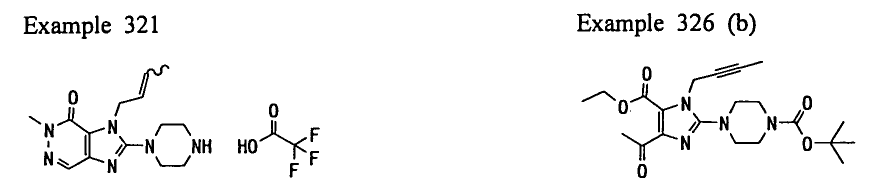

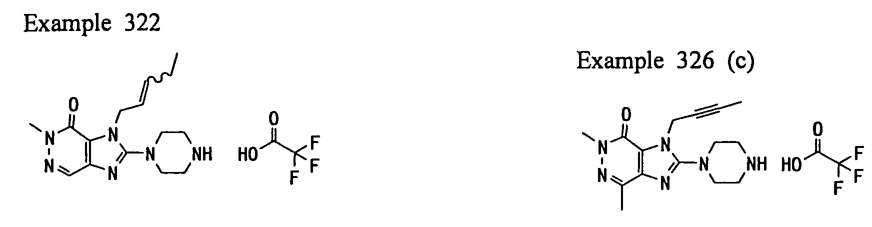

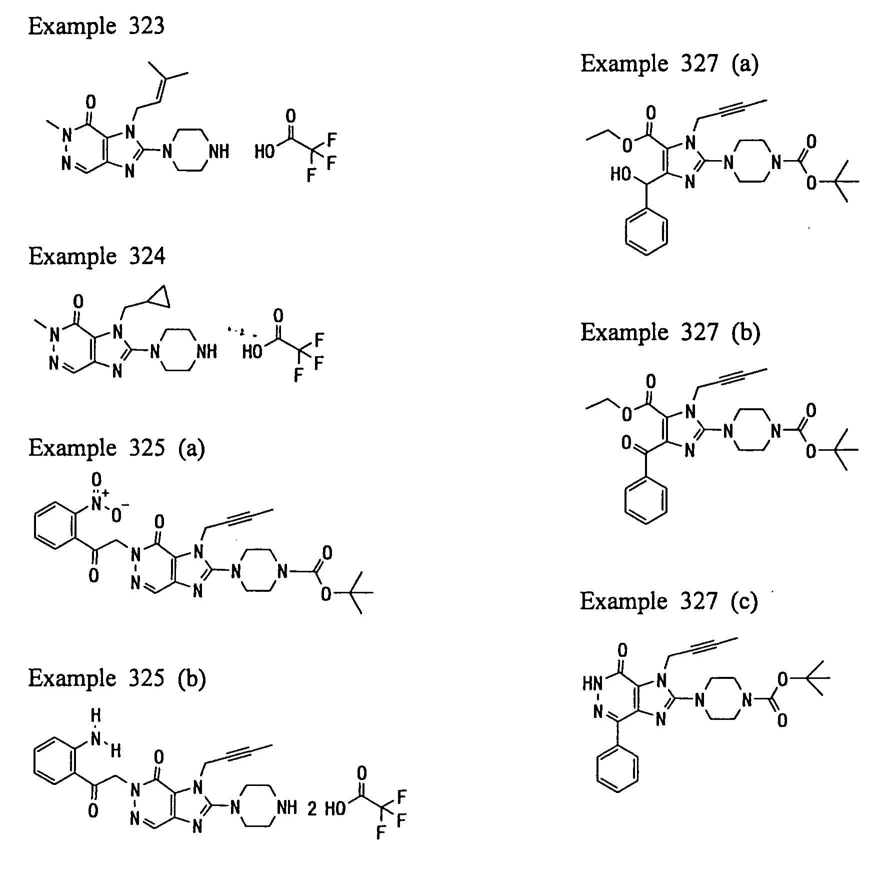

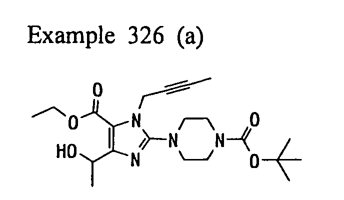

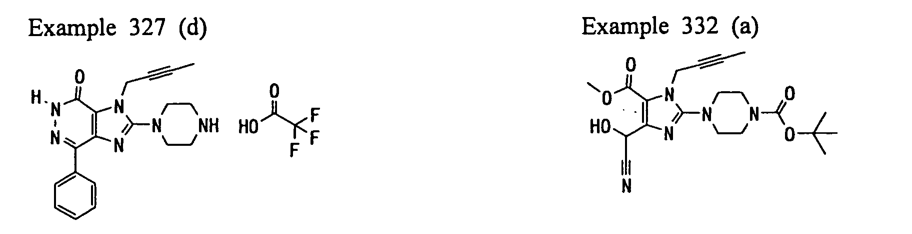

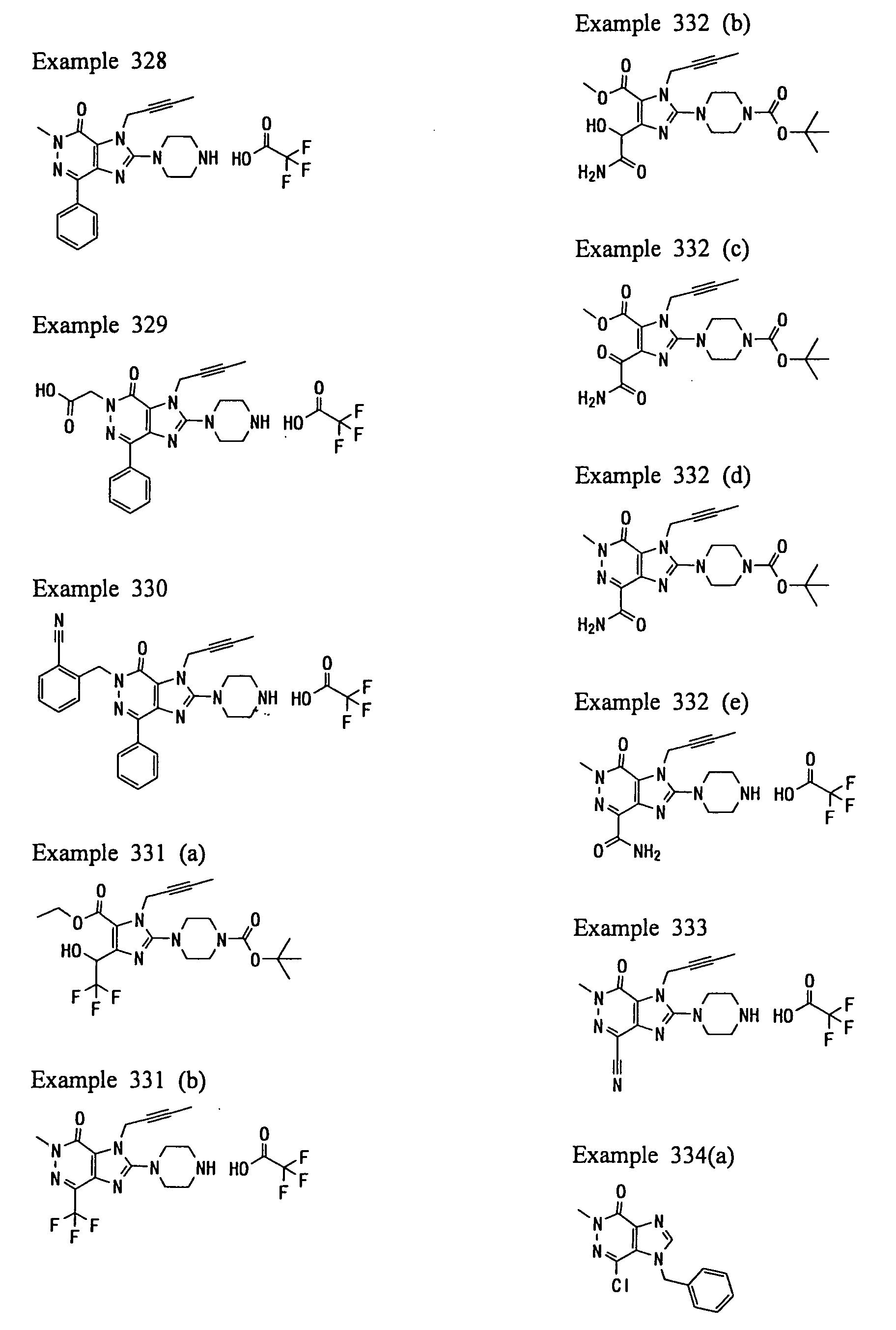

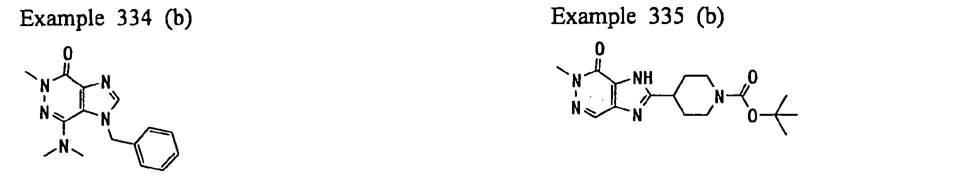

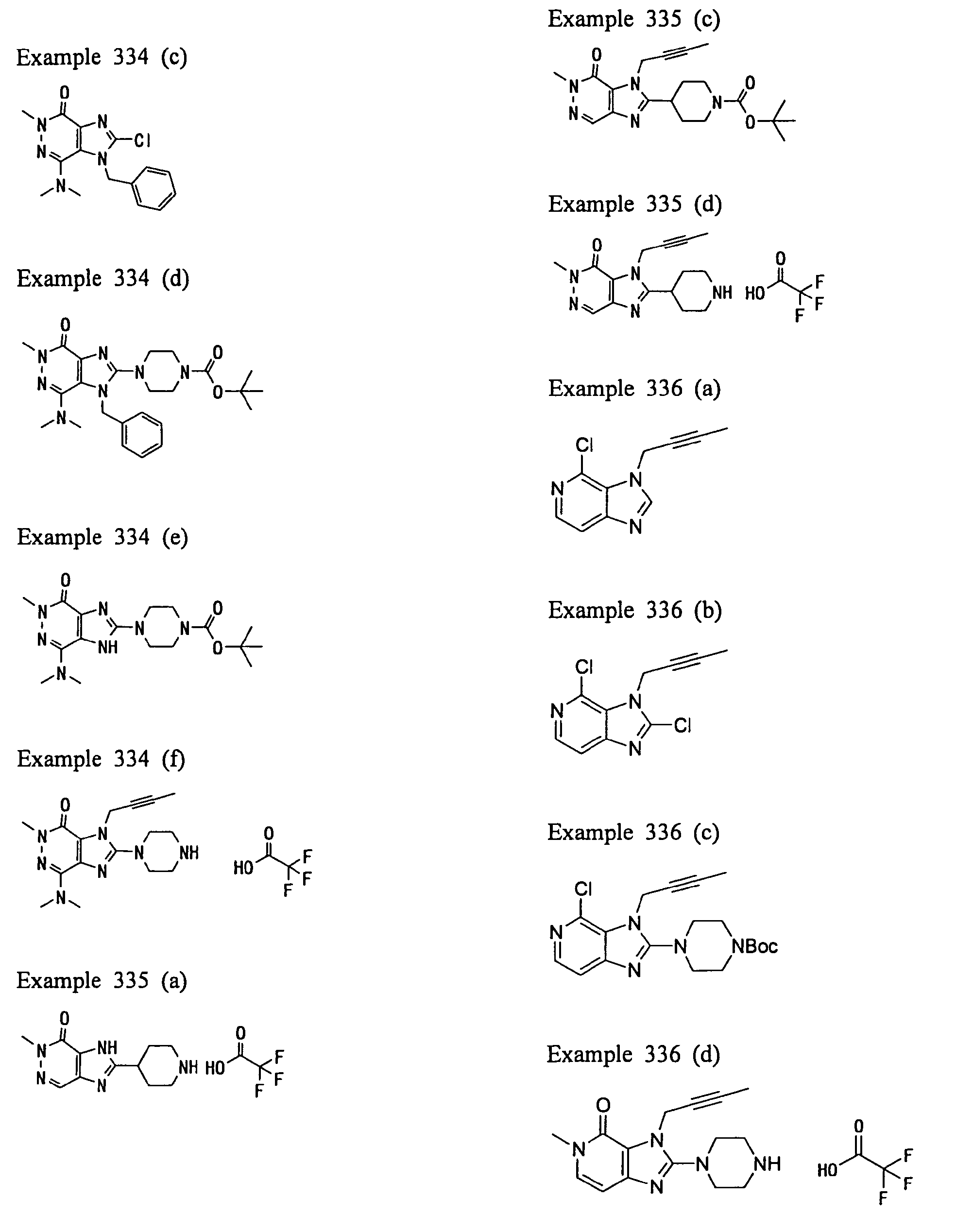

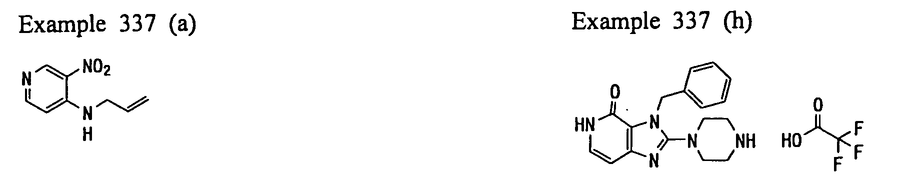

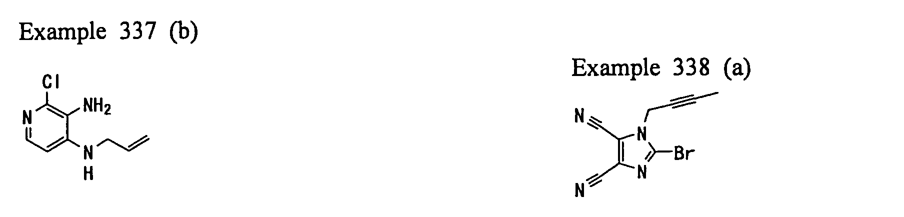

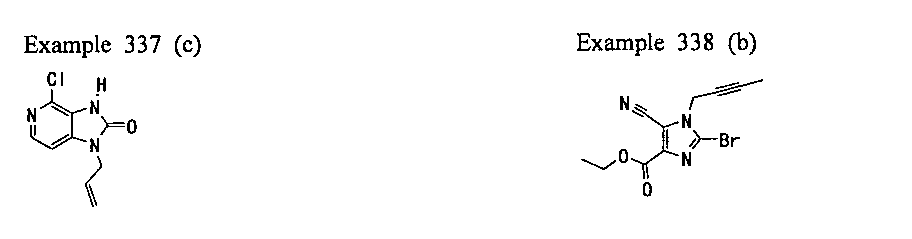

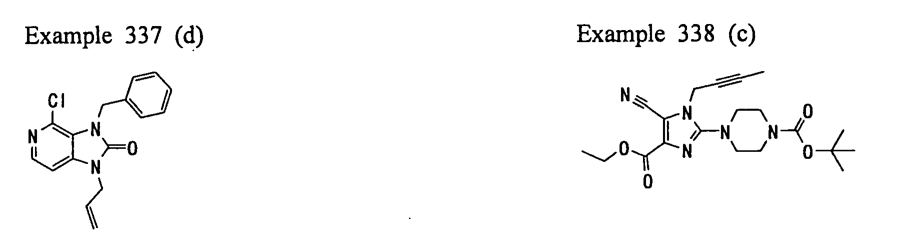

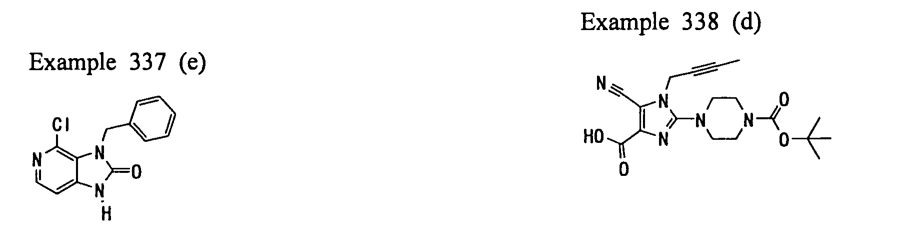

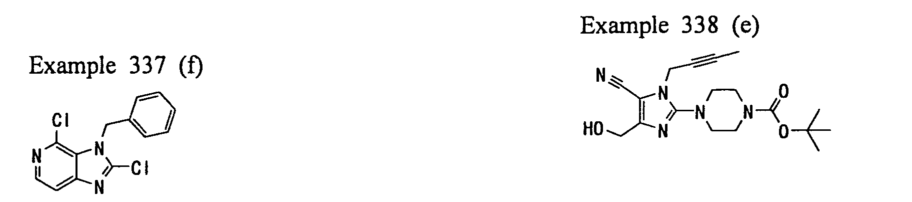

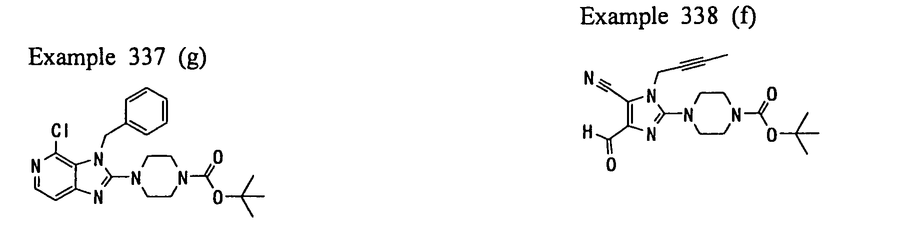

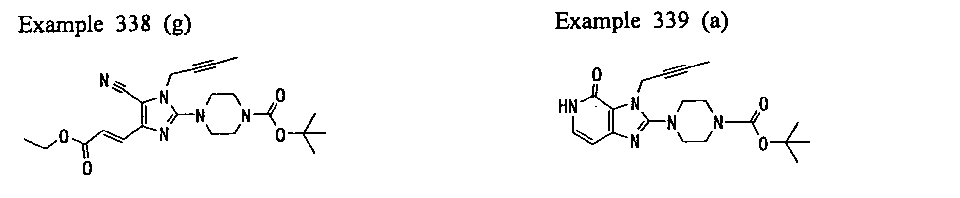

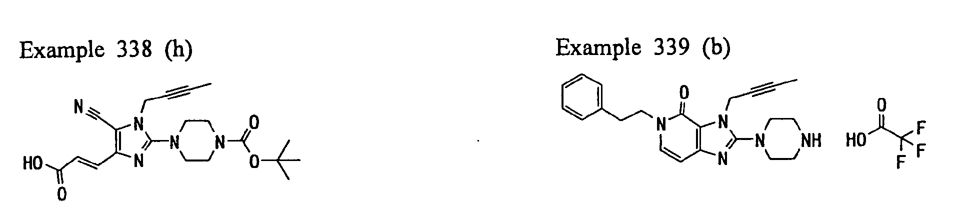

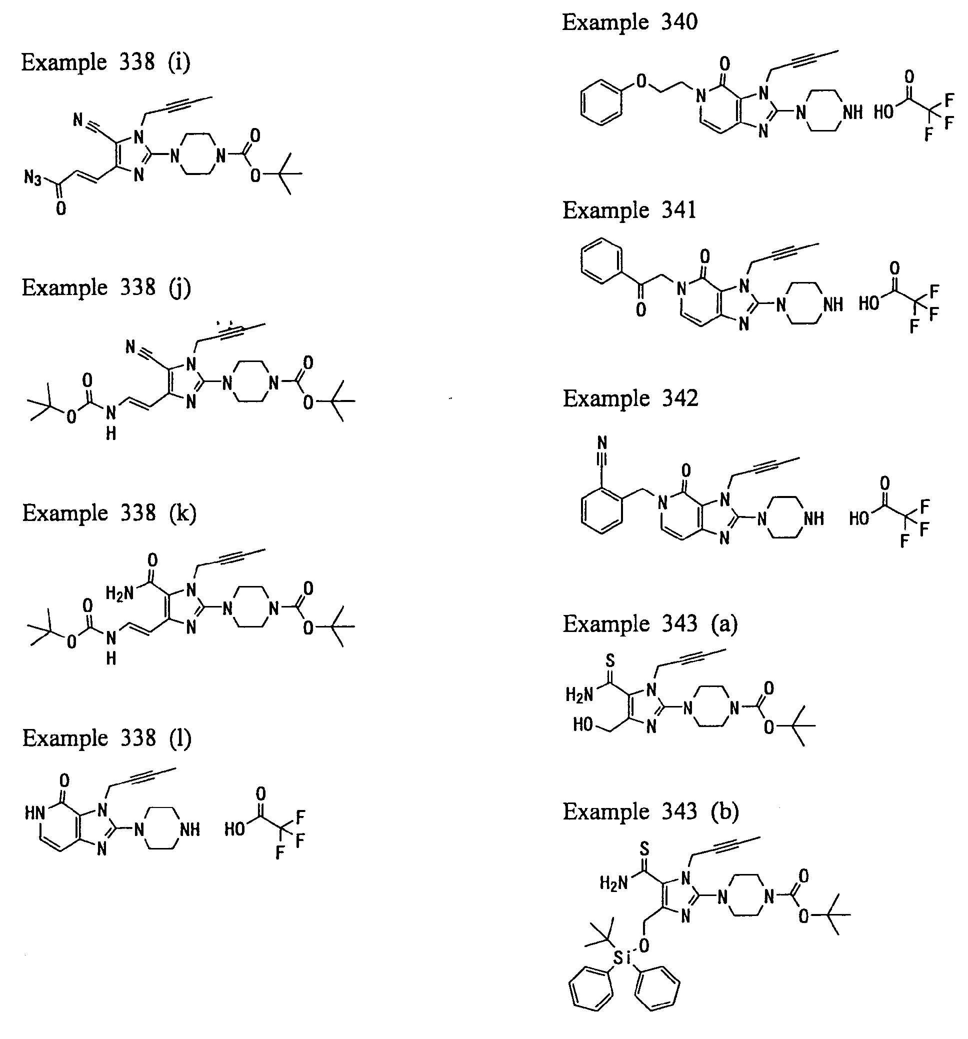

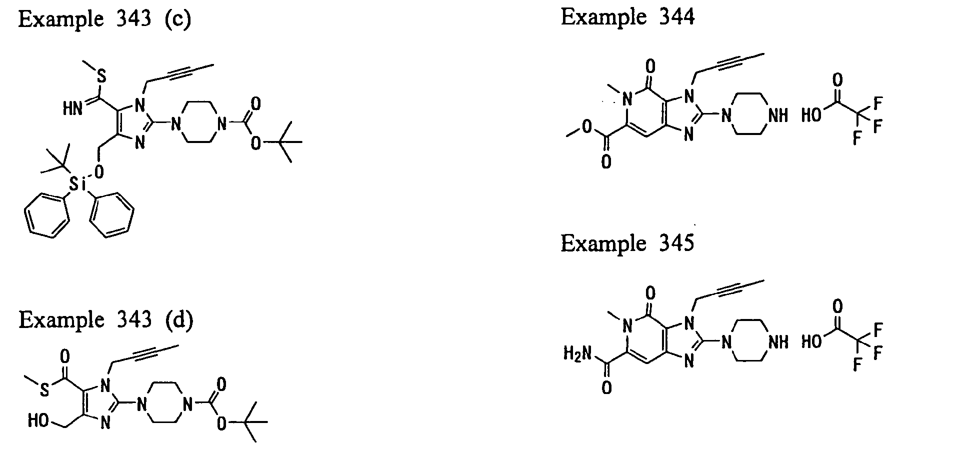

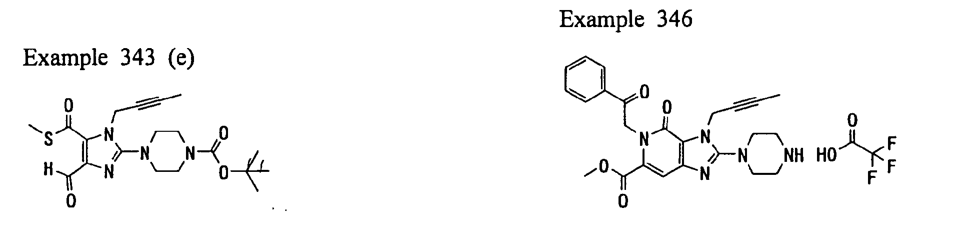

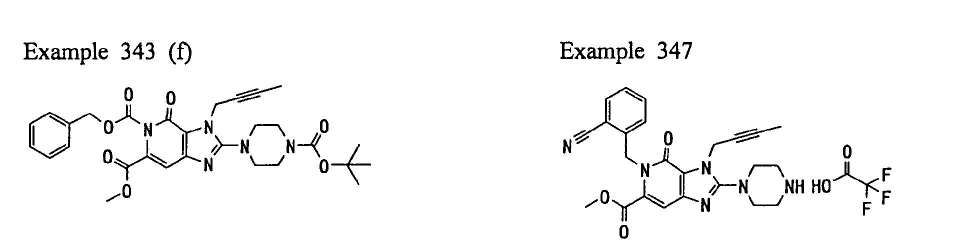

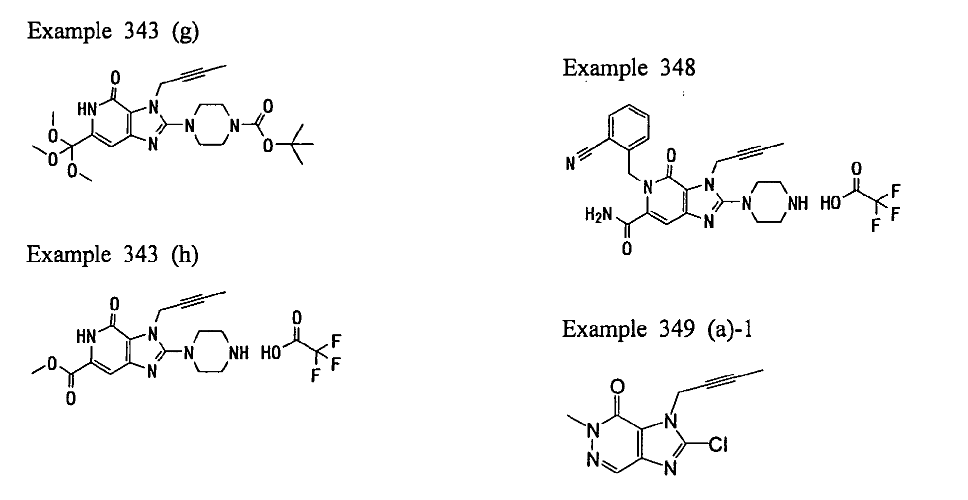

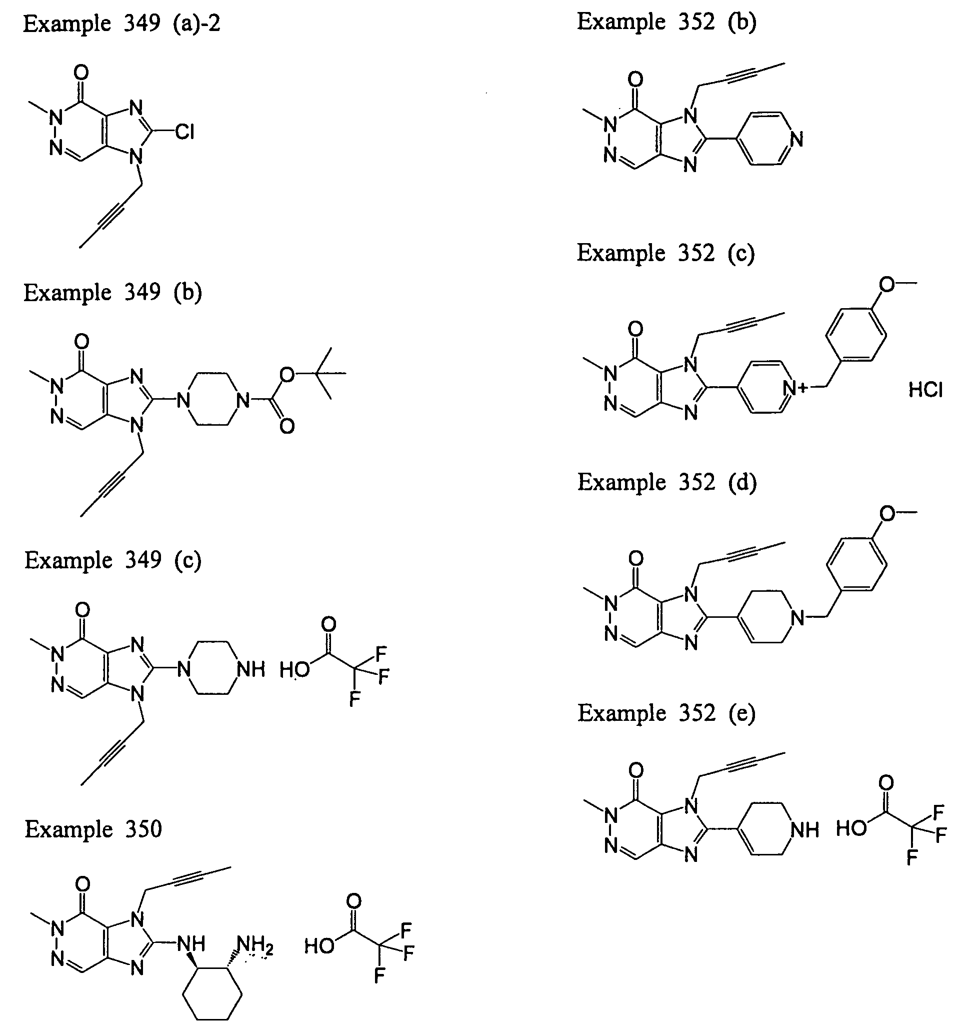

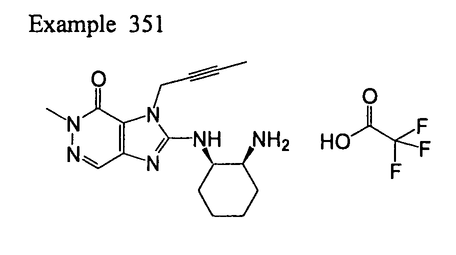

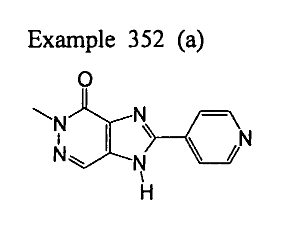

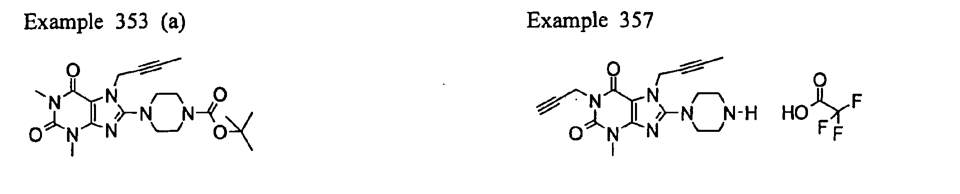

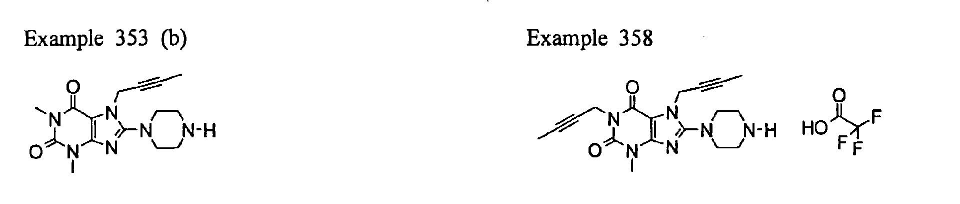

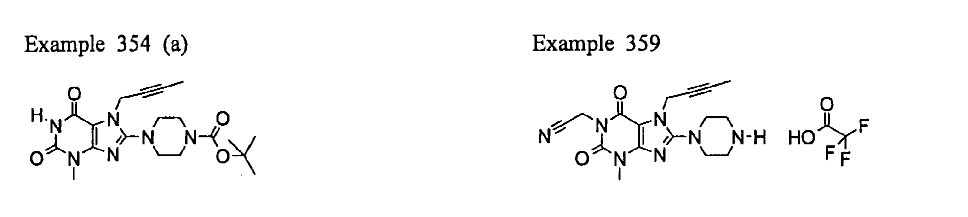

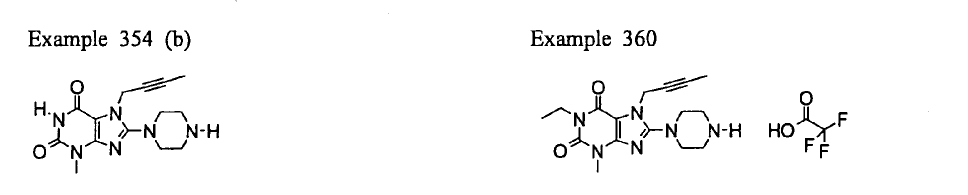

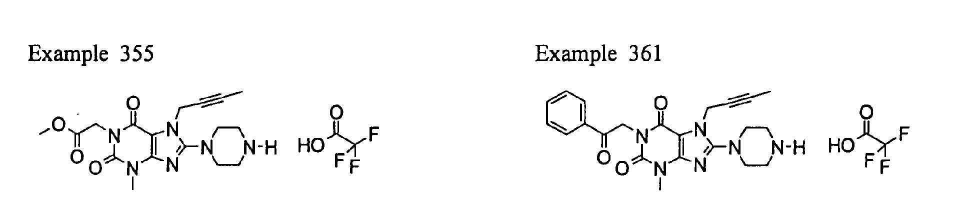

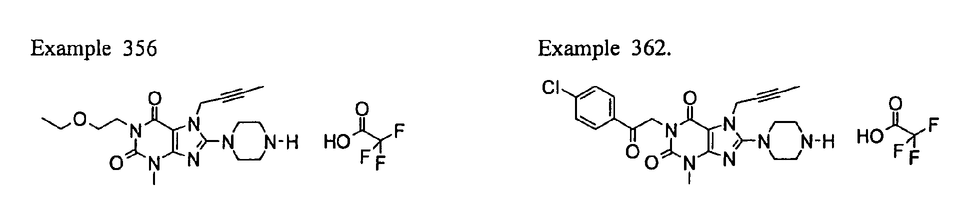

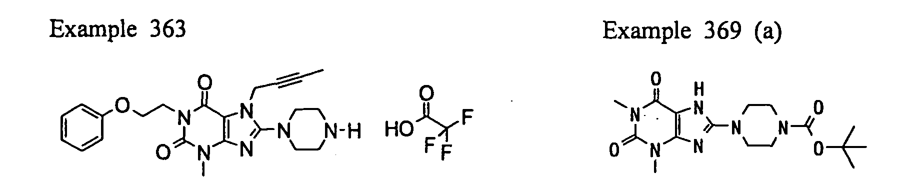

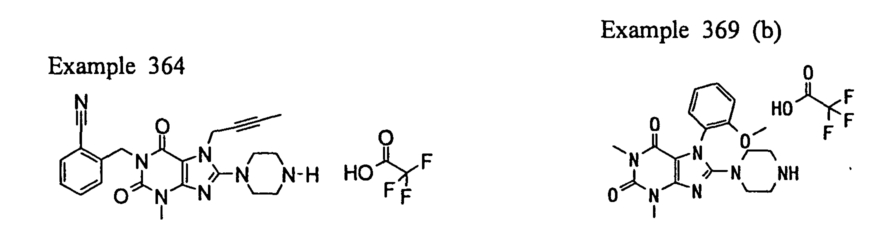

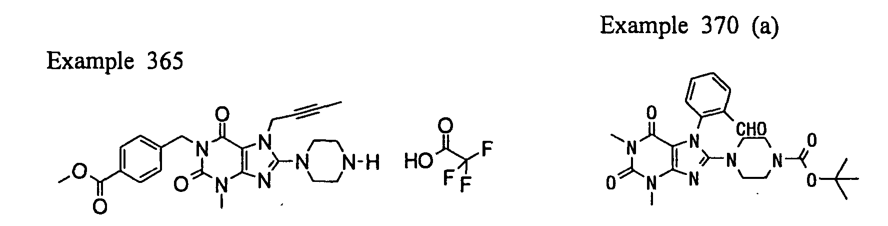

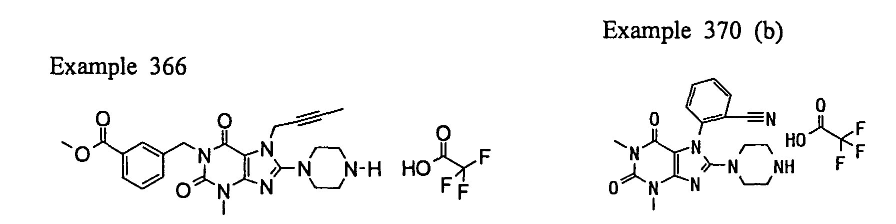

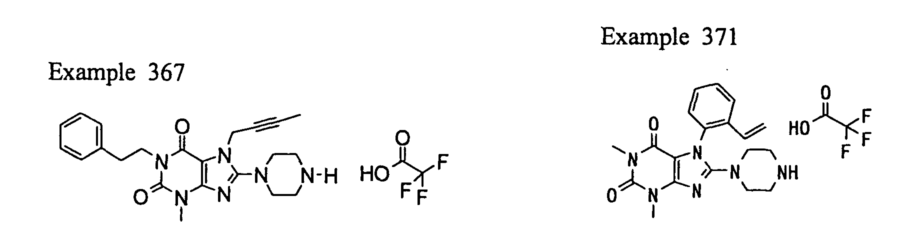

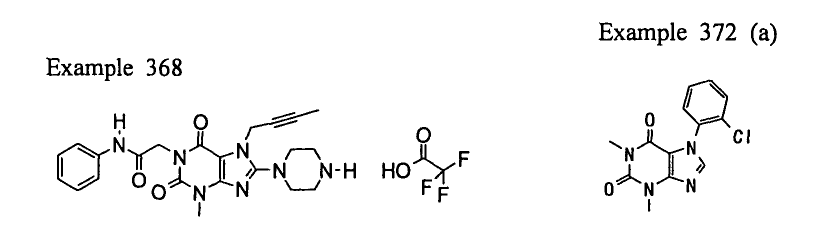

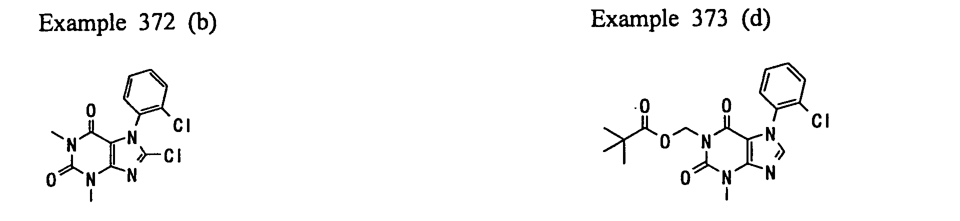

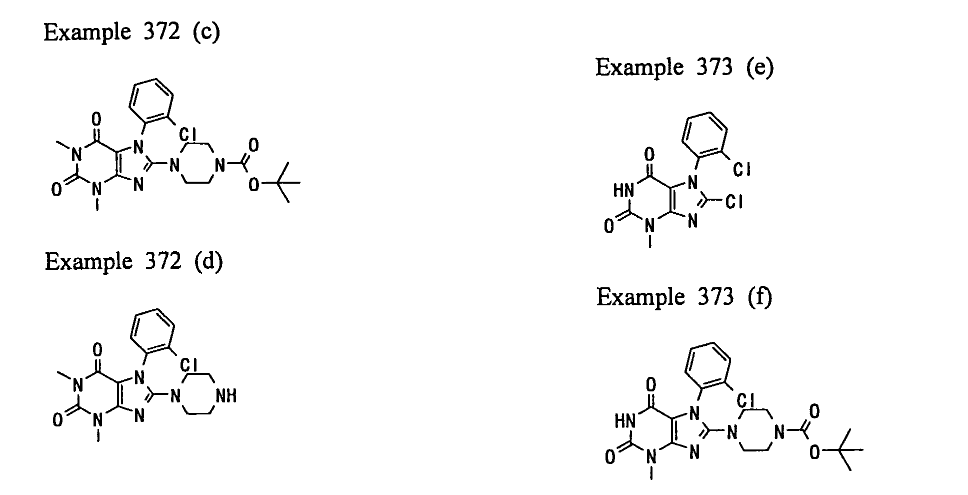

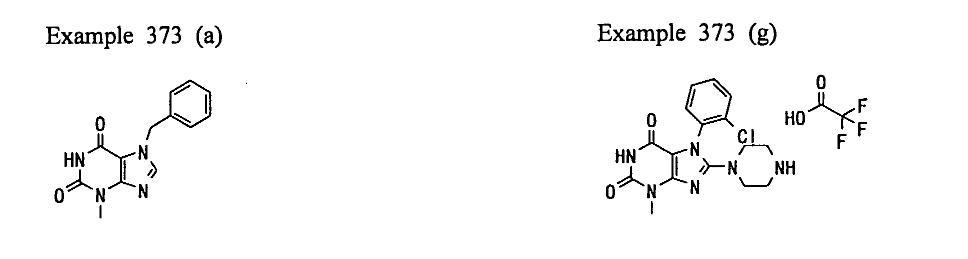

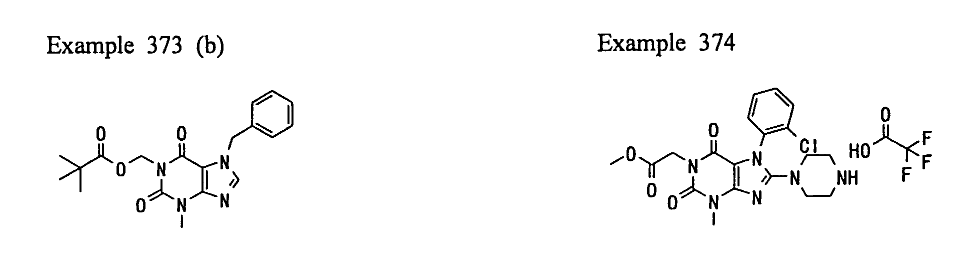

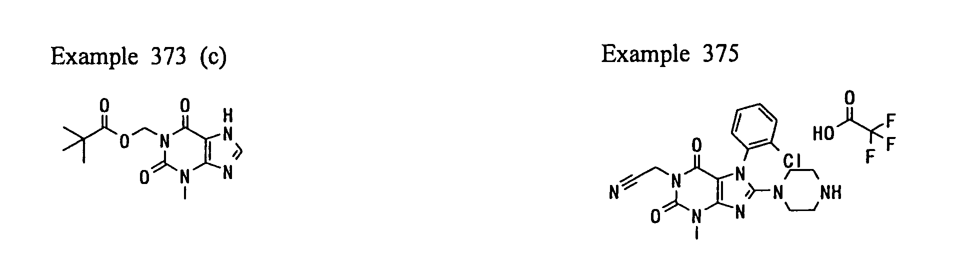

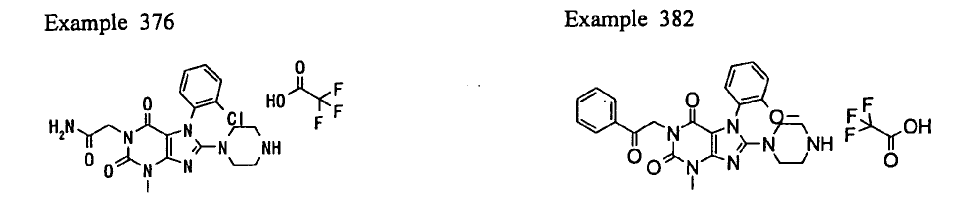

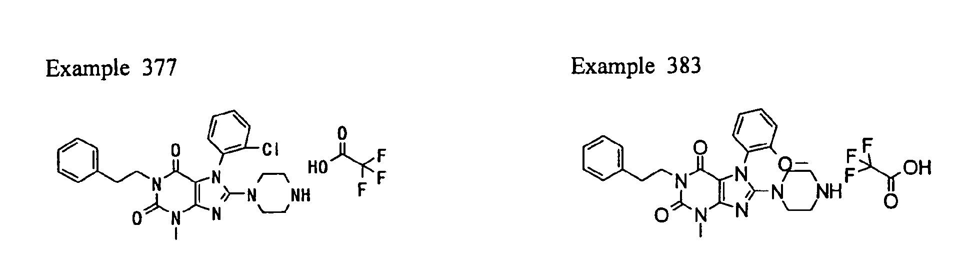

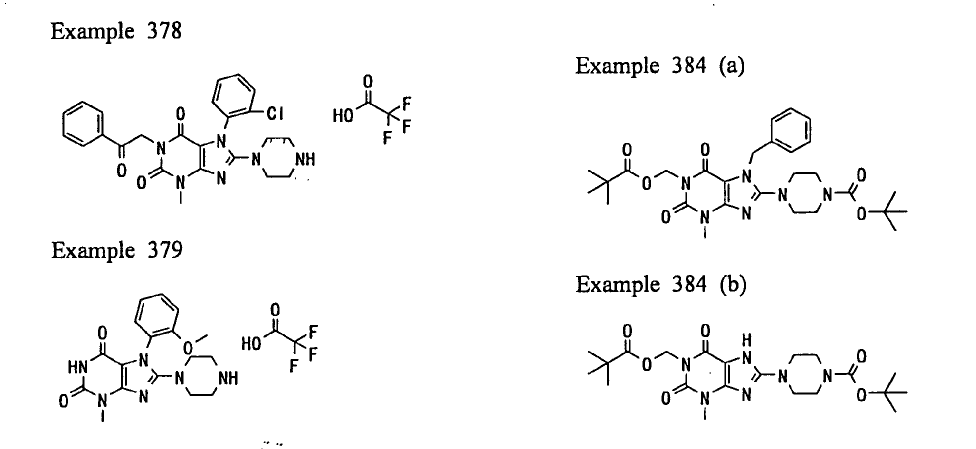

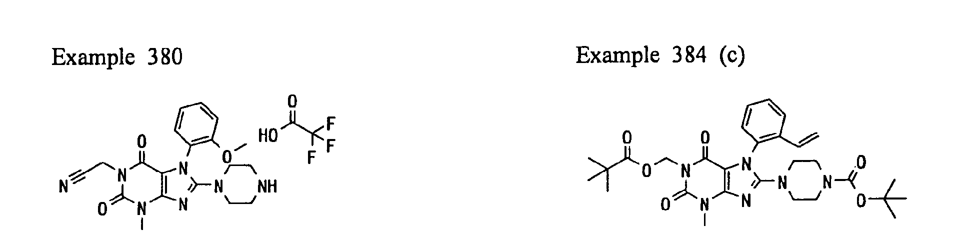

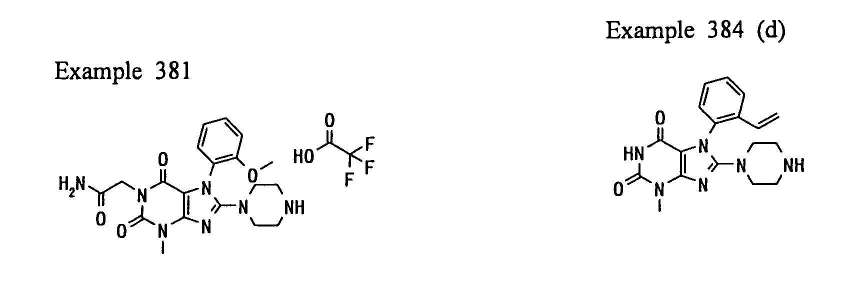

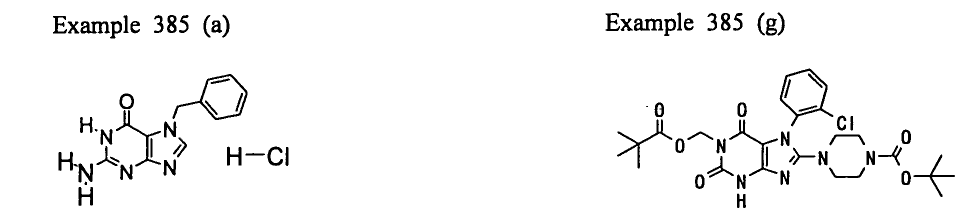

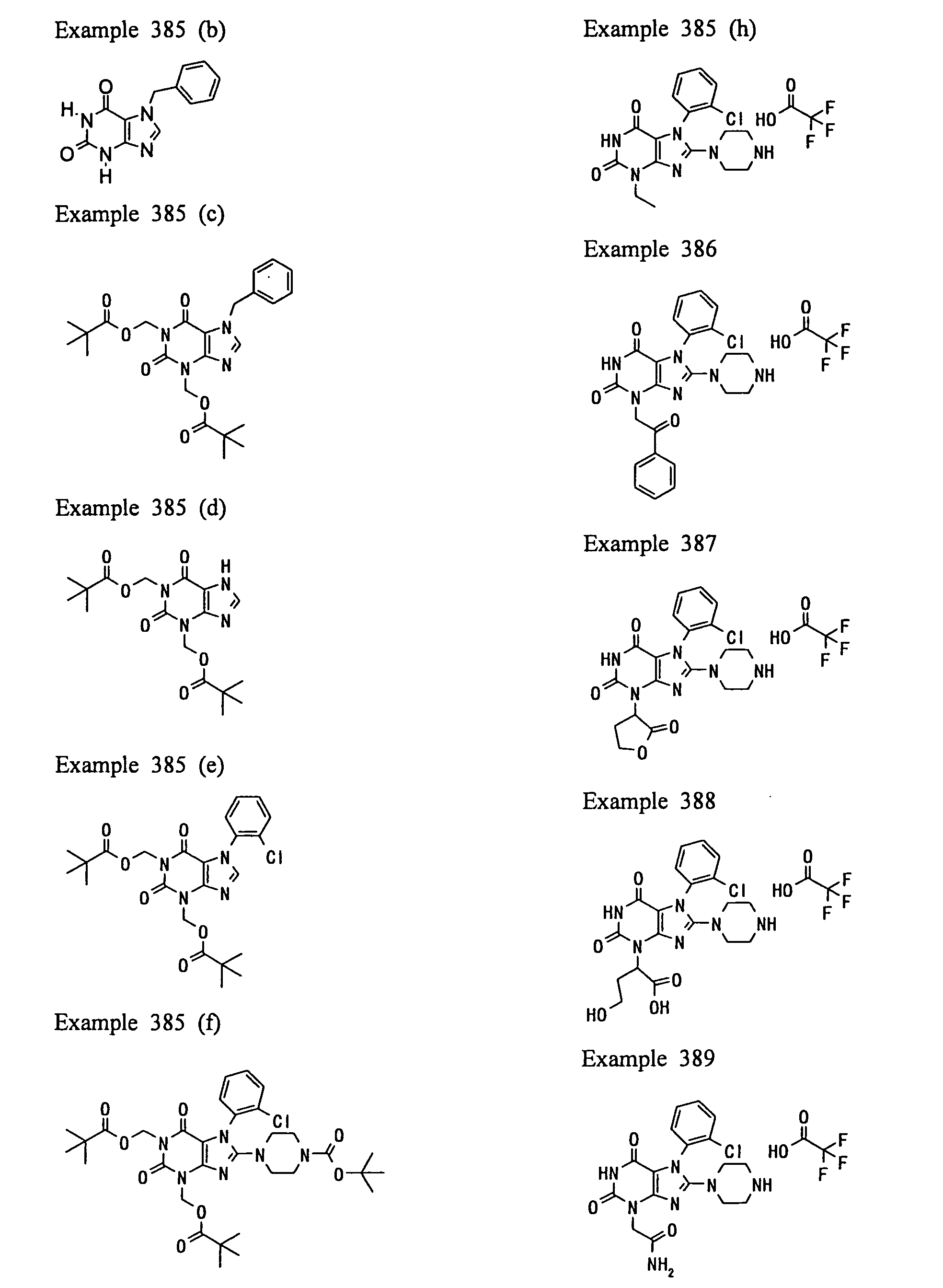

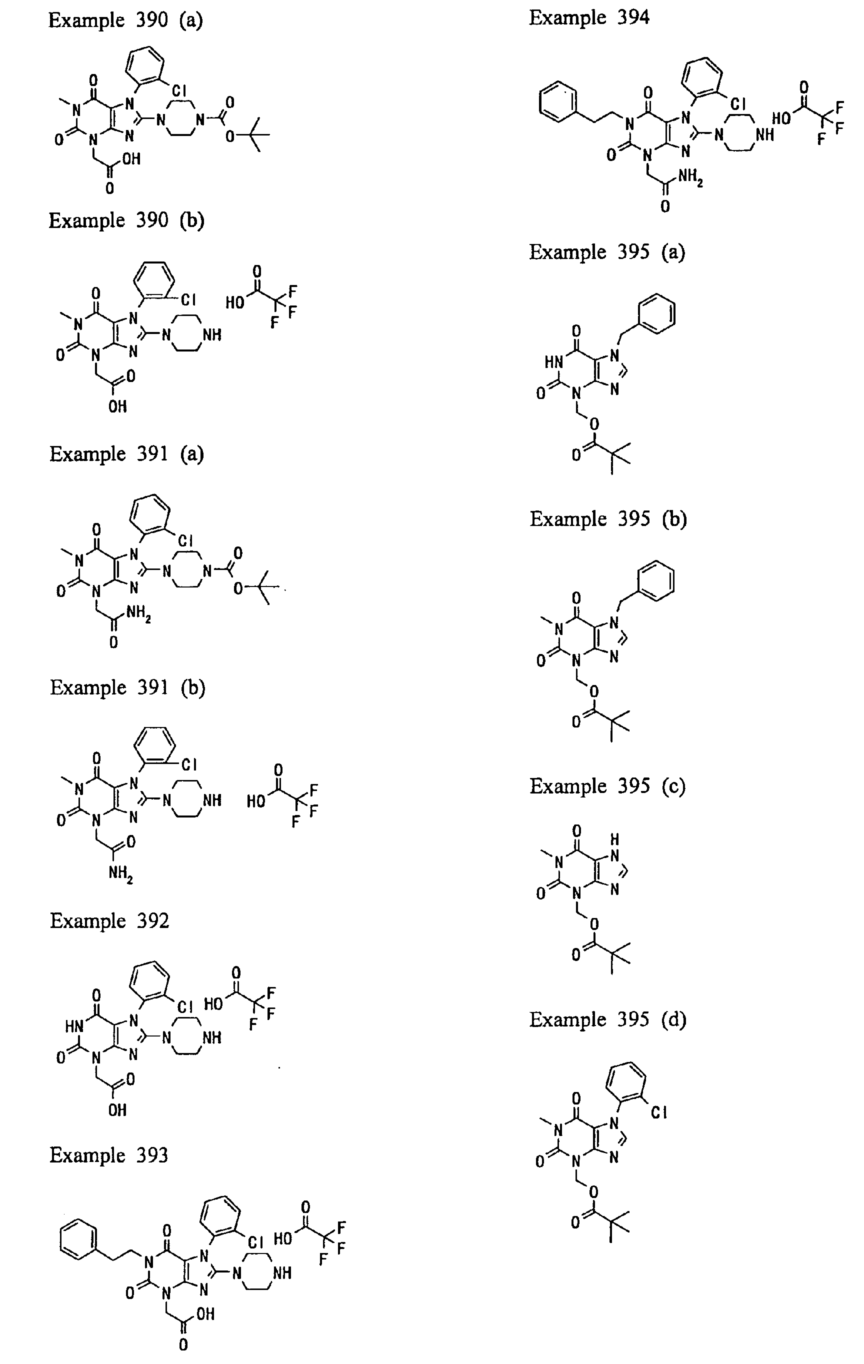

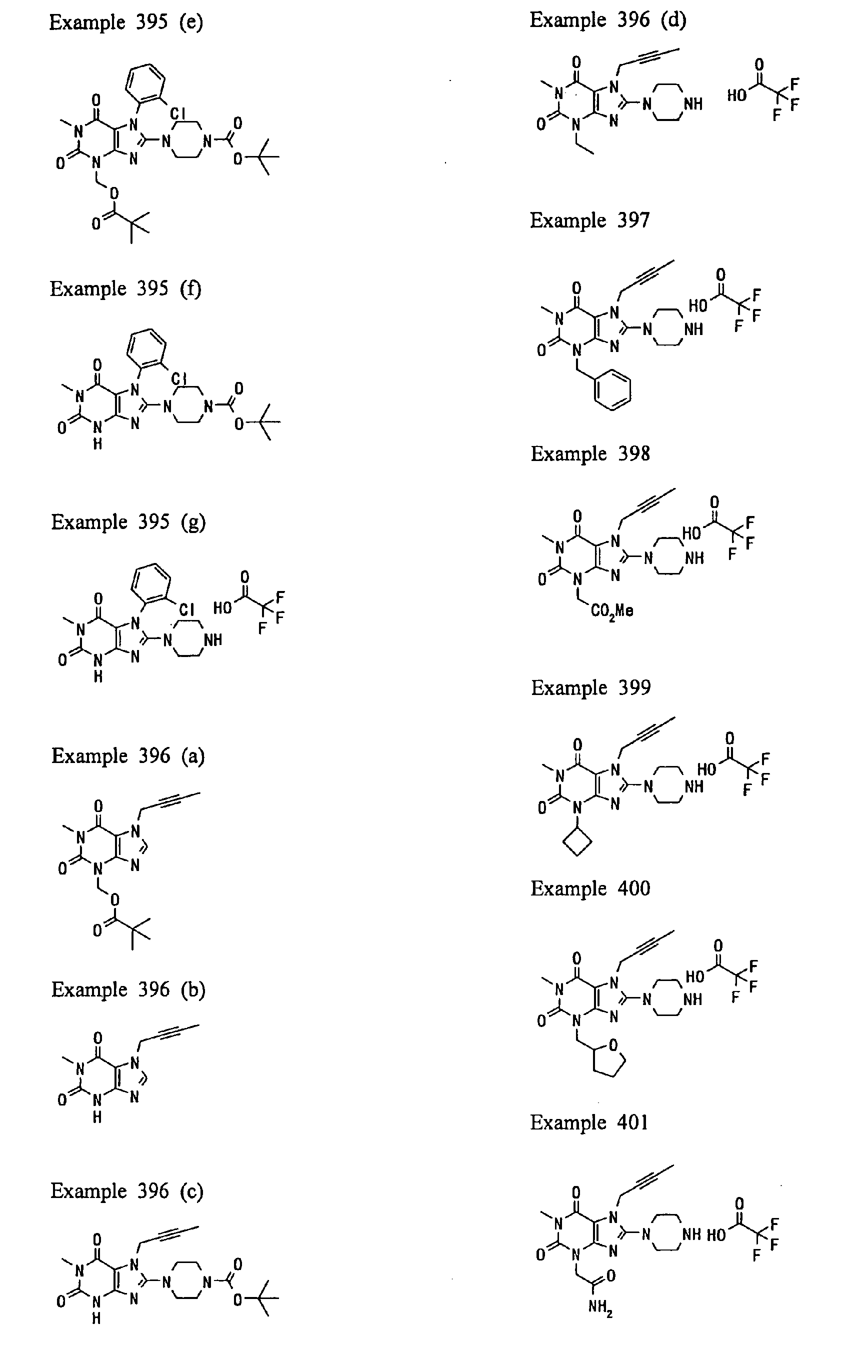

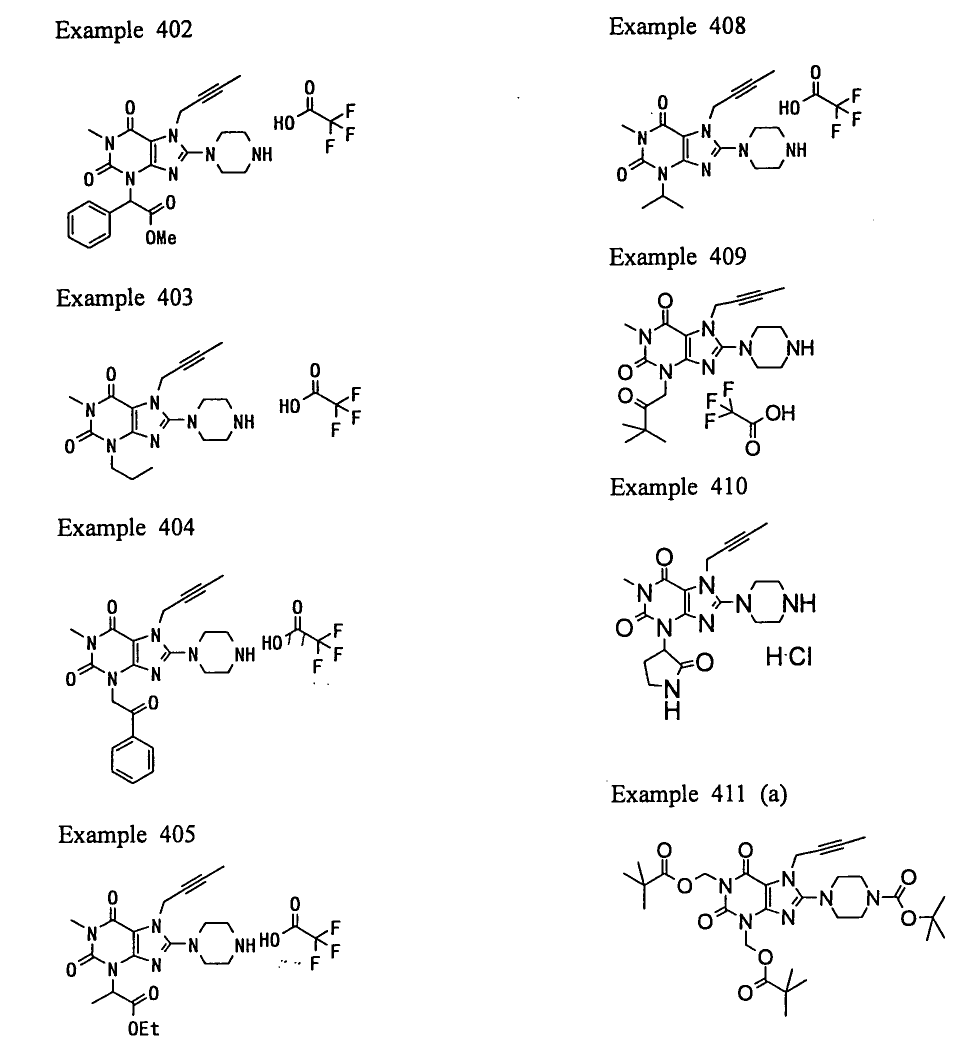

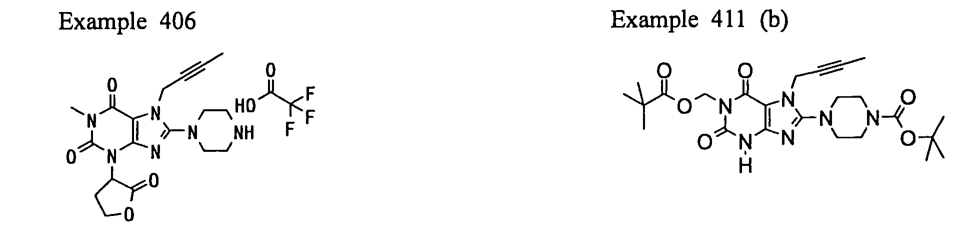

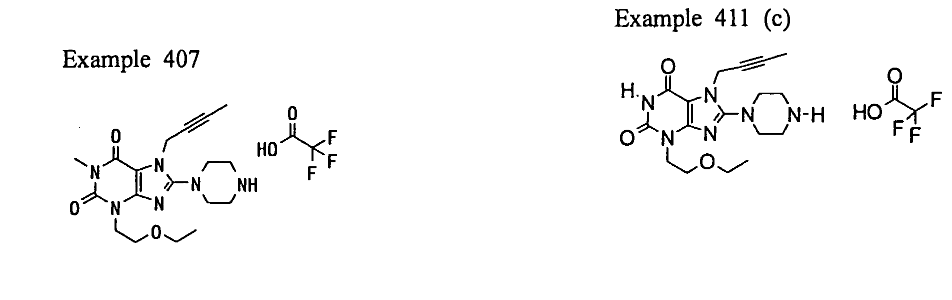

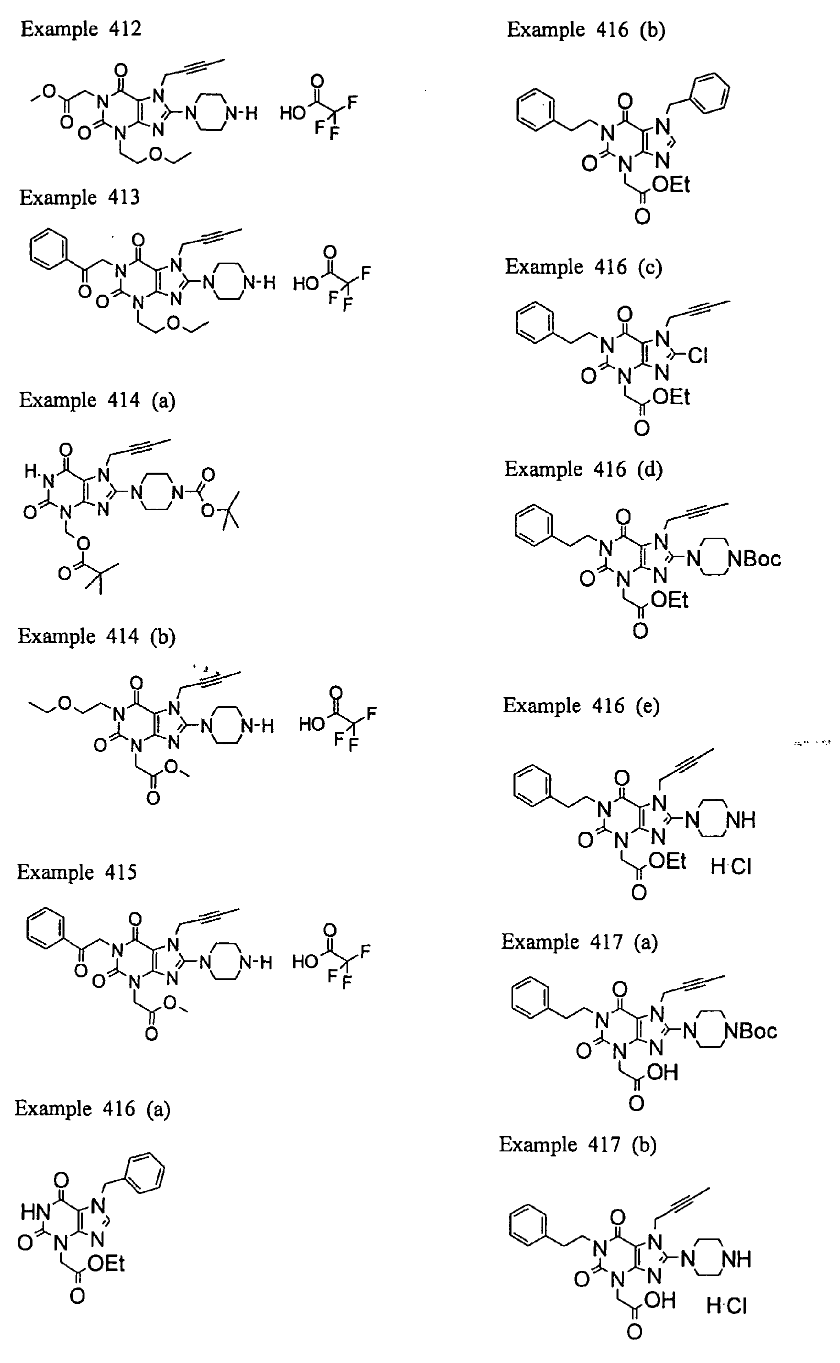

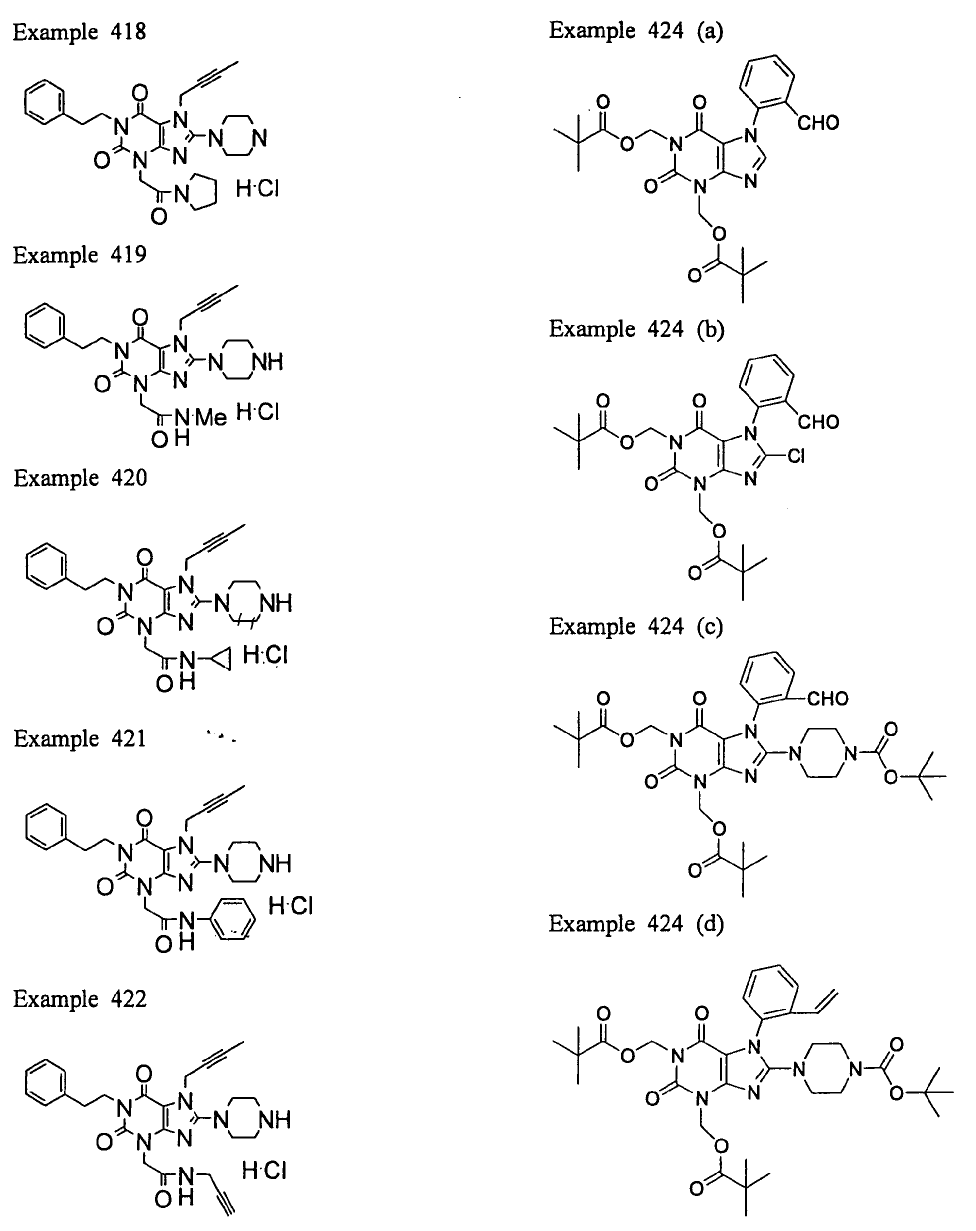

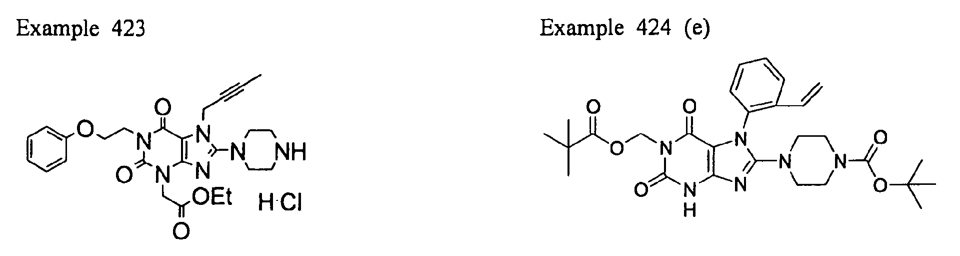

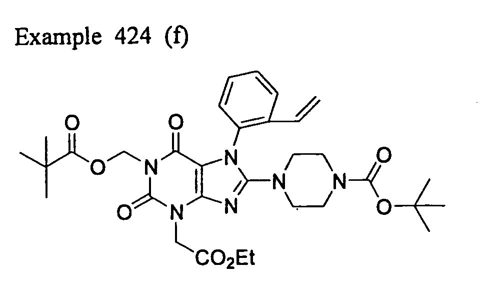

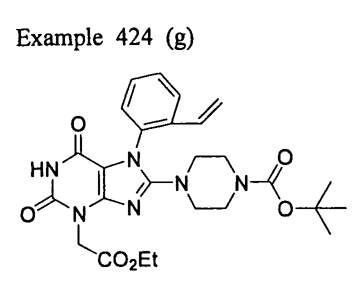

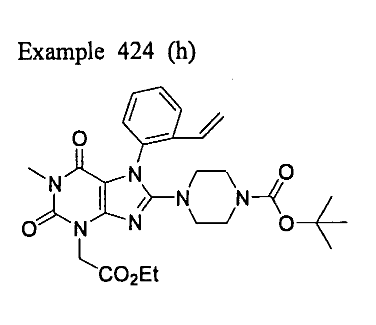

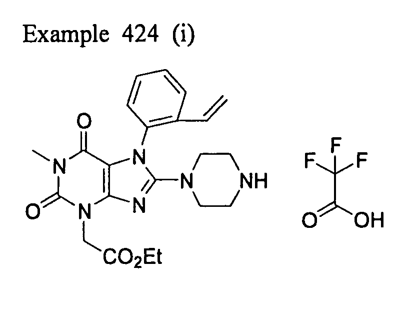

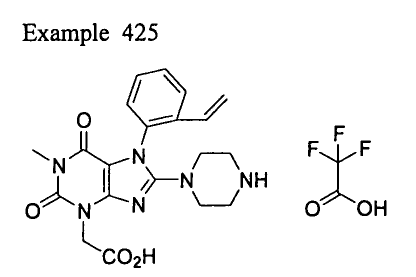

### [Assay Example 1]

### Assay for the DPPIV-inhibiting activity of the compound represented by formula (I)

DPPIV obtained from swine kidney was dissolved in a reaction buffer (50 mM Tris-HCl (pH 7.4)/0.1% BSA) at a final concentration of 10 mU/mL. A 110 µL aliquot of this enzyme solution was added to the reaction system, and then 15 µL of an agent was added. The reaction solution was incubated at room temperature for 20 minutes. 25 µL of a solution containing 2 mM Gly-Pro-p-nitroanilide was added to the reaction solution (at the final concentration of 0.33 mM) to start the enzyme reaction. The reaction time was 20 minutes. 25 µL of 1N phosphate solution was added to stop the reaction. Absorbance of the sample was measured at 405 nm. The degree of inhibition to the enzyme reaction was determined, and IC₅₀ was computed based on the absorbance.

**[Table 1]**

| Example No. | IC₅₀(nM) | Example No. | IC₅₀(nM) |
|---|---|---|---|
| Example 1 | 287 | Example4 | 211 |
| Example7 | 401 | Example9 | 141 |
| Example 12 | 183 | Example 13 | 126 |
| Example 16 | 272 | Example20 | 152 |
| Example 22 | 17 | Example 29 | 310 |
| Example 53 | 46. 9 | Example64 | 126 |
| Example73 | 33.4 | Example76 | 86.5 |
| Example79 | 35.7 | Example82 | 161 |
| Example83 | 27.4 | Example86 | 4.08 |
| Example88 | 2.89 | Example 98 | 9.69 |
| Example 109 | 1480 | Example 115 | 185 |
| Example119 | 154 | Example120 | 116 |
| Example 122 | 15.3 | Example129 | 116 |
| Example 142 | 68.5 | Example146 | 81. 7 |
| Example 159 | 37.7 | Example229 | 8.97 |
| Example 230 | 0.890 | Example 234 | 1. 74 |
| Example 235 | 1.44 | Example238 | 1.19 |
| Example 243 | 2. 15 | Example 248 | 6.40 |
| Example 266 | 1. 15 | Example 267 | 7.22 |
| Example 297 | 6.22 | Example311 | 77.5 |
| Example 341 | 7.32 | Example 353 | 283 |
| Example 354 | 285 | Example 355 | 147 |
| Example 357 | 323 | Example 358 | 357 |
| Example 359 | 353 | Example 361 | 0.654 |
| Example 364 | 9.48 | Example 367 | 4.56 |
| Example 377 | 8.77 | Example 378 | 9.52 |
| Example 382 | 6. 97 | Example 383 | 7.18 |
| Example 393 | 1. 2 | Example 394 | 2.16 |
| Example 396 | 197 | Example 398 | 237 |
| Example 400 | 183 | Example 402 | 354 |
| Example 403 | 266 | Example 404 | 276 |
| Example 405 | 359 | Example 407 | 275 |
| Example 408 | 340 | Example 409 | 222 |
| Example 410 | 64.9 | Example 413 | 1. 95 |
| Example 415 | 1. 81 | Example 416 | 4.02 |
| Example 417 | 0.864 | Example 418 | 1. 14 |
| Example 419 | 1. 55 | Example 420 | 1. 70 |
| Example 421 | 3. 37 | Example 422 | 0.472 |

### [Assay Example 2]

### Influences of metformin, buformin, and phenformin on the GLP-1 level in DPPIV-deficient rats

### Animals: DPPIV-deficient male Fisher rats (purchased from Charles River Japan, Inc.)

### Methods:

### [Preparation and administration of test compounds]

Each test compound was suspended in a solution of 0.5% methyl cellulose at the doses indicated in Table 2, and then administered orally at a volume of 5 mL/kg. The vehicle control group was orally administered a solution of 0.5% methyl cellulose at a volume of 5 mL/kg.

### [Blood collection and GLP-1 assay]

An unanesthetized rat was lightly cut at the caudal vein with a razor blade and bled immediately before, and at 1, 3, and 5 hours after the administration of a test compound or a solution of 0.5% methyl cellulose. 250 µL of blood was collected from the rat using a heparinized capillary and transferred into a centrifugation tube. The supernatant obtained by centrifugation (at 10000 g at 4°C for 2 minutes) was assayed for GLP-1 level using Active GLP-1 ELISA kit (Linco).

### Results:

The result is represented as an "average value ± standard error". The respective values were assessed and compared by Dunnett's test, which are shown in Table 2.

**[Table 2]**

| Test compound | Dose (mg/kg) | GLP-1 concentration at each time point (hr) after oral administration (% of Pre) | | | |
|---|---|---|---|---|---|
| | | 0 | 1 | 3 | 5 |
| Vehicle control | | 100±0.0 | 87.2±4.8 | 100.4±7.8 | 110.6±6.8 |
| Metformin | 30 | 100±0.0 | 99.9±3.7 | 106.6±5.0 | 116.3±2.7 |
| Metformin | 100 | 100±0.0 | 111.6±7.9 | 116.3±8.2 | 150.6±7.2 |
| Metformin | 300 | 100±0.0 | 140.0±11.5 | 199.3±32.4 | 227.1±35.5* |
| Buformin | 30 | 100±0.0 | 118.7±9.3 | 122.7±7.1 | 114.6±4.4 |
| Buformin | 100 | 100±0.0 | 163.6±19.6* | 171.2±9.1 | 195.8±36.6* |
| Phenformin | 30 | 100±0.0 | 125.3±10.7 | 120.0±7.2 | 126.7±10.7 |
| Phenformin | 100 | 100±0.0 | 316.9±26.4*** | 330.7±112.4* | 236.5±20.5* |

| | | | | | |
|---|---|---|---|---|---|
| *: P < 0.05 vs vehicle control group | | | | | |
| ***: P < 0.001 vs vehicle control group | | | | | |

The group of DPPIV-deficient rats administered metformin at a dose of 300 mg/kg, had a significantly elevated level of active GLP-1 in plasma at five hours after administration. The group of DPPIV-deficient rats administered buformin at a dose of 100 mg/kg, had a significantly elevated level of active GLP-1 in plasma at one and five hours after administration.
Furthermore, the group of DPPIV-deficient rats administered phenformin at a dose of 100 mg/kg, had a significantly elevated level of active GLP-1 in plasma at 1, 3, and 5 hours after administration.

### [Assay Example 3]

### Influences of metformin and the DPPIV inhibitor (valine pyrrolidide (Val-Pyr)), used singly or in combination, on GLP-1 level in normal rats

### Animals: DPPIV-intact normal male Fisher rats (purchased from CLEA Japan, Inc.)

### Methods:

### [Preparation and administration of test compounds]

Each test compound was suspended in a solution of 0.5% methyl cellulose at the doses indicated in Table 3, and then administered orally at a volume of 5 mL/kg. The vehicle control group was orally administered a solution of 0.5% methyl cellulose at a volume of 5 mL/kg.

### [Blood collection and GLP-1 assay]

An unanesthetized rat was lightly cut at the caudal vein with a razor blade and bled immediately before, and at 1, 3, and 5 hours after administration of a test compound or a solution of 0.5% methyl cellulose. 250 µL of blood was collected from the rat using a heparinized capillary and transferred into a centrifugation tube. The supernatant obtained by centrifugation (at 10000 g at 4°C for 2 minutes) was assayed for GLP-1 level using Active GLP-1 ELISA kit (Linco).

### Results:

The result is represented as an "average value ± standard error". The respective values were assessed and compared by Dunnett's test, which are shown in Table 3.

**[Table 3]**

| Test compound | Dose (mg/kg) | GLP-1 concentration at each time point (hr) after oral administration (% of Pre) | | | |
|---|---|---|---|---|---|
| | | 0 | 1 | 3 | 5 |
| Vehicle control | | 100±0.0 | 112±15 | 125±21 | 84±10 |
| Metformin | 300 | 100±0.0 | 117±9 | 149±24 | 94±10 |
| Val-Pyr | 30 | 100±0.0 | 127±6 | 136±20 | 91±2 |
| Metformin + Val-Pyr | 300 + 30 | 100±0.0 | 162±8*** | 215±19* | 177±15*** |

| | | | | | |
|---|---|---|---|---|---|
| *: P < 0.05 vs vehicle control group | | | | | |
| ***: P < 0.001 vs vehicle control group | | | | | |

When metformin or the DPPN inhibitor was given singly, there was no increase in the level of active GLP-1. However, the level of active GLP-1 was significantly elevated at 1, 3 and 5 hours after administration in the group administered metformin and DPPIV inhibitor in combination. This result suggests that the active GLP-1 level was elevated due to enhancement of GLP-1 secretion by metformin, and suppression of GLP-1 degradation by the DPPIV inhibitor.

### [Assay Example 4]

### Influences of metformin and the DPPIV inhibitor used singly (Examples 82, 119, 120, 122, 229, and 267) or in combination, on GLP-1 level in normal rats

### Animals: DPPIV-intact normal male Fisher rats (purchased from CLEA Japan, Inc.)

### Methods:

### [Preparation and administration of test compounds]

Each test compound was suspended in a solution of 0.5% methyl cellulose at the doses indicated in Tables 4 to 6, and then administered orally at a volume of 5 mL/kg. The vehicle control group was orally administered a solution of 0.5% methyl cellulose at a volume of 5 mL/kg.

### [Blood collection and GLP-1 assay]

An unanesthetized rat was lightly cut at the caudal vein with a razor blade and bled immediately before, and at 3 hours after the administration of a test compound or a solution of 0.5% methyl cellulose. 250 µL of blood was collected from the rat using a heparinized capillary and transferred into a centrifugation tube. The supernatant obtained by centrifugation (at 10000 g at 4°C for 2 minutes) was assayed for GLP-1 level using Active GLP-1 ELISA kit (Linco).

### Results:

The result is represented as an "average value ± standard error". The respective values were assessed and compared by Dunnett's test, which are shown in Tables 4 to 6.

**[Table 4]**

| Test compound | Dose (mg/kg) | GLP-1 concentration 3 hours after oral administration (% of Pre) |
|---|---|---|
| Vehicle control | | 98.8±2.9 |
| Example 119 | 10 | 98.9±2.2 |
| Example 122 | 10 | 108.2±6.6 |
| Metformin | 300 | 118.1±7.5 |
| Metformin + Example 119 | 300 + 10 | 162.5±7.4*** |
| Metformin + Example 122 | 300 + 10 | 168.1±13.1*** |

| | | |
|---|---|---|
| ***: P < 0.001 vs vehicle control group | | |

**[Table 5]**

| Test compound | Dose (mg/kg) | GLP-1 concentration 3 hours after oral administration (% of Pre) |
|---|---|---|
| Vehicle control | | 97.5±2.9 |
| Example229 | 10 | 102.5±1.7 |
| Example 120 | 10 | 104.8±2.9 |
| Metformin | 300 | 108.6±2.2 |
| Metformin + Example 229 | 300 + 10 | 153.7±13.4*** |
| Metformin + Example 120 | 300 + 10 | 166.4±16.5*** |

| | | |
|---|---|---|
| ***: P < 0.001 vs vehicle control group | | |

**[Table 6]**

| Test compound | Dose (mg/kg) | GLP-1 concentration 3 hours after oral administration (% of Pre) |
|---|---|---|
| Vehicle control | | 96.7±2.6 |
| Example82 | 20 | 97.3±2.1 |
| Example 267 | 10 | 110.0±9.0 |
| Metformin | 300 | 112.5±2.4 |
| Metformin + Example82 | 300 + 20 | 180.8±23.1*** |
| Metformin + Example 267 | 300 + 10 | 186.2±26.2*** |

| | | |
|---|---|---|
| ***: P < 0.01 vs vehicle control group | | |

When metformin or the DPPIV inhibitor was given singly, there was no increase in the level of active GLP-1. However, the level of active GLP-1 was significantly elevated 3 hours after administration in the group which received metformin and DPPIV inhibitor in combination. This result suggests that active GLP-1 level was elevated due to enhancement of GLP-1 secretion by metformin, and suppression of GLP-1 degradation by the DPPIV inhibitor.

### [Assay Example 5]

### Influences of metformin and the DPPIV inhibitor (valine pyrrolidide (Val-Pyr)) used singly or in combination, on glucose tolerance, insulin and GLP-1 levels, food intake, and body weight in Zucker fa/fa rats

### Animals: Zucker fa/fa rats, an animal model for typeII diabetes (purchased from Charles River Japan, Inc.)

### Methods:

### [Preparation and administration of test compounds]

Each test compound was dissolved in distilled water at the doses shown in the Tables indicated below, and then administered orally at a volume of 5 mL/kg. The vehicle control group was orally administered distilled water at a volume of 5 mL/kg. Each test compound or distilled water was given orally at the above dose, twice daily (at 10:00 a.m. and 4:00 p.m.), for 14 days. The rats were tested for glucose tolerance on the first day of the administration series. In the test, distilled water and the test compounds were given 0.5 hour before glucose load. [Procedure of blood collection and determination of the levels of blood glucose and GLP-1]

For the glucose tolerance test, an unanesthetized rat was lightly cut at the caudal vein with a razor blade and bled immediately before the administration of a test compound or distilled water, and immediately before, and at 0.5, 1, 2, and 3 hours after glucose load. 250 µL of blood was collected from the rat using a heparinized capillary and transferred into a centrifugation tube. The supernatant obtained by centrifugation (at 10000 g at 4°C for 2 minutes) was assayed for active GLP-1 level using Active GLP-1 ELISA kit (Linco). At the same time, 10 µL of blood was collected and mixed with 140 µL of a 0.6 M perchloric acid solution. The mixture was centrifuged (at 3000 g at 4°C for 10 minutes), and the resulting supernatant was assayed for glucose using Glucose Test Wako II (Wako Pure Chemical Industries, Inc.). The level of blood glucose alone was determined at the time of measurement 3 hours after glucose load.

### [Determination of food intake and body weight]

Food intake and body weight were determined at 4:00 p.m after the 14-day administration series. Total food intake and weight gain over 14 days were determined for each experimental group.

### Results:

The result is represented as an "average value ± standard error". The respective values were assessed and compared by Dunnett's test, which are shown in Tables 7 to 10.

**[Table 7]**

| Test compound Dose(mg/kg) | GLP-1 concentration at each time point (hr) after oral glucose administration (% of Pre) | | | | |
|---|---|---|---|---|---|
| | -0.5 | 0 | 0.5 | 1 | 2 |
| Vehicle control | 100.0±0.0 | 101.4±0.8 | 130.5±11.2 | 108.2±2.1 | 101.5±2.0 |
| Metformin(300) | 100.0±0.0 | 105.6±1.7 | 135.4±7.6 | 126.0±8.9 | 118.4±6.5 |
| Val-Pyr (30) | 100.0±0.0 | 119.5±3.6 | 217.6±24.6* | 197.5±20.4* | 128.3±5.4 |
| Metformin(300) + Val-Pyr (30) | 100.0±0.0 | 196.5±11.1*** | 345.7±40.7*** | 262.4±37.0*** | 272.6±21.2*** |

| | | | | | |
|---|---|---|---|---|---|
| *: P < 0.05, | | | | | |
| ***: P < 0.001 vs vehicle control group | | | | | |

**[Table 8]**

| Test compound Dose(mg/kg) | Blood glucose level at each time point (hr) after oral glucose administration (mg/dl) | | | | | |
|---|---|---|---|---|---|---|
| | -0.5 | 0 | 0.5 | 1 | 2 | 3 |
| Vehicle control | 101.4±3.4 | 115.7±3.1 | 199.9±14.5 | 226.9±14.9 | 186.6±8.1 | 120.9±5.4 |
| Metformin(300) | 108.9±5.6 | 117.4±5.5 | 160.6±9.7* | 177.5±10.6* | 159.8±8.6* | 122.4±3.7 |
| Val-Pyr (30) | 102.6±3.0 | 110.5±3.3 | 166.0±9.9 | 167.1±7.0*** | 139.3±3.3*** | 116.1±3.0 |
| Metformin(300) + Val-Pyr (30) | 99.0±4.6 | 103.2±3.9 | 119.1±6.6*** | 125.2±7.2*** | 114.6±4.5*** | 104.1±4.2*** |

| | | | | | | |
|---|---|---|---|---|---|---|
| *: P < 0.05, | | | | | | |
| ***: P < 0.001 vs vehicle control group | | | | | | |

**[Table 9]**

| Test compound Dose(mg/kg) | Insulin concentration at each time point (hr) after oral glucose administration (ng/ml) | | | | |
|---|---|---|---|---|---|
| | -0.5 | 0 | 0.5 | 1 | 2 |
| Vehicle control | 9.8±1.1 | 11.9±1.3 | 22.6±2.0 | 16.2±1.0 | 13.2±0.9 |
| Metformin (300) | 11.9±1.1 | 14.0±1.1 | 22.9±2.5 | 21.2±2.3 | 16.9±1.6 |
| Val-Pyr(30) | 8.8±1.1 | 13.1±1.2 | 32.4±3.2* | 27.7±5.0* | 14.4±2.6 |
| Metformin (300) + Val-Pyr (30) | 9.3±1.3 | 14.9±1.4 | 24.3±3.1 | 19.0±2.7 | 15.0±2.9 |

| | | | | | |
|---|---|---|---|---|---|
| *: P < 0.05 vs vehicle control group | | | | | |

**[Table 10]**

| Test compound | Dose (mg/kg) | Total food intake for 14 days (g) | Weight gain for 14 days (g) |
|---|---|---|---|
| Vehicle control | | 484.2±15.0 | 68.2±4.1 |
| Metformin | 300 | 495.1±8.9 | 64.5±3.5 |
| Val-Pyr | 30 | 491.8±11.1 | 60.9±4.4 |
| Metformin + Val-Pyr | 300 + 30 | 418.4±14.0* | 39.2±6.1*** |

| | | | |
|---|---|---|---|
| *: P < 0.05, | | | |
| ***: P < 0.001 vs vehicle control group | | | |

During the glucose tolerance test, the level of active GLP-1 was elevated significantly in the group administered the DPPIV inhibitor, while there was no significant increase in the active GLP-1 level in the group administered metformin. However, the level of active GLP-1 was increased synergistically in the group administered both metformin and the DPPIV inhibitor. This result suggests that the active GLP-1 level was increased through enhanced GLP-1 secretion induced by metformin, and suppressed GLP-1 degradation due to the DPPIV inhibitor, as described above.

The glucose tolerance test revealed that glucose tolerance was improved in each group singly administered either metformin or the DPPIV inhibitor. However, glucose tolerance was improved synergistically in the group administered metformin and the DPPIV inhibitor in combination, when compared with the groups administered either compound singly.

During the glucose tolerance test, the level of insulin was increased significantly in a glucose-dependent fashion in the group administered the DPPIV inhibitor singly, while there was no significant increase in the level of insulin in the groups administered either metformin alone, or metformin and the DPPIV inhibitor in combination. This result suggests that the effect observed in the metformin-administered group was based on the extra-pancreatic action of this agent, while the effect in the DPPIV inhibitor-administered group was based on the glucose-dependent increase in the insulin level, due to the increase in the level of active GLP-1. On the other hand, it is suggested that the group administered metformin and the DPPIV inhibitor in combination had synergistically improved glucose tolerance due to the enhanced susceptibility to insulin, based on the extra-pancreatic action of metformin, and the synergistic increase in the level of active GLP-1 resulting from the combined administration.

Furthermore, decreases in food intake and suppression of weight gain were observed only in the group administered metformin and the DPPIV inhibitor in combination for 14 days. It may be concluded that the synergistic increase in the level of active GLP-1 due to the combined use of metformin and the DPPIV inhibitor, led to the decrease in food intake via the hypothalamus, which in turn resulted in the suppression of weight gain.

In addition, synergistic decreases in the levels of blood glucose and insulin during fasting were found in the group administered metformin and the DPPIV inhibitor in combination for 14 days. It is conceivable that this resulted from enhanced glucose metabolism, due to the synergistic improvement in glucose tolerance and suppressed weight gain in the group administered metformin and the DPPIV inhibitor in combination. This suggests that the combined use of metformin and a DPPIV inhibitor is effective to treat typeII diabetes.

### [Assay Example 6]

### Influence of metformin on the level of GLP-2 in DPPIV-deficiency rats

### Animals: DPPIV-deficient male Fisher rats (purchased from Charles River Japan, Inc.)

### Methods:

### [Preparation and administration of test compound]

The test compound was suspended in a solution of 0.5% methyl cellulose at the dose indicated in Table 11, and then administered orally at a volume of 5 mL/kg. The vehicle control group was orally administered an aqueous solution of 0.5% methyl cellulose at a volume of 5 mL/kg.

### [Blood collection and determination of GLP-2 level]

An unanesthetized rat was lightly cut at the caudal vein with a razor blade and bled immediately before, and at 1, 3, and 5 hours after administration of a test compound or a solution of 0.5% methyl cellulose. 250 µL of blood was collected from the rat using a heparinized capillary and transferred into a centrifugation tube. The supernatant obtained by centrifugation (at 10000 g at 4°C for 2 minutes) was assayed for GLP-2 level using GLP-2 ELISA kit (Yanaihara Institute Inc.).

### Results:

The result is represented as an "average value ± standard error". The respective values were assessed and compared by t-test, which are shown in Table 11.

**[Table 11]**

| Test compound | Dose (mg/kg) | GLP-2 concentration at each time point (hr) after oral administration (ng/ml) | | | |
|---|---|---|---|---|---|
| | | 0 | 1 | 3 | 5 |
| Vehicle control | | 1.39±0.05 | 1.31±0.02 | 1.36±0.04 | 1.28±0.07 |
| Metformin | 300 | 1.32±0.02 | 1.65±0.06*** | 2.08±0.07*** | 2.15±0.05*** |

| | | | | | |
|---|---|---|---|---|---|
| ***: P < 0.001 vs vehicle control group | | | | | |

In the group administered metformin, the level of GLP-2 was significantly elevated in plasma at 1, 3, and 5 hours after administration in DPPIV-deficient rats. This result suggests that the combined use of metformin and the DPPIV inhibitor could synergistically enhance the action of GLP-2, and thus could be effective to treat gastrointestinal diseases.

### [Assay Example 7]

### Influences of metformin and the DPPIV inhibitor (valine pyrrolidide (Val-Pyr)), used singly or in combination, on the atrophy of small intestine caused by 5-fluorouracil (5-FU)

### Animals: BALB/c AnCrj mice (purchased from Charles River Japan, Inc.)

### Methods:

### [Preparation and administration of test compounds]

5-FU (purchased from Sigma) was suspended in a solution of 0.5% methyl cellulose, and then administered orally at a volume of 10 mL/kg/day (8 a.m. to 9 a.m.) for 3 days (60 mg/kg). Each test compound was suspended in a solution of 0.5% methyl cellulose at the doses indicated in Table 12, and then administered orally twice a day, at a volume of 10 mL/kg (8 a.m. to 9 a.m., and 3 p.m. to 4 p.m.). The vehicle control group was orally administered a solution of 0.5% methyl cellulose at a volume of 10 mL/kg. A group which did not receive 5-FU is defined as the normal control group.

### [Collection of samples of small intestine]

Mice were fasted for 18 hours following the afternoon administration on the third day of the administration series. On the following day, the mice were killed by cervical dislocation, and then the whole small intestine was excised and the wet weight was measured.

### Results:

The result is represented as an "average value ± standard error". The respective values were assessed and compared by Tukey's test, which are shown in Table 12.

**[Table 12]**

| 5-FU treatment (mg/kg) | Test compound | Dose (mg/kg) | Wet weight of small intestine (g) |
|---|---|---|---|
| | Normal control | | 0.700±0.009** |
| 60 | Vehicle control | | 0.622±0.005 |
| 60 | Metformin | 300 | 0.642±0.017 |
| 60 | Val-Pyr | 30 | 0.637±0.015 |
| 60 | Metformin + Val-Pyr | 300 + 30 | 0.693±0.015** |

| | | | |
|---|---|---|---|
| **: P < 0.01 vs vehicle control group | | | |

5-FU significantly decreased the wet weight of mouse small intestine. The administration of either metformin or the DPPIV inhibitor alone resulted in no alteration in the wet weight of small intestine in the group of mice treated with 5-FU. In contrast, the combined administration of metformin and the DPPIV inhibitor resulted in a significant increase in the wet weight of small intestine. The increase can be caused by the enhancement of GLP-2 activity resulting from the combined use of metformin and the DPPIV inhibitor. This suggests that the combined use of metformin and the DPPIV inhibitor can be used to treat gastrointestinal diseases, as the increase in the level of GLP-2 results in suppression of apoptosis and enhancement of growth of epithelial cells of the small intestine.

### Industrial Applicability

Pharmaceutical agents comprising a DPPIV inhibitor and a biguanide agent according to the present invention enhance the action of active circulating GLP-1 and/or active circulating GLP-2, and can be used as preventive and/or therapeutic agents for diabetes, obesity, hyperlipidemia, gastrointestinal diseases, and such. In addition, if the pharmaceutical agents according to the present invention are used in combination, the respective agents can be administered at lesser doses as compared with cases where each agent is given singly, which may reduce the risks of adverse side effects of biguanide agents (for example, symptoms of gastrointestinal system, such as diarrhea).

## Claims

1. A pharmaceutical agent comprising a dipeptidyl peptidase IV inhibitor and a biguanide agent in combination.

2. The pharmaceutical agent according to claim 1, which enhances the effects of active circulating glucagon-like peptide-1 (GLP-1) and/or active circulating glucagon-like peptide-2 (GLP-2).

3. A pharmaceutical agent that enhances the effects of active circulating GLP-2.

4. A pharmaceutical agent comprising a dipeptidyl peptidase IV inhibitor and the pharmaceutical agent according to claim 3 in combination.

5. The pharmaceutical agent according to claim 1 or 4, wherein the dipeptidyl peptidase IV inhibitor is a compound represented by the following formula, or a salt or hydrate thereof, 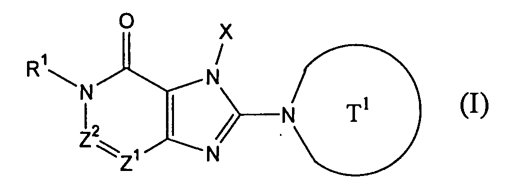 (wherein,
T¹ represents a monocyclic or bicyclic 4- to 12-membered heterocyclic group containing one or two nitrogen atoms in the ring, that may have one or more substituents;
X represents a C₁₋₆ alkyl group which may have one or more substituents, a C₂₋₆ alkenyl group which may have one or more substituents, a C₂₋₆ alkynyl group which may have one or more substituents, a C₆₋₁₀ aryl group which may have one or more substituents, a 5 to 10-membered heteroaryl group which may have one or more substituents, a C₆₋₁₀ aryl C₁₋₆ alkyl group which may have one or more substituents, or a 5 to 10-membered heteroaryl C₁₋₆ alkyl group which may have one or more substituents;
Z¹ and Z² each independently represent a nitrogen atom or a group represented by the formula -CR²=;
R¹ and R² each independently represent a group according to the formula -A⁰-A¹-A² (wherein A⁰ represents a single bond or a C₁₋₆ alkylene group, which may have 1 to 3 substituents selected from group B consisting of the substituents described below;
A¹ represents a single bond, an oxygen atom, a sulfur atom, a sulfinyl group, a sulfonyl group, a carbonyl group, a group represented by the formula -O-CO-, a group represented by the formula -CO-O-, a group represented by the formula -NR^{A}-, a group represented by the formula -CO-NR^{A}-, a group represented by the formula -NR^{A}-CO-, a group represented by the formula -SO₂-NR^{A}-, or a group represented by the formula -NR^{A}-SO₂-;
A² and R^{A} each independently represent a hydrogen atom, a halogen atom, a cyano group, a C₁₋₆ alkyl group, a C₃₋₈ cycloalkyl group, a C₂₋₆ alkenyl group, a C₂₋₆ alkynyl group, C₆₋₁₀ aryl group, a 5 to 10-membered heteroaryl group, a 4 to 8-membered heterocyclic group, a 5 to 10-membered heteroaryl C₁₋₆ alkyl group, a C₆₋₁₀ aryl C₁₋₆ alkyl group, or a C₂₋₇ alkylcarbonyl group;
however, A² and R^{A} each independently may have 1 to 3 substituents selected from the substituent group B described below:
when Z² is a group represented by the formula -CR²=, R¹, and R² may in combination form a 5 to 7-membered ring;
except in cases where: [1] R¹ is a hydrogen atom; Z¹ is a nitrogen atom; and Z² is -CH=; and [2] Z¹ is a nitrogen atom; and Z² is -C(OH)=;
<Substituent group B>
Substituent group B represents the group consisting of: a hydroxyl group, a mercapto group, a cyano group, a nitro group, a halogen atom, a trifluoromethyl group, a C₁₋₆ alkyl group which may have one or more substituents, a C₃₋₈ cycloalkyl group, a C₂₋₆ alkenyl group, a C₂₋₆ alkynyl group, a C₆₋₁₀ aryl group, a 5 to 10-membered heteroaryl group, a 4 to 8-membered heterocyclic group, a C₁₋₆ alkoxy group, a C₁₋₆ alkylthio group, a group represented by the formula -SO₂-NR^{B1}-R^{B2}, a group represented by the formula -NR^{B1}-CO-R^{B2}, a group represented by the formula -NR^{B1}-R^{B2} (where R^{B1} and R^{B2} each independently represent a hydrogen atom or a C₁₋₆ alkyl group), a group represented by the formula -CO-R^{B3} (where R^{B3} represents a 4 to 8-membered heterocyclic group), a group represented by the formula -CO-R^{B4}-R^{B5} and a group represented by the formula -CH₂-CO-R^{B4}-R^{B5} (where R^{B4} represents a single bond, an oxygen atom, or a group represented by the formula -NR^{B6}-; R^{B5} and R^{B6} each independently represent a hydrogen atom, a C₁₋₆ alkyl group, a C₃₋₈ cycloalkyl group, a C₂₋₆ alkenyl group, a C₂₋₆ alkynyl group, a C₆₋₁₀ aryl group, a 5 to 10-membered heteroaryl group, a 4 to 8-membered heterocyclic C₁₋₆ alkyl group, a C₆₋₁₀ aryl C₁₋₆ alkyl group, or a 5 to 10-membered heteroaryl C₁₋₆ alkyl group)).

6. The pharmaceutical agent according to claim 5, wherein T¹ is a piperazin-1-yl group or a 3-amino-piperidin-1-yl group.

7. The pharmaceutical agent according to claim 5, wherein T¹ is a piperazin-1-yl group.

8. The pharmaceutical agent according to any one of claims 5 to 7, wherein X is a 3-methyl-2-buten-1-yl group, a 2-butynyl group, a benzyl group, or a 2-chlorophenyl group.

9. The pharmaceutical agent according to any one of claims 5 to 7, wherein X is a 2-butynyl group.

10. The pharmaceutical agent according to any one of claims 5 to 9, wherein,
Z¹ is a nitrogen atom; and
Z² is a group represented by the formula -CR₂=
(where R² is as defined in claim 5).

11. The pharmaceutical agent according to any one of claims 5 to 9, wherein,
Z² is a nitrogen atom; and
Z¹ is a group represented by the formula -CR₂=
(where R² is as defined in claim 5).

12. The pharmaceutical agent according to any one of claims 5 to 11, wherein R¹ is either a methyl group, a cyanobenzyl group, a fluorocyanobenzyl group, a phenethyl group, a 2-methoxyethyl group, or a 4-methoxycarbonylpridin-2-yl group.

13. The pharmaceutical agent according to any one of claims 5 to 11, wherein R¹ is a methyl group, or a 2-cyanobenzyl group.

14. The pharmaceutical agent according to any one of claims 5 to 13, wherein R² is either a hydrogen atom, a cyano group, a methoxy group, a carbamoylphenyloxy group, or a group represented by the formula: 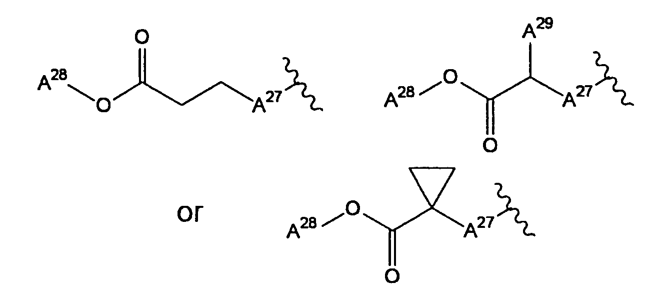 (where,
A²⁷ represents an oxygen atom, a sulfur atom, or -NH-;
A²⁸ and A²⁹ each independently represent a hydrogen atom or a C₁₋₆ alkyl group).

15. The pharmaceutical agent according to any one of claims 5 to 13, wherein R² is a hydrogen atom, a cyano group, or a 2-carbamoylphenyloxy group.

16. The pharmaceutical agent according to claim 5, wherein the compound represented by formula (I) is any one compound selected from:
(1) 7-(2-butynyl)-2-cyano-1-methyl-8-(piperazin-1-yl)-1,7-dihydropurin-6-one;
(2) 3-(2-butynyl)-5-methyl-2-(piperazin-1-yl)-3,5-dihydroimidazo[4,5-d]pyridazin-4-one;
(3) 2-(3-aminopiperidin-1-yl)-3-(2-butynyl)-5-methyl-3,5-dihydroimidazo[4,5-d]pyridazin-4-one;
(4) 2-[7-(2-butynyl)-1-methyl-6-oxo-8-(piperazin-1-yl)-6,7-dihydro-1H-purin-2-yloxy] benzamide;
(5) 7-(2-butynyl)-1-(2-cyanobenzyl)-6-oxo-8-(piperazin-1-yl)-6,7-dihydro-1H-purine-2-car bonitrile; and
(6) 2-[3-(2-butynyl)-4-oxo-2-(piperazin-1-yl)-3,4-dihydroimidazo[4,5-d] pyridazin-5-ylmethyl] benzonitrile;
or a salt or hydrate thereof.

17. The pharmaceutical agent according to claim 1 or 4, wherein the dipeptidyl peptidase IV inhibitor is a compound represented by the following formula, or a salt or hydrate thereof, 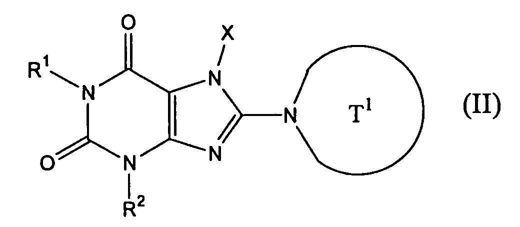 (wherein T¹, X, R¹, and R² are as defined in claim 5).

18. The pharmaceutical agent according to claim 17, wherein T¹ is a piperazin-1-yl group.

19. The pharmaceutical agent according to claim 17 or 18, wherein X is a 2-butynyl group or a 2-chlorophenyl group.

20. The pharmaceutical agent according to claim 17 or 18, wherein X is a 2-butynyl group.

21. The pharmaceutical agent according to any one of claims 17 to 20, wherein R¹ is a hydrogen atom, a methyl group, a 2-propynyl group, a 2-butynyl group, a cyanomethyl group, a phenethyl group, a phenoxyethyl group, or a group represented by the formula: 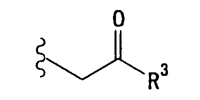 (where R³ represents a hydroxyl group, a C₁₋₆ alkoxy group, or a phenyl group).

22. The pharmaceutical agent according to any one of claims 17 to 21, wherein R² is a hydrogen atom, a C₁₋₆ alkyl group, an ethoxyethyl group, a tetrahydrofuranylmethyl group, or a group represented by the formula: 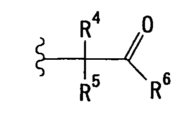 (where,
R⁴ and R⁵ are identical to or different from each other, and independently represent a hydrogen atom, a methyl group, or a phenyl group; and
R⁶ represents a hydroxyl group, a C₁₋₆ alkoxy group, or a phenyl group),
or a group represented by the formula:

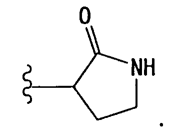

23. The pharmaceutical agent according to claim 17, wherein the compound represented by formula (II) is any one compound selected from:
(1) 7-(2-butynyl)-1,3-dimethyl-8-(piperazin-1-yl)-3,7-dihydropurine-2,6-dione;
(2) 7-(2-butynyl)-3-methyl-8-(piperazin-1-yl)-3,7-dihydropurine-2,6-dione;
(3) methyl [7-(2-butynyl)-3-methyl-2,6-dioxo-8-(piperazin-1-yl)-2,3,6,7-tetrahydropurin-1-yl] acetate;
(4) 7-(2-butynyl)-3-methyl-8-(piperazin-1-yl)-1-(2-propynyl)-3,7-dihydropurine-2,6-dione;
(5) 1,7-bis(2-butynyl)-3-methyl-8-(piperazin-1-yl)-3,7-dihydropurine-2,6-dione;
(6) [7-(2-butynyl)-3-methyl-2,6-dioxo-8-(piperazin-1-yl)-2,3,6,7-tetrahydropurin-1-yl] acetonitrile;
(7) 7-(2-butynyl)-3-methyl-1-[(2-oxo-2-phenyl)ethyl]-8-(piperazin-1-yl)-3,7-dihydropurine-2,6-dione;
(8) 7-(2-butynyl)-3-ethyl-1-methyl-8-(piperazin-1-yl)-3,7-dihydropurine-2,6-dione;
(9) methyl [7-(2-butynyl)-1-methyl-2,6-dioxo-8-(piperazin-1-yl)-1,2,6,7-tetrahydropurin-3-yl] acetate;
(10) 7-(2-butynyl)-3-(2-tetrahydrofuranyl)methyl-1-methyl-8-(piperazin-1-yl)-3,7-dihydropu rine-2,6-dione;
(11) methyl [7-(2-butynyl)-1-methyl-2,6-dioxo-8-(piperazin-1-yl)-1,2,6,7-tetrahydropurin-3-yl]phen ylacetate;
(12) 7-(2-butynyl)-3-propyl-1-methyl-8-(piperazin-1-yl)-3,7-dihydropurine-2,6-dione;
(13) 7-(2-butynyl)-3-(2-oxo-2-phenethyl)-1-methyl-8-(piperazin-1-yl)-3,7-dihydropurine-2,6 -dione;
(14) ethyl 2-[7-(2-butynyl)-1-methyl-2,6-dioxo-8-(piperazin-1-yl)-1,2,6,7-tetrahydropurin-3-yl] propionate;
(15) 7-(2-butynyl)-3-(2-ethoxyethyl)-1-methyl-8-(piperazin-1-yl)-3,7-dihydropurine-2,6-dio ne;
(16) 7-(2-butynyl)-3-isopropyl-1-methyl-8-(piperazin-1-yl)-3,7-dihydropurine-2,6-dione;
(17) 7-(2-butynyl)-3-(3,3-dimethyl-2-oxobutyl)-1-methyl-8-(piperazin-1-yl)-3,7-dihydropuri ne-2,6-dione;
(18) 7-(2-butynyl)-1-methyl-3-(2-oxopyrrolidin-3-yl)-8-(piperazin-1-yl)-3,7-dihydropurine-2 ,6-dione;
(19) 7-(2-butynyl)-3-(2-ethoxyethyl)-1-(2-oxo-2-phenylethyl)-8-(piperazin-1-yl)-3,7-dihydro purine-2,6-dione;
(20) methyl [7-(2-butynyl)-2,6-dioxo-1-(2-oxo-2-phenylethyl)-8-(piperazin-1-yl)-1,2,6,7-tetrahydro purin-3-yl] acetate;
(21) ethyl [7-(2-butynyl)-2,6-dioxo-1-(2-phenethyl)-8-(piperazin-1-yl)-1,2,6,7-tetrahydropurin-3-y 1] acetate;
(22) [7-(2-butynyl)-2,6-dioxo-1-(2-phenethyl)-8-(piperazin-1-yl)-1,2,6,7-tetrahydropurin-3-y l] acetate;
(23) 7-(2-butynyl)-3-[2-oxo-2-(pyrrolidin-1-yl)ethyl]-1-(2-phenethyl)-8-(piperazin-1-yl)-3,7-dihydropurine-2,6-dione;
(24) 2-[7-(2-butynyl)-2,6-dioxo-1-(2-phenethyl)-8-(piperazin-1-yl)-1,2,6,7-tetrahydropurin-3 -yl]-N-methylacetamide;
(25) 2-[7-(2-butynyl)-2,6-dioxo-1-(2-phenethyl)-8-(piperazin-1-yl)-1,2,6,7-tetrahydropurin-3 -yl]-N-cyclopropyl acetamide;
(26) 2-[7-(2-butynyl)-2,6-dioxo-1-(2-phenethyl)-8-(piperazin-1-yl)-1,2,6,7-tetrahydropurin-3 -yl]-N-phenylacetamide; and
(27) 2-[7-(2-butynyl)-2,6-dioxo-1-(2-phenethyl)-8-(piperazin-1-yl)-1,2,6,7-tetrahydropurin-3 -yl]-N-(2-propynyl) acetamide;
or a salt or hydrate thereof.

24. The pharmaceutical agent according to claim 1, wherein the biguanide agent is metformin.

25. The pharmaceutical agent according to claim 1 or 2, which is a preventive or therapeutic agent for a disease which is associated with active circulating GLP-1 and/or active circulating GLP-2.

26. The pharmaceutical agent according to claim 25, wherein the disease is at least any one selected from the group consisting of: diabetes, obesity, hyperlipidemia, and gastrointestinal diseases.

27. The pharmaceutical agent according to claim 3 or 4, which is a preventive or therapeutic agent for a disease which is associated with active circulating GLP-2.

28. The pharmaceutical agent according to claim 27, wherein the disease is a gastrointestinal disease.

29. A method for preventing or treating a disease which is associated with active circulating GLP-1 and/or active circulating GLP-2, which comprises administering the pharmaceutical agent according to claim 1 or 2 at an effective amount.

30. The use of the pharmaceutical agent according to claim 1 or 2 for producing a preventive or therapeutic agent for a disease which is associated with active circulating GLP-1 and/or active circulating GLP-2.

31. A method for preventing or treating a disease which is associated with active circulating GLP-2, which comprises administering the pharmaceutical agent according to claim 3 or 4 at an effective amount.

32. The use of the pharmaceutical agent according to claim 3 or 4 for producing a preventive or therapeutic agent for a disease which is associated with active circulating GLP-2.

33. A method for enhancing the effects of active circulating GLP-1 and/or active circulating GLP-2, which comprises using the pharmaceutical agent according to claim 1 or 2.

34. A method for enhancing the effects of active circulating GLP-2, which comprises using the pharmaceutical agent according to claim 3 or 4.
